(19) Europäisches Patentamt
European Patent Office
Office européen des brevets

(11) EP 4 512 811 A1

(12) **EUROPEAN PATENT APPLICATION**
published in accordance with Art. 153(4) EPC

(43) Date of publication:
**26.02.2025 Bulletin 2025/09**

(21) Application number: **23791956.8**

(22) Date of filing: **21.04.2023**

(51) International Patent Classification (IPC):
***C07D 498/04** (2006.01)* ***A61K 31/55** (2006.01)*
***A61K 31/553** (2006.01)* ***A61P 25/02** (2006.01)*
***A61P 25/16** (2006.01)* ***A61P 25/26** (2006.01)*
***A61P 25/28** (2006.01)* ***A61P 43/00** (2006.01)*
***C07D 519/00** (2006.01)*

(52) Cooperative Patent Classification (CPC):
**A61K 31/55; A61K 31/553; A61P 25/02; A61P 25/16; A61P 25/26; A61P 25/28; A61P 43/00; C07D 498/04; C07D 519/00**

(86) International application number:
**PCT/JP2023/015990**

(87) International publication number:
**WO 2023/204308 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.04.2022 JP 2022070484**

(71) Applicant: **Teijin Pharma Limited**
**Tokyo 100-0013 (JP)**

(72) Inventors:
- **OKADA, Kazuhisa**
  **Tokyo 100-0013 (JP)**
- **SASAKI, Kosuke**
  **Tokyo 100-0013 (JP)**
- **MUTOH, Hiroyuki**
  **Tokyo 100-0013 (JP)**
- **KIMURA, Kumi**
  **Tokyo 100-0013 (JP)**
- **FURUYA, Shiori**
  **Tokyo 100-0013 (JP)**

(74) Representative: **Lavoix**
**2, place d'Estienne d'Orves**
**75441 Paris Cedex 09 (FR)**

(54) **OXAZEPINE DERIVATIVE**

(57) Novel compounds are provided with excellent orexin type 2 receptor agonist activity. The compounds are represented by Formula (I) or pharmaceutically acceptable salts thereof.

[Formula 1]

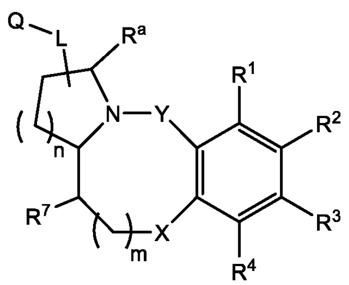

(I)

EP 4 512 811 A1

## Description

### TECHNICAL FIELD

The present invention relates to an oxazepine derivative and a pharmaceutically acceptable salt thereof, and specifically to a compound having orexin type 2 receptor agonist activity and useful for prevention or treatment of, e.g., narcolepsy, idiopathic hypersomnia, and hypersomnia.

### BACKGROUND ART

Orexin is a neuropeptide produced by certain nerve cells localized in the lateral subthalamic area of the brain and is generated from prepro-orexin by prohormone convertase. Orexin has two subtypes, orexin A and orexin B.

Unless otherwise noted, the "orexin" as used herein encompasses both orexin A and orexin B.

Orexin binds to orexin receptors as an intrinsic ligand for the orexin receptors, which are G protein-coupled receptors expressed primarily in the brain. Known orexin receptors are categorized as type 1 receptor (hereinafter referred to as OX1R) and type 2 receptor (hereinafter referred to as OX2R) (Non-Patent Literature 1).

Unless otherwise noted, the "orexin receptor" as used herein encompasses both OX1R and OX2R.

Known diseases associated with orexin or orexin type 2 receptor include narcolepsy, idiopathic hypersomnia, hypersomnia, and sleep apnea syndrome.

For example, it has been reported that narcolepsy-like symptoms produced in transgenic mice lacking endogenous orexin by denaturing orexin-producing neurons were ameliorated by intracerebroventricular administration of orexin peptide (Non-Patent Literature 2), and that narcolepsy-like symptoms were induced in mice lacking (KO) prepro-orexin, a precursor of orexin (Non-Patent Literature 3), suggesting that narcolepsy is induced by a deficiency of orexin.

Furthermore, it has been reported that hereditary narcolepsy occurred in dogs with mutations in OX2R (Non-Patent Literature 4), and that narcolepsy-like symptoms were also elicited in OX2R KO mice (Non-Patent Literature 5), strongly suggesting that stimulation of orexin type 2 receptor is involved in arousal maintenance effects.

Known diseases caused by abnormal orexin action include narcolepsy type 1 and narcolepsy type 2.

Unless otherwise noted, the "narcolepsy" as used herein encompasses both narcolepsy type 1 and narcolepsy type 2.

It has been reported that patients with narcolepsy type 1 have low orexin levels in cerebrospinal fluid (Non-Patent Literature 6). It has also been suggested that patients with sleep apnea syndrome have lower levels of orexin A concentration in plasma (Non-Patent Literature 7). It has further been reported that patients with Parkinson's disease experience drowsiness, which has been suggested to be caused by the loss of orexin-producing nerve cells (Non-Patent Literature 8).

Based on these backgrounds, it has been suggested that OX2R agonists may be used as promising therapeutic drugs for narcolepsy and other sleep disorders accompanied by hypersomnia (Non-Patent Literature 9).

It is therefore deemed that compounds with OX2R agonist activity can be used as prophylactic or therapeutic agents for narcolepsy, idiopathic hypersomnia, hypersomnia, sleep apnea syndrome, narcolepsy syndrome with narcolepsy-like symptoms, hypersomnia syndromes with daytime hypersomnia (e.g., Parkinson's disease, Lewy body dementia, Guillain-Barre syndrome, and Kleine-Levin syndrome).

In fact, it has been reported that compounds with OX2R agonist activity improve the symptoms of narcolepsy (Patent Literature 1). A known compound having OX2R agonist activity, TAK-925, has been clinically tested in healthy subjects and narcolepsy patients (via intravenous administration), and another known compound, TAK-994, has been reported to cause adverse reactions in clinical trials, but the details thereof are unknown.

### LIST OF CITATIONS

#### Patent Literature

[Patent Literature 1] WO2021/048822 A1

#### Non-Patent Literature

[Non-Patent Literature 1] Cell, Vol.92, 573-585, 1998
[Non-Patent Literature 2] Proc. Natl. Acad. Sci. USA, Vol.101, 4649-4654, 2004
[Non-Patent Literature 3] Cell, Vol.98, 437-451, 1998

[Non-Patent Literature 4] Cell, Vol.98, 365-376, 1999
[Non-Patent Literature 5] Neuron, Vol.38, 715-730, 2003
[Non-Patent Literature 6] The Lancet, Vol.355, 39-40, 2000
[Non-Patent Literature 7] Respiration, Vo.71, 575-579, 2004
[Non-Patent Literature 8] Brain, Vol.130, 1586-1595, 2007
[Non-Patent Literature 9] CNS Drugs, Vol.27, 83-90, 2013

## SUMMARY OF INVENTION

## PROBLEM TO BE SOLVED BY THE INVENTION

The problem addressed by the present invention is to provide a compound with excellent orexin type 2 receptor agonist activity.

## MEANS TO SOLVE THE PROBLEM

As a result of diligent research to solve the above problem, the present inventors have found novel compounds with orexin type 2 receptor agonist activity, and have also found that these compounds have therapeutic or preventive effects on diseases associated with orexin or orexin type 2 receptor, thereby completing the present invention.
Accordingly, the present invention includes the following aspects.
[1] A compound represented by Formula (I) or a pharmaceutically acceptable salt thereof.

[Formula 1]

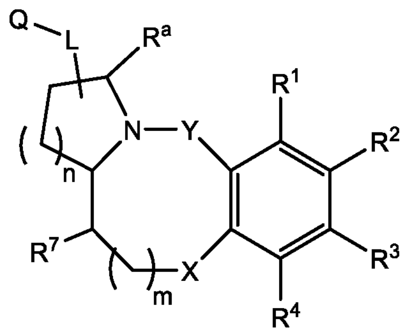

(I)

[In the formula,

$R^1$ represents a hydrogen atom, $C_{1-6}$ alkyl group, $C_{3-8}$ cycloalkyl group, 3- to 8-membered heterocyclyl group, hydroxy group, halogen, amino group, cyano group, carboxyl group, $C_{6-10}$ aryl group, 5- to 12-membered heteroaryl group, $C_{1-6}$ alkoxy group, $C_{3-8}$ cycloalkoxy group, 3- to 8-membered heterocyclyloxy group, $C_{6-10}$ aryloxy group, 5-to 12-membered heteroaryloxy group, $C_{3-8}$ cycloalkyl $C_{1-6}$ alkoxy group, 3- to 8-membered heterocyclyl $C_{1-6}$ alkoxy group, $C_{6-10}$ aryl $C_{1-6}$ alkoxy group, or 5- to 12-membered heteroaryl $C_{1-6}$ alkoxy group;
$R^1$ may be substituted with 1 to 6 [halogens, hydroxy groups, carboxyl groups, oxo groups, amino groups, cyano groups, $C_{1-6}$ alkyl groups which may be substituted with 1 to 3 halogens, or $C_{1-6}$ alkoxy groups which may be substituted with 1 to 3 halogens];
$R^2$, $R^3$ and $R^4$ each represent, independently of each other, a hydrogen atom, $C_{1-6}$ alkyl group which may be substituted with 1 to 6 halogens, $C_{3-8}$ cycloalkyl group which may be substituted with 1 to 6 halogens, hydroxy group, or halogen;
each of $R^2$, $R^3$ and $R^4$ may be substituted with 1 to 6 [halogens, hydroxy groups, carboxyl groups, oxo groups, amino groups, cyano groups, $C_{1-6}$ alkyl groups which may be substituted with 1 to 3 halogens, or $C_{1-6}$ alkoxy groups which may be substituted with 1 to 3 halogens];
each of the pairs of adjacent groups $R^1$ and $R^2$, $R^2$ and $R^3$, and $R^3$ and $R^4$ may each be linked to each other via a single bond to form a 5- to 8-membered ring;
$R^7$ represents a hydrogen atom, halogen, or $C_{1-6}$ alkyl group which may be substituted with 1 to 6 halogens;
$R^a$ represents a hydrogen atom, $C_{1-6}$ alkyl group which may be substituted with 1 to 6 halogens, hydroxy group, or

halogen;
X represents -O-, or $-CR^bR^c-$,
$R^b$ and $R^c$ each represent, independently of each other, a hydrogen atom, $C_{1-6}$ alkyl group which may be substituted with 1 to 6 halogens, or halogen,
Y represents $-CH_2-$ or -CO-,
n represents 1 or 2,
m represents 0 or 1,
L represents -O- or -NH-,
Q represents a 3- to 10-membered heterocyclyl group which may be substituted with 1 to 4 $R^{11}$, $C_{6-10}$ aryl group which may be substituted with 1 to 4 $R^{12}$, or 5- to 12-membered heteroaryl group which may be substituted with 1 to 4 $R^{13}$, and
$R^{11}$, $R^{12}$ and $R^{13}$ each represent, independently of each other, a halogen, hydroxy group, oxo group, $C_{1-6}$ alkyl group which may be substituted with 1 to 6 halogens, $C_{3-8}$ cycloalkyl group which may be substituted with 1 to 6 halogens, or $C_{1-6}$ alkoxy group which may be substituted with 1 to 6 halogens.]

[2] A compound or a pharmaceutically acceptable salt thereof according to [1], wherein

$R^1$ is $C_{1-6}$ alkyl group, $C_{3-8}$ cycloalkyl group, 3- to 8-membered heterocyclyl group, hydroxy group, $C_{6-10}$ aryl group, 5- to 12-membered heteroaryl group, $C_{1-6}$ alkoxy group, $C_{3-8}$ cycloalkoxy group, 3- to 8-membered heterocyclyloxy group, $C_{3-8}$ cycloalkyl $C_{1-6}$ alkoxy group, 3- to 8-membered heterocyclyl $C_{1-6}$ alkoxy group, or $C_{6-10}$ aryl $C_{1-6}$ alkoxy group, and
$R^1$ may be substituted with 1 to 3 [halogens, hydroxy groups, oxo groups, $C_{1-6}$ alkyl groups which may be substituted with 1 to 3 halogens, or $C_{1-6}$ alkoxy groups which may be substituted with 1 to 3 halogens].

[3] A compound or a pharmaceutically acceptable salt thereof according to [1] or [2], wherein $R^2$ is a hydrogen atom or halogen.
[4] A compound or a pharmaceutically acceptable salt thereof according to any one of [1] to [3], wherein $R^3$ is a hydrogen atom, $C_{1-6}$ alkyl group which may be substituted with 1 to 6 halogens, or halogen.
[5] A compound or a pharmaceutically acceptable salt thereof according to any one of [1] to [4], wherein $R^4$ is a hydrogen atom or halogen.
[6] A compound or a pharmaceutically acceptable salt thereof according to any one of [1] to [5], wherein $R^a$ is a hydrogen atom.
[7] A compound or a pharmaceutically acceptable salt thereof according to any one of [1] to [6], wherein n is 1.
[8] A compound or a pharmaceutically acceptable salt thereof according to any one of [1] to [7], wherein m is 0.
[9] A compound or a pharmaceutically acceptable salt thereof according to any one of [1] to [8], wherein L is -O-.
[10] A compound or a pharmaceutically acceptable salt thereof according to any one of [1] to [9], wherein Y is -CO-.
[11] A compound or a pharmaceutically acceptable salt thereof according to any one of [1] to [10], wherein Q is a 5- to 12-membered bicyclic heteroaryl group which may be substituted with 1 to 4 $R^{13}$.
[12] A compound or a pharmaceutically acceptable salt thereof according to any one of [1] to [11], wherein Q is a structure represented by any one selected from the group consisting of Formulae (II-1), (II-2), (II-3) and (II-4).

[Formula 2]

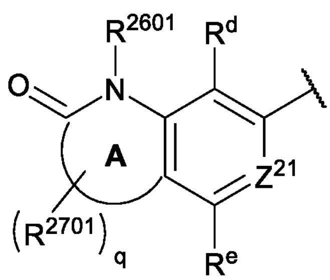
(II-1)

[Formula 3]

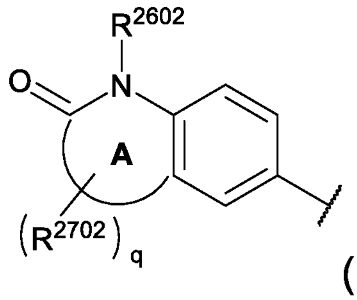

(II-2)

[Formula 4]

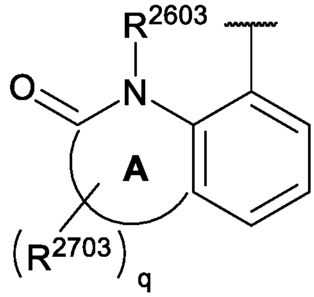

(II-3)

[Formula 5]

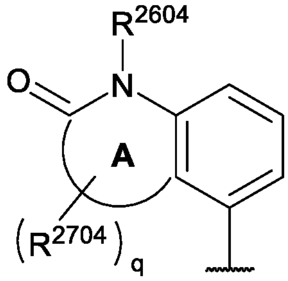

(II-4)

[In Formulae (II-1), (II-2), (II-3) and (II-4),

$Z^{21}$ represents a $CR^f$ or N,

Ring A represents a 6- to 8-membered monocyclic heterocyclyl group or 6- to 7-membered monocyclic heteroaryl group,

$R^{2701}$, $R^{2702}$, $R^{2703}$ and $R^{2704}$ each represent, independently of each other, a halogen or $C_{1-6}$ alkyl group which may be substituted with 1 to 3 halogens,

$R^d$, $R^e$, and $R^f$ each represent, independently of each other, a hydrogen atom, halogen, or $C_{1-6}$ alkyl group which may be substituted with 1 to 3 halogens,

$R^{2601}$, $R^{2602}$, $R^{2603}$ and $R^{2604}$ each represent, independently of each other, a hydrogen atom, $C_{1-6}$ alkyl group which may be substituted with 1 to 6 halogens, or $C_{3-8}$ cycloalkyl group which may be substituted with 1 to 6 halogens, and

q represents, independently of each other, an integer of 0 to 4.]

[13] A compound or a pharmaceutically acceptable salt thereof according to [1], which is a compound represented by Formula (III) or a pharmaceutically acceptable salt thereof.

[Formula 6]

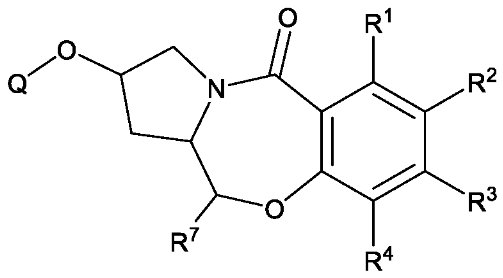

(III)

[In the formula,

$R^1$ represents a hydrogen atom, $C_{1-6}$ alkyl group, $C_{3-8}$ cycloalkyl group, 3- to 8-membered heterocyclyl group, hydroxy group, halogen, $C_{6-10}$ aryl group, 5- to 12-membered heteroaryl group, $C_{1-6}$ alkoxy group, $C_{3-8}$ cycloalkoxy group, 3- to 8-membered heterocyclyloxy group, $C_{6-10}$ aryloxy group, 5- to 12-membered heteroaryloxy group, $C_{3-8}$ cycloalkyl $C_{1-6}$ alkoxy group, 3- to 8-membered heterocyclyl $C_{1-6}$ alkoxy group, $C_{6-10}$ aryl $C_{1-6}$ alkoxy group, or 5- to 12-membered heteroaryl $C_{1-6}$ alkoxy group;
$R^1$ may be substituted with 1 to 6 [halogens, hydroxy groups, carboxyl groups, oxo groups, amino groups, cyano groups, $C_{1-6}$ alkyl groups which may be substituted with 1 to 3 halogens, or $C_{1-6}$ alkoxy groups which may be substituted with 1 to 3 halogens];
$R^2$, $R^3$ and $R^4$ each represent, independently of each other, a hydrogen atom, $C_{1-6}$ alkyl group which may be substituted with 1 to 6 halogens, or halogen;
$R^7$ represents a hydrogen atom, halogen, or $C_{1-6}$ alkyl group which may be substituted with 1 to 3 halogens;
Q represents a 3- to 10-membered heterocyclyl group which may be substituted with 1 to 4 $R^{11}$, $C_{6-10}$ aryl group which may be substituted with 1 to 4 $R^{12}$, or 5- to 12-membered heteroaryl group which may be substituted with 1 to 4 $R^{13}$, and
$R^{11}$, $R^{12}$ and $R^{13}$ each represent, independently of each other, a halogen, oxo group, or $C_{1-6}$ alkyl group which may be substituted with 1 to 3 halogens.]

[14] A compound or a pharmaceutically acceptable salt thereof according to any one of [1] to [13], wherein Q is a structure represented by any one selected from the group consisting of Formulae (IV), (V) and (VI).

[Formula 7]

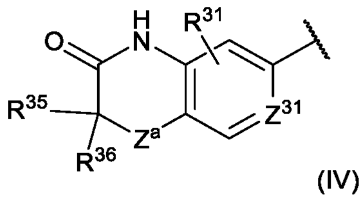

(IV)

[Formula 8]

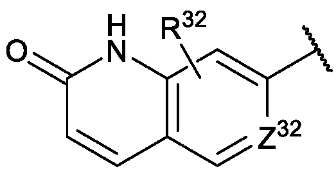

(V)

[Formula 9]

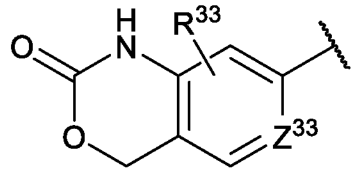

(VI)

[In Formulae (IV), (V) and (VI),

$R^{31}$, $R^{32}$, $R^{33}$, $R^{35}$ and $R^{36}$ each represent, independently of each other, a hydrogen atom, $C_{1-6}$ alkyl group which may be substituted with 1 to 6 halogens, hydroxy group, or halogen,
$Z^{31}$, $Z^{32}$ and $Z^{33}$ each represent, independently of each other, CH or N,
$Z^a$ represents O or $CR^g_2$, and
$R^g$ represents, independently of each other, a hydrogen atom, or $C_{1-6}$ alkyl group which may be substituted with 1 to 6 halogens.]

[15] A compound which is at least one selected from (1) to (50) below or a pharmaceutically acceptable salt thereof.

(1) (2S,11aR)-6-isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one
(2) (2S,11aR)-6-(cyclopentyloxy)-8-methyl-2-((2-oxo-1,2,3,4-tetrahydroquinolin-7-yl)oxy)-2,3, 11, 11a-tetrahydro-1H,5H-benzo[f]pyrrolo[2, 1-c][1,4]oxazepin-5-one
(3) (2S,11aR)-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(4) (2S,11aR)-6-(((S)-1-fluoropropan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one
(5) (2S,11aR)-6-isopropoxy-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one
(6) (2S,11aR)-6-(cyclopentyloxy)-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one
(7) (2S,11aR)-6-(cyclopentyloxy)-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one
(8) (2S,11aR)-8-methyl-2-((2-oxo-1,2,3,4-tetrahydro-1,6-naphthyridin-7-yl)oxy)-6-(((S)-1,1,1-trifluoropropan-2-yl)oxy)-2,3,11,11a-tetrahydro-1H,5H-benzo[f]pyrrolo[2,1-c][1,4]oxazepin-5-one
(9) (2S,11aR)-6-cyclobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(10) (2S,11aR)-8-methyl-2-((2-oxo-1,2,3,4-tetrahydro-1,6-naphthyridin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one
(11) (2S,11aR)-6-(((R)-1-fluoropropan-2-yl)oxy)-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one
(12) (2S,11aR)-6-(((R)-1,1-difluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(13) (2S,11aR)-6-(2,6-difluorophenyl)-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one
(14) (2S,11aR)-2-((8-fluoro-2-oxo-1,2,3,4-tetrahydro-1,6-naphthyridin-7-yl)oxy)-6-isopropoxy-8-methyl-2,3,11,11a-tetrahydro-1H,5H-benzo[f]pyrrolo[2,1-c][1,4]oxazepin-5-one
(15) (2S,11aR)-2-((8-fluoro-2-oxo-1,2,3,4-tetrahydroquinolin-7-yl)oxy)-6-isopropoxy-8-methyl-2,3, 11, 11a-tetrahydro-1H,5H-benzo[f]pyrrolo[2, 1-c][1,4]oxazepin-5-one
(16) (2S,11aR)-7-fluoro-6-isopropoxy-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one
(17) (2S,11aR)-6-isobutyl-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(18) (2S,11aR)-6-(cyclopentyloxy)-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(19) (2S,11aR)-8-methyl-2-((2-oxo-1,2-dihydro-1,6-naphthyridin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one

(20) (2S,11aR)-8-methyl-2-((2-oxo-1,2,3,4-tetrahydroquinolin-7-yl)oxy)-6-(((R)-1,1,1-trifluoropropan-2-yl)oxy)-2,3, 11, 11a-tetrahydro-1H,5H-benzo[f]pyrrolo[2, 1-c][1,4]oxazepin-5-one

(21) (2S,11aR)-2-((8-fluoro-2-oxo-1,2,3,4-tetrahydro-1,6-naphthyridin-7-yl)oxy)-8-methyl-6-(((R)-1,1,1-trifluoropropan-2-yl)oxy)-2,3,11,11a-tetrahydro-1H,5H-benzo[f]pyrrolo[2,1-c][1,4]oxazepin-5-one

(22) (2S,11aR)-8-chloro-6-isopropoxy-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(23) (2S,11aR)-6-isobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(24) (2S,11aR)-6-((R)-sec-butoxy)-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(25) (2S,11aR)-6-((S)-sec-butoxy)-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(26) (2S,11aR)-6-((2,2-difluorocyclopentyl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(27) (2S,11aR)-7,9-difluoro-6-isopropoxy-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(28) (2S,11aR)-7-fluoro-8-methyl-2-((2-oxo-1,2,3,4-tetrahydro-1,6-naphthyridin-7-yl)oxy)-6-(((R)-1, 1, 1-trifluoropropan-2-yl)oxy)-2,3, 11, 11a-tetrahydro-1H,5H-benzo[f]pyrrolo[2,1-c][1,4]oxazepin-5-one

(29) (2S,11aR)-6-(2-fluoro-2-methylpropoxy)-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(30) (2S,11aR)-8-methyl-2-((2-oxo-1,2,3,4-tetrahydro-1,6-naphthyridin-7-yl)oxy)-6-(pyrrolidin-1-yl)-2,3,11,11a-tetrahydro-1H,5H-benzo[f]pyrrolo[2,1-c][1,4]oxazepin-5-one

(31) (2S,11aR)-6-(cyclopropylmethoxy)-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(32) (2S,11aR)-7-fluoro-6-(2-fluoro-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(33) (2S,11aR)-7-fluoro-8-methyl-2-((2-oxo-1,2,3,4-tetrahydro-1,6-naphthyridin-7-yl)oxy)-6-(pyrrolidin-1-yl)-2,3, 11, 11a-tetrahydro-1H,5H-benzo[f]pyrrolo[2, 1-c][1,4]oxazepin-5-one

(34) (2S,11aR)-6-isopropoxy-2-((2-oxo-1,2,3,4-tetrahydro-1,6-naphthyridin-7-yl)oxy)-8-(trifluoromethyl)-2,3,11,11a-tetrahydro-1H,5H-benzo[f]pyrrolo[2,1-c][1,4]oxazepin-5-one

(35) (2S,11aR)-6-(cyclopropylmethoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(36) (2S,11aR)-7-fluoro-6-isobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(37) (2S,11aR)-7-fluoro-6-((1-fluorocyclopropyl)methoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(38) (2S,11aR)-9-fluoro-6-isopropoxy-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(39) (2S,11aR)-6-((R)-sec-butoxy)-7-fluoro-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(40) (2S,11aR)-6-((S)-sec-butoxy)-7-fluoro-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(41) (2S,11aR)-6-(cyclopentyloxy)-7-fluoro-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(42) (2S,11aR)-6-ethoxy-7-fluoro-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(43) (2S,11aR)-7-fluoro-8-methyl-2-((2-oxo-1,2,3,4-tetrahydro-1,6-naphthyridin-7-yl)oxy)-6-propoxy-2,3,11,11a-tetrahydro-1H,5H-benzo[f]pyrrolo[2,1-c][1,4]oxazepin-5-one

(44) (2S,11aR)-7-chloro-6-isopropoxy-8-methyl-2-((2-oxo-1,2,3,4-tetrahydro-1,6-naphthyridin-7-yl)oxy)-2,3, 11, 11a-tetrahydro-1H,5H-benzo[f]pyrrolo[2, 1-c][1,4]oxazepin-5-one

(45) (2S,11aR)-6-(((R)-1,1-difluoropropan-2-yl)oxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(46) (2S,11aR)-7-fluoro-6-(((R)-1-fluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(47) (2S,11aR)-7-fluoro-8-methyl-2-((2-oxo-1,2,3,4-tetrahydro-1,6-naphthyridin-7-yl)oxy)-6-(2,2,2-trifluoroethoxy)-2,3, 11,11 a-tetrahydro-1H,5H-benzo[f]pyrrolo[2, 1-c][1,4]oxazepin-5-one

(48) (2S,11aR)-7-fluoro-8-methyl-2-((2-oxo-1,2,3,4-tetrahydro-1,6-naphthyridin-7-yl)oxy)-6-(((S)-tetrahydrofuran-3-yl)oxy)-2,3,11,11a-tetrahydro-1H,5H-benzo[f]pyrrolo[2,1-c][1,4]oxazepin-5-one
(49) (2S,11aR)-7-fluoro-8-methyl-2-((2-oxo-1,2,3,4-tetrahydro-1,6-naphthyridin-7-yl)oxy)-6-(((R)-tetrahydrofuran-3-yl)oxy)-2,3, 11, 11a-tetrahydro-1H,5H-benzo[f]pyrrolo[2,1-c][1,4]oxazepin-5-one
(50) (2S,11aR)-6-((1,3-difluoropropan-2-yl)oxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

[16] An orexin type 2 receptor agonist comprising a compound or a pharmaceutically acceptable salt thereof according to any one of [1] to [15].
[17] A pharmaceutical composition comprising a compound or a pharmaceutically acceptable salt thereof according to any one of [1] to [15].
[18] A preventive drug or therapeutic drug for narcolepsy, idiopathic hypersomnia, hypersomnia, sleep apnea syndrome, narcolepsy syndrome with narcolepsy-like symptoms, hypersomnia associated with Parkinson's disease, hypersomnia associated with Guillain-Barre syndrome, hypersomnia associated with Kleine-Levin syndrome, or hypersomnia associated with Lewy body dementia, comprising a compound or a pharmaceutically acceptable salt thereof according to any one of [1] to [15].
In addition, the present invention may also include the following aspects.
[19] A compound or a pharmaceutically acceptable salt thereof according to any one of [1] to [12], wherein n is 1, m is 0, L is -O-, and Y is -CO-.
[20] Use of a compound or a pharmaceutically acceptable salt thereof according to any one of [1] to [15] in the manufacture of a therapeutic or preventive drug for a disease mediated by orexin type 2 receptor agonist activity.
[21] The use according to [20], wherein the disease mediated by orexin type 2 receptor agonist activity is selected from narcolepsy, idiopathic hypersomnia, hypersomnia, sleep apnea syndrome, narcolepsy syndrome with narcolepsy-like symptoms, hypersomnia associated with Parkinson's disease, hypersomnia associated with Guillain-Barre syndrome, hypersomnia associated with Kleine-Levin syndrome, and hypersomnia associated with Lewy body dementia.
[22] A compound or a pharmaceutically acceptable salt thereof according to any one of [1] to [15], for use in the treatment or prevention of a disease mediated by orexin type 2 receptor agonist activity.
[23] The compound or a pharmaceutically acceptable salt thereof for use according to [22], wherein the disease mediated by orexin type 2 receptor agonist activity is selected from narcolepsy, idiopathic hypersomnia, hypersomnia, sleep apnea syndrome, narcolepsy syndrome with narcolepsy-like symptoms, hypersomnia associated with Parkinson's disease, hypersomnia associated with Guillain-Barre syndrome, hypersomnia associated with Kleine-Levin syndrome, and hypersomnia associated with Lewy body dementia.

## EFFCTS OF THE INVENTION

The present invention provides compounds with excellent orexin type 2 receptor agonist activity.

## DESCRIPTION OF EMBODIMENTS

The present invention will be described below based on specific embodiments, but the invention is not limited to these embodiments in any way. All references cited herein, including patent publications, patent application publications, and non-patent documents, are incorporated herein by reference in their entirety for all purposes.
The term "pharmaceutically acceptable salt" as used herein is not limited to those salts that are acceptable for pharmacological uses and can form salts with the compounds of the present invention. Specific examples include: acid addition salts such as acetate, propionate, butyrate, formate, trifluoroacetate, maleate, fumarate, ditartrate, citrate, stearate, succinate, ethyl succinate, malonate, malate, benzoate, methane sulfonate, benzene sulfonate, p-toluene sulfonate, hydrochloride, hydrobromate, phosphorate, nitrate, sulfate, hydroiodate, nicotinate, and oxalate salts; inorganic base addition salts such as lithium, sodium, potassium, and calcium salts; organic base addition salts such as triethyl amine, pyridine, morpholine, and piperidine salts; and amino acid addition salts such as aspartate and glutamate salts.
The compounds of the present invention also include their anhydrides and solvates (including hydrates).
The compounds of the present invention also include isotopically labeled compounds. Such isotope elements include hydrogen, carbon, nitrogen, oxygen, fluorine, and chlorine isotopes.
The isotopically labeled compounds mentioned above can be prepared uniformly by performing the same procedure as disclosed in the examples below expect for using readily available isotopically labeled reagents in place of non-isotopically labeled reagents.
The compounds of the present invention may also be used in the form of prodrugs. Prodrugs are compounds that can be converted to the compounds of the present invention by reactions under physiological conditions in vivo by, e.g., enzymes or stomach acid, such as compounds that are enzymatically oxidized, reduced, hydrolyzed, etc., to the

compounds of the present invention, or compounds that are hydrolyzed by stomach acid, etc., to the compounds of the present invention.

In the definitions of substituents mentioned herein, the number of carbons may be denoted as, e.g., "$C_{1-6}$,". That is, "C1-6 alkyl," is synonymous with alkyl having 1 to 6 carbons.

Substituents which are not indicated herein explicitly with the term "may be substituted" refer to "unsubstituted" substituents.

On the other hand, when substituents are indicated herein explicitly with the term "may be substituted" the number of possible substituents, if any, is not limited and may be one or more as long as it is theoretically acceptable. The bonding position of each substituent may also be any position as long as it is scientifically acceptable, unless otherwise specified herein.

The term "$C_{1-6}$ alkyl" used herein refers to a saturated linear or branched chain hydrocarbon group having 1 to 6 carbons. Specific examples include methyl, ethyl, propyl, isopropyl, butyl, isobutyl, sec-butyl, tert-butyl, pentyl, isopentyl, neopentyl, and hexyl.

The term "$C_{3-8}$ cycloalkyl" as used herein refers to a non-aromatic cyclic hydrocarbon group having 3 to 8 carbons. The "$C_{3-8}$ cycloalkyl" includes those with partially unsaturated bonds and those with cross-linked structures. Specific examples include cyclopropyl, cyclobutyl, cyclopentyl, cyclohexyl, cycloheptyl, cyclooctyl, cyclopentenyl, and cyclohexenyl.

The term "3- to 10-membered heterocyclyl" as used herein refers to a monocyclic or bicyclic non-aromatic heterocyclic ring composed of 3 to 10 atoms containing, as atoms constituting the ring, 1 to 4 identical or different heteroatoms selected from the group consisting of oxygen, nitrogen and sulfur atoms, in addition to carbon atoms. The term "3-to 8-membered heterocyclyl" as used herein can also be understood in the same manner as mentioned above. The terms "3- to 10-membered heterocyclyl" and "3- to 8-membered heterocyclyl" include those with partially unsaturated bonds, partially cross-linked structures, and partial spiro ring structures. Specific examples include pyrrolidinyl, piperidinyl, morpholinyl, tetrahydrofuranyl, and tetrahydropyranyl.

The term "halogen" herein refers to a fluorine, chlorine, bromine or iodine atom.

The term "$C_{6-10}$ aryl" as used herein refers to a monocyclic or bicyclic aromatic hydrocarbon group whose ring-constituting atoms consist only of 6 to 10 carbon atoms. In the case of a bicyclic aryl group, if one ring is an aromatic ring, the other ring may be a non-aromatic ring structure. Specific examples include phenyl, 1-naphthyl, and 2-naphthyl.

The term "5- to 12-membered heteroaryl" as used herein refers to a monocyclic or bicyclic 5- to 12-membered aromatic heterocyclic ring containing 1 to 4 identical or different heteroatoms selected from the group consisting of an oxygen, nitrogen and sulfur atom in addition to one or more carbon atoms as ring-constituting atoms.

In the case of a bicyclic heteroaryl group, if one ring is an aromatic ring or aromatic heterocyclic ring, the other ring may be a non-aromatic ring structure. Specific examples include pyridyl, pyrimidinyl, pyrazinyl, pyridazinyl, pyrrolyl, pyrazolyl, azolyl, thiazolyl, indolyl, purinyl, tetrahydronaphthylidyl, 2-quinolyl, 4-quinolyl, and thiophenyl.

The term "$C_{1-6}$ alkoxy" as used herein refers to an oxy group substituted with a "$C_{1-6}$ alkyl" as mentioned above. Specific examples include methoxy, ethoxy, propoxy, isopropoxy, butoxy, isobutoxy, sec-butoxy, tert-butoxy, pentyloxy, isopentyloxy, and hexyloxy.

The term "$C_{3-8}$ cycloalkoxy" as used herein refers to an oxy group substituted with a "$C_{3-8}$ cycloalkyl" as mentioned above. Specific examples include cyclopropyloxy, cyclobutyloxy, cyclopentyloxy, cyclohexyloxy, cycloheptyloxy, and cyclooctyloxy.

The terms "3- to 8-membered heterocyclyloxy", "$C_{6-10}$ aryloxy", and "5- to 12-membered heteroaryloxy" as used herein refer to oxy groups substituted with a "3- to 8-membered heterocyclyl", "$C_{6-10}$ aryl", and "5- to 12-heteroaryl", respectively, as mentioned above.

The term "$C_{3-8}$ cycloalkyl $C_{1-6}$ alkoxy" as used herein refers to a "$C_{1-6}$ alkoxy" as mentioned above substituted with a "$C_{3-8}$ cycloalkyl" as mentioned above. Specific examples include combinations of any of the specific examples for the "$C_{3-8}$ cycloalkyl" mentioned above with any of the specific examples for the "$C_{1-6}$ alkoxy" mentioned above.

The term "3- to 8-membered heterocyclyl $C_{1-6}$ alkoxy" as used herein refers to a "$C_{1-6}$ alkoxy" as mentioned above substituted with a "3- to 8-membered heterocyclyl" as mentioned above. Specific examples include combinations of any of the specific examples for the "3- to 8-membered heterocyclyl" mentioned above with any of the specific examples for the "$C_{1-6}$ alkoxy" mentioned above.

The term "$C_{6-10}$ aryl $C_{1-6}$ alkoxy" as used herein refers to a "$C_{1-6}$ alkoxy" as mentioned above substituted with a "$C_{6-10}$ aryl" as mentioned above. Specific examples include combinations of any of the specific examples for the "$C_{6-10}$ aryl" mentioned above with any of the specific examples for the "$C_{1-6}$ alkoxy" mentioned above.

The term "$C_{5-12}$ heteroaryl $C_{1-6}$ alkoxy" as used herein refers to a "$C_{1-6}$ alkoxy" as mentioned above substituted with a "$C_{5-12}$ heteroaryl" as mentioned above. Specific examples include combinations of any of the specific examples for the "$C_{5-12}$ heteroaryl" mentioned above with any of the specific examples for the "$C_{1-6}$ alkoxy" mentioned above.

Preferable examples for $R^1$ in the compound represented by Formula (I) according to the present invention include $C_{1-6}$ alkyl group, $C_{3-8}$ cycloalkyl group, 3- to 8-membered heterocyclyl group, a hydroxy group, $C_{6-10}$ aryl group,

$C_{6-10}$ aryl group substituted with 1 to 6 halogens, $C_{6-10}$ aryl group substituted with 1 to 6 [$C_{1-6}$ alkyl groups substituted with 1 to 3 halogens], 5- to 12-membered heteroaryl group, 5- to 12-membered heteroaryl group substituted with 1 to 6 halogens, 5- to 12-membered heteroaryl group substituted with 1 to 6 [$C_{1-6}$ alkyl groups substituted with 1 to 3 halogens], $C_{1-6}$ alkoxy group, $C_{1-6}$ alkoxy group substituted with 1 to 6 halogens, $C_{1-6}$ alkoxy group substituted with 1 to 6 hydroxy groups, $C_{3-8}$ cycloalkoxy group, $C_{3-8}$ cycloalkoxy group substituted with 1 to 6 halogens, 3- to 8-membered heterocyclyloxy group, $C_{3-8}$ cycloalkyl $C_{1-6}$ alkoxy group, $C_{3-8}$ cycloalkyl $C_{1-6}$ alkoxy group substituted with 1 to 6 halogens, 3- to 8-membered heterocyclyl $C_{1-6}$ alkoxy group, and $C_{6-10}$ aryl $C_{1-6}$ alkoxy group.

Preferable examples for $R^2$ in the compound represented by Formula (I) according to the present invention include a hydrogen atom, $C_{1-6}$ alkyl group which may be substituted with 1 to 6 halogens, $C_{3-8}$ cycloalkyl group which may be substituted with 1 to 6 halogens, hydroxy group, and halogen, among which a hydrogen atom and halogen are more preferred.

Preferable examples for $R^3$ in the compound represented by Formula (I) according to the present invention include a hydrogen atom, $C_{1-6}$ alkyl group which may be substituted with 1 to 6 halogens, $C_{3-8}$ cycloalkyl group which may be substituted with 1 to 6 halogens, hydroxy group, and halogen, among which a hydrogen atom, halogen, $C_{1-6}$ alkyl group, and $C_{1-6}$ alkyl group which may be substituted with 1 to 6 halogens are more preferred.

Preferable examples for $R^4$ in the compound represented by Formula (I) according to the present invention include a hydrogen atom, $C_{1-6}$ alkyl group which may be substituted with 1 to 6 halogens, $C_{3-8}$ cycloalkyl group which may be substituted with 1 to 6 halogens, hydroxy group, and halogen, among which a hydrogen atom and halogen are more preferred.

Among $R^1$ to $R^4$ in Formula (I) according to the present invention mentioned above, each of the adjacent pairs of $R^1$ and $R^2$, $R^2$ and $R^3$, and $R^3$ and $R^4$ may be linked to each other via a single bond to form a 5- to 8-membered ring. Specific examples of compounds according to Formula (I) forming such rings include, but are not limited to, the following.

[Formula 7]

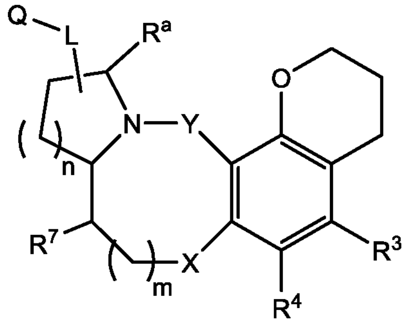

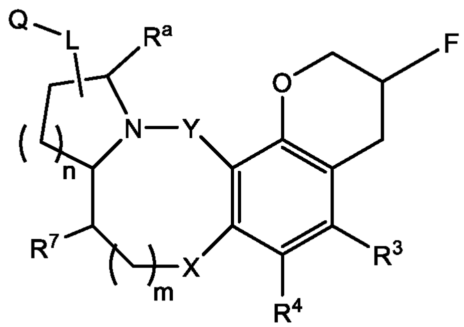

Preferable examples for $R^a$ in the compound represented by Formula (I) according to the present invention include a hydrogen atom.

Preferable examples for $R^7$ in the compound represented by Formula (I) according to the present invention include a hydrogen atom and $C_{1-6}$ alkyl group.

Preferable examples for X in the compound represented by Formula (I) according to the present invention include $-CR^bR^c-$ and -O-, of which -O- is more preferred. Preferable examples for each of $R^b$ and $R^c$ include a hydrogen atom.

Preferable examples for Y in the compound represented by Formula (I) according to the present invention include -CO-.

Preferable examples for n in the compound represented by Formula (I) according to the present invention include 1.

Preferable examples for m in the compound represented by Formula (I) according to the present invention include 0 and 1, of which 0 is more preferred.

Preferable examples for L in the compound represented by Formula (I) according to the present invention include -O- and -NH-, of which -O- is more preferred.

Preferable examples of the compounds represented by Formula (I) according to the present invention include the compounds represented by Formula (III) below.

[Formula 8]

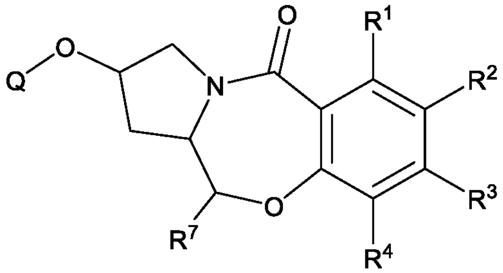

(III)

[In the formula,

$R^1$ represents a hydrogen atom, $C_{1-6}$ alkyl group, $C_{3-8}$ cycloalkyl group, 3- to 8-membered heterocyclyl group, hydroxy group, halogen, $C_{6-10}$ aryl group, 5- to 12-membered heteroaryl group, $C_{1-6}$ alkoxy group, $C_{3-8}$ cycloalkoxy group, 3- to 8-membered heterocyclyloxy group, $C_{6-10}$ aryloxy group, 5- to 12-membered heteroaryloxy group, $C_{3-8}$ cycloalkyl $C_{1-6}$ alkoxy group, 3- to 8-membered heterocyclyl $C_{1-6}$ alkoxy group, $C_{6-10}$ aryl $C_{1-6}$ alkoxy group, or 5- to 12-membered heteroaryl $C_{1-6}$ alkoxy group;

$R^1$ may be substituted with 1 to 6 [halogens, hydroxy groups, carboxyl groups, oxo groups, amino groups, cyano groups, $C_{1-6}$ alkyl groups which may be substituted with 1 to 3 halogens, or $C_{1-6}$ alkoxy groups which may be substituted with 1 to 3 halogens];

$R^2$, $R^3$ and $R^4$ each represent, independently of each other, a hydrogen atom, $C_{1-6}$ alkyl group which may be substituted with 1 to 6 halogens, or halogen;

$R^7$ represents a hydrogen atom, halogen, or $C_{1-6}$ alkyl group which may be substituted with 1 to 3 halogens;

Q represents a 3- to 10-membered heterocyclyl group which may be substituted with 1 to 4 $R^{11}$, $C_{6-10}$ aryl group which may be substituted with 1 to 4 $R^{12}$, or 5- to 12-membered heteroaryl group which may be substituted with 1 to 4 $R^{13}$,

$R^{11}$, $R^{12}$ and $R^{13}$ each represent, independently of each other, a halogen, oxo group, or $C_{1-6}$ alkyl group which may be substituted with 1 to 3 halogens.]

Specific examples, preferred examples, etc., in the compounds represented by Formula (III) of the present invention are the same as those for the compounds represented by Formula (I) of the present invention described above and described below.

When $R^1$ in the compound represented by Formula (I) according to the present invention is a "$C_{1-6}$ alkyl group", preferable examples of the "$C_{1-6}$ alkyl group" include isopropyl, isobutyl, and pentyl.

When $R^1$ in the compound represented by Formula (I) according to the present invention is a "$C_{3-8}$ cycloalkyl group", preferable examples of the "$C_{3-8}$ cycloalkyl group" include cyclohexyl and cyclopentyl.

When $R^1$ in the compound represented by Formula (I) according to the present invention is a "3- to 8-membered heterocyclyl group", preferable examples of the "3- to 8-membered heterocyclyl group" include pyrrolidine.

When $R^1$ in the compound represented by Formula (I) according to the present invention is a "$C_{6-10}$ aryl group", preferable examples of the "$C_{6-10}$ aryl group" include phenyl. The phenyl may be substituted with a substituent such as at least one fluorine atom or at least one methyl each substituted with at least one fluorine atom.

When $R^1$ in the compound represented by Formula (I) according to the present invention is a "5- to 12-membered heteroaryl", preferable examples of the "5- to 12-membered heteroaryl" include pyridyl or thienyl. The pyridyl may be substituted with a substituent such as at least one fluorine atom.

When $R^1$ in the compound represented by Formula (I) according to the present invention is a "$C_{1-6}$ alkoxy", preferable examples of the "$C_{1-6}$ alkoxy" include a methoxy, ethoxy, propoxy, isopropoxy, isobutoxy, sec-butoxy, pentyloxy, and neopentyloxy. The $C_{1-6}$ alkoxy (e.g., ethoxy, propoxy, isopropoxy, isobutoxy) may be substituted with a substituent such as at least one fluorine atom or at least one hydroxy group.

When $R^1$ in the compound represented by Formula (I) according to the present invention is a "$C_{3-8}$ cycloalkoxy", preferable examples of the "$C_{3-8}$ cycloalkoxy" include cyclobutyloxy, cyclopentyloxy, and bicycloheptyloxy. The cyclopentyloxy may be substituted with a substituent such as at least one fluorine atom.

When $R^1$ in the compound represented by Formula (I) according to the present invention is a "3- to 8-membered heterocyclyloxy", preferable examples of the "3- to 8-membered heterocyclyloxy" include tetrahydrofuranyloxy.

When $R^1$ in the compound represented by Formula (I) according to the present invention is a "$C_{3-8}$ cycloalkyl $C_{1-6}$ alkoxy", preferable examples of the "$C_{3-8}$ cycloalkyl $C_{1-6}$ alkoxy" include cyclopropyl methoxy. The cyclopropyl methoxy may be substituted with a substituent such as at least one fluorine atom.

When $R^1$ in the compound represented by Formula (I) according to the present invention is a "3- to 8-membered

heterocyclyl $C_{1-6}$ alkoxy", preferable examples of the "3-to 8-membered heterocyclyl $C_{1-6}$ alkoxy" include tetrahydrofuranylmethoxy and pyrrolidinylethoxy.

When $R^1$ in the compound represented by Formula (I) according to the present invention is a "$C_{6-10}$ aryl $C_{1-6}$ alkoxy", preferable examples of the "$C_{6-10}$ aryl $C_{1-6}$ alkoxy" include benzyloxy.

When $R^2$ in the compound represented by Formula (I) according to the present invention is a "halogen", preferable examples of the "halogen" include a fluorine atom and chlorine atom.

When $R^2$ in the compound represented by Formula (I) according to the present invention is a "$C_{1-6}$ alkyl group", preferable examples of the "$C_{1-6}$ alkyl group" include propyl.

When $R^3$ in the compound represented by Formula (I) according to the present invention is a "halogen", preferable examples of the "halogen" include a chlorine atom.

When $R^3$ in the compound represented by Formula (I) according to the present invention is a "$C_{1-6}$ alkyl group which may be substituted with 1 to 6 halogens", preferable examples of the "$C_{1-6}$ alkyl group which may be substituted with 1 to 6 halogens" include methyl or trifluoromethyl.

When $R^4$ in the compound represented by Formula (I) according to the present invention is a "halogen", preferable examples of the "halogen" include a fluorine atom or chlorine atom.

When $R^7$ in the compound represented by Formula (I) according to the present invention is a "$C_{1-6}$ alkyl group which may be substituted with 1 to 6 halogens", preferable examples of the "$C_{1-6}$ alkyl group which may be substituted with 1 to 6 halogens" include unsubstituted methyl.

Preferable examples for Q in the compound represented by Formula (I) according to the present invention include $C_{6-10}$ aryl group which may be substituted with 1 to 4 $R^{12}$ and 5- to 12-membered heteroaryl group which may be substituted with 1 to 4 $R^{13}$, among which bicyclic $C_{6-10}$ aryl group and 5- to 12-membered heteroaryl group are more preferred.

Preferable examples for each of $R^{12}$ and $R^{13}$ in Q of the compound represented by Formula (I) according to the present invention include an oxo group.

Preferable examples for Q in the compound represented by Formula (I) according to the present invention include the moieties represented by any one selected from the group consisting of Formulae (II-1), (II-2), (II-3) and (II-4) below.

[Formula 9]

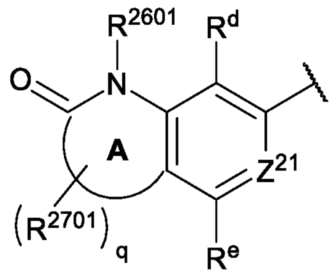

(II-1)

[Formula 10]

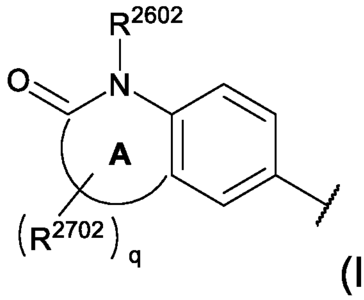

(II-2)

[Formula 11]

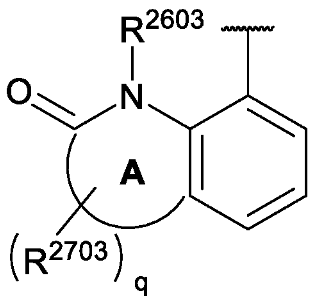

(II-3)

[Formula 12]

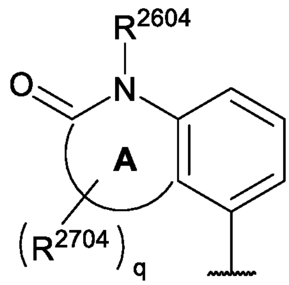

(II-4)

[In Formulae (II-1), (II-2), (II-3) and (II-4),

$Z^{21}$ represents $CR^f$ or N,
Ring A represents a 6- to 8-membered monocyclic heterocyclyl group or 6- to 7-membered monocyclic heteroaryl group,
$R^{2701}$, $R^{2702}$, $R^{2703}$ and $R^{2704}$ each represent, independently of each other, a halogen or $C_{1-6}$ alkyl group which may be substituted with 1 to 3 halogens,
$R^d$, $R^e$, and $R^f$ each represent, independently of each other, a hydrogen atom, halogen, or $C_{1-6}$ alkyl group which may be substituted with 1 to 3 halogens,
$R^{2601}$, $R^{2602}$, $R^{2603}$ and $R^{2604}$ each represent, independently of each other, a hydrogen atom, $C_{1-6}$ alkyl group which may be substituted with 1 to 6 halogens, or $C_{3-8}$ cycloalkyl group which may be substituted with 1 to 6 halogens, and
q each represent, independently of each other, an integer of 0 to 4.]

$R^{2701}$, $R^{2702}$, $R^{2703}$ and $R^{2704}$ in the compounds represented by Formulae (II-1), (II-2), (II-3) and (II-4), respectively, mean that 1 to 4 hydrogen atoms each bonded to a carbon atom constituting Ring A may be replaced by 1 to 4 $R^{2701}$, $R^{2702}$, $R^{2703}$ or $R^{2704}$. When Ring A is substituted with 1 to 4 $R^{2701}$, $R^{2702}$, $R^{2703}$ or $R^{2704}$, it is obvious that the $R^{2701}$, $R^{2702}$, $R^{2703}$ and $R^{2704}$ each replace a hydrogen atom attached to a carbon atom other than oxo group, N-$R^{2601}$, N-$R^{2602}$, N-$R^{2603}$, N-$R^{2604}$, and fused portions in the Ring A. In addition, q of ($R^{2701}$), ($R^{2702}$), ($R^{2703}$) and ($R^{2704}$) in each formula being an integer of 0 to 4 means that each moiety is either unsubstituted or substituted with 1 to 4 ($R^{2701}$), ($R^{2702}$), ($R^{2703}$) or ($R^{2704}$).

Preferable examples for $R^{2601}$, $R^{2602}$, $R^{2603}$ and $R^{2604}$ in the compounds represented by Formula (II-1), (II-2), (II-3) or (II-4) according to the present invention include a hydrogen atom. Preferable examples for $R^{2701}$, $R^{2702}$, $R^{2703}$ and $R^{2704}$ in the compounds represented by Formula (II-1), (II-2), (II-3) or (II-4) according to the present invention include a hydrogen atom, halogen, and $C_{1-6}$ alkyl group. Preferable examples of the "halogen" include a fluorine atom, and preferable examples of "$C_{1-6}$ alkyl group" include methyl.

Preferable examples for $R^d$ in the present invention in the compounds represented by Formula (II-1) according to the present invention include a halogen. Preferable examples of "halogen" include a fluorine atom and chlorine atom. Preferable examples for $R^e$ in the present invention in the compounds represented by Formula (II-1) according to the present invention include a hydrogen atom. Preferable examples for $R^f$ in the compounds represented by Formula (II-1) according to the present invention include a halogen. Preferable examples of the "halogen" include a fluorine atom.

Preferable examples of the "6- to 8-membered monocyclic heterocyclyl group or 6- to 7-membered monocyclic heteroaryl group" as A in the compounds represented by Formula (II-1), (II-2), (II-3) or (II-4) according to the present

invention include a tetrahydropyridine ring, dihydropyridine ring, dihydrooxazine ring or tetrahydroazepine ring.

Preferable examples for Q in the compound represented by Formula (I) according to the present invention include moieties represented by Formula (IV), (V) or (VI) below.

[Formula 13]

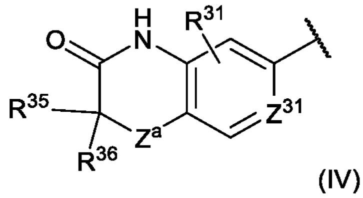

(IV)

[Formula 14]

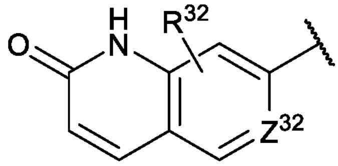

(V)

[Formula 15]

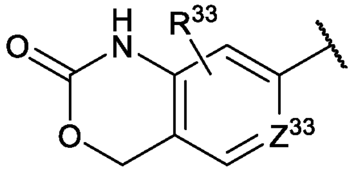

(VI)

[In Formulae (IV), (V) and (VI),

$R^{31}$, $R^{32}$, $R^{33}$, $R^{35}$ and $R^{36}$ each represent, independently of each other, a hydrogen atom, $C_{1-6}$ alkyl group which may be substituted with 1 to 6 halogens, hydroxy group or halogen,
$Z^{31}$, $Z^{32}$and $Z^{33}$ each represent, independently of each other, CH or N,
$Z^a$ represents O or $CR^g{}_2$,
$R^g$ represents, independently of each other, a hydrogen atom or $C_{1-6}$ alkyl group which may be substituted with 1 to 6 halogens.]

Preferable examples for $R^{31}$ in the compound represented by Formula (IV) according to the present invention include a hydrogen atom and halogen.

Preferable examples for each of $R^{35}$ and $R^{36}$ in the compound represented by Formula (IV) according to the present invention include a hydrogen atom, $C_{1-6}$ alkyl group, and halogen. Preferable examples of the "$C_{1-6}$ alkyl group" include methyl, and preferable examples of the "halogen" include a fluorine atom.

Preferable examples for $Z^a$ in the compound represented by Formula (IV) according to the present invention include $CR^g{}_2$. Preferable examples of $R^g$ include a hydrogen atom.

Preferable examples for $R^{32}$ in the compound represented by Formula (V) according to the present invention include a hydrogen atom.

Preferable examples for $R^{33}$ in the compound represented by Formula (VI) according to the present invention include a hydrogen atom.

When $R^{31}$ in the compound represented by Formula (IV) according to the present invention is a "halogen", preferable examples of the "halogen" include a fluorine atom and chlorine atom.

Examples of Q in the compounds represented by Formula (I) according to the present invention include the

moieties represented by any one of Q1 to Q19 below, among which the moieties represented by any one of Q1 to Q8 are preferred.

[Formula 16]

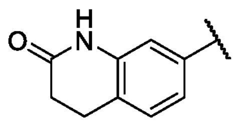
Q1

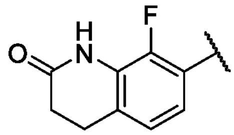
Q2

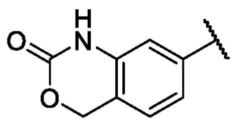
Q3

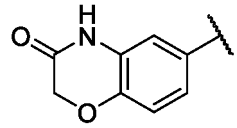
Q4

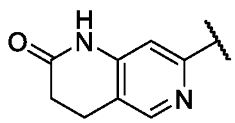
Q5

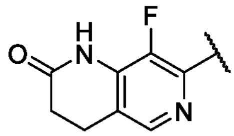
Q6

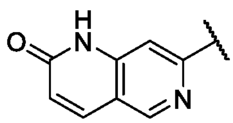
Q7

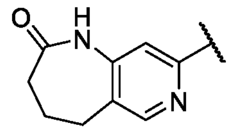
Q8

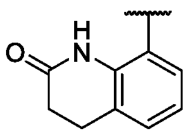
Q9

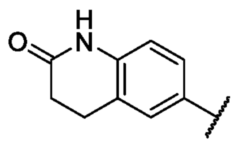
Q10

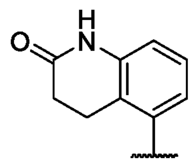
Q11

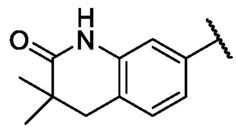
Q12

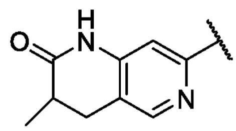
Q13

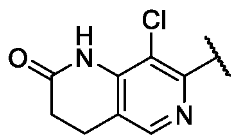
Q14

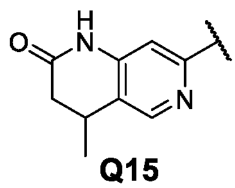
Q15

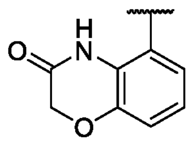
Q16

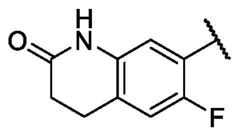
Q17

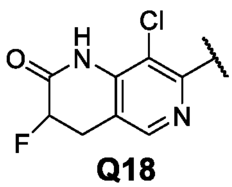
Q18

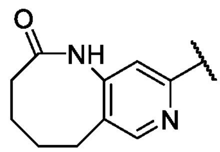
Q19

The definitions, specific examples, and preferred examples of each substituent in the compounds represented by Formula (I) of the present invention were explained above. In this regard, compounds having any combinations selected from the specific and preferred examples of each substituent mentioned above are also preferable as compounds represented by Formula (I) of the present invention.

The following are general synthetic methods for the compound represented by Formula (I) according to the present invention.

The compounds represented by Formula (I) according to the present invention (hereinafter also referred to as the compounds of the present invention) can be synthesized by a combination of methods known in the art, including the synthetic methods described below.

The compounds represented by Formula (I) according to the present invention and pharmaceutically acceptable salts thereof (hereinafter also referred to collectively as the compounds of the present invention) can be synthesized by a combination of methods known in the art, including the synthetic methods described below.

Each compound in each step of each reaction formula may form a salt. Examples of such salts include those similar to the salts of the compounds represented by Formula (I) of the invention.

Each of these salts can be converted to its free form or other types of salts of interest by known methods.

Conversely, if any compound obtained in each step is in a free form, it can be converted to a desired salt by known methods.

Protection and deprotection of each functional group in each step can be carried out with a protective group suitable for the functional group using a method commonly used in the art, such as those described in T. W. Greene & P. G. M. Wuts, "Protective Groups in Organic Synthesis, 5th Edition," (2014), John Wiley & Sons Inc., or those described in the Examples.

If a raw material or reagent for each compound in each step is commercially available, such a commercially available product can be used as it is.

Each compound obtained in each step may be isolated and purified by methods such as column chromatography, recrystallization, and distillation, or it may be used directly in the next step without isolation.

When the reaction formula of each step contains optical isomers, stereoisomers, and rotational isomers, these may also be included in the reaction formula, and each of these isomers can be obtained as a single product by synthesis and separation methods commonly used in the art. For example, if the compound represented by formula (I) of the present invention has optical isomers, each of the optical isomers divided from the compound may also be included in the scope of the compound represented by formula (I) of the present invention.

Typical synthetic methods for the compounds of the present invention will be explained below. The symbols in each formula are as defined in Formula (I).

*Production Method 1:

The compounds represented by Formula (I-1) can be produced by, e.g., the following methods.

[Formula 17]

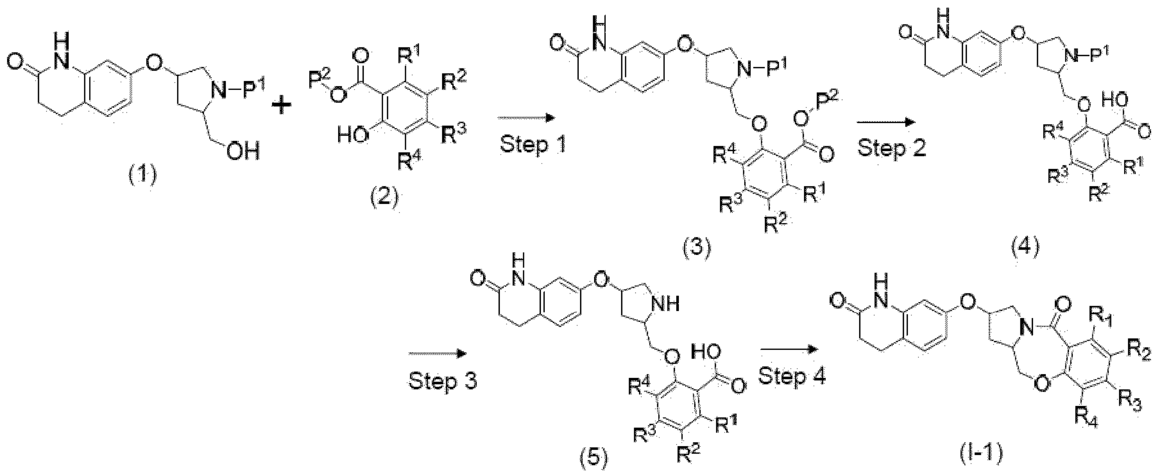


[Each symbol in the formula indicates the same meaning as mentioned above. $P^1$ and $P^2$ indicate appropriate protective groups.]

(Step 1)

This step is to subject Compound (1) and Compound (2) to a general dehydration-condensation reaction in the presence of a phosphine compound and an azo compound or in the presence of a phosphorane compound to thereby produce Compound (3).

The reaction in this process can be performed by a method known as the so-called Mitsunobu reaction. Examples of phosphine compounds used herein include triphenylphosphine and tributylphosphine. Examples of azo compounds used herein include diethyl azodicarboxylate and diisopropyl azodicarboxylate. Examples of phosphorane compounds used herein include cyanomethylene trimethylphosphorane, and cyanomethylene tributylphosphorane. The reaction may typically be carried out in an inert solvent, and examples of such inert solvents used herein include tetrahydrofuran, dichloromethane, benzene, toluene, and mixtures of these solvents. The reaction time may typically be from 1 hour to 24 hours, and the reaction temperature may be from -20°C to the boiling point of the solvent.

(Step 2)

This step is to remove the protective group $P^2$ of Compound (3) by a known method to thereby produce

Compound (4).

(Step 3)

This step is to remove the protective group $P^1$ of Compound (4) by a known method to thereby produce Compound (5).

(Step 4)

This step is to subject Compound (5) to general amide bond formation conditions to thereby produce Compound (I-1) according to the present invention. This reaction can be carried out using a condensing agent known to those skilled in the art or by an ester activation method, mixed acid anhydride method, acid chloride method, carbodiimide method, etc., available to those skilled in the art. Examples of such amide bond formation reagents used herein include thionyl chloride, oxalyl chloride, N,N'-dicyclohexyl carbodiimide, 1-methyl-2-chloropyridinium iodide, N,N'-carbonyl diimidazole, diphenylphosphoryl chloride, diphenylphosphorylazide, N,N'-disuccinimidyl carbonate, N,N'-disuccinimidyl oxalate, 1-ethyl-3-(3-dimethylaminopropyl)carbodiimide hydrochloride, 1H-benzotriazol-1-yloxytripyrrolidino phosphonium hexafluorophosphate, 2-(1H-benzotriazol-1-yl)-1,1,3,3-tetramethyluronium hexafluorophosphate, 2-(5-norbornene-2,3-dicarboxylimide)-1,1,3,3-tetramethyluronium tetrafluoroborate, O-(N-scusinimidyl)-1,1,3,3-tetramethyluronium tetrafluoroborate, bromotripyrrolidinophosphonium hexafluorophosphate, ethyl chloroformate, isobutyl chloroformate, and 2-(7-aza-1H-benzotriazol-1-yl)-1,1,3,3-tetramethyluronium hexafluorophosphate, propylphosphonic acid anhydride, among which 1-ethyl-3-(3-dimethylaminopropyl)carbodiimide hydrochloride or 2-(7-aza-1H-benzotriazol-1-yl)-1,1,3,3-tetramethyluronium hexafluorophosphate is preferred. In addition to the amide bond formation reagent mentioned above, it is also possible to use a base and/or a condensation aid in the amide bond formation reaction. Bases that can be used are not particularly limited as long as they are organic bases. Preferable examples include tertiary aliphatic amines, among which triethylamine and N,N-diisopropylethylamine are more preferable. Examples of condensation aids used herein include 1-hydroxy benzotriazole hydrate and N-hydroxy succinimide. Each of the base, the amide formation reagent, and/or the condensation aid used in this reaction may be either any one kind or a combination of any two or more. The reaction may typically be carried out in an inert solvent. Examples of such inert solvents used herein include tetrahydrofuran, acetonitrile, N,N-dimethyl formamide, ethyl acetate, 1,4-dioxane, benzene, toluene, dichloromethane, chloroform, carbon tetrachloride, 1,2-dichloroethane, and pyridine, as well as mixtures of these solvents. The reaction time may typically be from 0.5 hours to 96 hours, preferably from 1 hour to 24 hours. The reaction temperature may typically be from -78°C to 100°C, preferably from -20°C to the boiling point of the solvent.

*Production Method 2:

The compounds represented by Formula (I-1) can also be produced by, e.g., the following methods.

[Formula 18]

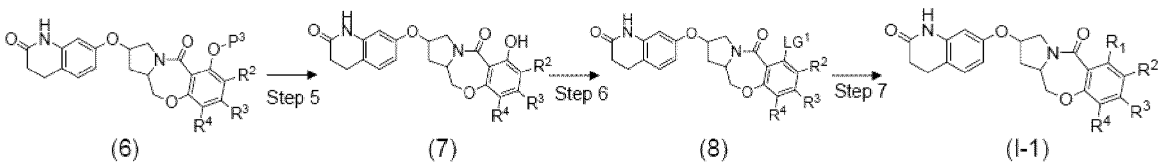


(6) (7) (8) (I-1)

[Each symbol in the formula indicates the same meaning as mentioned above. $P^3$ represents an appropriate protective group, and $LG^1$ represents an appropriate leaving group.]

(Step 5)

This step is to remove the protective group $P^3$ of Compound (6) by a known method to thereby produce Compound (7).

(Step 6)

This step is to subject the hydroxy group of Compound (7) to conditions for conversion reaction to a common leaving group to thereby produce Compound (8). Examples of such reaction conditions include conditions using methane sulfonyl chloride, p-toluene sulfonyl chloride, or trifluoromethane sulfonic anhydride. A base may also be used in this

reaction. Examples of bases used herein include: organic bases such as triethylamine, N,N-diisopropylethylamine, pyridine, and 1,8-diazabicyclo[5.4.0]undeca-7-ene; alkali metal hydride such as sodium hydride and potassium hydride; inorganic bases such as sodium bicarbonate, sodium carbonate, and potassium carbonate; metal alkoxides such as sodium methoxide and potassium tert-butoxide; organic metal reagents such as n-butyl lithium and isopropyl magnesium chloride; and metal amide reagents such as LDA and LHMDS. The reaction may typically be carried out in an inert solvent, and examples of such inert solvents used herein include tetrahydrofuran, acetonitrile, N,N-dimethyl formamide, ethyl acetate, 1,4-dioxane, benzene, toluene, dichloromethane, chloroform, carbon tetrachloride, 1,2-dichloroethane, and pyridine, as well as mixtures of these solvents. The reaction time may typically be from 1 hour to 24 hours, and the reaction temperature may be from -78°C to the boiling point of the solvent.

(Step 7)

This step is to subject the leaving group of Compound (8) to reaction with, e.g., a hydroxy compound, amine compound, organic boron compound, alkenyl compound, alkylyl compound, or organic cyanogen compound, in the presence of a base and a metal catalyst, and optionally in the presence of a phosphine ligand, to thereby produce Compound (I-1) according to the present invention. The hydroxy compound, amine compound, organic boron compound, alkenyl compound, alkylyl compound, or organic cyanogen compound to be used may be either purchased commercially or synthesized by known or similar methods. Examples of bases used herein include: inorganic bases such as sodium bicarbonate, sodium carbonate, potassium carbonate, cesium carbonate, potassium phosphate, and sodium hydroxide; organic salts such as ammonium acetate; metal alkoxides such as sodium methoxide and sodium tert-butoxide; and organic bases such as triethylamine, N,N-diisopropylethylamine, 1,4-diazabicyclo[2.2.2]octane, 1,8-diazabicyclo[5.4.0]undeca-7-ene, and pyridine. A base may not be used depending on the type of coupling. Examples of metal catalysts used herein include bis(tri-o-tolylphosphine) palladium (0), tetrakis(triphenylphosphine) palladium (0), bis(dibenzylideneacetone) palladium (0), tris(dibenzylideneacetone) dipalladium (0), [1,1'-bis(diphenylphosphino)ferrocene] palladium (II) dichloride, and XPhos Pd G3. It is also possible to combine, e.g., palladium acetate with an appropriate ligand selected from those described in "Palladium Reagents and Catalysts," (2004), John Wiley & Sons Inc., or analogous ligands. Examples of phosphine ligands used herein include tri(o-tolyl)phosphine, SPhos, XPhos, DPPF, BINAP, and XantPhos. A phosphine ligand may not be used depending on the type of coupling. The reaction may typically be carried out in an inert solvent, and examples of such inert solvents used herein include tetrahydrofuran, acetonitrile, N,N-dimethyl formamide, 1,4-dioxane, toluene, 1,2-dimethoxy ethane, and water, as well as mixtures of these solvents. The reaction time may typically be from 0.5 hours to 96 hours, preferably from 1 hour to 24 hours. The reaction temperature may typically be from 0°C to 200°C, preferably from room temperature to 100°C.

<u>*Production Method 3:</u>

The compounds represented by Formula (I-2) can be produced by, e.g., the following methods.

[Formula 19]

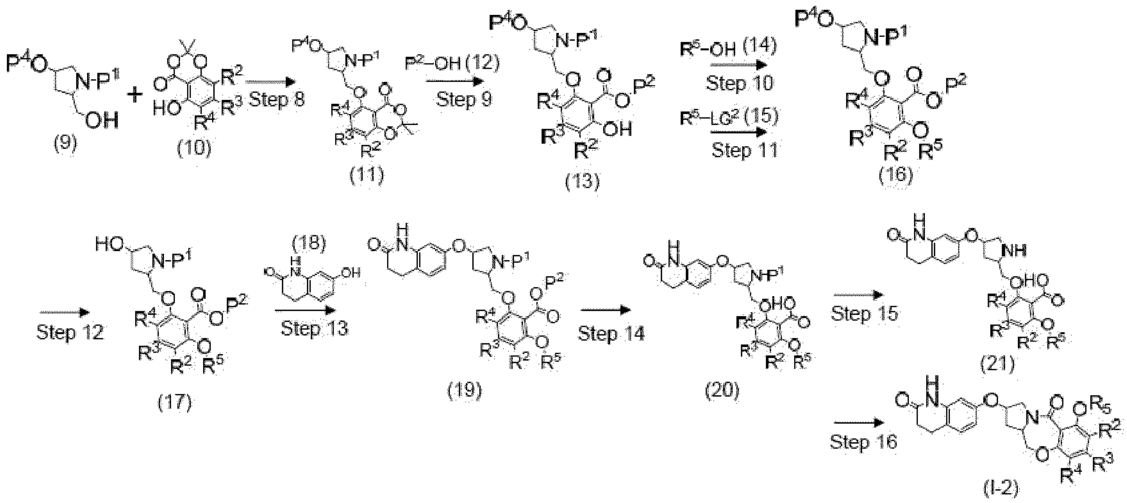


[Each symbol in the formula indicates the same meaning as mentioned above. $P^1$, $P^2$, and $P^4$ represent, independently of each other, an appropriate protective group, and $LG^2$ represents an appropriate leaving group. $R^5$ represents an

unsubstituted or substituted $C_{1-6}$ alkyl group, $C_{3-8}$ cycloalkyl group, 3- to 8-membered heterocyclyl group, $C_{6-10}$ aryl group, or $C_{5-12}$ heteroaryl group.]

(Step 8)

This step is to subject Compound (9) and Compound (10) to conditions similar to those in Step 1 of Production Method 1 to thereby produce Compound (11).

(Step 9)

This step is to subject Compound (11) to reaction with an alcohol represented by Formula (12) in the presence of a base to thereby produce Compound (13). Examples of bases used herein include: alkali metal hydrides such as sodium hydride and potassium hydride; alkali metal hydroxides such as lithium hydroxide, sodium hydroxide, and potassium hydroxide; alkali metal carbonates such as sodium carbonate, potassium carbonate, and cesium carbonate; alkali metal hydrocarbonates such as sodium bicarbonate and potassium bicarbonate; metal alkoxides such as sodium methoxide, sodium ethoxide, and potassium tert-butoxide; tertiary aliphatic amines such as trimethylamine, triethylamine, N,N-diisopropylethylamine, N-methyl morpholine, N-methyl pyrrolidine, N-methyl piperidine, N,N-dimethyl aniline, 1,8-diazabicyclo[5.4.0]undeca-7-ene, and 1,5-azabicyclo[4.3.0]nona-5-ene; and aromatic amines such as pyridine, 4-dimethylamino pyridine, picoline, lutidine, quinolin, and isoquinolin. Examples of alcohols represented by Formula (12) include methanol, ethanol, 2-propanol, and benzylalcohol, which are usually used also as a solvent. The reaction time may typically be from 1 hour to 24 hours, and the reaction temperature may be from -20°C to the boiling point of the solvent.

(Step 10)

This step is to subject Compound (13) and an alcohol represented by Formula (14) to conditions similar to those of Step 1 of Production Method 1 to thereby produce Compound (16). The alcohol represented by Formula (14) may be either purchased commercially or synthesized by known or similar methods. Examples thereof include methanol, ethanol, 2-propanol, butanol, neopentyl alcohol, and benzylalcohol.

(Step 11)

This step is to subject Compound (13), along with a compound having a leaving group represented by Formula (15), to conditions for nucleophilic substitution reaction in the presence of a base to thereby produce Compound (16). The compound represented by Formula (15) may be either purchased commercially or synthesized by known or similar methods. Examples thereof include: alkyl halides such as iodomethane, iodoethane, 2-iodopropane, benzyl bromide, (iodomethyl)cyclopropane, 1-chloro-2-methyl-2-propanol, and bromo cyclopentane; and alkyl sulfonate esters such as 1,1,1-trifluoropropan-2-yl trifluoromethane sulfonate. Examples of bases used herein include: alkali metal hydrides such as sodium hydride and potassium hydride; alkali metal hydroxides such as lithium hydroxide, sodium hydroxide, and potassium hydroxide; alkali metal carbonates such as sodium carbonate, potassium carbonate, and cesium carbonate; alkali metal hydrocarbonates such as sodium bicarbonate and potassium bicarbonate; metal alkoxides such as sodium methoxide, sodium ethoxide, and potassium tert-butoxide; tertiary aliphatic amines such as trimethylamine, triethylamine, N,N-diisopropylethylamine, N-methyl morpholine, N-methyl pyrrolidine, N-methyl piperidine, N,N-dimethyl aniline, 1,8-diazabicyclo[5.4.0]undeca-7-ene, and 1,5-azabicyclo[4.3.0]nona-5-ene; and aromatic amines such as pyridine, 4-dimethylamino pyridine, picoline, lutidine, quinolin, and isoquinolin. The reaction may typically be carried out in an inert solvent, and examples of such inert solvents used herein include tetrahydrofuran, acetonitrile, dimethyl sulfoxide, N,N-dimethyl formamide, 1,4-dioxane, benzene, toluene, acetone, methylethylketone, ethyl acetate, dichloromethane, chloroform, carbon tetrachloride, 1,2-dichloroethane, and pyridine, as well as mixtures of these solvents. The reaction time may typically be from 1 hour to 24 hours, and the reaction temperature may be from -20°C to the boiling point of the solvent.

(Step 12)

This step is to remove the protective group $P^4$ of Compound (16) by a known method to thereby produce Compound (17).

(Step 13)

This step is to subject Compound (17) and Compound (18) to conditions similar to those of Step 1 of Production

Method 1 to thereby produce Compound (19).

(Step 14)

This step is to subject Compound (19) to conditions similar to those of Step 2 of Production Method 1 to thereby produce Compound (20).

(Step 15)

This step is to subject Compound (20) to conditions similar to those of Step 3 of Production Method 1 to thereby produce Compound (21).

(Step 16)

This step is to subject Compound (21) to conditions similar to those of Step 4 of Production Method 1 to thereby produce Compound (I-2) according to the present invention.

*Production Method 4:

The compounds represented by Formula (19) can be produced by, e.g., the following methods.

[Formula 20]

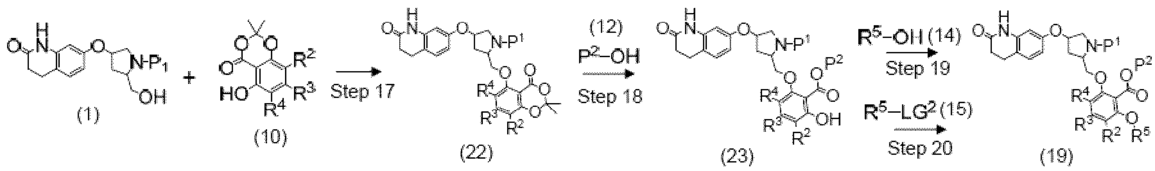


[Each symbol in the formula indicates the same meaning as mentioned above. $P^1$ and $P^2$ represent, independently of each other, an appropriate protective group, and $LG^2$ represents an appropriate leaving group. $R^5$ represents an unsubstituted or substituted $C_{1-6}$ alkyl group, $C_{3-8}$ cycloalkyl group, 3- to 8-membered heterocyclyl group, $C_{6-10}$ aryl group, or $C_{5-12}$ heteroaryl group.]

(Step 17)

This step is to subject Compound (1) and Compound (10) to conditions similar to those of Step 1 of Production Method 1 to thereby produce Compound (22).

(Step 18)

This step is to subject Compound (22) to conditions similar to those of Step 9 of Production Method 3 to thereby produce Compound (23).

(Step 19)

This step is to subject Compound (23) to conditions similar to those of Step 10 of Production Method 3 to thereby produce Compound (19).

(Step 20)

This step is to subject Compound (23) to conditions similar to those of Step 11 of Production Method 3 to thereby produce Compound (19).

*Production Method 5:

The compounds represented by Formula (I-3) can be produced by, e.g., the following methods.

[Formula 21]

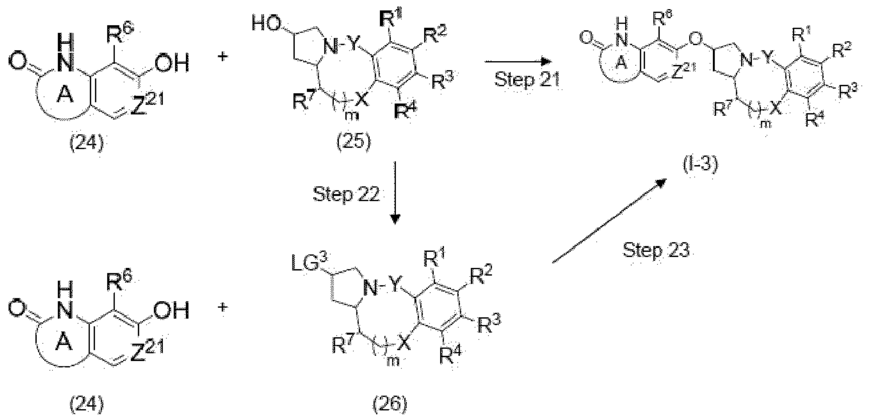


[Each symbol in the formula indicates the same meaning as mentioned above. $LG^3$ represents an appropriate leaving group. $R^6$ represents a hydrogen atom, unsubstituted or substituted $C_{1-6}$ alkyl group, unsubstituted or substituted $C_{3-8}$ cycloalkyl group, unsubstituted or substituted 3- to 8-membered heterocyclyl group, hydroxy group, or halogen.]

(Step 21)

This step is to subject Compound (24) and Compound (25) to conditions similar to those of Step 1 of Production Method 1 to thereby produce Compound (I-3) according to the present invention.

(Step 22)

This step is to subject Compound (25) to conditions for conversion reaction to a common leaving group similar to those of Step 6 of Production Method 2 to thereby produce Compound (26).

(Step 23)

This step is to subject Compound (24), along with Compound (26) having a leaving group, to conditions for nucleophilic substitution reaction in the presence of a base to thereby produce Compound (I-3) according to the present invention. Examples of bases used herein include: alkali metal hydrides such as sodium hydride and potassium hydride; alkali metal hydroxides such as lithium hydroxide, sodium hydroxide, potassium hydroxide; alkali metal carbonates such as sodium carbonate, potassium carbonate, and cesium carbonate; alkali metal hydrocarbonates such as sodium bicarbonate and potassium bicarbonate; metal alkoxides such as sodium methoxide, sodium ethoxide, and potassium tert-butoxide; tertiary aliphatic amines such as trimethylamine, triethylamine, N,N-diisopropylethylamine, N-methyl morpholine, N-methyl pyrrolidine, N-methyl piperidine, N,N-dimethyl aniline, 1,8-diazabicyclo[5.4.0]undeca-7-ene, and 1,5-diazabicyclo[4.3.0]nona-5-ene; and aromatic amines such as pyridine, 4-dimethylamino pyridine, picoline, lutidine, quinolin, and isoquinolin. The reaction may typically be carried out in an inert solvent, and examples of such inert solvents used herein include tetrahydrofuran, acetonitrile, dimethyl sulfoxide, N,N-dimethyl formamide, 1,4-dioxane, benzene, toluene, acetone, methyl ethyl ketone, ethyl acetate, dichloromethane, chloroform, carbon tetrachloride, 1,2-dichloroethane, and pyridine, as well as mixtures of these solvents. The reaction time may typically be from 1 hour to 24 hours, and the reaction temperature may be from -20°C to the boiling point of the solvent.

<u>*Production Method 6:</u>

The compounds represented by Formulae (I-4) and (I-5) can be produced by, e.g., the following methods.

[Formula 22]

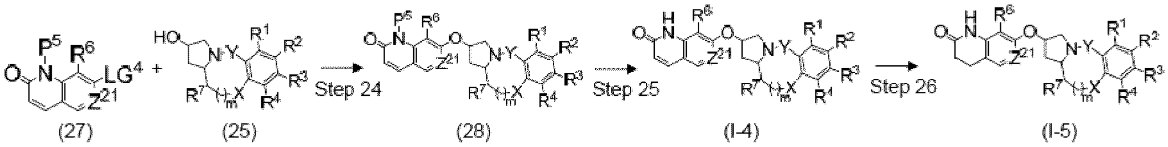

[Each symbol in the formula indicates the same meaning as mentioned above. $P^5$ represents an appropriate protective group, and $LG^4$ represents an appropriate leaving group. $R^6$ represents a hydrogen atom, unsubstituted or substituted $C_{1-6}$ alkyl group, unsubstituted or substituted $C_{3-8}$ cycloalkyl group, unsubstituted or substituted 3- to 8-membered heterocyclyl group, hydroxy group, or halogen.]

(Step 24)

This step is to subject Compound (27) having a leaving group and Compound (25) to conditions for nucleophilic substitution reaction similar to those of Step 23 of Production Method 5 to thereby produce Compound (28).

(Step 25)

This step is to remove the protective group $P^5$ of Compound (28) by a known method to thereby produce Compound (I-4) according to the present invention.

(Step 26)

This step is to subject Compound (I-4) to common conditions for catalytic hydrogenation reaction under a hydrogen atmosphere, if necessary, to thereby produce Compound (I-5) according to the present invention. Examples of such reaction conditions include conditions using, e.g., palladium carbon, hydroxide palladium (II), or Raney nickel. The reaction solvent used is not limited as long as it does not interfere with the reaction. Specific examples include methanol, ethanol, acetic acid, ethyl acetate, benzene, toluene, xylene, tetrahydrofuran, 1,4-dioxane, N,N-dimethyl formamide, dichloromethane, and acetonitrile, as well as mixtures of these solvents. The reaction time may typically be from 1 day to 14 days, and the reaction temperature is from room temperature to 100°C.

<u>*Production Method 7:</u>

The compounds represented by Formula (28) can also be produced by, e.g., the following methods.

[Formula 23]

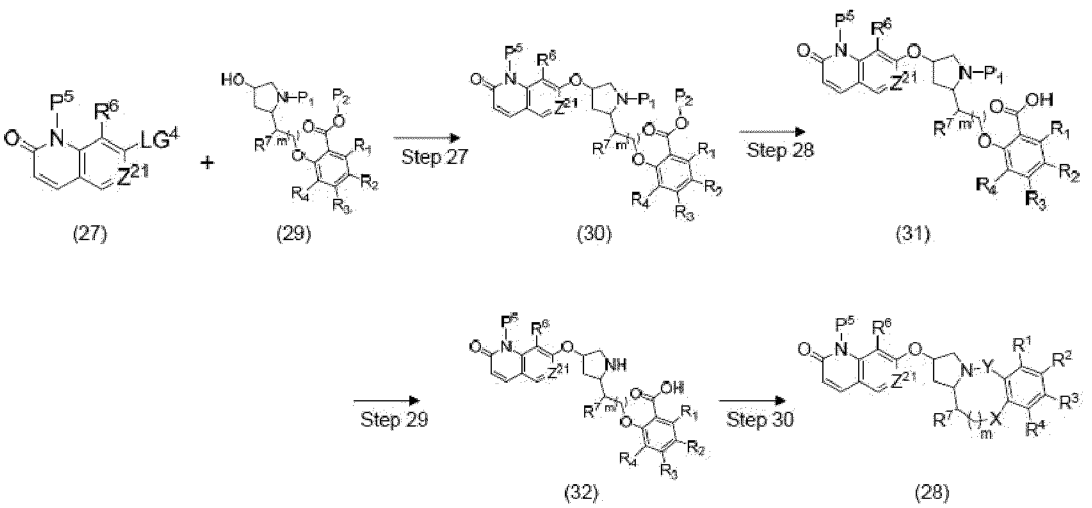


(27) (29) (30) (31) (32) (28)

[Each symbol in the formula indicates the same meaning as mentioned above. $P^1$, $P^2$ and $P^5$ represents, independently of each other, an appropriate protective group, and $LG^4$ represents an appropriate leaving group. $R^6$ represents a hydrogen atom, unsubstituted or substituted $C_{1-6}$ alkyl group, unsubstituted or substituted $C_{3-8}$ cycloalkyl group, unsubstituted or substituted 3- to 8-membered heterocyclyl group, hydroxy group, or halogen.]

(Step 27)

This step is to subject Compound (27) having a leaving group and Compound (29) to conditions for nucleophilic substitution reaction similar to those of Step 23 of Production Method 5 to thereby produce Compound (30).

(Step 28)

This step is to subject Compound (30) to conditions similar to those of Step 2 of Production Method 1 to thereby produce Compound (31).

(Step 29)

This step is to subject Compound (31) to conditions similar to those of Step 3 of Production Method 1 to thereby produce Compound (32).

(Step 30)

This step is to subject Compound (32) to conditions similar to those of Step 4 of Production Method 1 to thereby produce Compound (28).

*Production Method 8:

The compounds represented by Formula (I-6) can also be produced by, e.g., the following methods.

[Formula 24]

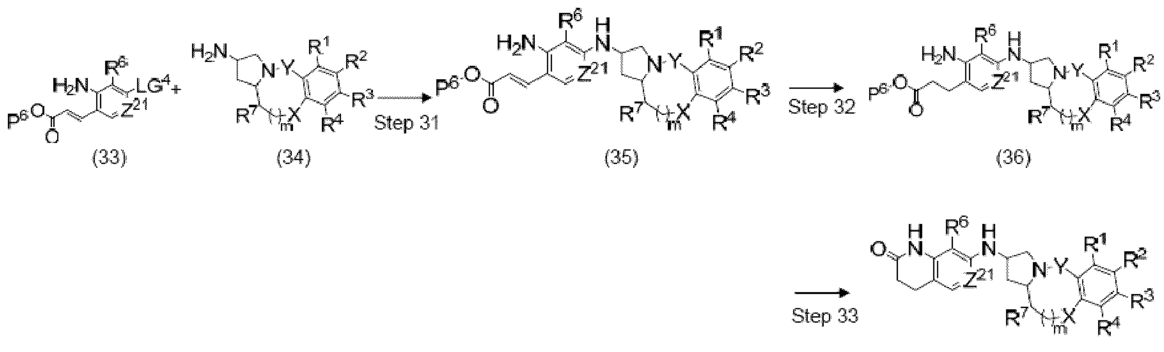


(33) (34) (35) (36) (I-6)

[Each symbol in the formula indicates the same meaning as mentioned above. $P^6$ represents an appropriate protective group, and $LG^4$ represents an appropriate leaving group. $R^6$ represents a hydrogen atom, unsubstituted or substituted $C_{1-6}$ alkyl group, unsubstituted or substituted $C_{3-8}$ cycloalkyl group, unsubstituted or substituted 3- to 8-membered heterocyclyl group, hydroxy group, or halogen.]

(Step 31)

This step is to subject Compound (33) having a leaving group and Compound (34) to conditions for nucleophilic substitution reaction in the presence of a base to thereby produce Compound (35). Examples of bases used herein include: alkali metal hydrides such as sodium hydride and potassium hydride; alkali metal hydroxides such as lithium hydroxide, sodium hydroxide, and potassium hydroxide; alkali metal carbonates such as sodium carbonate, potassium carbonate, and cesium carbonate; alkali metal hydrocarbonates such as sodium bicarbonate and potassium bicarbonate; metal alkoxides such as sodium methoxide, sodium ethoxide, and potassium tert-butoxide; tertiary aliphatic amines such as trimethylamine, triethylamine, N,N-diisopropylethylamine, N-methyl morpholine, N-methyl pyrrolidine, N-methyl piperidine, N,N-dimethyl aniline, 1,8-diazabicyclo[5.4.0]undeca-7-ene, and 1,5-diazabicyclo[4.3.0]nona-5-ene; and aromatic amines such as pyridine, 4-dimethylamino pyridine, picoline, lutidine, quinolin, and isoquinolin. The reaction may typically be carried out in an inert solvent, and examples of such inert solvents used herein include tetrahydrofuran, acetonitrile, dimethyl sulfoxide, N,N-dimethyl formamide, 1,4-dioxane, benzene, toluene, acetone, methylethylketone, ethyl acetate, dichloromethane, chloroform, carbon tetrachloride, 1,2-dichloroethane, and pyridine, as well as mixtures of these solvents. The reaction time may typically be from 1 hour to 96 hours, preferably from 1 hour to 24 hours. The reaction temperature may typically be from -20°C to 200°C, preferably from room temperature to the boiling point of the solvent.

(Step 32)

This step is to subject Compound (35) to common conditions for catalytic hydrogenation reaction similar to those of Step 26 of Production Method 6under a hydrogen atmosphere, if necessary, to thereby produce Compound (36).

(Step 33)

This step is to subject Compound (36) to general intramolecular cyclization reaction condition in the presence of an acid and, optionally, in the presence of a solvent to thereby produce Compound (I-6) according to the present invention. Examples of acids used herein include acetic acid, formic acid, sulfuric acid, trifluoroacetic acid, p-toluene sulfonic acid. The reaction solvent used is not limited as long as it does not interfere with the reaction. Specific examples include methanol, ethanol, acetic acid, ethyl acetate, benzene, toluene, tetrahydrofuran, 1,4-dioxane, N,N-dimethyl formamide, dichloromethane, acetonitrile, and water, as well as mixtures of these solvents. The reaction time may typically be from 1 hour to 24 hours, and the reaction temperature may be from 0°C to the boiling point of the solvent.

*Production Method 9:

The compounds represented by Formula (25) can also be produced by, e.g., the following methods.

[Formula 25]

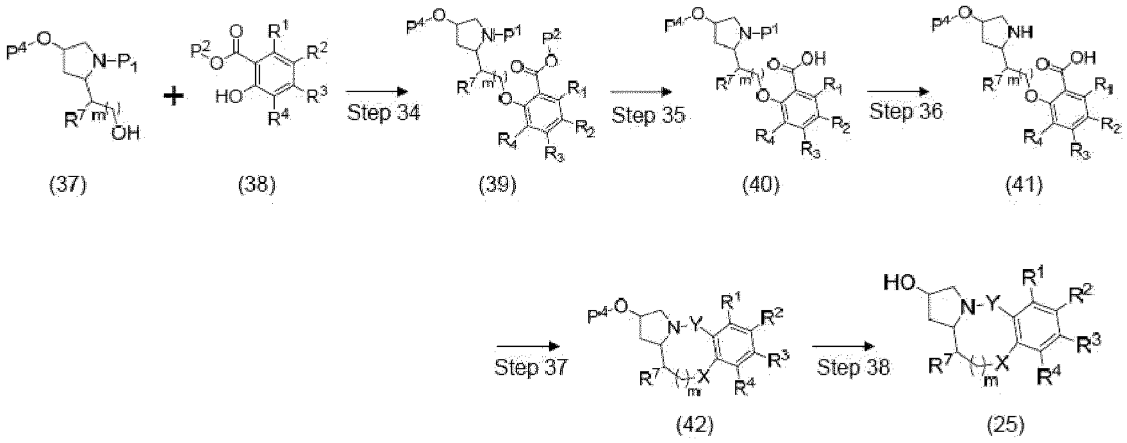


(37) (38) (39) (40) (41)

(42) (25)

[Each symbol in the formula indicates the same meaning as mentioned above. $P^1$, $P^2$ and $P^4$ represent, independently of each other, an appropriate protective group.]

(Step 34)

This step is to subject Compound (37) and Compound (38) to conditions similar to those of Step 1 of Production Method 1 to thereby produce Compound (39).

(Step 35)

This step is to subject Compound (39) to conditions similar to those of Step 2 of Production Method 1 to thereby produce Compound (40).

(Step 36)

This step is to subject Compound (40) to conditions similar to those of Step 3 of Production Method 1 to thereby produce Compound (41).

(Step 37)

This step is to subject Compound (41) to conditions similar to those of Step 4 of Production Method 1 to thereby produce Compound (42).

(Step 38)

This step is to remove the protective group $P^4$ of Compound (42) by a known method to thereby produce Compound (25).

*Production Method 10:

The compounds represented by Formula (42) can also be produced by, e.g., the following methods.

[Formula 26]

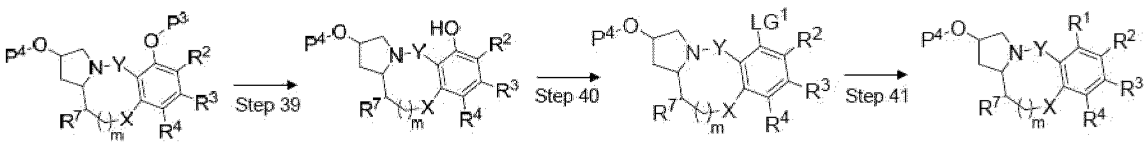


(43) (44) (45) (42)

[Each symbol in the formula indicates the same meaning as mentioned above. $P^3$ and $P^4$ represent, independently of each other, an appropriate protective group, and $LG^1$ represents an appropriate leaving group.]

(Step 39)

This step is to remove the protective group $P^4$ of Compound (43) by a known method to thereby produce Compound (44).

(Step 40)

This step is to subject Compound (44) to conditions for common conversion reaction to a leaving group similar to those of Step 6 of Production Method 2 to thereby produce Compound (45).

(Step 41)

This step is to subject Compound (45) to conditions similar to those of Step 7 of Production Method 2 to thereby produce Compound (42).

*Production Method 11:

The compounds represented by Formulae (27) and (33) can be produced by, e.g., the following methods.

[Formula 27]

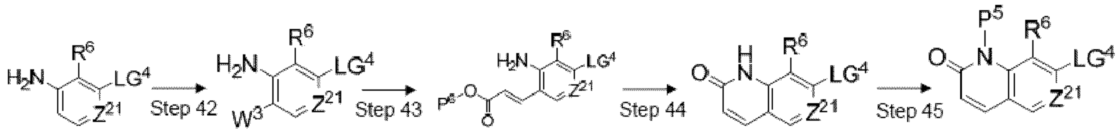


(46) (47) (33) (48) (27)

[Each symbol in the formula indicates the same meaning as mentioned above. $P^5$ and $P^6$ represent, independently of each other, an appropriate protective group. $LG^4$ represents an appropriate leaving group, and $W^3$ represents an appropriate halogen atom. $R^6$ represents a hydrogen atom, unsubstituted or substituted $C_{1-6}$ alkyl group, unsubstituted or substituted $C_{3-8}$ cycloalkyl group, unsubstituted or substituted 3- to 8-membered heterocyclyl group, hydroxy group, or halogen.]

(Step 42)

This step is to subject Compound (46) to common conditions for halogenation reaction to thereby produce Compound (47). Examples of halogenating reagents include chlorinating agents such as N-chlorosuccinimide, brominating agents such as N-bromosuccinimide, and iodinating agents such as N-iodosuccinimide. The reaction may typically be carried out in an inert solvent, and examples of such inert solvents used herein include tetrahydrofuran, acetonitrile, dimethyl sulfoxide, N,N-dimethyl formamide, 1,4-dioxane, acetone, methylethylketone, ethyl acetate, dichloromethane, chloroform, carbon tetrachloride, 1,2-dichloroethane, and water, as well as mixtures of these solvents. The reaction time may typically be from 1 hour to 96 hours, preferably from 1 hour to 24 hours. The reaction temperature may typically be from -78°C to 100°C, preferably from -20°C to the boiling point of the solvent.

(Step 43)

This step is to subject the halogen $W^3$ of Compound (47) to reaction with, e.g., an alkenyl compound in the presence of a base and a metal catalyst, and optionally in the presence of a phosphine ligand to thereby produce Compound (33). The alkenyl compound to be used may be either purchased commercially or synthesized by known or similar methods. Examples thereof include ethyl acrylate and t-butyl acrylate. Examples of bases used herein include: inorganic bases such as sodium bicarbonate, sodium carbonate, potassium carbonate, cesium carbonate, potassium phosphate, sodium hydroxide; organic salts such as ammonium acetate; metal alkoxides such as sodium methoxide and potassium tert-butoxide; organic bases such as triethylamine, N,N-diisopropylethylamine, 1,4-diazabicyclo[2.2.2]octane, 1,8-diazabicyclo[5.4.0]undeca-7-ene, and pyridine. A base may not be used depending on the type of coupling. Examples of metal catalysts used herein include bis(tri-o-tolylphosphine)palladium (0), tetrakis(triphenylphosphine)palladium (0), bis(dibenzylideneacetone)palladium (0), tris(dibenzylideneacetone)dipalladium (0), [1,1'-bis(diphenylphosphino)ferrocene]palladium (II) dichloride, and XPhos Pd G3. It is also possible to combine, e.g., palladium acetate with an appropriate ligand selected from those described in "Palladium Reagents and Catalysts," (2004), John Wiley & Sons Inc., or analogous ligands. Examples of phosphine ligands used herein include tri(o-tolyl)phosphine, SPhos, XPhos, DPPF, BINAP, and XantPhos. A phosphine ligand may not be used depending on the type of coupling. The reaction may typically be carried out in an inert solvent, and examples of such inert solvents used herein include tetrahydrofuran, acetonitrile, N,N-dimethyl formamide, 1,4-dioxane, toluene, 1,2-dimethoxy ethane, and water, as well as mixtures of these solvents. The reaction time may typically be from 0.5 hours to 96 hours, preferably from 1 hour to 24 hours. The reaction temperature may typically be from 0°C to 200°C, preferably from room temperature to 100°C.

(Step 44)

This step is to subject Compound (33) to common conditions for intramolecular cyclization reaction similar to those of Step 33 of Production Method 8 in the presence of an acids to thereby produce Compound (48).

(Step 45)

This step is to introduce an appropriate protective group $P^5$ to the amide group of Compound (48) by a known method to thereby produce Compound (27).

The compounds of the present invention have orexin type 2 receptor agonist activity, and are therefore useful in the treatment or prevention of diseases mediated by orexin type 2 receptor agonist activity. Specifically, the compound of the present invention is useful in the treatment or prevention of, e.g., narcolepsy, idiopathic hypersomnia, hypersomnia, sleep apnea syndrome, narcolepsy syndrome with narcolepsy-like symptoms, hypersomnia associated with Parkinson's disease, hypersomnia associated with Guillain-Barre syndrome, hypersomnia associated with Kleine-Levin syndrome, and hypersomnia associated with Lewy body dementia, and especially in the treatment or prevention of narcolepsy.

The compounds of the present invention may preferably have selectivity in orexin type 2 receptor agonist activity. For example, the compounds of the present invention may preferably have significantly higher orexin type 2 receptor agonist activity compared to orexin type 1 receptor agonist activity. In fact, certain compounds of the present invention represented by formula (I) have significantly higher orexin type 2 receptor agonist activity compared to orexin type 1 receptor agonist activity.

A therapeutic drug or preventive drug containing a compound of the present invention or a pharmaceutically acceptable salt thereof or a solvate thereof as an active ingredient may be prepared in the form of a pharmaceutical composition using carriers, bases and excipients and other additives normally used in the pharmaceutical formulation process. Such carriers, bases, and excipients may be either solid or liquid. Specific examples include lactose, magnesium stearate starch, talc, gelatin, agar, pectin, gum arabic, olive oil, sesame oil, cocoa butter, ethylene glycol, triglycerides of medium chain fatty acids, and other commonly used substances.

Administration of the compounds or pharmaceutical compositions of the present invention may be carried out either via oral administration in the form of, e.g., tablets, rounds, capsules, soft capsules, granules, dispersions, or liquids, or via parenteral administration by means of injection such as intravenous injection or intramuscular injection, using suppository, transdermally or intranasally, etc.

A compound of the present invention or a pharmaceutically acceptable salt thereof or a solvate thereof may be used in the form of an orexin type 2 receptor agonist drug containing the compound, etc., as an active ingredient. The term "agonist" as used herein refers to encompass various forms commonly used in the art, e.g., activators, agonists, stimulants, etc.,

Although depending on, e.g., the route of administration, the patient's age, sex, and severity of disease, and the frequency of administration, the therapeutic effective dose of the active ingredient in therapeutic agents, prophylactic agents, pharmaceutical compositions, and activators containing the compound of the invention as an active ingredient may usually be from 1 to 1500 mg/day, and the administration frequency may usually be from 1 to 3 times/day or 1 to 3 times/week. It is preferred to prepare formulations to satisfy these conditions.

However, since the dosage may also vary depending on various conditions, a dosage less than the above dosage may be sufficient in some cases, while a dosage in excess of the above range may be necessary in other cases.

## EXAMPLES

The present invention will be described more specifically by means of examples below. However, these examples do not limit the scope of the present invention.

The compound produced in an example is referred to by the same number as that of the example. Unless otherwise noted, reagents, starting materials, and solvents were purchased from vendors (e.g., Sigma-Aldrich, Fujifilm Wako Pure Chemicals, Tokyo Kasei, Fluka, Sigma, etc.) and used without further purification.

In the Reference Examples and the Examples below, the term "room temperature" usually refers to a temperature of from about 10°C to about 35°C. The ratios used herein for solvent mixtures are volume ratios unless otherwise indicated. The percentages used herein are weight percentages unless otherwise indicated, except that the yields are expressed in mol/mol% .

In the Reference Examples and the Examples below, column chromatography was carried out using silica gel columns and amino silica gel columns from Yamazen Corporation, and TLC observation was carried out using $60F_{254}$ from Merck (Merck) as TLC plates (silica gel) and NH2 Silica Gel 60F254 Plate Wako from Fujifilm Wako Pure Chemicals as TLC plates (NH silica gel).

The structures of the isolated novel compounds were confirmed by means of mass spectrometry using $^1$H-NMR and/or LC/MS.

For the compounds measured for $^1$H-NMR spectra (400MHz, DMSO-$d_6$, $CDCl_3$, or $CD_3OD$), their chemical shifts (δ: ppm) and coupling constants (J: Hz) are shown.

The LC/MS results are shown in terms of $[M+H]^+$ values (measured molecular weights: i.e., the measured values of the molecular masses (M) of the compounds to which protons ($H^+$) were added) and retention times Rt (min) observed using one of the following instruments and analytical conditions.

Method A

Mass spectrometry device: Agilent Technologies LC/MS 6130
HPLC: LC-1260
Column: Phenomenex Gemini C1, 3μm, 4.6mm × 30mm
UV: PDA detection (254nM)
Column temperature: 40°C
Detection voltage: 70V

Method B

Mass spectrometry device: Agilent Technologies LC/MS 6130
HPLC: LC-1260
Column: L-column2, 3μm, 3.0mm × 50mm
UV: PDA detection (254nM)

Column temperature: 40°C
Detection voltage: 70V

[Gradient conditions (volume ratios)]

Method A

Solvents:

A: $H_2O$/acetonitrile = 95/5, 0.05% TFA
B: $H_2O$/acetonitrile = 5/95, 0.05% TFA
Velocity: 1.0 mL/min

Gradients:

0 minutes, solvent B: 2%, solvent A: 98%
0 to 0.3 minutes, solvent B: 2% to 10%, solvent A: 98% to 90%
0.3 to 1.5 minutes, solvent B: 10% to 100%, solvent A: 90% to 0%
1.5 to 3.5 minutes, solvent B: 100%, solvent A: 0%
3.5 to 3.51 minutes, solvent B: 100% to 2%, solvent A: 0% to 98%
3.51 to 4.5 minutes, solvent B: 2%, solvent A: 98%

Method B

Solvents:

A: 100% $H_2O$, 0.05% TFA
B: 100% acetonitrile, 0.05% TFA
Velocity: 1.5 mL/min

Gradients:

0 to 0.3 minutes, solvent B: 2%, solvent A: 98%
0.3 to 2.3 minutes, solvent B: 2% to 95%, solvent A: 98% to 5%
2.3 to 3.5 minutes, solvent B: 95%, solvent A: 5%
3.5 to 3.51 minutes, solvent B: 95% to 2%, solvent A: 5% to 98%
3.51 to 4.0 minutes, solvent B: 2%, solvent A: 98%

The following analysis was performed using Method A, unless otherwise specified.
The following abbreviations may be used in the examples below.

$^1$H-NMR: Proton nuclear magnetic resonance
LC/MS: Liquid chromatography mass spectrometry
MS: Mass spectrometry
Rt: Retention time
min: Minute
s: Singlet
d: Doublet
t: Triplet
q: Quartet
dd: Double-doublet
ddd: Double-double-doublet
dt: Double-triplet
m: Multiplet

J: Coupling constant
Hz: Hertz
M: Molar concentration
DMF: N,N-Dimethyl formamide
DMSO: Dimethyl sulfoxide
THF: Tetrahydrofuran
LHMDS: Lithium bis(trimethylsilyl)amide
DIAD: Diisopropyl azodicarboxylate
WSC-HCl: 1-Ethyl-3-(3-dimethylaminopropyl)carbodiimide hydrochloride
HOBt: 1-Hydroxy benzotriazole
$Pd_2(dba)_3$: Tris(dibenzylideneacetone)dipalladium (0)
$Pd(dppf)Cl_2$ $CH_2Cl_2$: [1,1'-Bis(diphenylphosphino)ferrocene]palladium (II) dichloride dichloromethane adduct
XPhos: 2-Dicyclohexylphosphino-2',4',6'-triisopropyl-1,1'-biphenyl
Xantphos: 4,5-Bis(diphenylphosphino)-9,9-dimethyl xanthene
Selectfluor(registration trademark) : 1-Chloromethyl-4-fluoro-1,4-diazoniabicyclo[2.2.2]octane bis(tetrafluoroborate)
tBu: tert-Butyl
Boc: tert-Butoxycarbonyl
TBDPS: tert-Butyldiphenylsilyl
TFA: Trifluoroacetic acid
$CDCl_3$: Heavy chloroform
DMSO-$d_6$: Heavy dimethyl sulfoxide
$CD_3OD$: Heavy methanol

<Reference Example 1: Methyl 2-hydroxy-6-isopropoxybenzoate>

[Step 1] 5-Isopropoxy-2,2-dimethyl-4H-benzo[d][1,3]dioxin-4-one

5-Hydroxy-2,2-dimethyl-4H-benzo[d][1,3]dioxin-4-one (583 mg, 3.00 mmol) was dissolved in DMF (10 mL), combined with 2-iodopropane (447 μL, 4.50 mmol) and potassium carbonate (622 mg, 4.50 mmol), and agitated at 50°C overnight. The reaction mixture was then cooled to room temperature, combined with water, and extracted twice with heptane/ethyl acetate (1/1). The organic phase was dried with anhydrous magnesium sulfate, and concentrated under reduced pressure. The residue was purified with silica gel column chromatography (heptane/ethyl acetate) to thereby produce the title compound (512 mg).
LCMS: 237.2 $[M+H]^+$ (Rt= 2.207 min).

[Step 2] Methyl 2-hydroxy-6-isopropoxybenzoate

The compound obtained in Step 1 (512 mg, 2.17 mmol) was dissolved in methanol (6.6 mL), combined with potassium carbonate (898 mg, 6.50 mmol), and agitated overnight at room temperature. The reaction mixture was combined with water (30 mL) and 2M hydrochloric acid aqueous solution (8 mL), and extracted with dichloromethane. The organic phase was dried with anhydrous magnesium sulfate, and concentrated under reduced pressure. The residue was purified with silica gel column chromatography (heptane/ethyl acetate) to thereby produce the title compound (440 mg).
$^1$H-NMR ($CDCl_3$) δ: 11.37 (1H, s), 7.29 (1H, t, J = 8.5 Hz), 6.56 (1H, dd, J = 8.2, 0.9 Hz), 6.43 (1H, d, J = 8.2 Hz), 4.60-4.51 (1H, m), 3.93 (3H, s), 1.36 (6H, d, J = 6.4 Hz). LCMS: 211.2 $[M+H]^+$ (Rt= 2.169 min).

<Reference Example 2: Methyl 2-(benzyloxy)-6-hydroxy-4-methyl benzoate>

[Step 1] 5-Hydroxy-2,2,7-trimethyl-4H-benzo[d][1,3]dioxin-4-one

2,6-Dihydroxy-4-methyl benzoic acid (7.78g, 46.3 mmol) is dissolved in 1,2-dimethoxyethane (46 mL), to which acetone (4.1 mL, 55.5 mmol) and 4-dimethylaminopyridine (283 mg, 2.31 mmol) were added. Thionyl chloride (4.03 mL, 55.5 mmol) was added dropwise to the mixture at 0°C, and the temperature was raised to room temperature. The reaction mixture was agitated at room temperature for 20 hours, then cooled to 0°C, combined with 2M sodium hydroxide aqueous solution and saturated sodium bicarbonate aqueous solution, and extracted with ethyl acetate. The organic phase was washed twice with saturated sodium bicarbonate aqueous solution, dried with anhydrous magnesium sulfate, and concentrated under reduced pressure. The residue was purified with silica gel column chromatography (heptane/ethyl acetate) to thereby produce the title compound (7.40g).
$^1$H-NMR ($CDCl_3$) δ: 10.24 (1H, s), 6.46 (1H, s), 6.27 (1H, s), 2.31 (3H, s), 1.73 (6H, s). LCMS: 209.2 $[M+H]^+$ (Rt= 2.276 min).

[Step 2] 5-(Benzyloxy)-2,2,7-trimethyl-4H-benzo[d][1,3]dioxin-4-one

The compound obtained in Step 1 (300 mg, 1.44 mmol) and potassium carbonate (299 mg, 2.16 mmol) were dissolved in DMF (2.88 mL), and benzyl bromide (205 μL, 1.73 mmol) was added to the mixture dropwise. After the drop was finished, the mixture was agitated at room temperature for 2 hours, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (427 mg).

$^1$H-NMR ($CDCl_3$) δ: 7.56 (2H, d, J = 7.3 Hz), 7.41-7.38 (2H, m), 7.30 (1H, t, J = 7.3 Hz), 6.47 (1H, s), 6.38 (1H, s), 5.23 (2H, s), 2.32 (3H, s), 1.70 (6H, s).
LCMS: 299.1 $[M+H]^+$ (Rt= 2.370 min).

[Step 3] Methyl 2-(benzyloxy)-6-hydroxy-4-methyl benzoate

The compound obtained in Step 2 (427 mg, 1.43 mmol) was dissolved in methanol (4.77 mL), to which potassium carbonate (593 mg, 4.29 mmol) was added 50°C, and the resulting mixture was agitated for 1 hour. The reaction mixture was brought again to room temperature, combined with water, and extracted with ethyl acetate. The organic phase was washed twice with saturated saline solution, dried with anhydrous magnesium sulfate, and concentrated under reduced pressure to thereby produce the title compound (363 mg). LCMS: 273.1 $[M+H]^+$ (Rt= 2.393 min).

<Reference Example 3: Methyl 2-ethoxy-6-hydroxy-4-methyl benzoate>

The same procedures as those in Step 2 to Step 3 of Reference Example 2 were carried out except that the compound obtained in Step 1 of Reference Example 2 (200 mg, 0.96 mmol) and iodoethane (100 μL, 1.25 mmol) were used as raw materials, to thereby produce the title compound (194 mg).
LCMS: 211.1 $[M+H]^+$ (Rt= 2.256 min).

<Reference Example 4: Methyl 2-hydroxy-6-isopropoxy-4-methyl benzoate>

[Step 1] 5-Isopropoxy-2,2,7-trimethyl-4H-benzo[d][1,3]dioxin-4-one

The same procedures as those in Step 2 of Reference Example 2 were carried out except that the compound obtained in Step 1 of Reference Example 2 (500 mg, 2.40 mmol) and 2-iodopropane (310 μL, 3.12 mmol) were used as raw materials, to thereby produce the title compound (726 mg).

$^1$H-NMR ($CDCl_3$) δ: 6.43 (1H, s), 6.33 (1H, s), 4.67-4.58 (1H, m), 2.32 (3H, s), 1.68 (6H, s), 1.43 (6H, d, J = 5.9 Hz).
LCMS: 251.1 $[M+H]^+$ (Rt= 2.301 min).

[Step 2] Methyl 2-hydroxy-6-isopropoxy-4-methyl benzoate

The same procedures as those in Step 3 of Reference Example 2 were carried out except that the compound obtained in Step 1 (726 mg, 2.90 mmol) was used as raw materials, to thereby produce the title compound (598 mg).

$^1$H-NMR ($CDCl_3$) δ: 11.44 (1H, s), 6.40 (1H, s), 6.24 (1H, s), 4.58-4.49 (1H, m), 3.91 (3H, s), 2.28 (3H, s), 1.35 (6H, d, J = 5.9 Hz).
LCMS: 225.1 $[M+H]^+$ (Rt= 2.310 min).

<Reference Example 5: Methyl (S)-2-((1-((tert-butyldiphenylsilyl)oxy)propan-2-yl)oxy)-6-hydroxy-4-methyl benzoate>

[Step 1] (R)-1-((tert-Butyldiphenylsilyl)oxy)propan-2-ol

(R)-(-)-propan-1,2-diol (1.00 g, 13.1 mmol) and imidazole (1.34 g, 19.7 mmol) were dissolved in DMF (13.1 mL), and tert-butyldiphenyl chlorosilane (3.7 mL, 14.5 mmol) was added dropwise to the mixture. After the drop was finished, the mixture was agitated overnight at room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (3.93 g).

$^1H$-NMR ($CDCl_3$) δ: 7.68-7.65 (4H, m), 7.44-7.37 (6H, m), 3.96-3.86 (1H, m), 3.62 (1H, dd, J = 10.1, 3.2 Hz), 3.45 (1H, dd, J = 10.1, 7.8 Hz), 2.52 (1H, d, J = 3.2 Hz), 1.10 (3H, d, J = 6.4 Hz), 1.07 (9H, s).
LCMS: 315.1 $[M+H]^+$ (Rt= 2.577 min).

[Step 2] (S)-5-((1-((tert-Butyldiphenylsilyl)oxy)propan-2-yl)oxy)-2,2,7-trimethyl-4H-benzo[d][1,3]dioxin-4-one

The compound obtained in Step 1 (725 mg, 2.31 mmol), the compound obtained in Step 1 of Reference Example 2 (400 mg, 1.92 mmol), and triphenylphosphine (655 mg, 2.50 mmol) were dissolved in THF (5.77 mL), and DIAD (486 μL, 2.50 mmol) was added dropwise to the mixture. After the drop was finished, the mixture was agitated at room temperature for 1 hour, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (935 mg).

$^1H$-NMR ($CDCl_3$) δ: 7.68-7.64 (4H, m), 7.42-7.34 (6H, m), 6.47 (1H, s), 6.32 (1H, s), 4.65-4.58 (1H, m), 3.95 (1H, dd, J = 10.5, 5.9 Hz), 3.73 (1H, dd, J = 10.5, 5.5 Hz), 2.27 (3H, s), 1.67 (3H, s), 1.64 (3H, s), 1.41 (3H, d, J = 5.9 Hz), 1.01 (9H, s).
LCMS: 505.2 $[M+H]^+$ (Rt= 2.844 min).

[Step 3] Methyl (S)-2-((1-((tert-butyldiphenylsilyl)oxy)propan-2-yl)oxy)-6-hydroxy-4-methyl benzoate

The compound obtained in Step 2 (935 mg, 1.85 mmol) was dissolved in methanol (6.18 mL), combined with potassium carbonate (768 mg, 5.56 mmol), and agitated at room temperature for 15 hours. The reaction mixture was then combined with saturated saline solution, and extracted with ethyl acetate. The organic phase was washed twice with saturated saline solution, dried with anhydrous magnesium sulfate, and concentrated under reduced pressure to thereby produce the title compound (846 mg).

$^1H$-NMR ($CDCl_3$) δ: 11.43 (1H, s), 7.66-7.63 (4H, m), 7.42-7.34 (6H, m), 6.38 (1H, s), 6.24 (1H, s), 4.55-4.47 (1H, m), 3.85 (1H, dd, J = 10.5, 5.5 Hz), 3.79 (3H, s), 3.71 (1H, dd, J = 10.5, 5.0 Hz), 2.23 (3H, s), 1.35 (3H, d, J = 6.4 Hz), 1.03 (9H, s).
LCMS: 479.2 $[M+H]^+$ (Rt= 2.919 min).

<Reference Example 6: Methyl (R)-2-((1-((tert-butyldiphenylsilyl)oxy)propan-2-yl)oxy)-6-hydroxy-4-methyl benzoate>

The same procedures as those in Reference Example 5 were carried out except that (S)-(+)-propan-1,2-diol (231 mg, 3.04 mmol) was used as a raw material, to thereby produce the title compound (939 mg).

$^1H$-NMR ($CDCl_3$) δ: 11.45 (1H, s), 7.65-7.64 (4H, m), 7.44-7.34 (6H, m), 6.39 (1H, s), 6.24 (1H, s), 4.53-4.50 (1H, m), 3.85 (1H, dd, J = 10.3, 5.1 Hz), 3.80 (3H, s), 3.71 (1H, dd, J = 10.5, 5.3 Hz), 2.23 (3H, s), 1.35 (3H, d, J = 5.9 Hz), 1.03 (9H, s).
LCMS: 479.2 $[M+H]^+$ (Rt= 2.900 min).

<Reference Example 7: Methyl 2-hydroxy-4-methyl-6-((1,1,1-trifluoropropan-2-yl)oxy)benzoate>

[Step 1] 2,2,7-Trimethyl-5-((1,1,1-trifluoropropan-2-yl)oxy)-4H-benzo[d][1,3]dioxin-4-one

1,1,1-Trifluoropropan-2-ol (200 mg, 1.75 mmol) and pyridine (149 μL, 1.84 mmol) were dissolved in dichloromethane (3.5 mL). To the resulting solution, trifluoromethane sulfonic anhydride (288 μL, 1.75 mmol) was added dropwise at 0°C. After the drop was finished, the mixture was agitated overnight at room temperature, and the reaction mixture was then filtered. The filtrate was poured into the mixture of the compound obtained in Step 1 of Reference Example 2 (183 mg, 0.89 mmol), potassium carbonate (485 mg, 3.51 mmol), and DMF (3.51 mL), and the mixture was agitated at 60°C for 31 hours. The reaction mixture was cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with amino silica gel column chromatography (heptane/ethyl acetate) to thereby produce the title compound (106 mg).

$^1H$-NMR ($CDCl_3$) δ: 6.52 (1H, s), 6.50 (1H, s), 4.75-4.66 (1H, m), 2.35 (3H, s), 1.69 (6H, d, J = 2.7 Hz), 1.59 (3H, d, J = 6.4 Hz).

LCMS: 305.1 [M+H]+ (Rt= 2.320 min).

[Step 2] Methyl 2-hydroxy-4-methyl-6-((1,1,1-trifluoropropan-2-yl)oxy)benzoate

The compound obtained in Step 1 (106 mg, 0.35 mmol) was dissolved in methanol (1.74 mL), combined with potassium carbonate (240 mg, 1.74 mmol), and agitated for 1.5 hours at room temperature. The reaction mixture was then combined with saturated saline solution, and extracted with ethyl acetate. The organic phase was washed twice with saturated saline solution, dried with anhydrous magnesium sulfate, and concentrated under reduced pressure to thereby produce the title compound (91.7 mg).

$^1$H-NMR ($CDCl_3$) δ: 11.46 (1H, s), 6.51 (1H, s), 6.23 (1H, s), 4.71-4.62 (1H, m), 3.92 (3H, s), 2.30 (3H, s), 1.52 (3H, d, J = 6.4 Hz).
LCMS: 279.1 [M+H]+ (Rt= 2.319 min).

<Reference Example 8: Methyl (R)-2-hydroxy-4-methyl-6-((1,1,1-trifluoropropan-2-yl)oxy)benzoate>

[Step 1] (R)-2,2,7-Trimethyl-5-((1,1,1-trifluoropropan-2-yl)oxy)-4H-benzo[d][1,3]dioxin-4-one

The same procedures as those in Step 1 of Reference Example 7 were carried out except that (S)-1,1,1-trifluoro-2-propanol (10.0 g, 87.7 mmol) was used as a raw material, to thereby produce the title compound (6.02 g).

$^1$H-NMR ($CDCl_3$) δ: 6.52 (1H, s), 6.50 (1H, s), 4.75-4.66 (1H, m), 2.35 (3H, s), 1.70 (3H, s), 1.69 (3H, s), 1.59 (3H, d, J = 6.4 Hz).
LCMS: 305.1 [M+H]+ (Rt= 2.330 min).

[Step 2] Methyl (R)-2-hydroxy-4-methyl-6-((1,1,1-trifluoropropan-2-yl)oxy)benzoate

The same procedures as those in Step 2 of Reference Example 7 was carried out except that the compound obtained in Step 1 (9.08g, 29.9 mmol) was used as a raw material, to thereby produce the title compound (8.05g).

$^1$H-NMR ($CDCl_3$) δ: 11.46 (1H, s), 6.52 (1H, s), 6.23 (1H, s), 4.71-4.62 (1H, m), 3.92 (3H, s), 2.30 (3H, s), 1.52 (3H, d, J = 6.4 Hz).
LCMS: 279.1 [M+H]+ (Rt= 2.312 min).

<Reference Example 9: Methyl 2-(((2R)-bicyclo[2.2.1]heptan-2-yl)oxy)-6-hydroxy-4-methyl benzoate>

The same procedures as those in Step 2 to Step 3 of Reference Example 5 were carried out except that the compound obtained in Step 1 of Reference Example 2 (150 mg, 0.72 mmol) and exo-norborneol (105 mg, 0.94 mmol) were used as raw materials, to thereby produce the title compound (61.4 mg).
LCMS: 277.1 [M+H]+ (Rt= 2.543 min).

<Reference Example 10: Methyl 2-hydroxy-4-methyl-6-(neopentyloxy)benzoate>

The same procedures as those in Step 2 to Step 3 of Reference Example 5 were carried out except that the compound obtained in Step 1 of Reference Example 2 (300 mg, 1.44 mmol) and neopentylalcohol (165 mg, 1.87 mmol) were used as raw materials, to thereby produce the title compound (262 mg).
LCMS: 253.1 [M+H]+ (Rt= 2.540 min).

<Reference Example 11: Methyl 2-hydroxy-4-methyl-6-((tetrahydrofuran-2-yl)methoxy)benzoate>

The same procedures as those in Step 2 to Step 3 of Reference Example 5 were carried out except that the compound obtained in Step 1 of Reference Example 2 (150 mg, 0.72 mmol) and tetrahydrofurfurylalcohol (91.1 μL, 0.94 mmol) were used as raw materials, to thereby produce the title compound (138 mg).
LCMS: 267.1 [M+H]+ (Rt= 2.215 min).

<Reference Example 12: Methyl 2-hydroxy-6-isobutyl-4-methyl benzoate>

[Step 1] 2,2,7-Trimethyl-4-oxo-4H-benzo[d][1,3]dioxin-5-yl trifluoromethane sulfonate

The compound obtained in Step 1 of Reference Example 2 (1.50g, 7.20 mmol) and triethylamine (1.21 mL, 8.65 mmol) were dissolved in dichloromethane (14.4 mL), and trifluoromethane sulfonic anhydride (1.30 mL, 7.92 mmol) was added to the mixture dropwise at 0°C. After the drop was finished, the mixture was agitated at room temperature for 30 minutes, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (2.42 g).

$^1$H-NMR ($CDCl_3$) δ: 6.85 (1H, s), 6.80 (1H, s), 2.43 (3H, s), 1.74 (6H, s).
LCMS: 341.1 $[M+H]^+$ (Rt= 2.375 min).

[Step 2] 2,2,7-Trimethyl-5-(2-methyl-1-propen-1-yl)-4H-benzo[d][1,3]dioxin-4-one

The compound obtained in Step 1 (200 mg, 0.59 mmol), isopropenylboronic acid pinacol ester (214 mg, 1.18 mmol), and 2M sodium carbonate aqueous solution (882 μL, 1.76 mmol) were dissolved in 1,4-dioxane (1.77 mL), combined with XPhos Pd G3 (24.9 mg, 0.03 mmol), and agitated at 85°C for 2 hours. The reaction mixture was cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (120 mg).

$^1$H-NMR ($CDCl_3$) δ: 6.75 (2H, s), 6.63 (1H, s), 2.35 (3H, s), 1.95 (3H, d, J = 0.9 Hz), 1.77 (3H, d, J = 0.9 Hz), 1.69 (6H, s).
LCMS: 247.1 $[M+H]^+$ (Rt= 2.433 min).

[Step 3] 5-Isobutyl-2,2,7-trimethyl-4H-benzo[d][1,3]dioxin-4-one

The compound obtained in Step 2 (115 mg, 0.47 mmol) was dissolved in ethyl acetate (1.56 mL), combined with 10% palladium-activated carbon (11.5 mg), and agitated at room temperature for 1 hour under hydrogen atmosphere at normal pressure. The reaction mixture was filtered with cerite, and the filtrate was concentrated under reduced pressure to thereby produce the title compound (116 mg).

$^1$H-NMR ($CDCl_3$) δ: 6.69 (1H, s), 6.62 (1H, s), 2.95 (2H, d, J = 7.3 Hz), 2.33 (3H, s), 1.94-1.84 (1H, m), 1.68 (6H, s), 0.91 (6H, d, J = 6.9 Hz).
LCMS: 249.1 $[M+H]^+$ (Rt= 2.537 min).

[Step 4] Methyl 2-hydroxy-6-isobutyl-4-methyl benzoate

The compound obtained in Step 3 (120 mg, 0.48 mmol) was dissolved in methanol (1.61 mL), combined with potassium carbonate (200 mg, 1.45 mmol), and agitated at room temperature for 18 hours. The reaction mixture was combined with water, and extracted with ethyl acetate. The organic phase was washed twice with saturated saline solution, dried with anhydrous magnesium sulfate, and concentrated under reduced pressure to thereby produce the title compound (115 mg).

$^1$H-NMR ($CDCl_3$) δ: 11.23 (1H, s), 6.67 (1H, s), 6.49 (1H, s), 3.94 (3H, s), 2.74 (2H, d, J = 6.9 Hz), 2.28 (3H, s), 1.80-1.69 (1H, m), 0.88 (6H, d, J = 6.9 Hz).
LCMS: 223.2 $[M+H]^+$ (Rt= 2.484 min).

<Reference Example 13: Methyl 2-cyclohexyl-6-hydroxy-4-methyl benzoate>

The same procedures as those in Step 2 to Step 4 of Reference Example 12 were carried out except that the compound obtained in Step 1 of Reference Example 12 (150 mg, 0.44 mmol) and 1-cyclohexene boronic acid pinacol (138 mg, 0.66 mmol) were used as raw materials, to thereby produce the title compound (89.2 mg).
LCMS: 249.1 $[M+H]^+$ (Rt= 2.460 min).

<Reference Example 14: Methyl 2'-fluoro-3-hydroxy-5-methyl-[1,1'-biphenyl]-2-carboxylate>

The same procedures as those in Step 2 to Step 4 of Reference Example 12 were carried out except that the compound obtained in Step 1 of Reference Example 12 (150 mg, 0.44 mmol) and 2-fluorophenyl boronic acid (123 mg, 0.88 mmol) were used as raw materials, to thereby produce the title compound (97.1 mg).
LCMS: 261.1 $[M+H]^+$ (Rt= 2.391 min).

<Reference Example 15: Methyl 2',6'-difluoro-3-hydroxy-5-methyl-[1,1'-biphenyl]-2-carboxylate>

[Step 1] 2,2,7-Trimethyl-5-(4,4,5,5-tetramethyl-1,3,2-dioxaborolan-2-yl)-4H-benzo[d][1,3]dioxin-4-one

The compound obtained in Step 1 of Reference Example 12 (500 mg, 1.47 mmol), bis(pinacolato) diboron (485 mg, 1.91 mmol), and acetic acid potassium (433 mg, 4.41 mmol) were dissolved in 1,4-dioxane (7.35 mL), combined with $Pd(dppf)Cl_2 \cdot CH_2Cl_2$(538 mg, 0.74 mmol), and agitated at 100°C for 3 hours. The reaction mixture was cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (359 mg).

$^1$H-NMR ($CDCl_3$) $\delta$: 6.96 (1H, s), 6.74 (1H, s), 2.36 (3H, s), 1.71 (6H, s), 1.42 (12H, s).
LCMS: 319.2 $[M+H]^+$ (Rt= 2.390 min).

[Step 2] 5-(2,6-difluorophenyl)-2,2,7-trimethyl-4H-benzo[d][1,3]dioxin-4-one

The compound obtained in Step 1 (180 mg, 0.57 mmol), 1,3-difluoro-2-iodobenzene (204 mg, 0.85 mmol), 2M sodium carbonate aqueous solution (849 μL, 1.70 mmol) were dissolved in 1,4-dioxane (2.83 mL), combined with XPhos Pd G3 (23.9 mg, 0.03 mmol), and agitated for 1 hour at 100°C. The reaction mixture was cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (109 mg).

$^1$H-NMR ($CDCl_3$) $\delta$: 7.35-7.27 (1H, m), 7.00-6.92 (2H, m), 6.87 (2H, d, J = 9.6 Hz), 2.42 (3H, s), 1.74 (6H, s).
LCMS: 305.1 $[M+H]^+$ (Rt= 2.397 min).

[Step 3] Methyl 2',6'-difluoro-3-hydroxy-5-methyl-[1,1'-biphenyl]-2-carboxylate

The compound obtained in Step 2 (109 mg, 0.36 mmol) was dissolved in methanol (1.19 mL), combined with potassium carbonate (148 mg, 1.07 mmol), and agitated at 40°C for 2 hours. The reaction mixture was brought again to room temperature, then combined with saturated saline solution, and extracted with ethyl acetate. The organic phase was washed twice with saturated saline solution, dried with anhydrous magnesium sulfate, and concentrated under reduced pressure to thereby produce the title compound (97.3 mg).

$^1$H-NMR ($CDCl_3$) $\delta$: 11.17 (1H, s), 7.31-7.24 (1H, m), 6.95-6.90 (3H, m), 6.63 (1H, s), 3.55 (3H, s), 2.36 (3H, s).
LCMS: 279.1 $[M+H]^+$ (Rt= 2.397 min).

<Reference Example 16: Methyl 2'-(difluoromethyl)-3-hydroxy-5-methyl-[1,1'-biphenyl]-2-carboxylate>

The same procedures as those in Step 2 to Step 3 of Reference Example 15 were carried out except that the compound obtained in Step 1 of Reference Example 15 (174 mg, 0.55 mmol) and 1-bromo-2-(difluoromethyl)benzene (106 μL, 0.82 mmol) were used as raw materials, to thereby produce the title compound (87.1 mg).

$^1$H-NMR ($CDCl_3$) $\delta$: 11.17 (1H, s), 7.70-7.68 (1H, m), 7.45-7.44 (2H, m), 7.15-7.12 (1H, m), 6.88 (1H, s), 6.54 (1H, s), 6.32 (1H, t, J = 55.1 Hz), 3.40 (3H, s), 2.35 (3H, s).
LCMS: 293.7 $[M+H]^+$ (Rt= 2.415 min)

<Reference Example 17: Methyl 2-(3-fluoropyridin-2-yl)-6-hydroxy-4-methyl benzoate>

The same procedures as those in Step 2 to Step 3 of Reference Example 15 were carried out except that the compound obtained in Step 1 of Reference Example 15 (250 mg, 0.79 mmol) and 2-chloro-3-fluoropyridine (207 mg, 1.57 mmol) were used as raw materials, to thereby produce the title compound (99.5 mg).

$^1$H-NMR ($CDCl_3$) δ: 10.95 (1H, s), 8.46 (1H, td, J = 3.2, 1.5 Hz), 7.44-7.39 (1H, m), 7.32-7.28 (1H, m), 6.93 (1H, d, J = 0.9 Hz), 6.77 (1H, d, J = 0.9 Hz), 3.54 (3H, s), 2.37 (3H, s).
LCMS: 262.1 $[M+H]^+$ (Rt= 2.127 min).

<Reference Example 18: Methyl 2-hydroxy-4-methyl-6-(2-(trifluoromethyl)pyridin-3-yl)benzoate>

The same procedures as those in Step 2 and Step 4 of Reference Example 12 were carried out except that the compound obtained in Step 1 of Reference Example 12 (150 mg, 0.44 mmol) and 2-(trifluoromethyl)pyridin-3-boronic acid (126 mg, 0.66 mmol) were used as raw materials, to thereby produce the title compound (9.9 mg).
LCMS: 312.1 $[M+H]^+$ (Rt= 2.313 min).

<Reference Example 19: Methyl 2-hydroxy-4-methyl-6-(thiophen-2-yl)benzoate>

The same procedures as those in Step 2 and Step 4 of Reference Example 12 were carried out except that the compound obtained in Step 1 of Reference Example 12 (200 mg, 0.59 mmol) and 2-thienyl boronic acid (150 mg, 1.18 mmol) were used as raw materials, to thereby produce the title compound (136 mg).
LCMS: 249.1 $[M+H]^+$ (Rt= 2.242 min).

<Reference Example 20: tert-Butyl (2R,4R)-4-((tert-butyldiphenylsilyl)oxy)-2-(hydroxymethyl)pyrrolidin-1-carboxylate>

[Step 1] tert-Butyl (2R,4R)-2-((benzoyloxy)methyl)-4-hydroxy pyrrolidin-1-carboxylate

tert-Butyl (2R,4R)-4-hydroxy-2-(hydroxymethyl)pyrrolidin-1-carboxylate (2.17 g, 10.0 mmol) and pyridine (25.0 mL, 310 mmol) were dissolved in dichloromethane (60 mL). To the resulting solution, dichloromethane solution (20 mL) of benzoyl chloride (1.28 mL, 11.0 mmol) was added dropwise at -78°C for 10 minutes. After the drop was finished, the mixture was brought to room temperature, agitated overnight, combined with water, and extracted with dichloromethane. The organic phase was first washed with 1M hydrochloric acid aqueous solution, then washed twice with saturated sodium bicarbonate aqueous solution, dried with anhydrous magnesium sulfate, and concentrated under reduced pressure. The residue was purified with silica gel column chromatography (heptane/ethyl acetate) to thereby produce the title compound (2.74 g).
LCMS: 222.1 $[M+H-Boc]^+$ (Rt= 2.198 min).

[Step 2] tert-Butyl (2R,4R)-2-((benzoyloxy)methyl)-4-((tert-butyldiphenylsilyl)oxy)pyrrolidin-1-carboxylate

The compound obtained in Step 1 (508 mg, 1.58 mmol) was dissolved in DMF (5.27 mL), combined with imidazole (269 mg, 3.95 mmol) and tert-butyldiphenyl chlorosilane (486 μL, 1.90 mmol), and agitated overnight at room temperature. The reaction mixture was combined with water, and extracted with heptane/ethyl acetate (2/1). The organic phase was washed with water, dried with anhydrous magnesium sulfate, and concentrated under reduced pressure, to thereby produce a crude product of the title compound.
LCMS: 460.3 $[M+H-Boc]^+$ (Rt= 3.073 min).

[Step 3] tert-Butyl (2R,4R)-4-((tert-butyldiphenylsilyl)oxy)-2-(hydroxymethyl)pyrrolidin-1-carboxylate

The crude product of the compound obtained in Step 2 was dissolved in methanol (6.32 mL), combined with potassium carbonate (655 mg, 4.74 mmol), and agitated at room temperature for 3 hours. The reaction mixture was combined with water, and extracted with dichloromethane. The organic phase was washed with water twice, dried with anhydrous magnesium sulfate, and concentrated under reduced pressure. The residue was purified with silica gel column chromatography (heptane/ethyl acetate) to thereby produce the title compound (286 mg).
LCMS: 400.2 $[M+H-tBu]^+$ (Rt= 2.766 min).

<Reference Example 21: tert-Butyl (2S,4R)-4-((tert-butyldiphenylsilyl)oxy)-2-(hydroxymethyl)pyrrolidin-1-carboxylate>

The same procedures as those in Reference Example 20 were carried out except that tert-butyl (2S,4R)-4-hydroxy-2-(hydroxymethyl)pyrrolidin-1-carboxylate (1.09 g, 5.00 mmol) was used as a raw material, to thereby produce the title compound (1.87 g). LCMS: 400.2 $[M+H-tBu]^+$ (Rt= 2.735 min).

<Reference Example 22: tert-Butyl (2R,4S)-4-((tert-butyldiphenylsilyl)oxy)-2-(hydroxymethyl)pyrrolidin-1-carboxylate>

[Step 1] (2R,4S)-1-(tert-Butylcarbonyl)-4-((tert-butyldiphenylsilyl)oxy)pyrrolidin-2-carbonic acid

(2R,4S)-1-(tert-butoxycarbonyl)-4-hydroxy pyrrolidin-2-carbonic acid (2.31 g, 10.0 mmol) were dissolved in a mixture solvent of dichloromethane (50.0 mL) and DMF (10.0 mL), combined with imidazole (3.40 g, 50.0 mmol) and tert-butyldiphenyl chlorosilane (5.63 mL, 22.0 mmol), and agitated overnight at room temperature. The reaction mixture was combined with water, and extracted with dichloromethane. The organic phase was washed with saturated saline solution, dried with anhydrous magnesium sulfate, and concentrated under reduced pressure. The residue was dissolved in methanol (50.0 mL), combined with 4M lithium hydroxide aqueous solution (4.00 mL, 16.0 mmol), and agitated for 2 hours at room temperature. The reaction mixture was neutralized by addition of 1M hydrochloric acid aqueous solution, and extracted twice with dichloromethane. The organic phase was collected, washed with saturated saline solution, dried with anhydrous magnesium sulfate, and concentrated under reduced pressure. The residue was purified with silica gel column chromatography (heptane/ethyl acetate) to thereby produce the title compound (4.45 g).
LCMS: 370.2 $[M+H-Boc]^+$ (Rt= 2.601 min).

[Step 2] tert-Butyl (2R,4S)-4-((tert-butyldiphenylsilyl)oxy)-2-(hydroxymethyl)pyrrolidin-1-carboxylate

The compound obtained in Step 1 (4.45 g, 9.47 mmol) was dissolved in THF (19.0 mL). To the resulting solution, 0.9M borane-THF/THF solution (11.4 mL, 12.6 mmol) was added dropwise at 0°C. After the drop was finished, the mixture was brought to room temperature, agitated for 2 hours, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (4.12 g).
LCMS: 400.2 $[M+H-tBu]^+$ (Rt= 2.754 min).

<Reference Example 23: ((2R,4R)-4-((tert-Butyldiphenylsilyl)oxy)pyrrolidin-2-yl)methyl benzoate>

The compound obtained in Step 2 of Reference Example 20 (1.68 g, 3.00 mmol) was dissolved in dichloromethane (6 mL), combined with trifluoroacetic acid (6 mL), and agitated for 2 hours at room temperature. The reaction mixture was combined with saturated sodium bicarbonate aqueous solution, and extracted with dichloromethane. The organic phase was dried with sodium sulfate, and concentrated under reduced pressure. The residue was purified with amino silica gel column chromatography (heptane/ethyl acetate) to thereby produce the title compound (1.35 g).
LCMS: 460.15 $[M+H]^+$ (Rt= 2.138 min).

<Reference Example 24: (2R,11aR)-6-Isopropoxy-8-methyl-2,3,11,11a-tetrahydro-1H,5H-benzo[f]pyrrolo[2,1-c][1,4] oxazepin-2-ol>

[Step 1] 2-Hydroxy-6-isopropoxy-4-methyl benzaldehyde

The compound obtained in Step 1 of Reference Example 4 (1.25 g, 4.98 mmol) was dissolved in dichloromethane (25 mL). To the resulting solution, 1.5M diisobutylaluminum hydride (9.96 mL, 15.0 mmol) was added dropwise at -78°C for 20 minutes. After the drop was finished, the mixture was agitated for 3 hours with keeping the temperature at -78°C. Then, methanol (5 mL) and 1M hydrochloric acid aqueous solution (40 mL) were added dropwise to the mixture. The reaction mixture was brought to room temperature, agitated for 1 hour, and extracted with dichloromethane. The organic phase was washed twice with saturated saline solution, dried with anhydrous magnesium sulfate, and concentrated under reduced pressure. The residue was dissolved in THF (10 mL), combined with 2M hydrochloric acid aqueous solution (5 mL), agitated overnight, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (466 mg).
LCMS: 195.1 $[M+H]^+$ (Rt= 2.398 min).

[Step 2] ((2R,4R)-4-((tert-Butyldiphenylsilyl)oxy)-1-(2-hydroxy-6-isopropoxy-4-methyl benzyl)pyrrolidin-2-yl)methyl benzoate

The compound obtained in Step 1 (466 mg, 2.40 mmol) and the compound obtained in Reference Example 23 (1.04 g, 2.27 mmol) were dissolved in dichloromethane (11.4 mL), combined with acetic acid (156 μL, 2.72 mmol), and agitated for 1 hour at room temperature. The reaction mixture was combined with sodium triacetoxy borohydride (577 mg, 2.72 mmol), and the mixture was agitated overnight at room temperature, combined with saturated sodium bicarbonate aqueous solution, and extracted with dichloromethane. The organic phase was dried with sodium sulfate, and concentrated under reduced pressure. The residue was purified with silica gel column chromatography (heptane/ethyl acetate) to thereby produce the title compound (1.10 g).
LCMS: 638.4 $[M+H]^+$ (Rt= 2.242 min).

[Step 3] 2-(((2R,4R)-4-((tert-Butyldiphenylsilyl)oxy)-2-(hydroxymethyl)pyrrolidin-1-yl)methyl-3-isopropoxy-5-methylphenol

The compound obtained in Step 2 (1.10 g, 1.73 mmol) and potassium carbonate (955 mg, 6.91 mmol) were dissolved in a mixture solvent of methanol (8.64 mL) and dichloromethane (8.64 mL), and agitated for 4 hours at room temperature. The reaction mixture was combined with water, and and extracted with dichloromethane. The organic phase was washed with water, dried with anhydrous magnesium sulfate, and concentrated under reduced pressure. The residue was purified with silica gel column chromatography (heptane/ethyl acetate) to thereby produce the title compound (875 mg).
LCMS: 534.3 $[M+H]^+$ (Rt= 2.154 min).

[Step 4] (2R,11aR)-2-((tert-Butyldiphenylsilyl)oxy)-6-isopropoxy-8-methyl-2,3,11,11a-tetrahydro-1H,5H-benzo[f]pyrrolo [2,1-c][1,4]oxazepin

The compound obtained in Step 3 (820 mg, 1.54 mmol) and triphenylphosphine (806 mg, 3.07 mmol) were dissolved in THF (15.4 mL), and DIAD (598 μL, 3.07 mmol) was added dropwise at 0°C. After the drop was finished, the mixture was agitated overnight at room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (68.0 mg).
LCMS: 516.3 $[M+H]^+$ (Rt= 2.170 min).

[Step 5] (2R,11aR)-6-isopropoxy-8-methyl-2,3,11,11a-tetrahydro-1H,5H-benzo[f]pyrrolo[2,1-c][1,4]oxazepin-2-ol

The compound obtained in Step 4 (68.0 mg, 0.13 mmol) was dissolved in THF (527 μL), combined with 1M tetrabutyl ammonium fluoride/THF solution (0.264 mL, 0.26 mmol), and agitated overnight at room temperature. The reaction mixture was combined with saturated sodium bicarbonate aqueous solution, and extracted with ethyl acetate 5 times. The organic phase was washed with saturated saline solution, dried with sodium sulfate, and concentrated under reduced pressure. The residue was purified with amino silica gel column chromatography (heptane/ethyl acetate) to thereby produce the title compound (25.0 mg).
LCMS: 278.2 $[M+H]^+$ (Rt= 1.723 min).

<Reference Example 25: tert-Butyl (2R,4S)-4-(benzyloxy)-2-(hydroxymethyl)pyrrolidin-1-carboxylate>

[Step 1] (2R,4S)-4-(Benzyloxy)-1-(tert-butoxycarbonyl)pyrrolidin-2-carbonic acid

(2R,4S)-1-(tert-Butoxycarbonyl)-4-hydroxy pyrrolidin-2-carbonic acid (1.00 g, 4.32 mmol) was dissolved in THF (13 mL), combined with 50% sodium hydride (457 mg, 9.51 mmol) at 0°C, brought to room temperature, and agitated for 20 minutes. The reaction mixture was cooled again to 0°C, combined with benzyl bromide (565 μL, 4.76 mmol), and agitated at 50°C for 15 hours. The reaction mixture was cooled to room temperature, combined with water and 2M hydrochloric acid aqueous solution, and extracted with ethyl acetate. The organic phase was washed twice with saturated saline solution, dried with anhydrous magnesium sulfate, and concentrated under reduced pressure to thereby produce the title compound (1.19 g).

$^1$H-NMR ($CDCl_3$) δ: 7.38-7.30 (5H, m), 4.57-4.49 (3H, m), 4.17-4.15 (1H, m), 3.75-3.44 (2H, m), 2.57-2.47 (1H, m), 2.30-2.12 (1H, m), 1.49-1.43 (9H, m).

LCMS: 222.1 [M+H-Boc]$^+$ (Rt= 2.144 min).

[Step 2] tert-Butyl (2R,4S)-4-(benzyloxy)-2-(hydroxymethyl)pyrrolidin-1-carboxylate

The compound obtained in Step 1 (1.19 g, 3.70 mmol) was dissolved in THF (11.1 mL). To the resulting solution, 0.9M borane-THF/THF solution (10.3 mL, 9.25 mmol) was added dropwise at 0°C for 10 minutes. After the drop was finished, the mixture was brought to room temperature, agitated for 1 hour, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (922 mg). $^1$H-NMR ($CDCl_3$) δ: 7.37-7.28 (5H, m), 4.92 (1H, d, J = 8.2 Hz), 4.54-4.47 (2H, m), 4.17-4.05 (2H, m), 3.73-3.39 (3H, m), 2.22-2.17 (1H, m), 1.66-1.59 (1H, m), 1.47 (9H, s). LCMS: 252.1 [M+H-tBu]$^+$ (Rt= 2.202 min).

<Reference Example 26: tert-Butyl (2R,4R)-4-(benzyloxy)-2-(hydroxymethyl)pyrrolidin-1-carboxylate>

The same procedures as those in Reference Example 25 were carried out except that (2R,4R)-1-(tert-Butoxycarbonyl)-4-hydroxy pyrrolidin-2-carbonic acid was used as a raw material, to thereby produce the title compound. LCMS: 252.1 [M+H-tBu]$^+$ (Rt= 2.221 min).

<Reference Example 27: (2S,11aR)-6-(cyclopentyloxy)-2-hydroxy-2,3,11,11a-tetrahydro-1H,5H-benzo[f]pyrrolo[2,1-c][1,4]oxazepin-5-one>

[Step 1] Methyl 2-(cyclopentyloxy)-6-hydroxy benzoate

The same procedures as those in Step 2 to Step 3 of Reference Example 5 were carried out except that 5-hydroxy-2,2-dimethyl-4H-benzo[d][1,3]dioxin-4-one (500 mg, 2.58 mmol) and cyclopentanol (288 mg, 3.35 mmol) were used as raw materials, to thereby produce the title compound (382 mg).

$^1$H-NMR ($CDCl_3$) δ: 11.44 (1H, s), 7.29 (1H, t, J = 8.5 Hz), 6.55-6.53 (1H, m), 6.40 (1H, d, J = 8.7 Hz), 4.83-4.79 (1H, m), 3.92 (3H, s), 1.91-1.76 (6H, m), 1.69-1.59 (2H, m).
LCMS: 237.1 [M+H]$^+$ (Rt= 2.351 min).

[Step 2] tert-Butyl (2R,4S)-4-(benzyloxy)-2-((3-(cyclopentyloxy)-2-(methoxycarbonyl)phenoxy)methyl)pyrrolidin-1-carboxylate

The compound obtained in Step 1 (190 mg, 0.80 mmol), the compound obtained in Reference Example 25 (297 mg, 0.96 mmol), and triphenylphosphine (274 mg, 1.05 mmol) were dissolved in THF (2.4 mL), and to the resulting solution, DIAD (203 μL, 1.05 mmol) was added dropwise. After the drop was finished, the reaction mixture was agitated at 40°C for 1 hour, cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (389 mg). LCMS: 426.3 [M+H-Boc]$^+$ (Rt= 2.646 min).

[Step 3] 2-(((2R,4S)-4-(Benzyloxy)-1-(tert-butoxycarbonyl)pyrrolidin-2-yl)methoxy)-6-(cyclopentyloxy)benzoic acid

The compound obtained in Step 2 (389 mg, 0.74 mmol) was dissolved in a mixture solvent of methanol (2.22 mL), THF (0.74 mL), and water (1.48 mL), combined with potassium hydroxide (208 mg, 3.70 mmol), and agitated at 85°C for 6 days. The reaction mixture was then cooled to room temperature, combined with 1M hydrochloric acid aqueous solution, and extracted with ethyl acetate. The organic phase was washed twice with saturated saline solution, dried with anhydrous magnesium sulfate, and concentrated under reduced pressure to thereby produce the title compound (392 mg). LCMS: 412.2 [M+H-Boc]$^+$ (Rt= 2.458 min).

[Step 4] 2-(((2R,4S)-4-(Benzyloxy)pyrrolidin-2-yl)methoxy)-6-(cyclopentyloxy)benzoic acid hydrochloride

The compound obtained in Step 3 (392 mg, 0.77 mmol) was dissolved in dioxane (2.3 mL), and to the resulting mixture, 4M hydrochloric acid/1,4-dioxane solution (580 μL, 2.30 mmol) was added dropwise. After the drop was finished, the reaction mixture was agitated at 40°C for 20 hours, and concentrated under reduced pressure. The residue was azeotroped with 1,4-dioxane to thereby produce the title compound (297 mg).

LCMS: 412.2 $[M+H]^+$ (Rt= 1.891 min).

[Step 5] (2S,11aR)-2-(Benzyloxy)-6-(cyclopentyloxy)-2,3,11,11a-tetrahydro-1H,5H-benzo[f]pyrrolo[2,1-c][1,4]oxazepin-5-one

The compound obtained in Step 4 (297 mg, 0.66 mmol) and N,N-diisopropylethylamine (1.14 mL,6.64 mmol) were dissolved in DMF (2 mL), combined with WSC-HCl (191 mg, 1 mmol) and HOBt (152 mg, 1 mmol), and agitated at 40°C for 2 hours. The reaction mixture was then cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (209 mg).

$^1$H-NMR ($CDCl_3$) δ: 7.37-7.23 (6H, m), 6.76 (1H, d, J = 8.7 Hz), 6.62 (1H, d, J = 8.2 Hz), 4.78 (1H, s), 4.58 (1H, d, J = 11.9 Hz), 4.48 (1H, d, J = 11.9 Hz), 4.33-4.28 (1H, m), 4.13-4.02 (2H, m), 3.97-3.92 (3H, m), 2.27-2.20 (1H, m), 2.00-1.96 (1H, m), 1.91-1.76 (6H, m), 1.60-1.51 (2H, m).
LCMS: 394.2 $[M+H]^+$ (Rt= 2.368 min).

[Step 6] (2S,11aR)-6-(Cyclopentyloxy)-2-hydroxy-2,3,11,11a-tetrahydro-1H,5H-benzo[f]pyrrolo[2,1-c][1,4]oxazepin-5-one

The compound obtained in Step 5 (209 mg, 0.53 mmol) was dissolved in ethyl acetate (2.66 mL), combined with 20% hydroxide palladium-activated carbon (20.9 mg), and agitated at room temperature for 1 hour under hydrogen atmosphere at normal pressure. The reaction mixture was filtered with cerite, and the filtrate was concentrated under reduced pressure to thereby produce the title compound (153 mg).
LCMS: 304.2 $[M+H]^+$ (Rt= 1.948 min).

<Reference Example 28: (2R,11aR)-6-(Cyclopropylmethoxy)-2-hydroxy-8-methyl-2,3,11,11a-tetrahydro-1H,5H-benzo[f]pyrrolo[2,1-c][1,4]oxazepin-5-one>

[Step 1] Methyl 2-(cyclopropylmethoxy)-6-hydroxy-4-methyl benzoate

The same procedures as those in Step 2 to Step 3 of Reference Example 2 were carried out except that the compound obtained in Step 1 of Reference Example 2 (5.00 g, 24.0 mmol) and (bromomethyl) cyclopropane (3.03 mL, 31.2 mmol) were used as raw materials, to thereby produce the title compound (2.91 g).

$^1$H-NMR ($CDCl_3$) δ: 11.51 (1H, s), 6.41 (1H, s), 6.19 (1H, s), 3.93 (3H, s), 3.88 (2H, d, J = 6.4 Hz), 2.27 (3H, s), 1.31-1.22 (1H, m), 0.64-0.59 (2H, m), 0.43-0.39 (2H, m).
LCMS: 237.1 $[M+H]^+$ (Rt= 2.356 min).

[Step 2] (2R,11aR)-6-(Cyclopropylmethoxy)-2-hydroxy-8-methyl-2,3,11,11a-tetrahydro-1H,5H-benzo[f]pyrrolo[2,1-c][1,4]oxazepin-5-one

The same procedures as those in Step 2 to Step 6 of Reference Example 27 were carried out except that the compound obtained in Step 1 (147 mg, 0.62 mmol) and the compound obtained in Reference Example 26 (220 mg, 0.72 mmol) were used as raw materials, to thereby produce the title compound (84.7 mg).

$^1$H-NMR ($CDCl_3$) δ: 6.57 (1H, s), 6.51 (1H, s), 4.66-4.60 (2H, m), 4.06-3.81 (6H, m), 2.30 (3H, s), 2.28-2.21 (1H, m), 1.87-1.79 (2H, m), 1.35-1.28 (1H, m), 0.64-0.57 (2H, m), 0.41-0.33 (2H, m).
LCMS: 304.2 $[M+H]^+$ (Rt= 1.974 min).

<Reference Example 29: (2S,11aR)-2-Hydroxy-6-isobutoxy-8-methyl-2,3,11,11a-tetrahydro-1H,5H-benzo[f]pyrrolo[2,1-c][1,4]oxazepin-5-one>

The same procedures as those in Step 1 to Step 6 of Reference Example 27were carried out except that the compound obtained in Step 1 of Reference Example 2 (250 mg, 1.20 mmol) and 2-methyl-1-propanol (140 μL, 1.56 mmol) were used as raw materials, to thereby produce the title compound (70.2 mg).
LCMS: 306.2 $[M+H]^+$ (Rt= 2.006 min).

<Reference Example 30: (2S,11aR)-2-Hydroxy-6-(2-hydroxy-2-methylpropoxy)-8-methyl-2,3,11,11a-tetrahydro-1H,5H-benzo[f]pyrrolo[2,1-c][1,4]oxazepin-5-one>

[Step 1] tert-Butyl (2R,4S)-4-(benzyloxy)-2-(((2,2,7-trimethyl-4-oxo-4H-benzo[d][1,3]dioxin-5-yl)oxy)methyl)pyrrolidin-1-carboxylate

the compound obtained in Step 1 of Reference Example 2 (325 mg, 1.56 mmol), the compound obtained in Reference Example 25 (400 mg, 1.30 mmol), and triphenylphosphine (444 mg, 1.69 mmol) was dissolved in THF (3.91 mL), and to the resulting solution, DIAD (329 μL, 1.69 mmol) was added dropwise. After the drop was finished, the mixture was agitated at 40°C for 1 hour, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate), then with amino silica gel column chromatography (heptane/ethyl acetate), to thereby produce the title compound (565 mg). LCMS: 398.2 $[M+H-Boc]^+$ (Rt= 2.595 min).

[Step 2] tert-Butyl (2R,4S)-4-(benzyloxy)-2-((3-hydroxy-2-(methoxycarbonyl)-5-methylphenoxy)methyl)pyrrolidin-1-carboxylate

The compound obtained in Step 1 (565 mg, 1.14 mmol) was dissolved in methanol (10 mL), combined with potassium carbonate (471 mg, 3.41 mmol), and agitated at 40°C for 1.5 hours. The reaction mixture was then cooled to room temperature, combined with water, and extracted with ethyl acetate. The organic phase was washed twice with saturated saline solution, dried with anhydrous magnesium sulfate, and concentrated under reduced pressure to thereby produce the title compound (542 mg).
LCMS: 372.2 $[M+H-Boc]^+$ (Rt= 2.560 min).

[Step 3] tert-Butyl (2R,4S)-4-(benzyloxy)-2-((3-(2-hydroxy-2-methylpropoxy)-2-(methoxycarbonyl)-5-methylphenoxy) methyl)pyrrolidin-1-carboxylate

The compound obtained in Step 2 (180 mg, 0.38 mmol) and potassium carbonate (316 mg, 2.30 mmol) were dissolved in DMF (1.91 mL), combined with 1-chloro-2-methyl-2-propanol (156 μL, 1.53 mmol), and agitated at 100°C for 2 days. The reaction mixture was then cooled to room temperature, combined with water (50 mL), and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate), then with amino silica gel column chromatography (heptane/ethyl acetate), to thereby produce the title compound (124 mg).
LCMS: 444.2 $[M+H-Boc]^+$ (Rt= 2.471 min).

[Step 4] (2S,11aR)-2-hydroxy-6-(2-hydroxy-2-methylpropoxy)-8-methyl-2,3,11,11a-tetrahydro-1H,5H-benzo[f]pyrrolo [2,1-c][1,4]oxazepin-5-one

The same procedures as those in Step 3 to Step 6 of Reference Example 27 were carried out except that the compound obtained in Step 3 (124 mg, 0.23 mmol) was used as a raw material, to thereby produce the title compound (30.2 mg).
LCMS: 322.1 $[M+H-Boc]^+$ (Rt= 1.841 min).

<Reference Example 31: (2S,11aR)-2-hydroxy-6-isopropoxy-8-methyl-2,3,11,11a-tetrahydro-1H,5H-benzo[f]pyrrolo [2,1-c][1,4]oxazepin-5-one>

The same procedures as those in Step 2 to Step 6 of Reference Example 27 were carried out except that the compound obtained in Reference Example 4 (938 mg, 4.19 mmol) and the compound obtained in Reference Example 25 (1.01 g, 3.27 mmol) were used as raw materials, to thereby produce the title compound (375 mg).

$^1$H-NMR ($CDCl_3$) δ: 6.60 (1H, s), 6.47 (1H, s), 4.64-4.58 (1H, m), 4.56-4.48 (1H, m), 4.14-3.86 (4H, m), 3.80 (1H, dd, J = 12.6, 4.3 Hz), 2.30 (3H, s), 2.18-2.11 (2H, m), 1.86 (1H, dt, J = 13.0, 4.9 Hz), 1.37 (3H, d, J = 5.9 Hz), 1.33 (3H, d, J = 5.9 Hz).
LCMS: 292.1 $[M+H]^+$ (Rt= 1.890 min).

<Reference Example 32: (2R,11aR)-8-chloro-2-hydroxy-6-isopropoxy-2,3,11,11a-tetrahydro-1H,5H-benzo[f]pyrrolo[2,1-c][1,4]oxazepin-5-one>

[Step 1] 4-Chloro-2,6-dimethoxy benzoic acid

Methyl 4-chloro-2,6-difluorobenzoate (1.03 g, 5.00 mmol) was dissolved in methanol (10.0 mL), combined with 5M sodium methoxide/methanol solution (2.4 mL, 12.0 mmol), and agitated at 80°C overnight. The reaction mixture was cooled to room temperature, combined with 2M sodium hydroxide aqueous solution (10.0 mL, 20.0 mmol), and agitated at 60°C overnight. The reaction mixture was cooled to room temperature, combined with 2M hydrochloric acid aqueous solution (50 mL), and extracted twice with dichloromethane. The organic phase was washed twice with saturated saline solution, dried with anhydrous magnesium sulfate, and concentrated under reduced pressure to thereby produce the title compound (1.05 g).

$^1$H-NMR ($CDCl_3$) $\delta$: 6.60 (2H, s), 3.87 (6H, s).
LCMS: 217.1 $[M+H]^+$ (Rt= 1.955 min).

[Step 2] 4-Chloro-2,6-dihydroxy benzoic acid

The compound obtained in Step 1 (627 mg, 2.89 mmol) was dissolved in dichloromethane (5.79 mL), and to the resulting solution, 1M boron tribromide/dichloromethane solution (11.6 mL, 11.6 mmol) was added dropwise at -78°C. After the drop was finished, the reaction mixture was brought to room temperature, agitated overnight, then added to ice water and agitated for 30 minutes. The reaction mixture was filtered, and the resulting solid was washed with water to thereby produce the title compound (427 mg).
LCMS: 189.0 $[M+H]^+$ (Rt= 2.411 min).

[Step 3] 7-Chloro-5-hydroxy-2,2-dimethyl-4H-benzo[d][1,3]dioxin-4-one

The compound obtained in Step 2 (427 mg, 2.26 mmol), 4-dimethylaminopyridine (27.7 mg, 0.23 mmol), and acetone (333 μL, 4.53 mmol) were dissolved in 1,2-dimethoxyethane (11.3 mL), and to the resulting solution, thionyl chloride (329 μL, 4.53 mmol) was added dropwise at 0°C. After the drop was finished, the reaction mixture was heated to 50°C, and agitated for 6 hours. The reaction mixture was then cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was then purified with silica gel column chromatography (dichloromethane /ethyl acetate) to thereby produce the title compound (55.0 mg).
LCMS: 229.1 $[M+H]^+$ (Rt= 2.345 min).

[Step 4] tert-Butyl (2R,4R)-4-((tert-butyldiphenylsilyl)oxy)-2-(((7-chloro-2,2-dimethyl-4-oxo-4H-benzo[d][1,3]dioxin-5-yl)oxy)methyl)pyrrolidin-1-carboxylate

The compound obtained in Step 3 (151 mg, 0.66 mmol), the compound obtained in Reference Example 20 (286 mg, 0.63 mmol), and triphenylphosphine (198 mg, 0.75 mmol) was dissolved in THF (3.14 mL). To the resulting solution, DIAD (147 μL, 0.75 mmol) was added dropwise at 0°C. After the drop was finished, the mixture was agitated overnight at room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) and then with amino silica gel column chromatography (heptane/ethyl acetate), to thereby produce a crude product of the title compound.
LCMS: 566.3 $[M+H-Boc]^+$ (Rt= 3.118 min).

[Step 5] tert-Butyl (2R,4R)-4-((tert-butyldiphenylsilyl)oxy)-2-((5-chloro-3-hydroxy-2-(methoxycarbonyl)phenoxy)methyl)pyrrolidin-1-carboxylate

The same procedures as those in Step 2 of Reference Example 30 were carried out except that the crude product of the compound obtained in Step 4 was used as a raw material, to thereby produce the title compound (259 mg).
LCMS: 540.3 $[M+H-Boc]^+$ (Rt= 3.149 min).

[Step 6] tert-Butyl (2R,4R)-4-((tert-butyldiphenylsilyl)oxy)-2-((5-chloro-3-isopropoxy-2-(methoxycarbonyl)phenoxy) methyl)pyrrolidin-1-carboxylate

The same procedures as those in Step 3 of Reference Example 30 were carried out except that the compound obtained in Step 5 (259 mg, 0.40 mmol) and 2-iodopropane (121 μL, 1.21 mmol) were used as raw materials, to thereby produce the title compound (273 mg).
LCMS: 582.3 $[M+H-Boc]^+$ (Rt= 3.123 min).

[Step 7] 2-(((2R,4R)-1-(tert-Butoxycarbonyl)-4-hydroxy pyrrolidin-2-yl)methoxy)-4-chloro-6-isopropoxybenzoic acid

The compound obtained in Step 6 (273 mg, 0.40 mmol) was dissolved in ethanol (1.6 mL), combined with 2M sodium hydroxide aqueous solution (0.8 mL, 1.6 mmol), and agitated at 80°C overnight. The reaction mixture was cooled to room temperature, combined with 2M hydrochloric acid aqueous solution, and extracted twice with dichloromethane. The organic phase was washed twice with saturated saline solution, dried with anhydrous magnesium sulfate, and concentrated under reduced pressure to thereby produce a crude product of the title compound.
LCMS: 330.1 $[M+H-Boc]^+$ (Rt= 2.229 min).

[Step 8] 4-Chloro-2-(((2R,4R)-4-hydroxy pyrrolidin-2-yl)methoxy)-6-isopropoxybenzoic acid

The crude product of the compound obtained in Step 7 was dissolved in 1,4-dioxane (2 mL), combined with 4M hydrochloric acid/1,4-dioxane solution (2 mL, 8 mmol), and agitated overnight at room temperature. The reaction mixture was concentrated under reduced pressure, and the residue was azeotroped with 1,4-dioxane to thereby produce a crude product of the title compound.
LCMS: 330.1 $[M+H]^+$ (Rt= 1.718 min).

[Step 9] (2R,11aR)-8-Chloro-2-hydroxy-6-isopropoxy-2,3,11,11a-tetrahydro-1H,5H-benzo[f]pyrrolo[2,1-c][1,4]oxazepin-5-one

The crude product of the compound obtained in Step 8 and N,N-diisopropylethylamine (0.697 mL,4 mmol) were dissolved in DMF (4 mL), combined with WSC-HCl (115 mg, 0.6 mmol) and HOBt (91.9 mg, 0.6 mmol), and agitated at 40°C for 3 hours. The reaction mixture was then cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (89.0 mg). LCMS: 312.1 $[M+H]^+$ (Rt= 2.007 min).

<Reference Example 33: (2R,11aR)-2-Hydroxy-6-isopropoxy-8-(trifluoromethyl)-2,3,11,11a-tetrahydro-1H,5H-benzo[f]pyrrolo[2,1-c][1,4]oxazepin-5-one>

[Step 1] 1-(Benzyloxy)-3-fluoro-5-(trifluoromethyl)benzene

3-Fluoro-5-(trifluoromethyl)phenol (1.00 g, 5.55 mmol) and potassium carbonate (1.00 g, 7.22 mmol) were dissolved in DMF (11.0 mL), combined with benzyl bromide (0.80 mL, 6.67 mmol), and agitated overnight at room temperature. The reaction mixture was combined with water (20 mL), and extracted three times with heptane/ethyl acetate (2/1). The organic phase was collected, washed with saturated saline solution, dried with sodium sulfate, and concentrated under reduced pressure. The residue was purified with silica gel column chromatography (heptane/ethyl acetate) to thereby produce the title compound (1.50 g).

$^1$H-NMR ($CDCl_3$) δ: 7.43-7.34 (5H, m), 7.04 (1H, s), 6.94 (1H, d, J = 8.2 Hz), 6.85 (1H, dt, J = 10.2, 2.2 Hz), 5.08 (2H, s).
LCMS: 271.4 $[M+H]^+$ (Rt= 2.542 min).

[Step 2] 2-(Benzyloxy)-6-fluoro-4-(trifluoromethyl)benzoic acid

The compound obtained in Step 1 (1.50 g, 5.55 mmol) was dissolved in THF (20.0 mL), and to the resulting solution, 2M diisopropyl amide lithium /THF-heptane solution (3.30 mL, 6.60 mmol) was added dropwise at -78°C. After the drop was finished, the reaction mixture was agitated for 2 hours, combined with dry ice, raised to room temperature, and agitated overnight. The reaction mixture was combined with ethyl acetate, and extracted with water. The aqueous phase was combined with 1M hydrochloric acid aqueous solution, and extracted with ethyl acetate. The organic phase was dried

with sodium sulfate, and concentrated under reduced pressure to thereby produce the title compound (923 mg).
$^1$H-NMR ($CDCl_3$) δ: 7.44-7.34 (5H, m), 7.07 (2H, d, J = 7.3 Hz), 5.23 (2H, s).

[Step 3] Isopropyl 2-(benzyloxy)-6-fluoro-4-(trifluoromethyl)benzoate

The compound obtained in Step 2 (923 mg, 2.94 mmol) and potassium carbonate (1.53 g, 11.1 mmol) was dissolved in DMF (19 mL), combined with 2-iodopropane (0.58 mL, 5.83 mmol), and agitated overnight at room temperature. The reaction mixture was combined with water, and extracted with ethyl acetate. The organic phase was dried with sodium sulfate, and concentrated under reduced pressure. The residue was purified with silica gel column chromatography (heptane/ethyl acetate) to thereby produce the title compound (994 mg).

$^1$H-NMR ($CDCl_3$) δ: 7.42-7.32 (5H, m), 7.02-7.01 (2H, m), 5.33-5.23 (1H, m), 5.15 (2H, s), 1.30 (6H, d, J = 6.4 Hz).
LCMS: 357.1 $[M+H]^+$ (Rt= 2.569 min)

[Step 4] Isopropyl 2-(benzyloxy)-6-isopropoxy-4-(trifluoromethyl)benzoate

The compound obtained in Step 3 (994 mg, 2.79 mmol) was dissolved in 2-propanol (15 mL), combined with 50% sodium hydride (250 mg, 5.73 mmol), and agitated overnight at room temperature. The reaction mixture was combined with 2M hydrochloric acid aqueous solution, and extracted with ethyl acetate. The organic phase was dried with sodium sulfate, and concentrated under reduced pressure. The residue was purified with silica gel column chromatography (heptane/ethyl acetate) to thereby produce the title compound (227 mg).
LCMS: 397.2 $[M+H]^+$ (Rt= 2.600 min).

[Step 5] Isopropyl 2-hydroxy-6-isopropoxy-4-(trifluoromethyl)benzoate

The compound obtained in Step 4 (227 mg, 0.57 mmol) was dissolved in methanol (5.70 mL), combined with 10% palladium-activated carbon (22.7 mg), and agitated overnight under hydrogen atmosphere at normal pressure at room temperature. The reaction mixture was filtered with cerite, and the filtrate was concentrated under reduced pressure. The residue was purified with silica gel column chromatography (heptane/ethyl acetate) to thereby produce the title compound (100 mg).

$^1$H-NMR ($CDCl_3$) δ: 11.53 (1H, s), 6.79 (1H, d, J = 1.4 Hz), 6.59 (1H, s), 5.33-5.27 (1H, m), 4.67-4.61 (1H, m), 1.40-1.37 (12H, m).
LCMS: 307.1 $[M+H]^+$ (Rt= 2.447 min).

[Step 6] tert-Butyl (2R,4R)-4-((tert-butyldiphenylsilyl)oxy)-2-((3-isopropoxy-2-(isopropoxycarbonyl)-5-(trifluoromethyl) phenoxy)methyl)pyrrolidin-1-carboxylate

The compound obtained in Step 5 (100 mg, 0.33 mmol) and the compound obtained in Reference Example 20 (157 mg, 0.34 mmol) were dissolved in toluene (4 mL), combined with cyanomethylene tributylphosphorane (0.200 mL, 0.76 mmol), and agitated at 100°C overnight. The reaction mixture was then cooled to room temperature, and purified with silica gel column chromatography (heptane/ethyl acetate) to thereby produce the title compound (222 mg).
LCMS: 644.3 $[M+H-Boc]^+$ (Rt= 3.227 min).

[Step 7] 2-(((2R,4R)-1-(tert-Butoxycarbonyl)-4-hydroxy pyrrolidin-2-yl)methoxy)-6-isopropoxy-4-(trifluoromethyl)benzoic acid

The compound obtained in Step 6 (222 mg, 0.3 mmol) was dissolved in ethanol (3 mL), combined with 2M sodium hydroxide aqueous solution (0.6 mL, 1.19 mmol), and agitated at 60°C overnight. The reaction mixture was then cooled to room temperature, combined with 2M hydrochloric acid aqueous solution, and extracted with dichloromethane. The organic phase was dried with sodium sulfate, and concentrated under reduced pressure to thereby produce a crude product of the title compound.
LCMS: 406.2 $[M+H-tBu]^+$ (Rt= 2.488 min).

[Step 8] 2-(((2R,4R)-4-Hydroxy pyrrolidin-2-yl)methoxy)-6-isopropoxy-4-(trifluoromethyl)benzoic acid

The crude product of the compound obtained in Step 7 was dissolved in dichloromethane (1.49 mL), and to the resulting solution, trifluoroacetic acid (1.49 mL) was added dropwise. After the drop was finished, the mixture was agitated

at room temperature for 1 hour, the reaction mixture was azeotroped with toluene to thereby produce a crude product of the title compound.
LCMS: 406.2 $[M+H]^+$ (Rt= 1.901 min).

[Step 9] (2R,11aR)-2-Hydroxy-6-isopropoxy-8-(trifluoromethyl)-2,3,11,11a-tetrahydro-1H,5H-benzo[f]pyrrolo[2,1-c][1,4]oxazepin-5-one

The same procedures as those in Step 9 of Reference Example 32 were carried out except that the crude product of the compound obtained in Step 8 was used as a raw material, to thereby produce the title compound (52.3 mg).

$^1$H-NMR ($CDCl_3$) δ: 6.98 (1H, s), 6.93 (1H, s), 4.75-4.60 (3H, m), 4.11-3.84 (4H, m), 2.33-2.26 (1H, m), 2.01 (1H, br s), 1.87-1.83 (1H, m), 1.42 (3H, d, J = 5.9 Hz), 1.38 (3H, d, J = 5.9 Hz).
LCMS: 346.1 $[M+H]^+$ (Rt= 2.062 min).

<Reference Example 34: (2R,11aR)-2-Hydroxy-6-isopropoxy-8-methyl-2,3,11,11a-tetrahydro-1H,5H-benzo[f]pyrrolo[2,1-c][1,4]oxazepin-5-one>

The same procedures as those in Step 2 to Step 6 of Reference Example 27 were carried out except that the compound obtained in Reference Example 4 (355 mg, 1.58 mmol) and the compound obtained in Reference Example 26 (584 mg, 1.90 mmol) were used as raw materials, to thereby produce the title compound (268 mg).
LCMS: 292.2 $[M+H]^+$ (Rt= 1.930 min)

<Reference Example 35:(7aS,9S)-9-hydroxy-1-isopropoxy-3-methyl-6,7,7a,8,9,10-hexahydro-12H-benzo[b]pyrrolo[1,2-e][1,5]oxazocin-12-one>

[Step 1] tert-Butyl (2R,4S)-4-((tert-butyldiphenylsilyl)oxy)-2-formylpyrrolidin-1-carboxylate

The compound obtained in Reference Example 22 (2.00 g, 4.39 mmol) were dissolved in dichloromethane (22 mL), combined with Dess-Martin Periodinane (1.86 g, 4.39 mmol), and agitated for 2 hours at room temperature. The reaction mixture was combined with saturated sodium bicarbonate aqueous solution and sodium thiosulfate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (1.80 g).

$^1$H-NMR ($CDCl_3$) δ: 9.48-9.35 (1H, m), 7.62-7.58 (4H, m), 7.44-7.35 (6H, m), 4.44-4.25 (2H, m), 3.61-3.27 (2H, m), 2.08-2.02 (1H, m), 1.80-1.72 (1H, m), 1.45-1.43 (9H, m), 1.03 (9H, s).
LCMS: 354.9 $[M+H\text{-}Boc]^+$ (Rt= 2.946 min).

[Step 2] tert-Butyl (2R,4S)-4-((tert-butyldiphenylsilyl)oxy)-2-((E)-2-methoxy vinyl)pyrrolidin-1-carboxylate

(Methoxymethyl)triphenylphosphoniumchloride (2.87 g, 8.38 mmol) was dissolved in THF (6 mL), combined with 1M sodium bis(trimethylsilyl)amide/THF solution (7.96 mL, 7.96 mmol) at 0°C, and agitated at 0°C for 10 minutes. To the resulting reaction mixture, THF solution (7 mL) of the compound obtained in Step 1 (1.90 g, 4.19 mmol) was added dropwise. After the drop was finished, the reaction mixture was raised to room temperature, and agitated overnight. The reaction mixture was combined with saturated ammonium chloride aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (1.9 g). LCMS: 382.2 $[M+H\text{-}Boc]^+$ (Rt= 3.170 min).

[Step 3] tert-Butyl (2R,4S)-4-((tert-butyldiphenylsilyl)oxy)-2-(2-oxo ethyl)pyrrolidin-1-carboxylate

The compound obtained in Step 2 (2.16 g, 4.48 mmol) was dissolved in a mixture solvent of acetonitrile (30 mL) and water (7.5 mL), combined with trifluoroacetic acid (0.374 mL, 4.88 mmol), and agitated for 1 hour at room temperature. The reaction mixture was combined with saturated sodium bicarbonate aqueous solution, and extracted with ethyl acetate. The organic phase was washed twice with saturated saline solution, dried with sodium sulfate, and concentrated under reduced pressure to thereby produce a crude product of the title compound.
LCMS: 368.2 $[M+H\text{-}Boc]^+$ (Rt= 2.966 min).

[Step 4] tert-Butyl (2S,4S)-4-((tert-butyldiphenylsilyl)oxy)-2-(2-hydroxyethyl)pyrrolidin-1-carboxylate

The crude product of the compound obtained in Step 3 was dissolved in methanol (14.9 mL), combined with borosodium hydride (339 mg, 8.97 mmol), and agitated for 3 hours at room temperature. The reaction mixture was combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (1.95 g).
LCMS: 370.2 $[M+H-Boc]^+$ (Rt= 2.927 min).

[Step 5] tert-Butyl (2S,4S)-4-((tert-butyldiphenylsilyl)oxy)-2-(2-((2,2,7-trimethyl-4-oxo-4H-benzo[d][1,3]dioxin-5-yl)oxy) ethyl)pyrrolidin-1-carboxylate

The same procedures as those in Step 4 of Reference Example 32 were carried out except that the compound obtained in Step 4 (113 mg, 0.24 mmol) and the compound obtained in Step 1 of Reference Example 2 (55.1 mg, 0.26 mmol) were used as raw materials, to thereby produce the title compound (141 mg).
LCMS: 660.4 $[M+H]^+$ (Rt= 3.440 min).

[Step 6] tert-Butyl (2S,4S)-4-((tert-butyldiphenylsilyl)oxy)-2-(2-(3-isopropoxy-2-(methoxycarbonyl)-5-methylphenoxy) ethyl)pyrrolidin-1-carboxylate

The same procedures as those in Step 5 to Step 6 of Reference Example 32 were carried out except that the compound obtained in Step 5 (140 mg, 0.21 mmol) was used as a raw material, to thereby produce the title compound (132 mg).
LCMS: 676.3 $[M+H]^+$ (Rt= 3.484 min).

[Step 7] (7aS,9S)-9-hydroxy-1-isopropoxy-3-methyl-6,7,7a,8,9,10-hexahydro-12H-benzo[b]pyrrolo[1,2-e][1,5]oxazocin-12-one

The same procedures as those in Step 7 to Step 9 of Reference Example 32 were carried out except that the compound obtained in Step 6 (132 mg, 0.20 mmol) was used as a raw material, to thereby produce the title compound (6.3 mg).
LCMS: 306.2 $[M+H]^+$ (Rt= 1.899 min).

<Reference Example 36: (2S,11aR)-6-(((R)-1-Fluoropropan-2-yl)oxy)-2-hydroxy-8-methyl-2,3,11,11a-tetrahydro-1H,5H-benzo[f]pyrrolo[2,1-c][1,4]oxazepin-5-one>

[Step 1] tert-Butyl (2R,4S)-4-(benzyloxy)-2-((3-(((R)-1-((tert-butyldiphenylsilyl)oxy)propan-2-yl)oxy)-2-(methoxycarbonyl)-5-methylphenoxy)methyl)pyrrolidin-1-carboxylate

The same procedures as those in Step 2 of Reference Example 27 were carried out except that the compound obtained in Reference Example 25 (724 mg, 2.35 mmol) and the compound obtained in Reference Example 6 (939 mg, 1.96 mmol) were used as raw materials, to thereby produce the title compound (1.18 g).
LCMS: 668.4 $[M+H-Boc]^+$ (Rt= 3.135 min).

[Step 2] tert-Butyl (2R,4S)-4-(benzyloxy)-2-((3-(((R)-1-hydroxy propan-2-yl)oxy)-2-(methoxycarbonyl)-5-methylphenoxy)methyl)pyrrolidin-1-carboxylate

The compound obtained in Step 1 (1.18 g, 1.54 mmol) was dissolved in THF (5 mL), and to the resulting solution, 1M tetrabutyl ammonium fluoride/THF solution (2.3 mL, 2.31 mmol) was added dropwise at 0°C. After the drop was finished, the mixture was agitated for 1 hour at room temperature. The reaction mixture was combined with saturated ammonium chloride aqueous solution, and extracted with ethyl acetate. The organic phase was dried with anhydrous magnesium sulfate, and concentrated under reduced pressure. The residue was purified with silica gel column chromatography (heptane/ethyl acetate) to thereby produce the title compound (797 mg).
LCMS: 430.2 $[M+H-Boc]^+$ (Rt= 2.413 min).

[Step 3] tert-Butyl (2R,4S)-4-(benzyloxy)-2-((3-(((R)-1-fluoropropan-2-yl)oxy)-2-(methoxycarbonyl)-5-methylphenoxy) methyl)pyrrolidin-1-carboxylate

The compound obtained in Step 2 (297 mg, 0.56 mmol) was dissolved in dichloromethane (9.2 mL), and to the resulting solution, bis(2-methoxyethyl)aminosulfur trifluoride (197 μL, 1.12 mmol) was added dropwise at -78°C, and agitated for 21 hours at room temperature. The reaction mixture was combined with saturated sodium bicarbonate aqueous solution, and extracted with ethyl acetate. The organic phase was dried with anhydrous magnesium sulfate, and concentrated under reduced pressure. The residue was purified with silica gel column chromatography (heptane/ethyl acetate) to thereby produce the title compound (157 mg).

$^1$H-NMR ($CDCl_3$) δ: 7.33-7.28 (5H, m), 6.43-6.36 (2H, m), 4.61-4.20 (8H, m), 4.07-3.36 (6H, m), 2.32-2.30 (3H, m), 2.28-2.13 (2H, m), 1.47-1.44 (9H, m), 1.31 (3H, dd, J = 6.2, 1.1 Hz).
LCMS: 432.3 $[M+H-Boc]^+$ (Rt= 2.513 min).

[Step 4] (2S,11aR)-6-(((R)-1-Fluoropropan-2-yl)oxy)-2-hydroxy-8-methyl-2,3,11,11a-tetrahydro-1H,5H-benzo[f]pyrrolo [2,1-c][1,4]oxazepin-5-one

The same procedures as those in Step 3 to Step 6 of Reference Example 27 were carried out except that the compound obtained in Step 3 (157 mg, 0.30 mmol) were used as raw materials, to thereby produce the title compound (63.3 mg).

$^1$H-NMR ($CDCl_3$) δ: 6.69 (1H, s), 6.55 (1H, s), 4.66-4.38 (4H, m), 4.16-4.05 (2H, m), 4.00-3.94 (1H, m), 3.84 (2H, d, J = 4.6 Hz), 2.31 (3H, s), 2.19-2.12 (1H, m), 1.97 (1H, s), 1.85 (1H, dt, J = 13.3, 5.3 Hz), 1.38 (3H, dd, J = 6.2, 1.6 Hz).
LCMS: 310.2 $[M+H]^+$ (Rt= 1.881 min).

<Reference Example 37: (2S,11aR)-6-(((R)-1,1-difluoropropan-2-yl)oxy)-2-hydroxy-8-methyl-2,3,11,11a-tetrahydro-1H,5H-benzo[f]pyrrolo[2,1-c][1,4]oxazepin-5-one>

[Step 1] tert-Butyl (2R,4S)-4-(benzyloxy)-2-((2-(methoxycarbonyl)-5-methyl-3-(((R)-1-oxopropan-2-yl)oxy)phenoxy) methyl)pyrrolidin-1-carboxylate

The compound obtained in Step 2 of Reference Example 36 (500 mg, 0.94 mmol) was dissolved in dichloromethane (4.72 mL), combined with Dess-Martin Periodinane (481 mg, 1.13 mmol), and agitated for 2 hours at room temperature. The reaction mixture was combined with saturated sodium bicarbonate aqueous solution and sodium thiosulfate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (434 mg).
LCMS: 428.2 $[M+H-Boc]^+$ (Rt= 2.366 min).

[Step 2] tert-Butyl (2R,4S)-4-(benzyloxy)-2-((3-(((R)-1,1-difluoropropan-2-yl)oxy)-2-(methoxycarbonyl)-5-methylphenoxy)methyl)pyrrolidin-1-carboxylate

The compound obtained in Step 1 (434 mg, 0.82 mmol) were dissolved in dichloromethane (8.2 mL), combined with bis(2-methoxyethyl)aminosulfur trifluoride (0.43 mL, 2.47 mmol), agitated at 0°C for 1 hour, brought to room temperature, and agitated for 1 hour. The reaction mixture was combined with saturated sodium bicarbonate aqueous solution, and extracted with ethyl acetate. The organic phase was dried with anhydrous magnesium sulfate, and concentrated under reduced pressure. The residue was purified with silica gel column chromatography (heptane/ethyl acetate) to thereby produce the title compound (348 mg).
LCMS: 450.2 $[M+H-Boc]^+$ (Rt= 2.519 min).

[Step 3] (2S,11aR)-6-(((R)-1,1-Difluoropropan-2-yl)oxy)-2-hydroxy-8-methyl-2,3,11,11a-tetrahydro-1H,5H-benzo[f]pyrrolo[2,1-c][1,4]oxazepin-5-one

The same procedures as those in Step 3 to Step 6 of Reference Example 27 were carried out except that the compound obtained in Step 2 (348 mg, 0.63 mmol) was used as a raw material, to thereby produce the title compound (127 mg).
LCMS: 328.1 $[M+H]^+$ (Rt= 1.938 min).

<Reference Example 38: (2S,11aR)-2-hydroxy-8-methyl-6-(((R)-1,1,1-trifluoropropan-2-yl)oxy)-2,3,11,11a-tetrahydro-1H,5H-benzo[f]pyrrolo[2,1-c][1,4]oxazepin-5-one>

The same procedures as those in Step 2 to Step 6 of Reference Example 27 were carried out except that the compound obtained in Reference Example 8 (7.70 g, 27.7 mmol) and the compound obtained in Reference Example 25 (10.2 g, 33.2 mmol) were used as raw materials, to thereby produce the title compound (5.60 g).

$^1$H-NMR ($CDCl_3$) δ: 6.70 (1H, s), 6.62 (1H, s), 4.72-4.63 (1H, m), 4.63-4.57 (1H, m), 4.15 (1H, dd, J = 9.1, 4.1 Hz), 4.10-3.95 (2H, m), 3.88-3.79 (2H, m), 2.32 (3H, s), 2.20-2.13 (1H, m), 1.94 (1H, s), 1.86 (1H, dt, J = 13.4, 5.3 Hz), 1.59-1.57 (3H, m).
LCMS: 346.1 $[M+H]^+$ (Rt= 1.996 min).

<Reference Example 39: (2R,11aR)-2-Hydroxy-8-methyl-6-(((R)1,1,1-trifluoropropan-2-yl)oxy)-2,3,11,11a-tetrahydro-1H,5H-benzo[f]pyrrolo[2,1-c][1,4]oxazepin-5-one>

The same procedures as those in Step 2 to Step 6 of Reference Example 27 were carried out except that the compound obtained in Reference Example 8 (300 mg, 1.08 mmol) and the compound obtained in Reference Example 26 (398 mg, 1.29 mmol) were used as raw materials, to thereby produce the title compound (245 mg).

$^1$H-NMR ($CDCl_3$) δ: 6.70 (1H, s), 6.64 (1H, s), 4.74-4.61 (3H, m), 4.04 (1H, dd, J = 10.1, 5.0 Hz), 3.98-3.89 (2H, m), 3.84 (1H, dd, J = 13.3, 4.6 Hz), 2.32 (3H, s), 2.30-2.23 (1H, m), 1.89 (1H, d, J = 1.8 Hz), 1.81 (1H, dd, J = 14.2, 1.4 Hz), 1.59-1.57 (3H, m).
LCMS: 346.1 $[M+H]^+$ (Rt= 2.010 min).

<Reference Example 40: (2S,11aR)-2-Hydroxy-8-methyl-6-(((S)-1,1,1-trifluoropropan-2-yl)oxy)-2,3,11,11a-tetrahydro-1H,5H-benzo[f]pyrrolo[2,1-c][1,4]oxazepin-5-one>

[Step 1] Methyl (S)-2-hydroxy-4-methyl-6-((1,1,1-trifluoropropan-2-yl)oxy)benzoate

The same procedures as those in Reference Example 7 were carried out except that (R)-1,1,1-trifluoro-2-propanol (1.00 g, 8.77 mmol) was used as a raw material, to thereby produce the title compound (547 mg).

$^1$H-NMR ($CDCl_3$) δ: 11.46 (1H, s), 6.51 (1H, s), 6.23 (1H, s), 4.71-4.62 (1H, m), 3.92 (3H, s), 2.30 (3H, s), 1.52 (3H, d, J = 6.9 Hz).
LCMS: 279.1 $[M+H]^+$ (Rt= 2.316 min).

[Step 2] (2S,11aR)-2-Hydroxy-8-methyl-6-(((S)-1,1,1-trifluoropropan-2-yl)oxy)-2,3,11,11a-tetrahydro-1H,5H-benzo[f]pyrrolo[2,1-c][1,4]oxazepin-5-one

The same procedures as those in Step 2 to Step 6 of Reference Example 27 were carried out except that the compound obtained in Step 1 (274 mg, 0.98 mmol) and the compound obtained in Reference Example 25 (275 mg, 0.89 mmol) were used as raw materials, to thereby produce the title compound (153 mg).

$^1$H-NMR ($CDCl_3$) δ: 6.68 (1H, s), 6.64 (1H, s), 4.65-4.58 (2H, m), 4.18 (1H, dd, J = 9.6, 4.1 Hz), 4.08-3.97 (2H, m), 3.90-3.85 (1H, m), 3.81 (1H, dd, J = 12.6, 4.8 Hz), 2.32 (3H, s), 2.21-2.14 (1H, m), 1.87 (1H, dt, J = 13.3, 5.5 Hz), 1.79 (1H, d, J = 4.1 Hz), 1.46 (3H, d, J = 6.4 Hz).
LCMS: 346.1 $[M+H]^+$ (Rt= 2.001 min).

<Reference Example 41: benzyl (2R,4R)-4-((tert-butyldiphenylsilyl)oxy)-2-(hydroxymethyl)pyrrolidin-1-carboxylate>

[Step 1] (2R,4R)-1-((Benzyloxy)carbonyl)-4-hydroxy pyrrolidin-2-carbonic acid

cis-4-Hydroxy-D-proline(5.30 g, 40.4 mmol) was dissolved in a mixture solvent of saturated sodium bicarbonate aqueous solution (120 mL) and THF (20 mL), and to the resulting solution, THF (20 mL) solution benzyloxycarbonyl chloride (7.58 g, 44.5 mmol) was added dropwise at 0°C. After the drop was finished, the mixture was agitated for 2 hours with keeping the temperature at 0°C, combined with 3M hydrochloric acid aqueous solution, and extracted with ethyl acetate. The organic phase was washed twice with saturated saline solution, dried with anhydrous magnesium sulfate, and concentrated under reduced pressure. The residue was purified with silica gel column chromatography (ethyl

acetate/methanol) to thereby produce the title compound (9.93 g).

$^1$H-NMR ($CDCl_3$) $\delta$: 7.37-7.33 (5H, m), 5.24-5.11 (2H, m), 4.53-4.45 (2H, m), 3.74-3.54 (2H, m), 2.43-2.23 (2H, m). LCMS: 266.1 [M+H]$^+$ (Rt= 1.833 min).

[Step 2] (2R,4R)-1-((Benzyloxy)carbonyl)-4-((tert-butyldiphenylsilyl)oxy)pyrrolidin-2-carbonic acid

The compound obtained in Step 1 (9.93 g, 37.4 mmol) and imidazole (12.7 g, 187 mmol) were dissolved in a mixture solvent of dichloromethane (187 mL) and DMF (37.4 mL), combined with tert-butyldiphenyl chlorosilane (21.1 mL, 82.3 mmol), and agitated overnight at room temperature. The reaction mixture was combined with water, and extracted with dichloromethane. The organic phase was washed with saturated saline solution, and concentrated under reduced pressure. The residue was dissolved in a mixture solvent of methanol (125 mL) and water (46.8 mL), combined with 4M lithium hydroxide aqueous solution (15 mL), and agitated for 2 hours at room temperature. The reaction mixture was combined with 1M hydrochloric acid aqueous solution, and extracted with dichloromethane. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (17.5 g).
LCMS: 504.2 [M+H]$^+$ (Rt= 2.572 min).

[Step 3] Benzyl (2R,4R)-4-((tert-butyldiphenylsilyl)oxy)-2-(hydroxymethyl)pyrrolidin-1-carboxylate

The compound obtained in Step 2 (17.5 g, 34.8 mmol) was dissolved in THF (69.7 mL), and to the resulting solution, 1M borane-THF/THF solution (46.5 mL, 46.5 mmol) was added dropwise at 0°C. The reaction mixture was brought to room temperature, and agitated for 4 hours. The reaction mixture was combined with methanol, and concentrated under reduced pressure. The residue was mixed with water, and extracted with ethyl acetate. The organic phase was washed with saturated sodium bicarbonate aqueous solution, then with saturated saline solution, dried with anhydrous magnesium sulfate, and concentrated under reduced pressure. The residue was purified with silica gel column chromatography (heptane/ethyl acetate) to thereby produce the title compound (14.0 g). LCMS: 490.3 [M+H]$^+$ (Rt= 2.700 min).

<Reference Example 42: (2R,11aR)-6-((R)-sec-Butoxy)-2-hydroxy-8-methyl-2,3,11,11a-tetrahydro-1H,5H-benzo[f]pyrrolo[2,1-c][1,4]oxazepin-5-one>

[Step 1] Benzyl (2R,4R)-4-((tert-butyldiphenylsilyl)oxy)-2-(((2,2,7-trimethyl-4-oxo-4H-benzo[d][1,3]dioxin-5-yl)oxy)methyl)pyrrolidin-1-carboxylate

The same procedures as those in Step 1 of Reference Example 30 were carried out except that the compound obtained in Step 1 of Reference Example 2 (1.08 g, 5.2 mmol) and the compound obtained in Reference Example 41 (1.96 g, 4.00 mmol) were used as raw materials, to thereby produce a crude product of the title compound.
LCMS: 680.3 [M+H]$^+$ (Rt= 2.953 min).

[Step 2] Benzyl (2R,4R)-2-((2-((benzyloxy)carbonyl)-3-hydroxy-5-methylphenoxy)methyl)-4-((tert-butyldiphenylsilyl)oxy)pyrrolidin-1-carboxylate

To a mixture of 50% sodium hydride (640 mg, 16.0 mmol) and THF (20 mL), benzylalcohol (2.16 g, 20.0 mmol) was added dropwise at 0°C, and agitated for 1 hour at room temperature. To the reaction mixture cooled again to 0°C, THF (5 mL) solution of the crude product of the compound obtained in Step 1 was added dropwise. The mixture was with its temperature maintained at 0°C, agitated for 1 hour, and combined with saturated ammonium chloride aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (1.56 g). LCMS: 730.3 [M+H]$^+$ (Rt= 3.187 min).

[Step 3] Benzyl (2R,4R)-2-((2-((benzyloxy)carbonyl)-3-((R)-sec-butoxy)-5-methylphenoxy)methyl)-4-((tert-butyldiphenylsilyl)oxy)pyrrolidin-1-carboxylate

The compound obtained in Step 2 (438 mg, 0.60 mmol) and (S)-(+)-2-butanol (88.9 mg, 1.20 mmol) were dissolved in toluene (6 mL), combined with cyanomethylene tributylphosphorane (290 mg, 1.20 mmol), and agitated at 100°C overnight. The reaction mixture was then cooled to room temperature, concentrated under reduced pressure, and

the residue was purified with silica gel column chromatography (heptane/ethyl acetate) to thereby produce the title compound (462 mg).
LCMS: 786.4 $[M+H]^+$ (Rt= 3.235 min).

[Step 4] 2-((R)-sec-Butoxy)-6-(((2R,4R)-4-((tert-butyldiphenylsilyl)oxy)pyrrolidin-2-yl)methoxy)-4-methyl benzoic acid

The compound obtained in Step 3 (462 mg, 0.59 mmol) was dissolved in ethanol (5.88 mL), combined with 10% palladium-activated carbon (46.2 mg), and agitated overnight under hydrogen atmosphere at normal pressure at room temperature. The reaction mixture was filtered with cerite, and the filtrate was concentrated under reduced pressure, to thereby produce a crude product of the title compound.
LCMS: 562.4 $[M+H]^+$ (Rt= 2.172 min).

[Step 5] (2R,11aR)-6-((R)-sec-Butoxy)-2-((tert-butyldiphenylsilyl)oxy)-8-methyl-2,3,11,11a-tetrahydro-1H,5H-benzo[f]pyrrolo[2,1-c][1,4]oxazepin-5-one

The crude product of the compound obtained in Step 4 and triethylamine (328 μL, 2.35 mmol) were dissolved in THF (5.88 mL), combined with 1.7M propylphosphonic acid anhydride/ethyl acetate solution (519 μL, 0.88 mmol), and agitated for 2 hours at room temperature. The reaction mixture was combined with saturated sodium bicarbonate aqueous solution, and extracted three times with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (220 mg). LCMS: 544.3 $[M+H]^+$ (Rt= 2.838 min).

[Step 6] (2R,11aR)-6-((R)-sec-Butoxy)-2-hydroxy-8-methyl-2,3,11,11a-tetrahydro-1H,5H-benzo[f]pyrrolo[2,1-c][1,4]oxazepin-5-one

The compound obtained in Step 5 (220 mg, 0.40 mmol) was dissolved in THF (1.62 mL), combined with 1M tetrabutyl ammonium fluoride/THF solution (809 μL, 0.81 mmol), and agitated for 4 hours at room temperature. The reaction mixture was combined with saturated ammonium chloride aqueous solution, and extracted with ethyl acetate twice. The organic phase was washed twice with saturated saline solution, dried with anhydrous magnesium sulfate, and concentrated under reduced pressure. The residue was purified with silica gel column chromatography (ethyl acetate/-methanol) to thereby produce the title compound (111 mg).
LCMS: 306.1 $[M+H]^+$ (Rt= 2.007 min)

<Reference Example 43: (2R,11aR)-6-((S)-sec-Butoxy)-2-hydroxy-8-methyl-2,3,11,11a-tetrahydro-1H,5H-benzo[f]pyrrolo[2,1-c][1,4]oxazepin-5-one>

The same procedures as those in Step 3 to Step 6 of Reference Example 42 were carried out except that the compound obtained in Step 2 of Reference Example 42 (438 mg, 0.60 mmol) and (R)-(-)-2-butanol (88.9 mg, 1.20 mmol) were used as raw materials, to thereby produce the title compound (109 mg).
LCMS: 306.1 $[M+H]^+$ (Rt= 2.024 min).

<Reference Example 44: (2S,11aR)-6- Cyclobutoxy-2-hydroxy-8-methyl-2,3,11,11a-tetrahydro-1H,5H-benzo[f]pyrrolo[2,1-c][1,4]oxazepin-5-one>

[Step 1] Methyl 2-cyclobutoxy-6-hydroxy-4-methyl benzoate

The same procedures as those in Step 2 to Step 3 of Reference Example 5 were carried out except that the compound obtained in Step 1 of Reference Example 2 (250 mg, 1.20 mmol) and cyclobutanol (104 mg, 1.44 mmol) were used as raw materials, to thereby produce the title compound (216 mg).

$^1$H-NMR ($CDCl_3$) δ: 11.53 (1H, s), 6.39 (1H, s), 6.04 (1H, s), 4.69-4.62 (1H, m), 3.93 (3H, s), 2.51-2.43 (2H, m), 2.27 (3H, s), 2.24-2.16 (2H, m), 1.92-1.83 (1H, m), 1.76-1.64 (1H, m).
LCMS: 237.1 $[M+H]^+$ (Rt= 2.371 min).

[Step 2] (2S,11aR)-6-Cyclobutoxy-2-hydroxy-8-methyl-2,3,11,11a-tetrahydro-1H,5H-benzo[f]pyrrolo[2,1-c][1,4]oxazepin-5-one

The same procedures as those in Step 2 to Step 6 of Reference Example 27 were carried out except that the compound obtained in Step 1 (211 mg, 0.89 mmol) and the compound obtained in Reference Example 25 (250 mg, 0.81 mmol) were used as raw materials, to thereby produce the title compound (88.4 mg).

$^1$H-NMR ($CDCl_3$) $\delta$: 6.46 (1H, s), 6.41 (1H, s), 4.69-4.60 (2H, m), 4.15-4.05 (2H, m), 3.99-3.80 (3H, m), 2.45-2.10 (8H, m), 1.91-1.79 (3H, m), 1.71-1.60 (1H, m).
LCMS: 304.2 $[M+H]^+$ (Rt= 1.949 min).

<Reference Example 45: (2R,11aR)-6-((2,2-difluorocyclopentyl)oxy)-2-hydroxy-8-methyl-2,3,11,11a-tetrahydro-1H,5H-benzo[f]pyrrolo[2,1-c][1,4]oxazepin-5-one>

[Step 1] Methyl 2-(benzyloxy)-6-((2-hydroxy cyclopentyl)oxy)-4-methyl benzoate

The compound obtained in Reference Example 2 (817 mg, 3.00 mmol) and potassium carbonate (829 mg, 6.00 mmol) were dissolved in DMF (10.0 mL), combined with 1,2-epoxy cyclopentane (757 mg, 9.00 mmol), and agitated at 100°C overnight. The reaction mixture was then cooled to room temperature, combined with water, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (565 mg).
LCMS: 357.2 $[M+H]^+$ (Rt= 2.320 min).

[Step 2] Methyl 2-(benzyloxy)-4-methyl-6-((2-oxo cyclopentyl)oxy)methyl benzoate

The compound obtained in Step 1 (709 mg, 1.99 mmol) were dissolved in dichloromethane (10 mL), and to the resulting solution, Dess-Martin Periodinane (1.01 g, 2.39 mmol) were added at 0°C, and agitated overnight at room temperature. The reaction mixture was combined with 1M sodium thiosulfate aqueous solution and saturated sodium bicarbonate aqueous solution, agitated for further 30 minutes, and extracted with dichloromethane. The organic phase was dried with anhydrous magnesium sulfate, and concentrated under reduced pressure. The residue was purified with silica gel column chromatography (heptane/ethyl acetate) to thereby produce the title compound (653 mg). LCMS: 355.2 $[M+H]^+$ (Rt= 2.342 min).

[Step 3] Methyl 2-(benzyloxy)-6-((2,2-difluorocyclopentyl)oxy)-4-methyl benzoate

The compound obtained in Step 2 (653 mg, 1.84 mmol) was dissolved in dichloromethane (9.2 mL), combined with bis(2-methoxyethyl)aminosulfur trifluoride (1.7 mL, 9.21 mmol), and agitated for 6 hours at room temperature. The reaction mixture was combined with saturated sodium bicarbonate aqueous solution, and extracted with dichloromethane. The organic phase was dried with anhydrous magnesium sulfate, and concentrated under reduced pressure. The residue was purified with silica gel column chromatography (heptane/ethyl acetate) to thereby produce the title compound (616 mg). LCMS: 377.2 $[M+H]^+$ (Rt= 2.453 min).

[Step 4] Methyl 2-((2,2-difluorocyclopentyl)oxy)-6-hydroxy-4-methyl benzoate

The compound obtained in Step 3 (616 mg, 1.64 mmol) was dissolved in ethanol (8.18 mL), combined with 10% palladium-activated carbon (61.6 mg), and agitated overnight under hydrogen atmosphere at normal pressure at room temperature. The reaction mixture was filtered with cerite, and the filtrate was concentrated under reduced pressure. The residue was purified with silica gel column chromatography (heptane/ethyl acetate) to thereby produce the title compound (403 mg).

$^1$H-NMR ($CDCl_3$) $\delta$: 11.47 (1H, s), 6.46 (1H, s), 6.24 (1H, s), 4.60-4.56 (1H, m), 3.91 (3H, s), 2.35-2.11 (6H, m), 2.03-1.92 (2H, m), 1.86-1.74 (1H, m).
LCMS: 287.1 $[M+H]^+$ (Rt= 2.341 min).

[Step 5] (2R,11aR)-6-((2,2-Difluorocyclopentyl)oxy)-2-hydroxy-8-methyl-2,3,11,11a-tetrahydro-1H,5H-benzo[f]pyrrolo[2,1-c][1,4]oxazepin-5-one

The same procedures as those in Step 2 to Step 3 of Reference Example 27 and Step 4 to Step 5 of Reference Example 42 were carried out except that the compound obtained in Step 4 (391 mg, 1.84 mmol) and the compound obtained in Reference Example 41 (637 mg, 1.30 mmol) were used as raw materials, , to thereby produce the title compound (222 mg).
LCMS: 354.1 $[M+H]^+$ (Rt= 2.027 min)

<Reference Example 46: (2S,11aR)-2-Hydroxy-6-isobutyl-8-methyl-2,3,11,11a-tetrahydro-1H,5H-benzo[f]pyrrolo[2,1-c][1,4]oxazepin-5-one>

[Step 1] Methyl 2-hydroxy-4-methyl-6-(2-methyl-1-propen-1-yl)benzoate

The same procedures as those in Step 4 of Reference Example 12 were carried out except that the compound obtained in Step 2 of Reference Example 12 (165 mg, 0.67 mmol) was used as a raw material, to thereby produce the title compound (142 mg).

$^1$H-NMR ($CDCl_3$) $\delta$: 11.23 (1H, s), 6.69 (1H, s), 6.49 (1H, s), 6.43 (1H, s), 3.88 (3H, s), 2.30 (3H, s), 1.88 (3H, s),1.65 (3H, s).
LCMS: 221.1 $[M+H]^+$ (Rt= 2.423 min).

[Step 2] (2S,11aR)-2-Hydroxy-6-isobutyl-8-methyl-2,3,11,11a-tetrahydro-1H,5H-benzo[f]pyrrolo[2,1-c][1,4]oxazepin-5-one

The same procedures as those in Step 2 to Step 6 of Reference Example 27 were carried out except that the compound obtained in Step 1 (142 mg, 0.65 mmol) and the compound obtained in Reference Example 25 (238 mg, 0.77 mmol) were used as raw materials, to thereby produce the title compound (78.6 mg).

$^1$H-NMR ($CDCl_3$) $\delta$: 6.83 (1H, s), 6.72 (1H, s), 4.63-4.61 (1H, m), 4.16 (1H, dd, J = 8.5, 3.0 Hz), 4.04-3.93 (2H, m), 3.85-3.84 (2H, m), 3.19 (1H, dd, J = 13.0, 7.5 Hz), 2.39 (1H, dd, J = 13.3, 6.9 Hz), 2.31 (3H, s), 2.18-2.12 (1H, m), 1.88 (1H, dt, J = 13.4, 5.1 Hz), 1.82-1.76 (1H, m), 1.64 (1H, s), 0.85 (6H, t, J = 6.2 Hz).
LCMS: 290.1 $[M+H]^+$ (Rt= 2.089 min).

<Reference Example 47: (2S,11aR)-6-(2,6-Difluorophenyl)-2-hydroxy-8-methyl-2,3,11,11a-tetrahydro-1H,5H-benzo[f]pyrrolo[2,1-c][1,4]oxazepin-5-one>

The same procedures as those in Step 2 to Step 6 of Reference Example 27 were carried out except that the compound obtained in Reference Example 15 (131 mg, 0.47 mmol) and the compound obtained in Reference Example 25 (173 mg, 0.56 mmol) were used as raw materials, to thereby produce the title compound (77.4 mg).

$^1$H-NMR ($CDCl_3$) $\delta$: 7.29-7.22 (1H, m), 7.02-6.95 (3H, m), 6.87-6.82 (1H, m), 4.58-4.55 (1H, m), 4.28 (1H, dd, J = 9.8, 4.8 Hz), 4.25-4.17 (1H, m), 4.08-4.03 (1H, m), 3.82-3.77 (1H, m), 3.72 (1H, dd, J = 12.8, 4.6 Hz), 2.39 (3H, s), 2.26-2.19 (1H, m), 1.87 (1H, dt, J = 13.3, 5.7 Hz), 1.71 (1H, d, J = 4.1 Hz).
LCMS: 346.1 $[M+H]^+$ (Rt= 2.061 min).

<Reference Example 48: (2S,11aR)-2-hydroxy-8-methyl-6-(pyrrolidin-1-yl)-2,3,11,11a-tetrahydro-1H,5H-benzo[f]pyrrolo[2,1-c][1,4]oxazepin-5-one>

[Step 1] (2S,11aR)-2-(Benzyloxy)-6-(cyclopropylmethoxy)-8-methyl-2,3,11,11a-tetrahydro-1H,5H-benzo[f]pyrrolo[2,1-c][1,4]oxazepin-5-one

The same procedures as those in Step 2 to Step 5 of Reference Example 27 were carried out except that the compound obtained in Step 1 of Reference Example 28 (110 mg, 0.47 mmol) and the compound obtained in Reference Example 25 (157 mg, 0.51 mmol) were used as raw materials, to thereby produce the title compound (101 mg).

$^1$H-NMR ($CDCl_3$) $\delta$: 7.37-7.28 (5H, m), 6.56 (1H, s), 6.47 (1H, s), 4.59 (1H, d, J = 11.4 Hz), 4.49 (1H, d, J = 11.9 Hz), 4.33-4.27 (1H, m), 4.12-4.02 (2H, m), 3.96-3.91 (4H, m), 3.81 (1H, dd, J = 10.1, 6.9 Hz), 2.30 (3H, s), 2.26-2.19 (1H, m),

1.88 (1H, ddd, J = 13.3, 5.9, 4.1 Hz), 1.32-1.29 (1H, m), 0.60-0.56 (2H, m), 0.37-0.34 (2H, m).
LCMS: 394.2 $[M+H]^+$ (Rt= 2.344 min).

[Step 2] (2S,11aR)-2-(Benzyloxy)-6-hydroxy-8-methyl-2,3,11,11a-tetrahydro-1H,5H-benzo[f]pyrrolo[2,1-c][1,4]oxazepin-5-one

The compound obtained in Step 1 (138 mg, 0.35 mmol) was dissolved in ethanol (1.75 mL), combined with 4M hydrochloric acid/1,4-dioxane solution (437 μL, 1.75 mmol), and agitated at 50°C overnight. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (102 mg). $^1$H-NMR ($CDCl_3$) δ: 7.37-7.28 (5H, m), 6.46 (1H, d, J = 0.9 Hz), 6.28 (1H, d, J = 1.4 Hz), 4.58 (1H, d, J = 11.9 Hz), 4.51 (1H, d, J = 11.9 Hz), 4.44 (1H, dd, J = 11.9, 1.8 Hz), 4.25-4.18 (2H, m), 4.01 (1H, d, J = 13.7 Hz), 3.95 (1H, dd, J = 11.9, 8.7 Hz), 3.81 (1H, dd, J = 13.7, 4.1 Hz), 2.34 (1H, tdd, J = 8.6, 4.3, 2.1 Hz), 2.24 (3H, s), 1.73 (1H, ddd, J = 14.2, 9.8, 3.4 Hz).
LCMS: 340.2 $[M+H]^+$ (Rt= 2.418 min).

[Step 3] (2S,11aR)-2-(Benzyloxy)-8-methyl-5-oxo-2,3,11,11a-tetrahydro-1H,5H-benzo[f]pyrrolo[2,1-c][1,4]oxazepin-6-yl trifluoromethane sulfonate

The same procedures as those in Step 1 of Reference Example 12 were carried out except that the compound obtained in Step 2 (102 mg, 0.30 mmol) was used as a raw material, to thereby produce the title compound (126 mg).

$^1$H-NMR ($CDCl_3$) δ: 7.36-7.29 (5H, m), 6.90 (1H, s), 6.89 (1H, s), 4.60 (1H, d, J = 11.4 Hz), 4.45 (1H, d, J = 11.9 Hz), 4.29-4.24 (2H, m), 4.16-4.04 (3H, m), 3.76 (1H, dd, J = 12.8, 4.6 Hz), 2.37 (3H, s), 2.33-2.27 (1H, m), 1.86 (1H, dt, J = 13.3, 5.5 Hz).
LCMS: 472.1 $[M+H]^+$ (Rt= 2.406 min).

[Step 4] (2S,11aR)-2-(Benzyloxy)-8-methyl-6-(pyrrolidin-1-yl)-2,3,11,11a-tetrahydro-1H,5H-benzo[f]pyrrolo[2,1-c][1,4]oxazepin-5-one

The compound obtained in Step 3 (126 mg, 0.27 mmol), pyrrolidine (111 μL, 1.33 mmol), and cesium carbonate (175 mg, 0.53 mmol) were dissolved in 1,2-dimethoxyethane (1.33 mL), combined with XPhos (20.3 mg, 0.04 mmol), $Pd_2(dba)_3$ (9.75 mg, 0.01 mmol), and agitated at 80°C for 4 hours. The reaction mixture was then cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (48.0 mg).

$^1$H-NMR ($CDCl_3$) δ: 7.35-7.28 (5H, m), 6.35 (1H, s), 6.23 (1H, s), 4.56 (1H, d, J = 11.4 Hz), 4.47 (1H, d, J = 11.4 Hz), 4.33-4.26 (2H, m), 4.15-4.07 (2H, m), 3.90 (1H, t, J = 11.0 Hz), 3.80 (1H, dd, J = 12.8, 4.6 Hz), 3.44-3.38 (2H, m), 3.02-2.98 (2H, m), 2.34-2.28 (1H, m), 2.26 (3H, s), 1.92-1.73 (5H, m).
LCMS: 393.2 $[M+H]^+$ (Rt= 1.950 min).

[Step 5] (2S,11aR)-2-Hydroxy-8-methyl-6-(pyrrolidin-1-yl)-2,3,11,11a-tetrahydro-1H,5H-benzo[f]pyrrolo[2,1-c][1,4]oxazepin-5-one

The same procedures as those in Step 6 of Reference Example 27 were carried out except that the compound obtained in Step 4 (48.0 mg, 0.12 mmol) was used as a raw material, to thereby produce the title compound (30.1 mg). LCMS: 303.2 $[M+H]^+$ (Rt= 1.627 min).

<Reference Example 49: (2S,11aR)-7-Fluoro-2-hydroxy-6-methoxy-8-methyl-2,3,11,11a-tetrahydro-1H,5H-benzo[f]pyrrolo[2,1-c][1,4]oxazepin-5-one>

[Step 1] Methyl 2-(cyclopropylmethoxy)-3-fluoro-6-hydroxy-4-methyl benzoate

The compound obtained in Step 1 of Reference Example 28 (10.3 g, 43.4 mmol) was dissolved in acetonitrile (86.0 mL), and to the resulting solution, Selectfluor(registration trademark) (23.1 g, 65.1 mmol) was added for 20 minutes, and

the reaction mixture was brought to room temperature and agitated for 14 hours. The reaction mixture was filtered, and the filtrate was concentrated under reduced pressure. The residue was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with amino silica gel column chromatography (heptane/ethyl acetate) to thereby produce the title compound (5.51 g).

$^1$H-NMR ($CDCl_3$) δ: 10.88 (1H, s), 6.55 (1H, dd, J = 5.9, 0.9 Hz), 3.97 (3H, s), 3.86 (2H, d, J = 6.9 Hz), 2.26 (3H, d, J = 2.3 Hz), 1.34-1.24 (1H, m), 0.64-0.59 (2H, m), 0.34-0.30 (2H, m).
LCMS: 255.1 [M+H]$^+$ (Rt= 2.343 min).

[Step 2] tert-Butyl (2R,4S)-4-(benzyloxy)-2-((3-(cyclopropylmethoxy)-4-fluoro-2-(methoxycarbonyl)-5-methylphenoxy) methyl)pyrrolidin-1-carboxylate

The compound obtained in Step 1 (5.51 g, 21.7 mmol), the compound obtained in Reference Example 25 (8.00 g, 26.0 mmol), and triphenylphosphine (7.39 g, 28.2 mmol) were dissolved in THF (54.2 mL), and to the resulting solution, DIAD (5.49 mL, 28.2 mmol) was added dropwise at 0°C. After the drop was finished, the reaction mixture was agitated at 40°C overnight, cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate), then with amino silica gel column chromatography (heptane/ethyl acetate), to thereby produce the title compound (9.80 g). $^1$H-NMR ($CDCl_3$) δ: 7.37-7.28 (5H, m), 6.47-6.39 (1H, m), 4.56-4.46 (2H, m), 4.24-4.20 (3H, m), 4.02-3.97 (1H, m), 3.90 (2H, d, J = 7.3 Hz), 3.86 (3H, s), 3.50-3.40 (2H, m), 2.25-2.14 (5H, m), 1.45 (9H, d, J = 8.7 Hz), 1.23-1.18 (1H, m), 0.59-0.54 (2H, m), 0.30-0.26 (2H, m).
LCMS: 444.3[M+H-Boc]$^+$ (Rt= 2.673 min).

[Step 3] (2S,11aR)-2-(Benzyloxy)-6-(cyclopropylmethoxy)-7-fluoro-8-methyl-2,3,11,11a-tetrahydro-1H,5H-benzo[f]pyrrolo[2,1-c][1,4]oxazepin-5-one

The same procedures as those in Step 3 to Step 5 of Reference Example 27 were carried out except that the compound obtained in Step 2 (9.80 g, 18.0 mmol) was used as a raw material, to thereby produce the title compound (7.18 g).

$^1$H-NMR ($CDCl_3$) δ: 7.35-7.31 (5H, m), 6.60 (1H, d, J = 5.9 Hz), 4.47-4.58 (2H, m), 4.31-4.25 (1H, m), 4.08-4.01 (3H, m), 3.94-3.84 (4H, m), 2.24-2.19 (4H, m), 1.87 (1H, dt, J = 13.0, 5.0 Hz), 1.31-1.29 (1H, m), 0.57-0.46 (2H, m), 0.33-0.25 (2H, m).
LCMS: 412.2 [M+H]$^+$ (Rt= 2.391 min).

[Step 4] (2S,11aR)-2-(Benzyloxy)-7-fluoro-6-hydroxy-8-methyl-2,3,11,11a-tetrahydro-1H,5H-benzo[f]pyrrolo[2,1-c][1,4] oxazepin-5-one

The compound obtained in Step 3 (7.18 g, 17.4 mmol) was dissolved in ethanol (58 mL), combined with 4M hydrochloric acid/ethyl acetate solution (21.8 mL), and agitated at 50°C for 22 hours. The reaction mixture was concentrated under reduced pressure, the residue was purified with silica gel column chromatography (heptane/ethyl acetate) to thereby produce the title compound (5.07 g).

$^1$H-NMR ($CDCl_3$) δ: 7.37-7.28 (5H, m), 6.24 (1H, d, J = 5.9 Hz), 4.60-4.50 (2H, m), 4.44 (1H, dd, J = 11.9, 1.8 Hz), 4.23-4.20 (2H, m), 3.99-3.85 (3H, m), 2.35 (1H, dd, J = 13.0, 6.2 Hz), 2.24 (3H, d, J = 1.8 Hz), 1.73 (1H, td, J = 14.2, 10.1 Hz).
LCMS: 358.2 [M+H]$^+$ (Rt= 2.439 min).

[Step 5] (2S,11aR)-2-(Benzyloxy)-7-fluoro-6-methoxy-8-methyl-2,3,11,11a-tetrahydro-1H,5H-benzo[f]pyrrolo[2,1-c] [1,4]oxazepin-5-one

The compound obtained in Step 4 (50.0 mg, 0.14 mmol) and potassium carbonate (38.7 mg, 0.28 mmol) were dissolved in DMF (700 μL), combined with iodomethane (13.1 μL, 0.21 mmol), and agitated for 19 hours at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography

(heptane/ethyl acetate) to thereby produce the title compound (52.1 mg).

$^1$H-NMR ($CDCl_3$) δ: 7.37-7.28 (5H, m), 6.60 (1H, d, J = 5.9 Hz), 4.57 (1H, d, J = 11.9 Hz), 4.50 (1H, d, J = 11.9 Hz), 4.31-4.25 (1H, m), 4.15-4.00 (2H, m), 3.99 (3H, d, J = 1.4 Hz), 3.96-3.90 (2H, m), 3.85 (1H, dd, J = 12.3, 5.5 Hz), 2.26-2.20 (4H, m), 1.87 (1H, dt, J = 13.3, 5.0 Hz).
LCMS: 372.2 $[M+H]^+$ (Rt= 2.282 min).

[Step 6] (2S,11aR)-7-Fluoro-2-hydroxy-6-methoxy-8-methyl-2,3,11,11a-tetrahydro-1H,5H-benzo[f]pyrrolo[2,1-c][1,4]oxazepin-5-one

The compound obtained in Step 5 (52.1 mg, 0.14 mmol) was dissolved in ethyl acetate (1.4 mL), and to the resulting solution, 20% hydroxide palladium-activated carbon (2.61 mg) was added under hydrogen atmosphere at normal pressure, and the mixture was agitated for 4 hours at room temperature. The reaction mixture was filtered with cerite, and the filtrate was concentrated under reduced pressure to thereby produce the title compound (40.6 mg).

$^1$H-NMR ($CDCl_3$) δ: 6.62 (1H, d, J = 5.5 Hz), 4.64-4.58 (1H, m), 4.17-3.94 (6H, m), 3.89 (1H, dd, J = 12.6, 3.9 Hz), 3.82 (1H, dd, J = 12.6, 4.8 Hz), 2.27 (3H, d, J = 1.8 Hz), 2.20-2.14 (1H, m), 1.86 (1H, dt, J = 13.4, 5.3 Hz), 1.81 (1H, br s).
LCMS: 282.1 $[M+H]^+$ (Rt= 1.880 min).

<Reference Example 50: (2S,11aR)-6-Ethoxy-7-fluoro-2-hydroxy-8-methyl-2,3,11,11a-tetrahydro-1H,5H-benzo[f]pyrrolo[2,1-c][1,4]oxazepin-5-one>

The same procedures as those in Step 5 to Step 6 of Reference Example 49 were carried out except that the compound obtained in Step 4 of Reference Example 49 (50.0 mg, 0.14 mmol) and iodoethane (16.9 μL, 0.21 mmol) were used as raw materials, to thereby produce the title compound (40.2 mg).

$^1$H-NMR ($CDCl_3$) δ: 6.61 (1H, d, J = 5.9 Hz), 4.62-4.59 (1H, m), 4.30-4.02 (4H, m), 3.98-3.93 (1H, m), 3.84 (2H, d, J = 4.6 Hz), 2.26 (3H, d, J = 2.3 Hz), 2.19-2.13 (1H, m), 1.97 (1H, br s), 1.86 (1H, dt, J = 13.3, 5.3 Hz), 1.40 (3H, t, J = 6.9 Hz).
LCMS: 296.1 $[M+H]^+$ (Rt= 1.926 min).

<Reference Example 51: (2S,11aR)-7-Fluoro-2-hydroxy-8-methyl-6-propoxy-2,3,11,11a-tetrahydro-1H,5H-benzo[f]pyrrolo[2,1-c][1,4]oxazepin-5-one>

[Step 1] (2S,11aR)-2-(benzyloxy)-7-fluoro-8-methyl-6-propoxy-2,3,11,11a-tetrahydro-1H,5H-benzo[f]pyrrolo[2,1-c][1,4]oxazepin-5-one

The compound obtained in Step 4 of Reference Example 49 (120 mg, 0.34 mmol), 1-propanol (30.2 μL, 0.40 mmol), and triphenylphosphine (115 mg, 0.44 mmol) were dissolved in THF (1 mL), and to the resulting solution, DIAD (85 μL, 0.44 mmol) was added dropwise. After the drop was finished, the mixture was agitated for 2 hours at room temperature, combined with saturated sodium bicarbonate aqueous solution, and extracted with ethyl acetate. The organic phase was washed twice with saturated saline solution, dried with sodium sulfate, and vacuum-concentrated. The residue was purified with silica gel column chromatography (heptane/ethyl acetate) to thereby produce the title compound (187 mg).

$^1$H-NMR ($CDCl_3$) δ: 7.37-7.28 (5H, m), 6.59 (1H, d, J = 5.9 Hz), 5.01-4.95 (1H, m), 4.57 (1H, d, J = 11.9 Hz), 4.49 (1H, d, J = 11.4 Hz), 4.31-4.26 (1H, m), 4.16-3.89 (5H, m), 3.86 (1H, dd, J = 12.6, 5.3 Hz), 2.26-2.20 (4H, m), 1.87 (1H, dt, J = 13.1, 5.1 Hz), 1.78 (2H, ddd, J = 14.4, 7.1, 2.3 Hz), 0.98 (3H, t, J = 7.5 Hz).
LCMS: 400.2 $[M+H]^+$ (Rt= 2.422 min)

[Step 2] (2S,11aR)-7-Fluoro-2-hydroxy-8-methyl-6-propoxy-2,3,11,11a-tetrahydro-1H,5H-benzo[f]pyrrolo[2,1-c][1,4]oxazepin-5-one

The same procedures as those in Step 6 of Reference Example 49 were carried out except that the compound obtained in Step 1 (187 mg, 0.47 mmol) was used as a raw material, to thereby produce the title compound (164 mg).

$^1$H-NMR ($CDCl_3$) δ: 6.61 (1H, d, J = 5.9 Hz), 5.01-4.95 (1H, m), 4.63-4.59 (1H, m), 4.21-4.12 (2H, m), 4.09-3.92 (2H, m), 3.84 (2H, d, J = 4.6 Hz), 2.26 (3H, d, J = 1.8 Hz), 2.19-2.13 (1H, m), 1.90-1.76 (4H, m), 1.00 (3H, t, J = 7.3 Hz).
LCMS: 310.2 $[M+H]^+$ (Rt= 2.023 min)

<Reference Example 52: (2S,11aR)-6-Butoxy-7-fluoro-2-hydroxy-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (100 mg, 0.28 mmol) and 1-butanol (38.0 μL, 0.42 mmol) were used as raw materials, to thereby produce the title compound (135 mg).
LCMS: 324.2 $[M+H]^+$ (Rt= 2.106 min).

<Reference Example 53: (2S,11aR)-7-Fluoro-2-hydroxy-8-methyl-6-(pentyloxy)-2,3,11,11a-tetrahydro-1H,5H-benzo[f]pyrrolo[2,1-c][1,4]oxazepin-5-one>

The same procedures as those in Step 5 to Step 6 of Reference Example 49 were carried out except that the compound obtained in Step 4 of Reference Example 49 (100 mg, 0.28 mmol) and 1-bromopentane (51.5 μL, 0.42 mmol) were used as raw materials, to thereby produce the title compound (85.3 mg).
LCMS: 338.2 $[M+H]^+$ (Rt= 2.203 min).

<Reference Example 54: (2S,11aR)-7-Fluoro-6-(3-fluoropropoxy)-2-hydroxy-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (75.0 mg, 0.21 mmol) and 3-fluoro-1-propanol (23.7 μL, 0.31 mmol) were used as raw materials, to thereby produce the title compound (50.8 mg).
LCMS: 328.8 $[M+H]^+$ (Rt= 1.956 min).

<Reference Example 55: (2S,11aR)-7-Fluoro-2-hydroxy-6-isopropoxy-8-methyl-2,3,11,11a-tetrahydro-1H,5H-benzo[f]pyrrolo[2,1-c][1,4]oxazepin-5-one>

[Step 1] Methyl 3-fluoro-6-hydroxy-2-isopropoxy-4-methyl benzoate

The same procedures as those in Step 1 of Reference Example 49 were carried out except that the compound obtained in Reference Example 4 (53.0 mg, 0.24 mmol) was used as a raw material, to thereby produce the title compound (18.2 mg).

$^1$H-NMR ($CDCl_3$) δ: 10.93 (1H, s), 6.53 (1H, d, J = 5.5 Hz), 4.46-4.37 (1H, m), 3.96 (3H, s), 2.26 (3H, d, J = 2.3 Hz), 1.31 (6H, dd, J = 5.9, 0.9 Hz).
LCMS: 243.1 $[M+H]^+$ (Rt= 2.317 min).

[Step 2] (2S,11aR)-7-Fluoro-2-hydroxy-6-isopropoxy-8-methyl-2,3,11,11a-tetrahydro-1H,5H-benzo[f]pyrrolo[2,1-c][1,4]oxazepin-5-one

The same procedures as those in Step 2 to Step 6 of Reference Example 27 were carried out except that the compound obtained in Step 1 (252 mg, 1.04 mmol) and the compound obtained in Reference Example 25 (384 mg, 1.25 mmol) were used as raw materials, to thereby produce the title compound (122 mg).

$^1$H-NMR ($CDCl_3$) δ: 6.61 (1H, d, J = 5.9 Hz), 4.61 (1H, s), 4.53-4.47 (1H, m), 4.14 (1H, dd, J = 9.6, 4.6 Hz), 4.07-3.92 (2H, m), 3.88-3.80 (2H, m), 2.26 (3H, d, J = 2.3 Hz), 2.19-2.13 (1H, m), 1.89-1.83 (1H, m), 1.35 (3H, d, J = 5.9 Hz), 1.28 (3H, d, J = 6.4 Hz).
LCMS: 310.2 $[M+H]^+$ (Rt= 1.971 min).

<Reference Example 56: (2S,11aR)-9-Fluoro-2-hydroxy-6-isopropoxy-8-methyl-2,3,11,11a-tetrahydro-1H,5H-benzo[f]pyrrolo[2,1-c][1,4]oxazepin-5-one>

[Step 1] 8-Fluoro-5-isopropoxy-2,2,7-trimethyl-4H-benzo[d][1,3]dioxin-4-one

The same procedures as those in Step 1 of Reference Example 49 were carried out except that the compound obtained in Step 1 of Reference Example 4 (5.05 g, 20.2 mmol) was used as a raw material, and the residue was purified with silica gel column chromatography (heptane/ethyl acetate) to thereby produce the title compound (290 mg). LCMS: 269.1 $[M+H]^+$ (Rt= 2.331 min).

[Step 2] Methyl 3-fluoro-2-hydroxy-6-isopropoxy-4-methyl benzoate

The same procedures as those in Step 3 of Reference Example 2 were carried out except that the compound obtained in Step 1 (290 mg, 1.08 mmol) was used as a raw material, to thereby produce the title compound (246 mg).

$^1$H-NMR ($CDCl_3$) δ: 11.49 (1H, s), 6.21 (1H, d, J = 5.5 Hz), 4.51-4.42 (1H, m), 3.93 (3H, s), 2.28 (3H, d, J = 2.3 Hz), 1.33 (6H, d, J = 5.9 Hz).
LCMS: 243.1 $[M+H]^+$ (Rt= 2.300 min).

[Step 3] (2S,11aR)-9-Fluoro-2-hydroxy-6-isopropoxy-8-methyl-2,3,11,11a-tetrahydro-1H,5H-benzo[f]pyrrolo[2,1-c][1,4] oxazepin-5-one

The same procedures as those in Step 2 to Step 6 of Reference Example 27 were carried out except that the compound obtained in Step 2 (246 mg, 1.01 mmol) and the compound obtained in Reference Example 25 (374 mg, 1.22 mmol) were used as raw materials, to thereby produce the title compound (236 mg).
LCMS: 310.2 $[M+H]^+$ (Rt= 1.970 min).

<Reference Example 57: (2S,11aR)-7,9-Difluoro-2-hydroxy-6-isopropoxy-8-methyl-2,3,11,11a-tetrahydro-1H,5H-benzo[f]pyrrolo[2,1-c][1,4]oxazepin-5-one>

[Step 1] 6,8-Difluoro-5-isopropoxy-2,2,7-trimethyl-4H-benzo[d][1,3]dioxin-4-one

The compound obtained in Step 1 of Reference Example 4 (1 g, 4.00 mmol) was dissolved in acetonitrile (7.99 mL), and to the resulting solution, Selectfluor(registration trademark) (3.54 g, 9.99 mmol) was added at 0°C, and agitated at 60°C for 1 hour. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (223 mg).

$^1$H-NMR ($CDCl_3$) δ: 4.55-4.46 (1H, m), 2.28 (3H, t, J = 2.3 Hz), 1.73 (6H, s), 1.36 (6H, dd, J = 5.9, 0.9 Hz).
LCMS: 287.1 $[M+H]^+$ (Rt= 2.404 min).

[Step 2] (2S,11aR)-7,9-Difluoro-2-hydroxy-6-isopropoxy-8-methyl-2,3,11,11a-tetrahydro-1H,5H-benzo[f]pyrrolo[2,1-c] [1,4]oxazepin-5-one

The same procedures as those in Step 2 to Step 3 of Reference Example 56 were carried out except that the compound obtained in Step 1 (223 mg, 0.78 mmol) was used as a raw material, to thereby produce the title compound (106 mg).

$^1$H-NMR ($CDCl_3$) δ: 4.65-4.59 (1H, m), 4.47-4.41 (1H, m), 4.24 (1H, dd, J = 9.1, 3.2 Hz), 4.08-3.98 (2H, m), 3.88 (1H, dd, J = 12.8, 3.2 Hz), 3.82 (1H, dd, J = 12.8, 5.0 Hz), 2.23 (3H, t, J = 2.1 Hz), 2.21-2.15 (1H, m), 1.89 (1H, dt, J = 13.4, 5.1 Hz), 1.80 (1H, d, J = 4.1 Hz), 1.35 (3H, d, J = 5.9 Hz), 1.26 (3H, d, J = 5.9 Hz).
LCMS: 328.1 $[M+H]^+$ (Rt= 2.006 min).

<Reference Example 58: (2R,11aR)-7-Chloro-6-ethoxy-2-hydroxy-8-methyl-2,3,11,11a-tetrahydro-1H,5H-benzo[f]pyrrolo[2,1-c][1,4]oxazepin-5-one>

[Step 1] 8-Chloro-5-hydroxy-2,2,7-trimethyl-4H-benzo[d][1,3]dioxin-4-one

The compound obtained in Step 1 of Reference Example 2 (700 mg, 3.36 mmol) was dissolved in acetonitrile (16.8 mL), combined with N-chlorosuccinimide (584 mg, 4.37 mmol), and agitated at 50°C for 5 hours. The reaction mixture was concentrated under reduced pressure, the residue was purified with amino silica gel column chromatography (heptane/ethyl acetate) to thereby produce the title compound (523 mg).
LCMS: 243.1 $[M+H]^+$ (Rt= 2.395 min).

[Step 2] tert-Butyl (2R,4R)-4-((tert-butyldiphenylsilyl)oxy)-2-(((8-chloro-2,2,7-trimethyl-4-oxo-4H-benzo[d][1,3]dioxin-5-yl)oxy)methyl)pyrrolidin-1-carboxylate

The compound obtained in Step 1 (547 mg, 1.20 mmol), the compound obtained in Reference Example 20 (320 mg, 1.32 mmol), and triphenylphosphine (378 mg, 1.44 mmol) were dissolved in THF (6 mL), amd to the resulting solution, DIAD (280 μL, 1.44 mmol) was added dropwise at 0°C. After the drop was finished, the mixture was agitated overnight at room temperature, combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (460 mg).
LCMS: 580.3 $[M+H-Boc]^+$ (Rt= 3.120 min).

[Step 3] tert-Butyl (2R,4R)-4-((tert-butyldiphenylsilyl)oxy)-2-((4-chloro-3-hydroxy-2-(methoxycarbonyl)-5-methylphenoxy)methyl)pyrrolidin-1-carboxylate

The same procedures as those in Step 2 of Reference Example 30 were carried out except that the compound obtained in Step 2 (460 mg, 0.68 mmol) was used as a raw material, to thereby produce a crude product of the title compound.

[Step 4] tert-Butyl (2R,4R)-4-((tert-butyldiphenylsilyl)oxy)-2-((4-chloro-3-ethoxy-2-(methoxycarbonyl)-5-methylphenoxy)methyl)pyrrolidin-1-carboxylate

The same procedures as those in Step 3 of Reference Example 30 were carried out except that the crude product of the compound obtained in Step 3 and iodoethane (81.5 μL, 1.01 mmol) were used as raw materials, to thereby produce the title compound (407 mg). LCMS: 582.3 $[M+H-Boc]^+$ (Rt= 3.235 min).

[Step 5] (2R,11aR)-7-Chloro-6-ethoxy-2-hydroxy-8-methyl-2,3,11,11a-tetrahydro-1H,5H-benzo[f]pyrrolo[2,1-c][1,4]oxazepin-5-one

The same procedures as those in Step 7 to Step 9 of Reference Example 32 were carried out except that the compound obtained in Step 4 (407 mg, 0.60 mmol) was used as a raw material, to thereby produce the title compound (100 mg).
$^1$H-NMR ($CDCl_3$) δ: 6.77 (1H, s), 4.66-4.64 (2H, m), 4.34-4.30 (1H, m), 4.18-4.15 (1H, m), 4.03 (1H, dd, J = 10.3, 5.3 Hz), 3.95-3.92 (2H, m), 3.88-3.82 (1H, m), 2.37 (3H, s), 2.26-2.19 (1H, m), 1.83-1.80 (1H, m), 1.43 (3H, t, J = 6.9 Hz).

<Reference Example 59: (2R,11aR)-7-Chloro-2-hydroxy-6-isopropoxy-8-methyl-2,3,11,11a-tetrahydro-1H,5H-benzo[f]pyrrolo[2,1-c][1,4]oxazepin-5-one>

[Step 1] 6-Chloro-5-isopropoxy-2,2,7-trimethyl-4H-benzo[d][1,3]dioxin-4-one

The same procedures as those in Step 1 of Reference Example 58 were carried out except that the compound obtained in Step 1 of Reference Example 4 (800 mg, 3.20 mmol) was used as a raw material, to thereby produce the title compound (275 mg). LCMS:285.1 $[M+H]^+$ (Rt= 2.391 min).

[Step 2] Methyl 3-chloro-6-hydroxy-2-isopropoxy-4-methyl benzoate

The same procedures as those in Step 3 of Reference Example 2 were carried out except that the compound obtained in Step 1 (300 mg, 1.05 mmol) was used as a raw material, to thereby produce the title compound (358 mg).

$^1$H-NMR ($CDCl_3$) δ: 6.02 (1H, s), 4.29-4.23 (1H, m), 3.57 (3H, s), 2.02 (3H, s), 1.15 (6H, d, J = 5.9 Hz).
LCMS:259.1 $[M+H]^+$ (Rt=2.362 min).

[Step 3] (2S,11aR)-2-(Benzyloxy)-7-chloro-6-isopropoxy-8-methyl-2,3,11,11a-tetrahydro-1H,5H-benzo[f]pyrrolo[2,1-c][1,4]oxazepin-5-one

The same procedures as those in Step 2 to Step 5 of Reference Example 27 were carried out except that the compound obtained in Step 2 (273 mg, 1.05 mmol) and the compound obtained in Reference Example 25 (389 mg, 1.27 mmol) were used as raw materials, to thereby produce the title compound (128 mg).

LCMS: 416.2 $[M+H]^+$ (Rt= 2.453 min).

[Step 4] (2S,11aR)-7-Chloro-2-hydroxy-6-isopropoxy-8-methyl-2,3,11,11a-tetrahydro-1H,5H-benzo[f]pyrrolo[2,1-c][1,4]oxazepin-5-one

The compound obtained in Step 3 (100 mg, 0.24 mmol) was dissolved in methanol (12 mL), combined with 10% palladium-activated carbon (25.6 mg), and agitated for 1 hour at room temperature under hydrogen atmosphere at normal pressure. The reaction mixture was filtered with cerite, and the filtrate was concentrated under reduced pressure to thereby produce the title compound (81.2 mg).
LCMS: 326.1 $[M+H]^+$ (Rt= 2.014 min).

[Step 5] (2R,11aR)-7-Chloro-2-hydroxy-6-isopropoxy-8-methyl-2,3,11,11a-tetrahydro-1H,5H-benzo[f]pyrrolo[2,1-c][1,4]oxazepin-5-one

The compound obtained in Step 4 (81.2 mg, 0.25 mmol), acetic acid (21.4 μL, 0.374 mmol), and triphenylphosphine (85 mg, 0.324 mmol) was dissolved in THF (1.25 mL), and to the resulting solution, DIAD (63.1 μL, 0.324 mmol) was added dropwise at 0°C. After the drop was finished, the mixture was agitated for 3 hours at room temperature, combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate). The resulting compound was dissolved in methanol (1.07 mL), combined with potassium carbonate (59.4 mg, 0.43 mmol), and agitated overnight at room temperature. The reaction mixture was combined with water, and extracted with ethyl acetate. The organic phase was washed twice with saturated saline solution, dried with sodium sulfate, and concentrated under reduced pressure to thereby produce the title compound (69.2 mg).
LCMS: 326.1 $[M+H]^+$ (Rt= 2.014 min).

<Reference Example 60: (2R,11aR)-7,9-Dichloro-2-hydroxy-6-isopropoxy-8-methyl-2,3,11,11a-tetrahydro-1H,5H-benzo[f]pyrrolo[2,1-c][1,4]oxazepin-5-one>

[Step 1] 6,8-Dichloro-5-hydroxy-2,2,7-trimethyl-4H-benzo[d][1,3]dioxin-4-one

The same procedures as those in Step 1 of Reference Example 58 were carried out except that the compound obtained in Step 1 of Reference Example 2 (700 mg, 3.36 mmol) was used as a raw material, to thereby produce the title compound.
LCMS: 277.0 $[M+H]^+$ (Rt= 2.493 min).

[Step 2] tert-Butyl (2R,4R)-4-((tert-butyldiphenylsilyl)oxy)-2-((2,4-dichloro-5-hydroxy-6-(methoxycarbonyl)-3-methylphenoxy)methyl)pyrrolidin-1-carboxylate

The same procedures as those in Step 2 to Step 3 of Reference Example 58 were carried out except that the crude product of the compound obtained in Step 1 and the compound obtained in Reference Example 20 (547 mg, 1.20 mmol) were used as raw materials, to thereby produce a crude product of the title compound.
LCMS: 632.3 $[M+H-tBu]^+$ (Rt= 3.629 min).

[Step 3] tert-Butyl (2R,4R)-4-((tert-butyldiphenylsilyl)oxy)-2-((2,4-dichloro-5-isopropoxy-6-(methoxycarbonyl)-3-methylphenoxy)methyl)pyrrolidin-1-carboxylate

The same procedures as those in Step 3 of Reference Example 30 were carried out except that the crude product of the compound obtained in Step 2 and 2-iodopropane (18.1 μL, 0.18 mmol) were used as raw materials, to thereby produce the title compound (99.4 mg).

[Step 4] (2R,11aR)-7,9-Dichloro-2-hydroxy-6-isopropoxy-8-methyl-2,3,11,11a-tetrahydro-1H,5H-benzo[f]pyrrolo[2,1-c][1,4]oxazepin-5-one

The same procedures as those in Step 7 to Step 9 of Reference Example 32 were carried out except that the compound obtained in Step 3 (99.4 mg, 0.14 mmol) was used as a raw material, to thereby produce the title compound (27.4 mg).
LCMS: 360.1 $[M+H]^+$ (Rt= 2.190 min).

<Reference Example 61: (2R,11aR)-6-(Cyclopropylmethoxy)-7-fluoro-8-methyl-5-oxo-2,3,11,11a-tetrahydro-1H,5H-benzo[f]pyrrolo[2,1-c][1,4]oxazepin-2-yl 4-methyl benzene sulfonate>

[Step 1] (2S,11aR)-6-(Cyclopropylmethoxy)-7-fluoro-2-hydroxy-8-methyl-2,3,11,11a-tetrahydro-1H,5H-benzo[f]pyrrolo[2,1-c][1,4]oxazepin-5-one

The compound obtained in Step 3 of Reference Example 49 (150 mg, 0.36 mmol) was dissolved in ethyl acetate (1.80 mL), combined with 20% hydroxide palladium-activated carbon (15.0 mg), and agitated for 1 hour at room temperature under hydrogen atmosphere at normal pressure. The reaction mixture was filtered with cerite, and the filtrate was concentrated under reduced pressure to thereby produce the title compound (116 mg).
LCMS: 322.2 $[M+H]^+$ (Rt= 2.020 min).

[Step 2] (2R,11aR)-6-(cyclopropylmethoxy)-7-fluoro-2-hydroxy-8-methyl-2,3,11,11a-tetrahydro-1H,5H-benzo[f]pyrrolo[2,1-c][1,4]oxazepin-5-one

The compound obtained in Step 1 (116 mg, 0.36 mmol), benzoic acid (52.8 mg, 0.43 mmol), and triphenylphosphine (113 mg, 0.43 mmol) were dissolved in THF (1.08 mL), and to the resulting solution, DIAD (84.2 μL, 0.43 mmol) was added dropwise. After the drop was finished, the mixture was agitated for 1 hour at room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was amino purified with silica gel column chromatography (heptane/ethyl acetate). The resulting compound was dissolved in ethanol (1.9 mL), combined with 2M sodium hydroxide aqueous solution (0.380 mL, 0.76 mmol), and agitated overnight at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (77.9 mg).

$^1$H-NMR ($CDCl_3$) δ: 6.64 (1H, d, J = 5.9 Hz), 4.64-4.58 (2H, m), 4.13-4.11 (1H, m), 4.02 (1H, dd, J = 10.1, 5.0 Hz), 3.92-3.87 (4H, m), 2.24-2.20 (4H, m), 1.86-1.79 (2H, m), 1.42-1.33 (1H, m), 0.59-0.55 (2H, m), 0.40-0.35 (2H, m).
LCMS: 322.2 $[M+H]^+$ (Rt= 2.027 min).

[Step 3] (2R,11aR)-6-(cyclopropylmethoxy)-7-fluoro-8-methyl-5-oxo-2,3,11,11a-tetrahydro-1H,5H-benzo[f]pyrrolo[2,1-c][1,4]oxazepin-2-yl 4-methyl benzene sulfonate

The compound obtained in Step 2 (147 mg, 0.46 mmol), pyridine (73.7 μL, 0.92 mmol), and 4-dimethylaminopyridine (11.2 mg, 0.09 mmol) were dissolved in dichloromethane (2.29 mL), combined with p-toluene sulfonyl chloride (131 mg, 0.69 mmol), and agitated at 40°C for 5 days. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with amino silica gel column chromatography (heptane/ethyl acetate) to thereby produce the title compound (109 mg).

$^1$H-NMR ($CDCl_3$) δ: 7.81 (2H, d, J = 8.2 Hz), 7.37 (2H, d, J = 7.8 Hz), 6.64 (1H, d, J = 5.9 Hz), 5.22-5.20 (1H, m), 4.38 (1H, dd, J = 11.7, 10.3 Hz), 4.06 (1H, dd, J = 9.6, 7.3 Hz), 4.01 (1H, dd, J = 10.3, 5.3 Hz), 3.98-3.84 (3H, m), 3.78 (1H, dd, J = 14.4, 4.8 Hz), 2.47 (3H, s), 2.38-2.31 (1H, m), 2.25 (3H, d, J = 1.8 Hz), 2.14-2.10 (1H, m), 1.37-1.30 (1H, m), 0.57-0.54 (2H, m), 0.39-0.31 (2H, m).
LCMS: 476.2 $[M+H]^+$ (Rt= 2.375 min).

<Reference Example 62: (2S,11aR)-7-Fluoro-2-hydroxy-8-methyl-6-(2,2,2-trifluoroethoxy)-2,3,11,11a-tetrahydro-1H,5H-benzo[f]pyrrolo[2,1-c][1,4]oxazepin-5-one>

The same procedures as those in Step 5 to Step 6 of Reference Example 49 were carried out except that the compound obtained in Step 4 of Reference Example 49 (100 mg, 0.28 mmol) and trifluoromethane sulfonic acid 2,2,2-trifluoroethyl (80.7 μL, 0.56 mmol) were used as raw materials, to thereby produce the title compound (99.3 mg). LCMS: 350.1 $[M+H]^+$ (Rt= 2.051 min).

<Reference Example 63: (2R,11aR)-7-Ffluoro-6-((1-fluorocyclopropyl)methoxy)-8-methyl-5-oxo-2,3,11,11a-tetrahydro-1H,5H-benzo[f]pyrrolo[2,1-c][1,4]oxazepin-2-yl4-methyl benzene sulfonate>

[Step 1] (2S,11aR)-2-(benzyloxy)-7-Fluoro-6-((1-fluorocyclopropyl)methoxy)-8-methyl-2,3,11,11a-tetrahydro-1H,5H-benzo[f]pyrrolo[2,1-c][1,4]oxazepin-5-one

The compound obtained in Step 4 of Reference Example 49 (147 mg, 0.41 mmol), (1-fluorocyclopropyl)methanol (44.6 mg, 0.50 mmol), and triphenylphosphine (130 mg, 0.50 mmol) were dissolved in THF (1.24 mL), and to the resulting solution, DIAD (96.3 μL, 0.50 mmol) was added dropwise. After the drop was finished, and agitated overnight at 40°C. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (224 mg).
LCMS: 430.2 $[M+H]^+$ (Rt= 2.357 min).

[Step 2] (2S,11aR)-7-Fluoro-6-((1-fluorocyclopropyl)methoxy)-2-hydroxy-8-methyl-2,3,11,11a-tetrahydro-1H,5H-benzo[f]pyrrolo[2,1-c][1,4]oxazepin-5-one

The same procedures as those in Step 1 of Reference Example 61 were carried out except that the compound obtained in Step 1 (224 mg, 0.52 mmol) was used as a raw material, to thereby produce the title compound (188 mg).
LCMS: 340.1 $[M+H]^+$ (Rt= 2.008 min).

[Step 3] (2R,11aR)-7-Fluoro-6-((1-fluorocyclopropyl)methoxy)-8-methyl-5-oxo-2,3,11,11a-tetrahydro-1H,5H-benzo[f]pyrrolo[2,1-c][1,4]oxazepin-2-yl 4-methyl benzene sulfonate

The same procedures as those in Step 2 to Step 3 of Reference Example 61 were carried out except that the compound obtained in Step 2 (49.5 mg, 0.15 mmol) was used as a raw material, to thereby produce the title compound (40.5 mg).

$^1$H-NMR ($CDCl_3$) δ: 7.81 (2H, d, J = 8.2 Hz), 7.38 (2H, d, J = 8.2 Hz), 6.68 (1H, d, J = 5.9 Hz), 5.22-5.20 (1H, m), 4.51-4.30 (3H, m), 4.04-3.92 (2H, m), 3.87 (1H, d, J = 14.2 Hz), 3.77 (1H, dd, J = 14.4, 5.3 Hz), 2.47 (3H, s), 2.40-2.32 (1H, m), 2.27 (3H, s), 2.14-2.11 (1H, m), 1.13-1.09 (2H, m), 0.99-0.76 (2H, m).
LCMS: 494.2 $[M+H]^+$ (Rt= 2.370 min).

<Reference Example 64: (2S,11aR)-7-Fluoro-2-hydroxy-6-isobutoxy-8-methyl-2,3,11,11a-tetrahydro-1H,5H-benzo[f]pyrrolo[2,1-c][1,4]oxazepin-5-one>

The same procedures as those in Step 5 to Step 6 of Reference Example 49 were carried out except that the compound obtained in Step 4 of Reference Example 49 (150 mg, 0.42 mmol) and 1-bromo-2-methyl propane (67.9 μL, 0.63 mmol) were used as raw materials, to thereby produce the title compound (132 mg).
LCMS: 324.2 $[M+H]^+$ (Rt= 2.102 min).

<Reference Example 65: (2S,11aR)-7-Fluoro-6-(2-fluoro-2-methylpropoxy)-2-hydroxy-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (200 mg, 0.56 mmol) and 2-fluoro-2-methyl-1-propanol (79.9 μL, 0.84 mmol) were used as raw materials, to thereby produce the title compound (92.3 mg).
LCMS: 342.1 $[M+H]^+$ (Rt= 2.030 min).

<Reference Example 66: (2R,11aR)-6-((1,3-Difluoropropan-2-yl)oxy)-7-fluoro-2-hydroxy-8-methyl-2,3,11,11a-tetrahydro-1H,5H-benzo[f]pyrrolo[2,1-c][1,4]oxazepin-5-one>

[Step 1] (2S,11aR)-6-((1,3-Difluoropropan-2-yl)oxy)-7-fluoro-2-hydroxy-8-methyl-2,3,11,11a-tetrahydro-1H,5H-benzo[f]pyrrolo[2,1-c][1,4]oxazepin-5-one

The same procedures as those in Reference Example 51 were carried out except that the compound obtained in Step 4 of Reference Example 49 (200 mg, 0.56 mmol) and 1,3-difluoropropan-2-ol (65.0 μL, 0.84 mmol) were used as raw

materials, to thereby produce the title compound (109 mg).
LCMS: 346.2 $[M+H]^+$ (Rt= 1.960 min).

[Step 2] (2R,11aR)-6-((1,3-Difluoropropan-2-yl)oxy)-7-fluoro-2-hydroxy-8-methyl-2,3,11,11a-tetrahydro-1H,5H-benzo [f]pyrrolo[2,1-c][1,4]oxazepin-5-one

The same procedures as those in Step 2 of Reference Example 61 were carried out except that the compound obtained in Step 1 (42.3 mg, 0.12 mmol) was used as a raw material, to thereby produce the title compound (25.2 mg).

$^1$H-NMR ($CDCl_3$) δ: 6.71 (1H, d, J = 5.9 Hz), 4.92-4.86 (1H, m), 4.81-4.59 (6H, m), 4.03 (1H, dd, J = 10.1, 5.0 Hz), 3.93-3.87 (2H, m), 3.82 (1H, dd, J = 13.3, 4.6 Hz), 2.28-2.21 (4H, m), 1.84-1.80 (1H, m).
LCMS: 346.2 $[M+H]^+$ (Rt= 1.977 min).

<Reference Example 67: (2S,11aR)-6-((R)-sec-Butoxy)-7-fluoro-2-hydroxy-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (100 mg, 0.28 mmol) and (S)-(+)-2-butanol (33.0 μL, 0.36 mmol) were used as raw materials, to thereby produce the title compound (103 mg).
LCMS: 324.2 $[M+H]^+$ (Rt= 2.036 min).

<Reference Example 68: (2S,11aR)-6-((S)-sec-Butoxy)-7-fluoro-2-hydroxy-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (100 mg, 0.28 mmol) and (R)-(-)-2-butanol (67.0 μL, 0.73 mmol) were used as raw materials, to thereby produce the title compound (73.5 mg).

$^1$H-NMR ($CDCl_3$) δ: 6.61 (1H, d, J = 5.9 Hz), 4.60 (1H, dd, J = 8.2, 4.1 Hz), 4.27-4.21 (1H, m), 4.01-3.90 (5H, m), 2.25 (3H, d, J = 1.8 Hz), 2.21-2.13 (1H, m), 1.88-1.76 (3H, m), 1.69-1.62 (1H, m), 1.20 (3H, d, J = 6.4 Hz), 0.97 (3H, t, J = 7.3 Hz).
LCMS: 324.2 $[M+H]^+$ (Rt= 2.037 min).

<Reference Example 69: (2S,11aR)-7-Fluoro-6-(((R)-1-fluoropropan-2-yl)oxy)-2-hydroxy-8-methyl-2,3,11,11a-tetrahydro-1H,5H-benzo[f]pyrrolo[2,1-c][1,4]oxazepin-5-one>

[Step 1] Methyl (R)-2-((1-((tert-butyldiphenylsilyl)oxy)propan-2-yl)oxy)-3-fluoro-6-hydroxy-4-methyl benzoate

The compound obtained in Reference Example 6 (1.62 g, 3.39 mmol) was dissolved in acetonitrile (11 mL), and to the resulting solution, Selectfluor(registration trademark) (1.80 g, 5.09 mmol) was added at 0°C, and the mixture was agitated overnight at room temperature. The reaction mixture was combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (832 mg).
LCMS: 497.3 $[M+H]^+$ (Rt= 2.928 min).

[Step 2] tert-Butyl (2R,4S)-4-(benzyloxy)-2-((3-(((R)-1-((tert-butyldiphenylsilyl)oxy)propan-2-yl)oxy)-4-fluoro-2-(methoxycarbonyl)-5-methylphenoxy)methyl)pyrrolidin-1-carboxylate

The compound obtained in Step 1 (832 mg, 1.68 mmol), the compound obtained in Reference Example 25 (618 mg, 2.01 mmol), and triphenylphosphine (571 mg, 2.18 mmol) were dissolved in THF (4.2 mL), and to the resulting solution, DIAD (424 μL, 2.18 mmol) was added dropwise. After the drop was finished, the mixture was agitated at 45°C for 4 hours, combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (1.21 g).
LCMS: 686.4 $[M+H-Boc]^+$ (Rt= 3.296 min).

[Step 3] tert-Butyl (2R,4S)-4-(benzyloxy)-2-((4-fluoro-3-(((R)-1-hydroxy propan-2-yl)oxy)-2-(methoxycarbonyl)-5-methylphenoxy)methyl)pyrrolidin-1-carboxylate

The compound obtained in Step 2 (1.21 g, 1.54 mmol) was dissolved in THF (5 mL), and to the resuling solution, 1M tetrabutyl ammonium fluoride/THF solution (2.31 mL, 2.31 mmol) was added dropwise at 0°C. After the drop was finished, the mixture was agitated for 2.5 hours at room temperature, combined with saturated ammonia chloride aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (818 mg).
$^1$H-NMR ($CDCl_3$) δ: 7.35-7.26 (5H, m), 6.46-6.38 (1H, m), 4.51-4.46 (3H, m), 4.25-3.55 (11H, m), 3.39-3.41 (1H, m), 2.24-2.18 (5H, m), 1.47-1.45 (9H, m), 1.32 (3H, d, J = 6.4 Hz).

[Step 4] tert-Butyl (2R,4S)-4-(benzyloxy)-2-((4-fluoro-3-(((R)-1-fluoropropan-2-yl)oxy)-2-(methoxycarbonyl)-5-methylphenoxy)methyl)pyrrolidin-1-carboxylate

The compound obtained in Step 3 (400 mg, 0.73 mmol) was dissolved in dichloromethane (7.3 mL), combined with bis(2-methoxyethyl)aminosulfur trifluoride (257 μL, 1.46 mmol), and agitated overnight at room temperature. The reaction mixture was combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (150 mg).
$^1$H-NMR ($CDCl_3$) δ: 7.32-7.29 (5H, m), 6.49-6.42 (1H, m), 4.59-4.46 (4H, m), 4.40 (1H, d, J = 4.1 Hz), 4.25-3.96 (4H, m), 3.85 (3H, s), 3.84-3.34 (2H, m), 2.23-2.18 (5H, m), 1.47-1.45 (9H, m), 1.32 (3H, d, J = 6.4 Hz).

[Step 5] (2S,11aR)-7-Fluoro-6-(((R)-1-fluoropropan-2-yl)oxy)-2-hydroxy-8-methyl-2,3,11,11a-tetrahydro-1H,5H-benzo[f]pyrrolo[2,1-c][1,4]oxazepin-5-one

The same procedures as those in Step 3 to Step 6 of Reference Example 27 were carried out except that the compound obtained in Step 4 (146 mg, 0.35 mmol) was used as a raw material, to thereby produce the title compound (115 mg).
LCMS: 328.2 $[M+H]^+$ (Rt= 1.937 min).

<Reference Example 70: (2S,11aR)-6-(((R)-1,1-Difluoropropan-2-yl)oxy)-7-fluoro-2-hydroxy-8-methyl-2,3,11,11a-tetrahydro-1H,5H-benzo[f]pyrrolo[2,1-c][1,4]oxazepin-5-one>

[Step 1] tert-Butyl (2R,4S)-4-(benzyloxy)-2-((4-fluoro-2-(methoxycarbonyl)-5-methyl-3-(((R)-1-oxopropan-2-yl)oxy)phenoxy)methyl)pyrrolidin-1-carboxylate

The compound obtained in Step 3 of Reference Example 69 (400 mg, 0.73 mmol) was dissolved in dichloromethane (3.7 mL), combined with Dess-Martin Periodinane (372 mg, 0.88 mmol), and agitated for 2 hours at room temperature. The reaction mixture was then combined with saturated sodium bicarbonate aqueous solution and sodium thiosulfate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (257 mg).
$^1$H-NMR ($CDCl_3$) δ: 9.85 (1H, s), 7.35-7.26 (5H, m), 6.54-6.45 (1H, m), 4.57-4.46 (3H, m), 4.25-3.79 (7H, m), 3.56-3.37 (2H, m), 2.25-2.05 (5H, m), 1.47-1.45 (9H, m), 1.32 (3H, d, J = 6.4 Hz).

[Step 2] tert-Butyl (2R,4S)-4-(benzyloxy)-2-((3-(((R)-1,1-difluoropropan-2-yl)oxy)-4-fluoro-2-(methoxycarbonyl)-5-methylphenoxy)methyl)pyrrolidin-1-carboxylate

The compound obtained in Step 1 (257 mg, 0.47 mmol) was dissolved in dichloromethane (4.70 mL), and to the resulting solution, bis(2-methoxyethyl)aminosulfur trifluoride (248 μL, 1.41 mmol) was added at 0°C, agitated for 1 hour with its temperature maintained at 0°C, brought to room temperature, and agitated for 2 hours. The reaction mixture was combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (218 mg).
$^1$H-NMR ($CDCl_3$) δ: 7.35-7.30 (5H, m), 6.52-6.44 (1H, m), 5.88 (1H, td, J = 55.6, 2.7 Hz), 4.56-4.39 (3H, m), 4.27-4.18 (3H,

m), 4.02-3.99 (1H, m), 3.83-3.53 (4H, m), 3.42-3.36 (1H, m), 2.27-2.25 (3H, m), 2.21-2.15 (2H, m), 1.45 (9H, d, J = 8.7 Hz), 1.37 (3H, d, J = 6.4 Hz).

[Step 3] (2S,11aR)-6-(((R)-1,1-Difluoropropan-2-yl)oxy)-7-fluoro-2-hydroxy-8-methyl-2,3,11,11a-tetrahydro-1H,5H-benzo[f]pyrrolo[2,1-c][1,4]oxazepin-5-one

The same procedures as those in Step 3 to Step 6 of Reference Example 27 were carried out except that the compound obtained in Step 2 (217 mg, 0.38 mmol) was used as a raw material, to thereby produce the title compound (116 mg).

$^1$H-NMR ($CDCl_3$) δ: 6.67 (1H, d, J = 5.9 Hz), 5.84 (1H, td, J = 55.9, 4.9 Hz), 4.60-4.57 (1H, m), 4.39-4.32 (1H, m), 4.16 (1H, dd, J = 9.1, 3.7 Hz), 4.06-3.94 (2H, m), 3.88 (1H, dq, J = 12.6, 1.6 Hz), 3.79 (1H, dd, J = 12.6, 4.8 Hz), 2.27 (3H, d, J = 1.8 Hz), 2.20-2.15 (1H, m), 1.89-1.82 (2H, m), 1.48 (3H, d, J = 6.4 Hz).
LCMS: 346.1 $[M+H]^+$ (Rt= 2.022 min).

<Reference Example 71: (2S,11aR)-7-Fluoro-2-hydroxy-8-methyl-6-(((R)-1,1,1-trifluoropropan-2-yl)oxy)-2,3,11,11a-tetrahydro-1H,5H-benzo[f]pyrrolo[2,1-c][1,4]oxazepin-5-one>

[Step 1] (R)-6-Fluoro-2,2,7-trimethyl-5-((1,1,1-trifluoropropan-2-yl)oxy)-4H-benzo[d][1,3]dioxin-4-one

The compound obtained in Step 1 of Reference Example 8 (400 mg, 1.31 mmol) was dissolved in acetonitrile (6.57 mL), and to the resulting solution, Selectfluor(registration trademark) (931 mg, 2.63 mmol) was added at 0°C, and agitated for 48 hours at room temperature. The reaction mixture was filtered, and the filtrate was concentrated under reduced pressure. The residue was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (258 mg).

$^1$H-NMR ($CDCl_3$) δ: 6.60 (1H, d, J = 5.5 Hz), 4.72-4.62 (1H, m), 2.32-2.32 (3H, m), 1.69 (6H, d, J = 7.8 Hz), 1.62 (3H, d, J = 6.4 Hz).
LCMS: 323.1 $[M+H]^+$ (Rt= 2.387 min).

[Step 2] Methyl (R)-3-fluoro-6-hydroxy-4-methyl-2-((1,1,1-trifluoropropan-2-yl)oxy)benzoate

The same procedures as those in Step 2 of Reference Example 7 were carried out except that the compound obtained in Step 1 (258 mg, 0.80 mmol) was used as a raw material, to thereby produce the title compound (177 mg).

$^1$H-NMR ($CDCl_3$) δ: 11.01 (1H, s), 6.60 (1H, d, J = 5.9 Hz), 4.62-4.52 (1H, m), 3.95 (3H, s), 2.28 (3H, d, J = 2.3 Hz), 1.54 (3H, d, J = 6.4 Hz).
LCMS: 297.0 $[M+H]^+$ (Rt= 2.352 min)

[Step 3] (2S,11aR)-7-Fluoro-2-hydroxy-8-methyl-6-(((R)-1,1,1-trifluoropropan-2-yl)oxy)-2,3,11,11a-tetrahydro-1H,5H-benzo[f]pyrrolo[2,1-c][1,4]oxazepin-5-one

The same procedures as those in Step 2 to Step 6 of Reference Example 27 were carried out except that the compound obtained in Step 2 (2.25 g, 7.59 mmol) and the compound obtained in Reference Example 25 (2.80 mg, 9.11 mmol) were used as raw materials, to thereby produce the title compound (1.57 g).
LCMS: 364.1 $[M+H]^+$ (Rt= 2.050 min).

<Reference Example 72: (2S,11aR)-6-(Cyclopentyloxy)-7-fluoro-2-hydroxy-8-methyl-2,3,11,11a-tetrahydro-1H,5H-benzo[f]pyrrolo[2,1-c][1,4]oxazepin-5-one>

The same procedures as those in Step 5 to Step 6 of Reference Example 49 were carried out except that the compound obtained in Step 4 of Reference Example 49 (50.0 mg, 0.14 mmol) and bromo cyclopentane (30.0 μL, 0.28 mmol) were used as raw materials, to thereby produce the title compound (41.4 mg).
LCMS: 336.2 $[M+H]^+$ (Rt= 2.075 min)

<Reference Example 73: (2S,11aR)-7-Fluoro-2-hydroxy-8-methyl-6-(((S)-tetrahydrofuran-3-yl)oxy)-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (70.0 mg, 0.20 mmol) and (R)-3-hydroxy tetrahydrofuran (23.3 μL, 0.29 mmol) were used as raw materials, to thereby produce the title compound (33.8 mg).

$^1$H-NMR ($CDCl_3$) δ: 6.65 (1H, d, J = 5.9 Hz), 5.04-5.01 (1H, m), 4.61-4.59 (1H, m), 4.20-4.16 (2H, m), 4.06-3.75 (7H, m), 2.27-2.14 (5H, m), 2.03-1.98 (2H, m), 1.87-1.83 (1H, m).
LCMS: 338.2 $[M+H]^+$ (Rt= 1.853 min).

<Reference Example 74: (2S,11aR)-7-Fluoro-2-hydroxy-8-methyl-6-(((R)-tetrahydrofuran-3-yl)oxy)-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (100 mg, 0.28 mmol) and (S)-3-hydroxy tetrahydrofuran (33.3 μL, 0.42 mmol) were used as raw materials, to thereby produce the title compound (59.6 mg).
LCMS: 338.2 $[M+H]^+$ (Rt= 1.871 min)

<Reference Example 75:(2R,11S,11aR)-7-Fluoro-2-hydroxy-6-isopropoxy-8,11-dimethyl-2,3,11,11a-tetrahydro-1H,5H-benzo[f]pyrrolo[2,1-c][1,4]oxazepin-5-one>

[Step 1] tert-Butyl (2R,4R)-4-((tert-butyldiphenylsilyl)oxy)-2-formylpyrrolidin-1-carboxylate

The same procedures as those in Step 1 of Reference Example 35 were carried out except that the compound obtained in Reference Example 20 (4.56 g, 10.0 mmol) was used as a raw material, to thereby produce the title compound (4.34 g).
LCMS: 454.7 $[M+H]^+$ (Rt= 2.808 min).

[Step 2] tert-Butyl (2R,4R)-4-((tert-butyldiphenylsilyl)oxy)-2-((R)-1-hydroxyethyl)pyrrolidin-1-carboxylate

The compound obtained in Step 1 (4.34 g, 9.57 mmol) was dissolved in THF (32 mL), and to the resulting solution, 3M methyl magnesium bromide/diethyl ether solution (3.83 mL, 11.5 mmol) was added dropwise at -78°C. After the drop was finished, the reaction mixture was brought to room temperature, and agitated overnight. The reaction mixture was combined with saturated ammonium chloride aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (1.49 g).

$^1$H-NMR ($CDCl_3$) δ: 7.66-7.62 (4H, m), 7.47-7.38 (6H, m), 5.60-5.25 (1H, m), 4.49-4.05 (2H, m), 3.73-3.18 (3H, m), 1.99 (1H, br s), 1.71-1.67 (1H, m), 1.43 (9H, s), 1.16-1.15 (3H, m), 1.07-1.06 (9H, m).
LCMS: 414.2 $[M+H-tBu]^+$ (Rt= 2.848 min)

[Step 3] (2R,11S,11aR)-7-Fluoro-2-hydroxy-6-isopropoxy-8,11-dimethyl-2,3,11,11a-tetrahydro-1H,5H-benzo[f]pyrrolo[2,1-c][1,4]oxazepin-5-one

The same procedures as those in Step 6 to Step 9 of Reference Example 33 were carried out except that the compound obtained in Step 2 (564 mg, 1.20 mmol) was used as a raw material, to thereby produce the title compound (155 mg).
LCMS: 324.2 $[M+H]^+$ (Rt= 2.033 min)

<Reference Example 76:(2R,11R,11aR)-7-Fluoro-2-hydroxy-6-isopropoxy-8,11-dimethyl-2,3,11,11a-tetrahydro-1H,5H-benzo[f]pyrrolo[2,1-c][1,4]oxazepin-5-one>

[Step 1] tert-Butyl (2R,4R)-4-((tert-butyldiphenylsilyl)oxy)-2-((S)-1-hydroxyethyl)pyrrolidin-1-carboxylate

The same procedures as those in Step 2 of Reference Example 75 were carried out except that the compound obtained in Step 1 of Reference Example 75 (4.34 g, 9.57 mmol) was used as a raw material, to thereby produce the title

compound (1.28 g).

$^1$H-NMR ($CDCl_3$) δ: 7.66 (4H, t, J = 6.4 Hz), 7.48-7.38 (6H, m), 4.33-3.12 (5H, m), 2.06 (1H, br s), 1.79 (1H, br s), 1.42 (9H, s), 1.14 (3H, d, J = 6.4 Hz), 1.07 (9H, s).
LCMS: 414.2 $[M+H-tBu]^+$ (Rt= 2.848 min).

[Step 2] (2R,11R,11aR)-7-Fluoro-2-hydroxy-6-isopropoxy-8,11-dimethyl-2,3,11,11a-tetrahydro-1H,5H-benzo[f]pyrrolo[2,1-c][1,4]oxazepin-5-one

The same procedures as those in Step 3 of Reference Example 75 were carried out except that the compound obtained in Step 1 (705 mg, 1.50 mmol) was used as a raw material, to thereby produce the title compound (13.0 mg). LCMS: 324.2 $[M+H]^+$ (Rt= 2.022 min).

<Reference Example 77: (2S,11aR)-7-Fluoro-2-hydroxy-8-methyl-6-(pyrrolidin-1-yl)-2,3,11,11a-tetrahydro-1H,5H-benzo[f]pyrrolo[2,1-c][1,4]oxazepin-5-one>

[Step 1] (2S,11aR)-2-(Benzyloxy)-7-fluoro-8-methyl-5-oxo-2,3,11,11a-tetrahydro-1H,5H-benzo[f]pyrrolo[2,1-c][1,4]oxazepin-6-yl trifluoromethane sulfonate

The compound obtained in Step 4 of Reference Example 49 (110 mg, 0.31 mmol) and triethylamine (64.4 μL, 0.47 mmol) were dissolved in dichloromethane (1.5 mL), and to the resulting solution, trifluoromethane sulfonic anhydride (65.4 μL, 0.40 mmol) was added dropwise at 0°C. After the drop was finished, the mixture was agitated for 1.5 hours at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (141 mg).

$^1$H-NMR ($CDCl_3$) δ: 7.36-7.27 (5H, m), 6.93 (1H, d, J = 5.9 Hz), 4.60 (1H, d, J = 11.4 Hz), 4.45 (1H, d, J = 11.9 Hz), 4.30-4.22 (2H, m), 4.18-4.02 (3H, m), 3.74 (1H, dd, J = 12.8, 4.6 Hz), 2.33-2.28 (4H, m), 1.86 (1H, dt, J = 13.3, 5.7 Hz).
LCMS: 490.1 $[M+H]^+$ (Rt= 2.450 min).

[Step 2] (2S,11aR)-2-(Benzyloxy)-7-fluoro-8-methyl-6-(pyrrolidin-1-yl)-2,3,11,11a-tetrahydro-1H,5H-benzo[f]pyrrolo[2,1-c][1,4]oxazepin-5-one

The same procedures as those in Step 4 of Reference Example 48 were carried out except that the compound obtained in Step 1 (141 mg, 0.29 mmol) and pyrrolidine (120 μL, 1.45 mmol) were used as raw materials, to thereby produce the title compound (62.5 mg).

$^1$H-NMR ($CDCl_3$) δ: 7.36-7.27 (5H, m), 6.23 (1H, d, J = 5.5 Hz), 4.56 (1H, d, J = 11.4 Hz), 4.47 (1H, d, J = 11.4 Hz), 4.33-4.29 (1H, m), 4.25-4.18 (1H, m), 4.08-4.02 (2H, m), 3.88-3.78 (2H, m), 3.70-3.63 (2H, m), 3.14-3.10 (2H, m), 2.34-2.28 (1H, m), 2.17 (3H, d, J = 2.7 Hz), 1.90-1.69 (5H, m).
LCMS: 411.2 $[M+H]^+$ (Rt= 1.945 min).

[Step 3] (2S,11aR)-7-Fluoro-2-hydroxy-8-methyl-6-(pyrrolidin-1-yl)-2,3,11,11a-tetrahydro-1H,5H-benzo[f]pyrrolo[2,1-c][1,4]oxazepin-5-one

The same procedures as those in Step 6 of Reference Example 27 were carried out except that the compound obtained in Step 2 (62.5 mg, 0.15 mmol) was used as a raw material, to thereby produce the title compound (42.1 mg). LCMS: 321.2 $[M+H]^+$ (Rt= 1.632 min).

<Reference Example 78: (2S,11aR)-7-Fluoro-2-hydroxy-6-isopropyl-8-methyl-2,3,11,11a-tetrahydro-1H,5H-benzo[f]pyrrolo[2,1-c][1,4]oxazepin-5-one>

[Step 1] (2S,11aR)-2-(Benzyloxy)-7-fluoro-8-methyl-6-(propen-2-yl)-2,3,11,11a-tetrahydro-1H,5H-benzo[f]pyrrolo[2,1-c][1,4]oxazepin-5-one

The compound obtained in Step 1 of Reference Example 77 (130 mg, 0.27 mmol), isopropenylboronic acid pinacol ester (99.8 μL, 0.53 mmol), and 2M sodium carbonate aqueous solution (398 μL, 0.80 mmol) were dissolved in 1,4-dioxane (1.06 mL), combined with XPhos Pd G3 (11.2 mg, 0.01 mmol), and agitated at 90°C for 1 hour. The reaction mixture was cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (90.0 mg).

$^1$H-NMR ($CDCl_3$) δ: 7.32-7.30 (5H, m), 6.78 (1H, d, J = 6.4 Hz), 5.17 (1H, s), 4.75 (1H, s), 4.55 (1H, d, J = 11.4 Hz), 4.45 (1H, d, J = 11.4 Hz), 4.29-4.25 (1H, m), 4.18-3.93 (4H, m), 3.71 (1H, dd, J = 12.6, 4.3 Hz), 2.31-2.25 (4H, m), 2.14 (3H, s), 1.87-1.84 (1H, m).
LCMS: 382.2 $[M+H]^+$ (Rt= 2.414 min).

[Step 2] (2S,11aR)-7-Fluoro-2-hydroxy-6-isopropyl-8-methyl-2,3,11,11a-tetrahydro-1H,5H-benzo[f]pyrrolo[2,1-c][1,4]oxazepin-5-one

The compound obtained in Step 1 (90.0 mg, 0.24 mmol) was dissolved in ethyl acetate (1.18 mL), combined with 20% hydroxide palladium-activated carbon (4.50 mg), and agitated for 7 days under hydrogen atmosphere at normal pressure at room temperature. The reaction mixture was filtered with cerite, and the filtrate was concentrated under reduced pressure. The residue was purified with silica gel column chromatography (heptane/ethyl acetate) to thereby produce the title compound (38.3 mg).

$^1$H-NMR ($CDCl_3$) δ: 6.73 (1H, d, J = 6.4 Hz), 4.66-4.64 (1H, m), 4.11-3.99 (2H, m), 3.94-3.88 (2H, m), 3.77 (1H, dd, J = 12.3, 4.1 Hz), 3.53-3.42 (1H, m), 2.24 (3H, d, J = 2.7 Hz), 2.21-2.15 (1H, m), 1.93-1.90 (1H, m), 1.72 (1H, d, J = 4.6 Hz), 1.39 (3H, d, J = 6.9 Hz), 1.30-1.28 (3H, m).
LCMS: 294.2 $[M+H]^+$ (Rt= 2.069 min).

<Reference Example 79: (2S,11aR)-6-Cyclopentyl-7-fluoro-2-hydroxy-8-methyl-2,3,11,11a-tetrahydro-1H,5H-benzo[f]pyrrolo[2,1-c][1,4]oxazepin-5-one>

The same procedures as those in Reference Example 78 were carried out except that the compound obtained in Step 1 of Reference Example 77 (130 mg, 0.27 mmol) and 1-cyclopentenyl boronic acid (55.1 μL, 0.53 mmol) were used as raw materials, to thereby produce the title compound (72.0 mg).
LCMS: 320.2 $[M+H]^+$ (Rt= 2.165 min).

<Reference Example 80: tert-Butyl (2R,4S)-4-((tert-butyldiphenylsilyl)oxy)-2-ethynylpyrrolidin-1-carboxylate>

A mixture of potassium carbonate (305 mg, 2.20 mmol), dimethyl (1-diazo-2-oxopropyl)phosphonate (199 μL, 1.32 mmol), and methanol (3.68 mL) was agitated for 10 minutes at room temperature. The mixture was combined with the compound obtained in Step 1 of Reference Example 35 (500 mg, 1.10 mmol), and agitated for 3 hours at room temperature. The reaction mixture was then combined with saturated saline solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (379 mg).

$^1$H-NMR ($CDCl_3$) δ: 7.61 (4H, t, J = 5.9 Hz), 7.45-7.35 (6H, m), 4.62-4.50 (1H, m), 4.44-4.39 (1H, m), 3.42-3.34 (2H, m), 2.23-2.17 (2H, m), 1.97 (1H, br s), 1.25 (9H, s), 1.02 (9H, s).
LCMS: 350.2 $[M+H-Boc]^+$ (Rt= 3.105 min).

<Reference Example 81: (2S,11aS)-7-Fluoro-2-hydroxy-6-isopropoxy-8-methyl-1,2,3,10,11,11a-hexahydro-5H-benzo[e]pyrrolo[1,2-a]azepin-5-one>

[Step 1] Methyl 3-fluoro-2-isopropoxy-4-methyl-6-(((trifluoromethyl)sulfonyl)oxy)benzoate

The compound obtained in Step 1 of Reference Example 55 (580 mg, 2.39 mmol) and N,N-diisopropylethylamine (495 μL, 2.87 mmol) were dissolved in dichloromethane (3.19 mL), and to the resulting solution, trifluoromethane sulfonic anhydride (431 μL, 2.63 mmol) was added at 0°C, and the mixture was agitated for 1 hour with its temperature maintained at 0°C. The reaction mixture was combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to

thereby produce the title compound (486 mg).
LCMS: 375.1 $[M+H]^+$ (Rt= 2.583 min).

[Step 2] tert-Butyl (2R,4S)-4-((tert-butyldiphenylsilyl)oxy)-2-((4-fluoro-3-isopropoxy-2-(methoxycarbonyl)-5-methylphenyl)ethynyl)pyrrolidin-1-carboxylate

The compound obtained in Step 1 (100 mg, 0.27 mmol), the compound obtained in Reference Example 80 (126 mg, 0.28 mmol), and diethylamine (84.0 μL, 0.80 mmol) were dissolved in DMF (1.07 mL), combined with copper iodide (10.2 mg, 0.05 mmol) and dichlorobis(triphenylphosphine) palladium (II) (18.8 mg, 0.03 mmol), and agitated at 80°C overnight under nitrogen atmosphere. The reaction mixture was cooled to room temperature, combined with water, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (113 mg).
LCMS: 574.2 $[M+H-Boc]^+$ (Rt= 3.742 min).

[Step 3] tert-Butyl (2S,4S)-4-((tert-butyldiphenylsilyl)oxy)-2-(4-fluoro-3-isopropoxy-2-(methoxycarbonyl)-5-methylphenethyl)pyrrolidin-1-carboxylate

The compound obtained in Step 2 (113 mg, 0.17 mmol) was dissolved in methanol (3.35 mL), combined with 10% palladium-activated carbon (17.8 mg), and agitated for 5 hours under hydrogen atmosphere at normal pressure at room temperature. The reaction mixture was filtered with cerite, and the filtrate was concentrated under reduced pressure to thereby produce the title compound (112 mg).
LCMS: 578.3 $[M+H-Boc]^+$ (Rt= 3.924 min).

[Step 4] (2S,11aS)-7-fluoro-2-hydroxy-6-isopropoxy-8-methyl-1,2,3,10,11,11a-hexahydro-5H-benzo[e]pyrrolo[1,2-a]azepin-5-one

The same procedures as those in Step 7 to Step 9 of Reference Example 32 were carried out except that the compound obtained in Step 3 (112 mg, 0.17 mmol) was used as a raw material, to thereby produce the title compound (29.0 mg).

$^1$H-NMR ($CDCl_3$) δ: 6.66 (1H, d, J = 6.9 Hz), 4.62-4.55 (1H, m), 4.49-4.40 (1H, m), 3.83 (1H, dd, J = 12.6, 5.3 Hz), 3.74 (1H, dd, J = 12.6, 4.8 Hz), 3.71-3.63 (1H, m), 2.79-2.69 (1H, m), 2.56 (1H, dd, J = 13.7, 6.4 Hz), 2.25 (3H, d, J = 2.3 Hz), 2.19-2.12 (1H, m), 2.04-1.94 (2H, m), 1.90-1.86 (1H, m), 1.84-1.75 (1H, m), 1.34 (3H, d, J = 5.9 Hz), 1.26 (3H, d, J = 6.4 Hz).
LCMS:308.2 $[M+H]^+$ (Rt= 2.024 min).

<Reference Example 82: (2S,11aR)-2-Amino-7-fluoro-6-isopropoxy-8-methyl-2,3,11,11a-tetrahydro-1H,5H-benzo[f]pyrrolo[2,1-c][1,4]oxazepin-5-one>

[Step 1] (2R,11aR)-7-Fluoro-2-hydroxy-6-isopropoxy-8-methyl-2,3,11,11a-tetrahydro-1H,5H-benzo[f]pyrrolo[2,1-c][1,4]oxazepin-5-one

The same procedures as those in Step 6 to Step 9 of Reference Example 33 were carried out except that the compound obtained in Reference Example 20 (1.80 g, 3.96 mmol) was used as a raw material, to thereby produce the title compound (894 mg). LCMS:310.1 $[M+H]^+$ (Rt= 1.983 min).

[Step 2] (2S,11aR)-2-azide -7-fluoro-6-isopropoxy-8-methyl-2,3,11,11a-tetrahydro-1H,5H-benzo[f]pyrrolo[2,1-c][1,4]oxazepin-5-one

The compound obtained in Step 1 (309 mg, 1.00 mmol) and triethylamine (181 μL, 1.30 mmol) were dissolved in dichloromethane (5.00 mL), combined with methane sulfonyl chloride (92.9 μL, 1.20 mmol), and agitated for 1 hour at room temperature. The reaction mixture was combined with water, and extracted with dichloromethane. The organic phase was washed with 0.5M hydrochloric acid aqueous solution, then with saline solution, dried with anhydrous magnesium sulfate, and concentrated under reduced pressure. The residue was dissolved in DMF (5.00 mL), combined with sodium azide (130 mg, 2.00 mmol), and agitated at 80°C overnight. The reaction mixture was then cooled to room temperature, combined with saturated sodium bicarbonate aqueous solution, and extracted with ethyl acetate. The organic phase was washed twice with saline solution, dried with anhydrous magnesium sulfate, and concentrated under reduced pressure.

The residue was purified with silica gel column chromatography (heptane/ethyl acetate) to thereby produce the title compound (331 mg).
LCMS: 335.2 $[M+H]^+$ (Rt= 2.259 min).

[Step 3] (2S,11aR)-2-Amino-7-fluoro-6-isopropoxy-8-methyl-2,3,11,11a-tetrahydro-1H,5H-benzo[f]pyrrolo[2,1-c][1,4] oxazepin-5-one

The compound obtained in Step 2 (331 mg, 0.99 mmol) was dissolved in ethanol (4.95 mL), combined with 10% palladium-activated carbon (33.1 mg), and agitated for 4 days under hydrogen atmosphere at normal pressure at room temperature. The reaction mixture was filtered with cerite, and the filtrate was concentrated under reduced pressure. The residue was purified with amino silica gel column chromatography (heptane/ethyl acetate) to thereby produce the title compound (227 mg).
LCMS: 309.2 $[M+H]^+$ (Rt= 1.693 min).

<Reference Example 83: tert-Butyl (2R,4S)-2-(hydroxymethyl)-4-((2-oxo-1,2,3,4-tetrahydroquinolin-7-yl)oxy)pyrrolidin-1-carboxylate>

[Step 1] tert-Butyl (2R,4R)-2-((benzoyloxy)methyl)-4-hydroxy pyrrolidin-1-carboxylate

tert-butyl (2R,4R)-4-hydroxy-2-(hydroxymethyl)pyrrolidin-1-carboxylate (897 mg, 4.13 mmol) and pyridine (1.00 mL, 12.4 mmol) were dissolved in dichloromethane (20.7 mL), and to the resulting solution, benzoyl chloride (524 μL, 4.54 mmol) was added dropwise at -78°C. After the drop was finished, the reaction mixture was brought to room temperature, agitated for 19 hours, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (737 mg).

$^1$H-NMR ($CDCl_3$) δ: 8.06-8.04 (2H, m), 7.57 (1H, t, J = 7.3 Hz), 7.45 (2H, t, J = 7.5 Hz), 4.63-4.49 (3H, m), 4.31-4.20 (1H, m), 3.71-3.60 (1H, m), 3.45-3.42 (1H, m), 2.23 (1H, br s), 2.09-1.99 (1H, m), 1.46 (9H, s).
LCMS: 222.1 $[M+H-Boc]^+$ (Rt= 2.202 min).

[Step 2] tert-Butyl (2R,4S)-2-((benzoyloxy)methyl)-4-((2-oxo-1,2,3,4-tetrahydroquinolin-7-yl)oxy)pyrrolidin-1-carboxylate

The compound obtained in Step 1 (643 mg, 2.00 mmol), 7-hydroxy-3,4-dihydroquinolin-2(1H)-one (424 mg, 2.60 mmol), and triphenylphosphine (630 mg, 2.40 mmol) were dissolved in THF (10 mL), and to the resulting solution, DIAD (467 μL, 2.40 mmol) was added dropwise at 0°C. After the drop was finished, the reaction mixture was the mixture was brought to room temperature, agitated overnight, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (767 mg). $^1$H-NMR ($CDCl_3$) δ: 8.03 (2H, d, J = 7.8 Hz), 7.61-7.55 (2H, m), 7.46 (2H, t, J = 7.8 Hz), 7.04 (1H, d, J = 8.7 Hz), 6.46 (1H, dd, J = 8.2, 2.3 Hz), 6.25 (1H, d, J = 1.8 Hz), 4.94-4.87 (1H, m), 4.56-4.32 (3H, m), 3.92-3.70 (1H, m), 3.62-3.59 (1H, m), 2.90 (2H, t, J = 7.5 Hz), 2.61 (2H, t, J = 7.5 Hz), 2.39-2.24 (2H, br m), 1.49-1.46 (9H, m).
LCMS: 367.1 $[M+H-Boc]^+$ (Rt= 2.337 min).

[Step 3] tert-Butyl (2R,4S)-2-(hydroxymethyl)-4-((2-oxo-1,2,3,4-tetrahydroquinolin-7-yl)oxy)pyrrolidin-1-carboxylate

The compound obtained in Step 2 (1.17 g, 2.51 mmol) was dissolved in methanol (8.38 mL), combined with potassium carbonate (1.04 g, 7.54 mmol), and agitated overnight at room temperature. The reaction mixture was combined with water, and extracted with dichloromethane. The organic phase was dried with anhydrous magnesium sulfate, and concentrated under reduced pressure. The residue was purified with silica gel column chromatography (heptane/ethyl acetate/methanol)to thereby produce the title compound (846 mg).

$^1$H-NMR ($CDCl_3$) δ: 7.99 (1H, s), 7.06 (1H, d, J = 8.2 Hz), 6.48 (1H, dd, J = 8.2, 2.7 Hz), 6.30 (1H, d, J = 2.3 Hz), 4.83-4.76 (2H, m), 4.17 (1H, br s), 3.87-3.72 (2H, m), 3.63-3.55 (2H, m), 2.91 (2H, t, J = 7.5 Hz), 2.63 (2H, t, J = 7.3 Hz), 2.32-2.26 (1H, m), 1.81-1.75 (1H, m), 1.47 (9H, s).
LCMS: 263.1 $[M+H-tBu]^+$ (Rt= 2.015 min).

<Reference Example 84: 7-Chloro-1-((2-(trimethylsilyl)ethoxy)methyl)-1,6-naphthyridin-2(1H)-one>

[Step 1] Ethyl (E)-3-(4-amino-6-chloropyridin-3-yl)acrylate

2-Chloro-5-iodopyridin-4-amine (2.00 g, 7.86 mmol), ethyl acrylate (941 μL, 8.65 mmol) and triethylamine (2.19 mL, 15.7 mmol) were dissolved in acetonitrile (26.2 mL), combined with palladium acetate (II) (70.6 mg, 0.31 mmol) and tri(o-tolyl)phosphine (191 mg, 0.63 mmol), and agitated at 70°C for 3 hours. The reaction mixture was cooled to room temperature and filtered with cerite, and the filtrate was concentrated under reduced pressure. The residue was purified with silica gel column chromatography (heptane/ethyl acetate), then with amino silica gel column chromatography (heptane/ethyl acetate), to thereby produce the title compound (1.51 g).

$^1$H-NMR ($CDCl_3$) δ: 8.21 (1H, s), 7.62 (1H, d, J = 16.0 Hz), 6.60 (1H, s), 6.39 (1H, d, J = 16.0 Hz), 4.58 (2H, br s), 4.27 (2H, q, J = 7.2 Hz),1.34 (3H, t, J = 7.1 Hz).
LCMS: 227.1 $[M+H]^+$ (Rt= 1.716 min).

[Step 2] 7-Chloro-1,6-naphthyridin-2(1H)-one

The compound obtained in Step 1 (1.45 g, 6.40 mmol) was dissolved in acetic acid (12.8 mL), combined with tributylphosphine (1.6 mL, 6.40 mmol), and agitated at 100°C for 5 hours. The reaction mixture was cooled to room temperature, and concentrated under reduced pressure. The resuling solid was washed with ethyl acetate to thereby produce the title compound (948 mg).

$^1$H-NMR (DMSO-d6) δ: 12.11 (1H, br s), 8.68 (1H, s), 7.99 (1H, d, J = 9.6 Hz), 7.20 (1H, s), 6.59 (1H, d, J = 9.6 Hz).
LCMS: 181.0 $[M+H]^+$ (Rt= 1.660 min).

[Step 3] 7-Chloro-1-((2-(trimethylsilyl)ethoxy)methyl)-1,6-naphthyridin-2(1H)-one

The compound obtained in Step 2 (1.01 g, 5.59 mmol) was dissolved in DMF (56 mL), combined with 50% sodium hydride (349 mg, 7.27 mmol), and agitated for 1 hour at room temperature. The resulting mixture was then combined with 2-(trimethylsilyl)ethoxymethyl chloride (1.28 mL, 7.27 mmol), and agitated overnight at room temperature. The reaction mixture was combined with saturated ammonium chloride aqueous solution, and extracted with heptane/ethyl acetate (1/1). The organic phase was washed twice with saturated saline solution, dried with anhydrous magnesium sulfate, and concentrated under reduced pressure. The residue was purified with silica gel column chromatography (heptane/ethyl acetate) to thereby produce the title compound (776 mg). $^1$H-NMR ($CDCl_3$) δ: 8.55 (1H, s), 7.71 (1H, d, J = 9.6 Hz), 7.48 (1H, s), 6.69 (1H, d, J = 9.6 Hz), 5.66 (2H, s), 3.67 (2H, t, J = 8.2 Hz), 0.97-0.93 (2H, m), -0.02 (9H, s).
LCMS: 311.1 $[M+H]^+$ (Rt= 2.413 min).

<Reference Example 85: 7-Hydroxy-3,4-dihydro-1,6-naphthyridin-2(1H)-one>

[Step 1] 5-Iodo-2-methoxy pyridin-4-amine

2-Chloro-5-iodopyridin-4-amine (2.54 g, 10.0 mmol) was combined with 5M sodium methoxide/methanol solution (10.0 mL, 50.0 mmol), and agitated at 80°C overnight. The reaction mixture was cooled to room temperature, combined with saline solution, and extracted with dichloromethane. The organic phase was dried with sodium sulfate, and concentrated under reduced pressure. The residue was purified with amino silica gel column chromatography (heptane/ethyl acetate) to thereby produce the title compound (1.17 g).
LCMS: 251.0 $[M+H]^+$ (Rt= 0.826 min).

[Step 2] Ethyl (E)-3-(4-amino-6-methoxy pyridin-3-yl)acrylate

The same procedures as those in Step 1 of Reference Example 84 were carried out except that the compound obtained in Step 1 (773 mg, 3.09 mmol) and ethyl acrylate (404 μL, 3.71 mmol) were used as raw materials, to thereby produce the title compound (506 mg).
LCMS: 223.1 $[M+H]^+$ (Rt= 1.577 min).

[Step 3] Ethyl 3-(4-amino-6-methoxy pyridin-3-yl)propanoate

The compound obtained in Step 2 (506 mg, 2.28 mmol) was dissolved in methanol (11.4 mL), combined with 10%

palladium-activated carbon (50.6 mg), and agitated overnight under hydrogen atmosphere at normal pressure at room temperature. The reaction mixture was filtered with cerite, and the filtrate was concentrated under reduced pressure, to thereby produce a crude product of the title compound.
LCMS: 225.1 $[M+H]^+$ (Rt= 1.506 min).

[Step 4] 7-Methoxy-3,4-dihydro-1,6-naphthyridin-2(1H)-one

The crude product of the compound obtained in Step 3 was combined with acetic acid (7.60 mL), and agitated at 100°C overnight. The reaction mixture was then cooled to room temperature, combined with saturated sodium bicarbonate aqueous solution, and extracted with dichloromethane. The organic phase was dried with anhydrous magnesium sulfate, and concentrated under reduced pressure. The residue was purified with silica gel column chromatography (heptane/ethyl acetate) to thereby produce the title compound (240 mg).

$^1$H-NMR (DMSO-d6) δ: 10.35 (1H, s), 7.86 (1H, s), 6.18 (1H, s), 3.76 (3H, s), 2.80 (2H, t, J = 7.5 Hz), 2.49-2.45 (2H, m).
LCMS: 179.1 $[M+H]^+$ (Rt= 0.596 min).

[Step 5] 7-Hydroxy-3,4-dihydro-1,6-naphthyridin-2(1H)-one

The compound obtained in Step 4 (412 mg, 2.31 mmol) was mixed with 6M hydrochloric acid aqueous solution (2.31 mL) and 4M hydrochloric acid/1,4-dioxane solution (7.71 mL), and agitated at 100°C overnight. The reaction mixture was then cooled to room temperature, combined with dichloromethane, and extracted with water. The aqueous phase was concentrated under reduced pressure, and the residue was suspended in methanol, and filtered. The filtrate was dried with sodium sulfate and concentrated under reduced pressure, and the residue was purified with amino silica gel column chromatography to thereby produce the title compound (296 mg).

$^1$H-NMR (DMSO-d6) δ: 10.96 (1H, s), 7.77 (1H, s), 6.42 (1H, s), 2.82 (2H, t, J = 7.5 Hz), 2.52 (2H, t, J = 7.8 Hz).
LCMS: 165.1 $[M+H]^+$ (Rt= 0.723 min).

<Reference Example 86: 8-Fluoro-7-hydroxy-3,4-dihydroquinolin-2(1H)-one>

[Step 1] 1-(Benzyloxy)-4-bromo-2,3-difluorobenzene

4-Bromo-2,3-difluorophenol (2.09 g, 10.0 mmol) and potassium carbonate (2.07 g, 15.0 mmol) were dissolved in DMF (20.0 mL), and to the resulting solution, benzyl bromide (1.56 mL, 13.0 mmol) was added dropwise, and the resulting mixture was agitated at 80°C overnight. The reaction mixture was cooled to room temperature, combined with water, and extracted with heptane/ethyl acetate (2/1). The organic phase was dried with anhydrous magnesium sulfate, and concentrated under reduced pressure. The residue was purified with silica gel chromatography (heptane/ethyl acetate) to thereby produce the title compound (2.98 g).
$^1$H-NMR ($CDCl_3$) δ: 7.43-7.32 (5H, m), 7.20-7.15 (1H, m), 6.72-6.68 (1H, m), 5.14 (2H, s).

[Step 2] 3-(Benzyloxy)-6-bromo-N-(2,4-dimethoxy benzyl)-2-fluoroaniline

The compound obtained in Step 1 (2.98 g, 9.96 mmol) was dissolved in N-methyl pyrrolidone (16.6 mL), combined with 2,4-dimethoxy benzylamine (4.5 mL, 29.9 mmol), and agitated at 130°C overnight. The reaction mixture was cooled to room temperature, combined with water, and extracted with heptane/ethyl acetate (2/1). The organic phase was washed twice with saturated saline solution, dried with anhydrous magnesium sulfate, and concentrated under reduced pressure. The residue was purified with silica gel column chromatography (heptane/ethyl acetate), to thereby produce a crude product of the title compound.
LCMS: 446.1 $[M+H]^+$ (Rt= 2.583 min).

[Step 3] 3-(Benzyloxy)-6-bromo-2-fluoroaniline

The crude product of the compound obtained in Step 2 was dissolved in dichloromethane (10.0 mL), and to the resulting solution, trifluoroacetic acid (10.0 mL) was added dropwise, and the resulting mixture was agitated overnight at room temperature. The reaction mixture was combined with methanol (100ml), the precipitate was filtered, and the filtrate was concentrated under reduced pressure. The residue was combined with saturated sodium bicarbonate aqueous solution, and extracted with dichloromethane. The organic phase was dried with sodium sulfate, and concentrated under reduced pressure to thereby produce the title compound (893 mg).

LCMS: 296.0 $[M+H]^+$ (Rt= 2.389 min).

[Step 4] 3-(Benzyloxy)-6-bromo-N-(1-ethoxy cyclopropyl)-2-fluoroaniline

The compound obtained in Step 3 (296 mg, 1.00 mmol) and acetic acid (229 μL, 4.00 mmol) was dissolved in ethanol (5 mL), combined with (1-ethoxy cyclopropoxy)trimethyl silane (402 μL, 2.00 mmol), and refluxed with heating overnight under nitrogen atmosphere. The reaction mixture was then cooled to room temperature, concentrated under reduced pressure, and purified with silica gel column chromatography (heptane/ethyl acetate) to thereby produce the title compound (291 mg).
LCMS: 380.1 $[M+H]^+$ (Rt= 2.555 min).

[Step 5] 7-(Benzyloxy)-8-fluoro-3,4-dihydroquinolin-2(1H)-one

The compound obtained in Step 4 (291 mg, 0.77 mmol) and potassium carbonate (207 mg, 1.50 mmol) were dissolved in DMF (5.00 mL), combined with $Pd_2(dba)_3$ (45.8 mg, 0.05 mmol) and XPhos (119 mg, 0.25 mmol), and agitated overnight under nitrogen atmosphere at 95°C. The reaction mixture was cooled to room temperature, combined with 1M hydrochloric acid aqueous solution, and extracted with ethyl acetate. The organic phase was washed twice with saturated saline solution, dried with anhydrous magnesium sulfate, and concentrated under reduced pressure. The residue was purified with silica gel chromatography (heptane/ethyl acetate) to thereby produce the title compound (107 mg).
LCMS: 272.1 $[M+H]^+$ (Rt= 2.163 min).

[Step 6] 8-Fluoro-7-hydroxy-3,4-dihydroquinolin-2(1H)-one

The compound obtained in Step 5 (107 mg, 0.39 mmol) was dissolved in a mixture solvent of ethanol (3.90 mL) and THF (3.90 mL), combined with 10% palladium-activated carbon (21.4 mg), and agitated overnight under hydrogen atmosphere at normal pressure at room temperature. The reaction mixture was filtered with cerite, and the filtrate was concentrated under reduced pressure. The residue was purified with silica gel chromatography (heptane/ethyl acetate) to thereby produce the title compound (61.0 mg).

$^1$H-NMR (DMSO-d6) δ: 9.96 (1H, s), 9.67 (1H, s), 6.74 (1H, d, J = 7.8 Hz), 6.47 (1H, t, J = 8.2 Hz), 2.78 (2H, t, J = 7.3 Hz), 2.43-2.39 (2H, m).
LCMS: 182.1 $[M+H]^+$ (Rt= 1.665 min).

<Reference Example 87: 8-Fluoro-7-hydroxy-3,4-dihydro-1,6-naphthyridin-2(1H)-one>

[Step 1] 2,3-Difluoro-5-iodopyridin-4-amine

2,3-Difluoropyridin-4-amine (500 mg, 3.84 mmol) and N-iodosuccinimide (1.04 g, 4.61 mmol) were dissolved in acetonitrile (7.69 mL), combined with p-toluene sulfonic acid monohydrate (36.6 mg, 0.19 mmol), agitated at 80°C for 16 hours. The reaction mixture was then cooled to room temperature, combined with saturated sodium carbonate aqueous solution, and extracted with ethyl acetate. The organic phase was washed with saturated sodium sulfite aqueous solution, then with saline solution, dried with anhydrous magnesium sulfate, and concentrated under reduced pressure to thereby produce the title compound (872 mg).

$^1$H-NMR ($CDCl_3$) δ: 7.97 (1H, s), 4.87 (2H, br s).
LCMS: 256.9 $[M+H]^+$ (Rt= 2.033 min).

[Step 2] 3-fluoro-5-iodo-2-methoxy pyridin-4-amine

The compound obtained in Step 1 (383 mg, 1.50 mmol) was dissolved in methanol (3.74 mL), combined with 5M sodium methoxide/methanol solution (598 μL, 2.99 mmol), and agitated at 80°C overnight. The reaction mixture was then cooled to room temperature, combined with water, and extracted with dichloromethane. The organic phase was washed twice with saturated saline solution, dried with sodium sulfate, and concentrated under reduced pressure to thereby produce the title compound (391 mg).
LCMS: 269.0 $[M+H]^+$ (Rt= 2.058 min).

[Step 3] 8-fluoro-7-methoxy-3,4-dihydro-1,6-naphthyridin-2(1H)-one

The same procedures as those in Step 2 to Step 4 of Reference Example 85 were carried out except that the compound obtained in Step 2 (391 mg, 1.46 mmol) and ethyl acrylate (191 μL, 1.75 mmol) were used as raw materials, to thereby produce the title compound (225 mg).
LCMS: 197.1 $[M+H]^+$ (Rt= 1.756 min).

[Step 4] 8-fluoro-7-hydroxy-3,4-dihydro-1,6-naphthyridin-2(1H)-one

The compound obtained in Step 3 (98.1 mg, 0.50 mmol) was dissolved in 4M hydrochloric acid/1,4-dioxane solution (2.0 mL), and agitated at 90°C overnight. The reaction mixture was then cooled to room temperature, then combined with saturated saline solution, and filtered with dichloromethane. The residue was washed with water, then dichloromethane, and dried under reduced pressure to thereby produce the title compound (60.0 mg).

$^1$H-NMR (DMSO-d6) δ: 11.56 (1H, br s), 10.45 (1H, br s), 7.08 (1H, s), 2.71 (2H, t, J = 7.2 Hz), 2.46 (2H, t, J = 7.2 Hz).
LCMS: 183.1 $[M+H]^+$ (Rt= 0.720 min).

<Reference Example 88:7-hydroxy-1,4-dihydro-2H-benzo[d][1,3]oxazin-2-one>

[Step 1] Ethyl 4-(benzyloxy)-2-nitrobenzoate

Ethyl 4-fluoro-2-nitrobenzoate (1.03 g, 4.83 mmol) and potassium carbonate (1.00 g, 7.25 mmol) were dissolved in DMF (9.67 mL), combined with benzylalcohol (599 μL, 5.80 mmol), and agitated at 60°C overnight. The reaction mixture was then cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate), then with amino silica gel column chromatography (heptane/ethyl acetate), to thereby produce the title compound (791 mg).
LCMS: 302.1 $[M+H]^+$ (Rt= 2.415 min).

[Step 2] (4-(Benzyloxy)-2-nitrophenyl)methanol

The compound obtained in Step 1 (791 mg, 2.63 mmol) was dissolved in a mixture solvent of THF (7.5 mL) and methanol (0.88 mL), and to the resulting solution, 4M borohydride lithium/THF solution (1.31 mL, 5.25 mmol) was added dropwise at 0°C. After the drop was finished, the mixture was agitated for 4 hours at room temperature, combined with saturated ammonium chloride aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (683 mg).

$^1$H-NMR (DMSO-d6) δ: 7.69 (1H, d, J = 2.7 Hz), 7.58 (1H, d, J = 8.7 Hz), 7.44-7.36 (5H, m), 7.27-7.24 (1H, m), 5.14 (2H, s), 4.86 (2H, d, J = 6.4 Hz), 2.55 (1H, t, J = 6.6 Hz).
LCMS: 242.1 $[M+H-H_2O]^+$ (Rt= 2.220 min).

[Step 3] (2-Amino-4-(benzyloxy)phenyl)methanol

The compound obtained in Step 2 (683 mg, 2.64 mmol) and ammonium chloride (705 mg, 13.2 mmol) was dissolved in a mixture solvent of water (1.10 mL) and ethanol (4.40 mL), combined with iron (736 mg, 13.2 mmol), and agitated at 50°C for 16 hours. The reaction mixture was then cooled to room temperature, combined with ethyl acetate, and filtered with cerite. The filtrate was extracted with ethyl acetate, and the organic phase was washed twice with saturated saline solution, dried with anhydrous magnesium sulfate, and concentrated under reduced pressure. The residue was purified with silica gel column chromatography (heptane/ethyl acetate) to thereby produce the title compound (280 mg).
LCMS: 212.1 $[M+H-H_2O]^+$ (Rt= 1.808 min).

[Step 4] 7-(Benzyloxy)-1,4-dihydro-2H-benzo[d][1,3]oxazin-2-one

The compound obtained in Step 3 (280 mg, 1.22 mmol) was dissolved in THF (3.70 mL), and to the resulting solution, THF (2 mL) solution of triphosgene (435 mg, 1.47 mmol) was added dropwise at 0°C. The reaction mixture was

agitated at 0°C for 15 minutes, combined with triethylamine (595 μL, 4.27 mmol), and agitated for 30 minutes at 0°C. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (254 mg).
LCMS: 256.1 $[M+H]^+$ (Rt= 2.143 min).

[Step 5] 7-Hydroxy 1,4-dihydro-2H-benzo[d][1,3]oxazin-2-one

The compound obtained in Step 4 (254 mg, 0.993 mmol) was dissolved in ethyl acetate (5.00 mL), combined with 10% palladium-activated carbon (50.7 mg), and agitated for 4 hours under hydrogen atmosphere at normal pressure at room temperature. The reaction mixture was filtered with cerite, and the filtrate was concentrated under reduced pressure. The residue was purified with silica gel column chromatography (ethyl acetate/methanol) to thereby produce the title compound (79.7 mg).

$^1$H-NMR (DMSO-d6) δ: 9.98 (1H, s), 9.55 (1H, s), 6.95 (1H, d, J = 7.8 Hz), 6.37 (1H, dd, J = 8.2, 2.3 Hz), 6.33 (1H, d, J = 2.3 Hz), 5.12 (2H, s).
LCMS: 166.1 $[M+H]^+$ (Rt= 1.597 min).

<Reference Example 89: 6-Hydroxy-2H-benzo[b][1,4]oxazin-3(4H)-one>

2H-Benzo[b][1,4]oxazin-3(4H)-one (500 mg, 3.35 mmol) was dissolved in trifluoroacetic acid (6.70 mL), combined with trifluoroacetic acid (4.00 mL) solution [bis(trifluoroacetoxy)iodo]benzene (1.73 g, 4.02 mmol), and agitated at 80°C for 30 minutes. The reaction mixture was combined with water and filtered, and the residue was purified with silica gel column chromatography (heptane/ethyl acetate) to thereby produce the title compound (338 mg).

$^1$H-NMR (DMSO-d6) δ: 10.54 (1H, s), 9.15 (1H, s), 6.72 (1H, d, J = 8.7 Hz), 6.35 (1H, d, J = 2.7 Hz), 6.27 (1H, dd, J = 8.7, 2.7 Hz), 4.42 (2H, s).
LCMS: 166.1 $[M+H]^+$ (Rt= 1.635 min).

<Reference Example 90: 8-Hydroxy-1,3,4,5-tetrahydro-2H-pyrido[4,3-b]azepin-2-one>

[Step 1] tert-Butyl (E)-4-(4-amino-6-methoxy pyridin-3-yl)-3-butenoate

The same procedures as those in Step 2 of Reference Example 85 were carried out except that the compound obtained in Step 1 of Reference Example 85 (748 mg, 2.99 mmol) and tert-butyl 3-butenoate (970 μL, 5.98 mmol) were used as raw materials, to thereby produce the title compound (486 mg).
LCMS: 265.1 $[M+H]^+$ (Rt= 1.704 min).

[Step 2] tert-Butyl 4-(4-amino-6-methoxy pyridin-3-yl)butanoate

The same procedures as those in Step 3 of Reference Example 85 were carried out except that the compound obtained in Step 1 (486 mg, 1.84 mmol) was used as a raw material, to thereby produce a crude product of the title compound.
LCMS: 267.2 $[M+H]^+$ (Rt= 1.715 min).

[Step 3] 4-(4-Amino-6-methoxy pyridin-3-yl)butane acid

The crude product of the compound obtained in Step 2 was dissolved in THF (9.19 mL), combined with potassium tert-butoxide (247 mg, 2.20 mmol), and agitated at 50°C overnight. The reaction mixture was combined with saturated ammonium chloride aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (200 mg).
LCMS: 211.6 $[M+H]^+$ (Rt= 0.524 min).

[Step 4] 8-Methoxy-1,3,4,5-tetrahydro-2H-pyrido[4,3-b]azepin-2-one

The compound obtained in Step 3 (200 mg, 0.95 mmol) and N,N-diisopropylethylamine (0.497 mL, 2.85 mmol)

were dissolved in DMF (4.76 mL), combined with WSC-HCl (274 mg, 1.43 mmol) and HOBt (193 mg, 1.43 mmol), and agitated overnight at room temperature. The reaction mixture was combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (46.2 mg). LCMS: 193.2 $[M+H]^+$ (Rt= 1.605 min).

[Step 5] 8-hydroxy-1,3,4,5-tetrahydro-2H-pyrido[4,3-b]azepin-2-one

The compound obtained in Step 4 (46.2 mg) was dissolved in acetonitrile (2.40 mL), combined with potassium iodide (79.8 mg, 0.481 mmol) and trimethyl chlorosilane (61 μL, 0.481 mmol), and agitated at 80°C overnight. The reaction mixture was then cooled to room temperature, and filtered. The filtrate was combined with water, and washed three times with dichloromethane. The aqueous phase was concentrated under reduced pressure, the residue was amino purified with silica gel column chromatography (ethyl acetate/methanol) to thereby produce the title compound (7.50 mg).
LCMS: 179.5 $[M+H]^+$ (Rt= 1.072 min).

<Reference Example 91: tert-Butyl (E)-3-(4-amino-5,6-difluoropyridin-3-yl)acrylate>

The same procedures as those in Step 1 of Reference Example 84 were carried out except that the compound obtained in Step 1 of Reference Example 87 (1.28 g, 5.00 mmol) and tert-butyl acrylate (769 mg, 6.00 mmol) were used as raw materials, to thereby produce the title compound (1.01 g).
LCMS: 257.1 $[M+H]^+$ (Rt= 2.289 min).

<Reference Example 92: tert-Butyl (2R,5S)-2-(hydroxymethyl)-5-((2-oxo-1,2,3,4-tetrahydroquinolin-7-yl)oxy)piperidine-1-carboxylate>

[Step 1] 1-(tert-Butyl) 2-methyl (2R,5S)-5-((2-oxo-1,2,3,4-tetrahydroquinolin-7-yl)oxy)piperidine-1,2-dicarboxylate

The same procedures as those in Step 2 of Reference Example 83 were carried out except that 1-(tert-butyl) 2-methyl (2R,5R)-5-hydroxy piperidine-1,2-dicarboxylate (58.5 mg, 0.23 mmol) and 7-hydroxy-3,4-dihydroquinolin-2(1H)-one (44.2 mg, 0.27 mmol) were used as raw materials, to thereby produce the title compound (33.9 mg).

$^1$H-NMR ($CDCl_3$) δ: 7.31 (1H, s), 7.06 (1H, d, J = 8.2 Hz), 6.51 (1H, dd, J = 8.2, 2.3 Hz), 6.28 (1H, s), 4.92 (1H, d, J = 102.0 Hz), 4.37 (1H, s), 4.28 (1H, d, J = 14.6 Hz), 3.77 (3H, d, J = 13.7 Hz), 3.22 (1H, d, J = 12.8 Hz), 2.90 (2H, t, J = 7.5 Hz), 2.61 (2H, t, J = 7.3 Hz), 2.26-2.14 (1H, m), 2.02 (2H, d, J = 14.2 Hz), 1.38 (9H, d, J = 45.7 Hz).
LCMS: 305.1 $[M+H-Boc]^+$ (Rt= 2.190 min).

[Step 2] tert-Butyl (2R,5S)-2-(hydroxymethyl)-5-((2-oxo-1,2,3,4-tetrahydroquinolin-7-yl)oxy)piperidine-1-carboxylate

The same procedures as those in Step 4 of Reference Example 35 were carried out except that the compound obtained in Step 1 (101 mg, 0.25 mmol) was used as a raw material, to thereby produce the title compound (39.7 mg).

$^1$H-NMR ($CDCl_3$) δ: 7.36 (1H, s), 7.06 (1H, d, J = 8.2 Hz), 6.52 (1H, dd, J = 8.2, 2.3 Hz), 6.28 (1H, d, J = 2.3 Hz), 4.38-4.32 (2H, m), 4.21 (1H, d, J = 14.6 Hz), 3.83-3.80 (1H, m), 3.70-3.64 (1H, m), 3.12 (1H, d, J = 13.7 Hz), 2.90 (2H, t, J = 7.5 Hz), 2.62-2.60 (2H, m), 2.13-2.03 (2H, m), 1.93-1.78 (2H, m), 1.53-1.50 (1H, m), 1.36 (9H, s).
LCMS: 321.1 $[M+H-tBu]^+$ (Rt= 1.967 min).

<Reference Example 93: tert-Butyl (2R,4R)-2-(hydroxymethyl)-4-((2-oxo-1,2,3,4-tetrahydroquinolin-7-yl)oxy)piperidine-1-carboxylate>

The same procedures as those in Reference Example 92 were carried out except that 1-(tert-butyl) 2-methyl (2R,4S)-4-hydroxy piperidine-1,2-dicarboxylate (200 mg, 0.77 mmol) and 7-hydroxy-3,4-dihydroquinolin-2(1H)-one (151 mg, 0.93 mmol) were used as raw materials, to thereby produce the title compound (62.4 mg).

$^1$H-NMR ($CDCl_3$) δ: 8.02 (1H, br s), 7.04 (1H, d, J = 8.2 Hz), 6.52 (1H, dd, J = 8.2, 2.3 Hz), 6.35 (1H, d, J = 2.3 Hz), 4.50-4.45 (2H, m), 4.13-4.11 (1H, m), 3.78-3.76 (2H, m), 3.07-3.04 (1H, m), 2.90 (2H, t, J = 7.5 Hz), 2.64-2.62 (2H, m), 2.58 (1H, br s), 2.27-2.23 (1H, m), 2.06-2.03 (1H, m), 1.71-1.60 (2H, m), 1.48 (9H, s).
LCMS: 321.1 $[M+H-tBu]^+$ (Rt= 2.066 min).

<Reference Example 94: 7-Hydroxy-3,3-dimethyl-3,4-dihydroquinolin-2(1H)-one>

[Step 1] 7-(Benzyloxy)-1-(4-methoxy benzyl)-3,4-dihydroquinolin-2(1H)-one

7-(Benzyloxy)-3,4-dihydroquinolin-2(1H)-one (500 mg, 1.97 mmol) was dissolved in DMF (9.87 mL), and to the resulting solution, 50% sodium hydride (114 mg, 2.37 mmol) were added at 0°C. The mixture was then brought to room temperature, and agitated for 15 minutes. The reaction mixture was combined with 4-methoxy benzylchloride (404 μL, 2.96 mmol), and agitated overnight at room temperature. The reaction mixture was combined with saturated ammonium chloride aqueous solution, and extracted three times with ethyl acetate. The organic phase was collected, washed with saturated saline solution, dried with sodium sulfate, and concentrated under reduced pressure. The resuling solid was washed with ethyl acetate to thereby produce the title compound (675 mg).

$^1$H-NMR ($CDCl_3$) δ: 7.37-7.29 (5H, m), 7.11 (2H, d, J = 8.7 Hz), 7.04 (1H, d, J = 9.1 Hz), 6.82 (2H, dt, J = 9.3, 2.5 Hz), 6.58-6.55 (2H, m), 5.05 (2H, s), 4.93 (2H, s), 3.78 (3H, s), 2.88 (2H, dd, J = 8.7, 5.9 Hz), 2.74 (2H, dd, J = 8.5, 5.7).
LCMS: 374.2 $[M+H]^+$ (Rt= 2.448 min).

[Step 2] 7-(Benzyloxy)-1-(4-methoxy benzyl)-3-methyl-3,4-dihydroquinolin-2(1H)-one

The compound obtained in Step 1 (496 mg, 1.33 mmol) was dissolved in THF (6.64 mL), and to the resulting solution, 1M LHMDS/toluene solution (1.46 mL, 1.46 mmol) were added at 0°C, and agitated at 0°C for 30 minutes. The reaction mixture was combined with iodomethane (99.2 μL, 1.59 mmol), and agitated at 0°C for 3 hours. The reaction mixture was combined with saturated ammonium chloride aqueous solution, and extracted three times with ethyl acetate. The organic phase was collected, washed with saturated saline solution, dried with sodium sulfate, and concentrated under reduced pressure. The residue was purified with silica gel column chromatography (heptane/ethyl acetate) to thereby produce the title compound (335 mg).
LCMS: 388.2 $[M+H]^+$ (Rt= 2.551 min).

[Step 3] 7-(Benzyloxy)-1-(4-methoxy benzyl)-3,3-dimethyl-3,4-dihydroquinolin-2(1H)-one

The compound obtained in Step 2 (468 mg, 1.21 mmol) was dissolved in THF (6.04 mL), and to the resulting solution, 1M LHMDS/toluene solution (1.33 mL, 1.33 mmol) were added at 0°C, and agitated at 0°C for 30 minutes. The reaction mixture was combined with iodomethane (90.3 μL, 1.45 mmol), and agitated at 0°C for 3 hours. The reaction mixture was combined with saturated ammonium chloride aqueous solution, and extracted three times with ethyl acetate. The organic phase was collected, washed with saturated saline solution, dried with sodium sulfate, and concentrated under reduced pressure. The residue was purified with silica gel column chromatography (heptane/ethyl acetate) to thereby produce the title compound (405 mg).

$^1$H-NMR ($CDCl_3$) δ: 7.38-7.30 (5H, m), 7.08 (2H, d, J = 8.7 Hz), 7.04-6.99 (1H, m), 6.85-6.80 (2H, m), 6.59-6.52 (2H, m), 5.02 (2H, s), 4.92 (2H, s), 3.77 (3H, s), 2.74 (2H, s), 1.23 (6H, s).
LCMS: 402.2 $[M+H]^+$ (Rt= 2.606 min).

[Step 4] 7-Hydroxy-3,3-dimethyl-3,4-dihydroquinolin-2(1H)-one

The compound obtained in Step 3 (180 mg, 0.45 mmol) was dissolved in trifluoroacetic acid (750 μL), combined with anisole (48.7 μL, 0.45 mmol), and agitated at 70°C overnight. The reaction mixture was concentrated under reduced pressure, and the residue was combined with triethylamine and saturated saline solution, and extracted with dichloromethane. The organic phase was washed with saturated saline solution, dried with sodium sulfate, and concentrated under reduced pressure. The resuling solid was washed with dichloromethane to thereby produce the title compound (22.6 mg).

$^1$H-NMR ($CD_3OD$) δ: 6.93 (1H, d, J = 8.2 Hz), 6.40 (1H, dd, J = 8.0, 2.5 Hz), 6.33 (1H, d, J = 2.3 Hz), 2.67 (2H, s), 1.12 (6H, s).
LCMS: 192.10 $[M+H]^+$ (Rt= 1.865 min).

<Reference Example 95: 7-Hydroxy-3-methyl-3,4-dihydro-1,6-naphthyridin-2(1H)-one>

The same procedures as those in Step 2 to Step 5 of Reference Example 85 were carried out except that the compound obtained in Step 1 of Reference Example 85 (50.0 mg, 0.20 mmol) and methyl methacrylate (85.0 μL, 0.80

mmol) were used as raw materials, to thereby produce the title compound (17.1 mg).

$^1$H-NMR ($CD_3OD$) δ: 7.90 (1H, s), 6.50 (1H, s), 3.10-3.08 (1H, m), 2.74-2.71 (2H, m), 1.26 (3H, d, J = 6.4 Hz).
LCMS: 179.1 $[M+H]^+$ (Rt= 1.105 min).

<Reference Example 96: N-Benzyl-3-chloro-2-fluoro-5-iodopyridin-4-amine>

[Step 1] tert-Butyl (3-chloro-2-fluoropyridin-4-yl) carbamate

3-Chloro-2-fluoro-4-iodopyridine (515 mg, 2.00 mmol) was dissolved in toluene (10.0 mL), combined with tert-butyl carbamate (281 mg, 2.40 mmol), cesium carbonate (1.30g, 4.00 mmol), XantPhos (347 mg, 0.60 mmol), and $Pd_2(dba)_3$ (275 mg, 0.30 mmol), and agitated under nitrogen atmosphere at 100°C overnight. The reaction mixture was then cooled to room temperature, filtered with cerite, and the filtrate was concentrated under reduced pressure. The residue was purified with silica gel column chromatography (heptane/ethyl acetate) to thereby produce the title compound (430 mg).
LCMS: 247.0 $[M+H]^+$ (Rt= 2.478 min).

[Step 2] N-Benzyl-3-chloro-2-fluoropyridin-4-amine

The same procedures as those in Step 2 of Reference Example 2 and Step 4 of Reference Example 27 were carried out except that the compound obtained in Step 1 (430 mg, 1.74 mmol) was used as a raw material, to thereby produce the title compound (414 mg).
LCMS: 237.0$[M+H]^+$ (Rt= 2.289 min).

[Step 3] N-Benzyl-3-chloro-2-fluoro-5-iodopyridin-4-amine

The same procedures as those in Step 1 of Reference Example 87 were carried out except that the compound obtained in Step 2 (414 mg, 1.75 mmol) was used as a raw material, to thereby produce the title compound (378 mg).

$^1$H-NMR ($CDCl_3$) δ: 8.14 (1H, s), 7.42-7.30 (5H, m), 5.05 (1H, br s), 4.90 (2H, d, J = 5.9 Hz).
LCMS: 363.0 $[M+H]^+$ (Rt= 2.538 min).

<Reference Example 97: 6-Fluoro-7-hydroxy-3,4-dihydroquinolin-2(IH)-one>

[Step 1] 6,7-Difluoro-1-((2-(trimethylsilyl)ethoxy)methyl)quinolin-2(1H)-one

The same procedures as those in Reference Example 84 were carried out except that 4,5-difluoro-2-iodoaniline (1.27 g, 5.00 mmol) and ethyl acrylate (601 mg, 6.00 mmol) were used as raw materials, to thereby produce the title compound (682 mg).

$^1$H-NMR ($CDCl_3$) δ: 7.57 (1H, d, J = 9.6 Hz), 7.45 (1H, dd, J = 12.3, 6.9 Hz), 7.33 (1H, dd, J = 9.8, 8.5 Hz), 6.65 (1H, d, J = 9.6 Hz), 5.69 (2H, s), 3.67 (2H, t, J = 8.2 Hz), 0.94 (2H, t, J = 8.2 Hz), -0.03 (9H, s).
LCMS: 312.1 $[M+H]^+$ (Rt= 2.609 min) (Method B).

[Step 2] 7-(Benzyloxy)-6-fluoro-1-((2-(trimethylsilyl)ethoxy)methyl)quinolin-2(1H)-one

To a mixture of 50% sodium hydride (36.0 mg, 0.90 mmol) and THF (0.30 mL), DMF solution (1.50 mL) of benzylalcohol (92.7 μL, 0.90 mmol) was added dropwise, and the mixture was agitated for 1 hour at room temperature. To the resulting reaction mixture, the compound obtained in Step 1 (93.4 mg, 0.30 mmol) was added, and agitated overnight at room temperature. The reaction mixture was combined with saturated ammonium chloride aqueous solution, and extracted with ethyl acetate twice. The organic phase was collected, washed with saturated saline solution, dried with anhydrous magnesium sulfate, and concentrated under reduced pressure. The residue was purified with silica gel column chromatography (heptane/ethyl acetate) to thereby produce the title compound (159 mg).

$^1$H-NMR ($CDCl_3$) δ: 7.54-7.21 (8H, m), 6.55 (1H, d, J = 9.6 Hz), 5.67 (2H, s), 5.25 (2H, s), 3.68-3.64 (2H, m), 0.93-0.89 (2H, m), -0.02 (9H, s).
LCMS: 400.1 $[M+H]^+$ (Rt= 2.755 min) (Method B).

[Step 3] 7-(Benzyloxy)-6-fluoroquinolin-2(1H)-one

The compound obtained in Step 2 (294 mg, 0.74 mmol) was dissolved in THF (4.91 mL), combined with 6M hydrochloric acid aqueous solution (2.45 mL), and agitated at 50°C overnight. The reaction mixture was then cooled to room temperature, and concentrated under reduced pressure. The resuling solid was washed with ethyl acetate to thereby produce the title compound (261 mg).

$^1$H-NMR (DMSO-$d_6$) δ: 11.72 (1H, br s), 7.77 (1H, d, J = 9.6 Hz), 7.56 (1H, d, J = 11.4 Hz), 7.50-7.48 (2H, m), 7.43-7.36 (3H, m), 7.11 (1H, d, J = 7.3 Hz), 6.37 (1H, d, J = 9.6 Hz), 5.19 (2H, s).
LCMS: 270.1 $[M+H]^+$ (Rt= 2.039 min) (Method B).

[Step 4] 6-Fluoro-7-hydroxy-3,4-dihydroquinolin-2(1H)-one

The same procedures as those in Step 6 of Reference Example 27 were carried out except that the compound obtained in Step 3 (261 mg, 0.97 mmol) was used as a raw material, to thereby produce the title compound (28.0 mg).

$^1$H-NMR (DMSO-$d_6$) δ: 9.93 (1H, s), 9.69 (1H, s), 6.93 (1H, d, J = 11.0 Hz), 6.49 (1H, d, J = 7.8 Hz), 2.72 (2H, t, J = 7.3 Hz), 2.36 (2H, t, J = 7.5 Hz).
LCMS: 182.10 $[M+H]^+$ (Rt= 1.507 min) (Method B).

<Reference Example 98: (2S,11aR)-7-Fluoro-2-hydroxy-8-methyl-6-pentyl-2,3,11,11a-tetrahydro-1H,5H-benzo[f]pyrrolo[2,1-c][1,4]oxazepin-5-one>

The same procedures as those in Reference Example 78 were carried out except that the compound obtained in Step 1 of Reference Example 77 (100 mg, 0.20 mmol) and 1-penten-1-ylboronic acid (46.6 mg, 0.41 mmol) were used as raw materials, to thereby produce the title compound (40.2 mg).
LCMS: 322.2 $[M+H]^+$ (Rt= 2.277 min).

<Reference Example 99: (2S,11aR)-7-Fluoro-6-(2-fluorophenyl)-2-hydroxy-8-methyl-2,3,11,11a-tetrahydro-1H,5H-benzo[f]pyrrolo[2,1-c][1,4]oxazepin-5-one>

The same procedures as those in Reference Example 78 were carried out except that the compound obtained in Step 1 of Reference Example 77 (123 mg, 0.25 mmol) and 2-fluorophenyl boronic acid (70.2 mg, 0.50 mmol) were used as raw materials, to thereby produce the title compound (56.2 mg).
LCMS: 346.1 $[M+H]^+$ (Rt= 2.049 min).

<Reference Example 100: (2S,11aR)-2-Amino-6-isopropoxy-8-methyl-2,3,11,11a-tetrahydro-1H,5H-benzo[f]pyrrolo[2,1-c][1,4]oxazepin-5-one>

The same procedures as those in Step 2 to Step 3 of Reference Example 82 were carried out except that the compound obtained in Reference Example 34 (437 mg, 1.50 mmol) was used as a raw material, to thereby produce the title compound (337 mg). LCMS: 291.1 $[M+H]^+$ (Rt= 1.640 min).

<Reference Example 101: (2R,11aR)-7-Fluoro-6-isopropoxy-8-methyl-5-oxo-2,3,11,11a-tetrahydro-1H,5H-benzo[f]pyrrolo[2,1-c][1,4]oxazepin-2-yl 4-methyl benzene sulfonate>

The compound obtained in Step 1 of Reference Example 82 (100 mg, 0.32 mmol) was dissolved in THF (3.23 mL), combined with 50% sodium hydride (31.0 mg, 0.65 mmol), and agitated for 30 minutes at room temperature. The reaction mixture was combined with p-toluene sulfonyl chloride (185 mg, 0.97 mmol), agitated for 3 hours at room temperature, combined with saturated ammonium chloride aqueous solution, and extracted with ethyl acetate. The organic phase was washed twice with saturated saline solution, dried with sodium sulfate, and concentrated under reduced pressure. The residue was purified with silica gel column chromatography (heptane/ethyl acetate) to thereby produce the title compound (137 mg).

$^1$H-NMR ($CDCl_3$) δ: 7.80 (2H, d, J = 8.2 Hz), 7.37 (2H, d, J = 8.2 Hz), 6.63 (1H, d, J = 5.5 Hz), 5.23 (1H, t, J = 5.0 Hz), 4.63-4.55 (1H, m), 4.37 (1H, dd, J = 11.9, 10.1 Hz), 4.00 (1H, dd, J = 10.3, 5.3 Hz), 3.97-3.90 (1H, m), 3.87 (1H, d, J = 14.6 Hz), 3.77 (1H, dd, J = 14.6, 5.0 Hz), 2.47 (3H, s), 2.41-2.32 (1H, m), 2.25 (3H, d, J = 2.3 Hz), 2.13 (1H, d, J = 15.1 Hz), 1.33 (3H, d, J = 5.9 Hz), 1.28 (3H, d, J = 5.7 Hz).

LCMS: 464.2 [M+H]+ (Rt= 2.370 min).

<Reference Example 102: (2S,11aR)-7-Fluoro-2-hydroxy-6-(((S)-1-(methoxymethoxy)propan-2-yl)oxy)-8-methyl-2,3,11,11a-tetrahydro-1H,5H-benzo[f]pyrrolo[2,1-c][1,4]oxazepin-5-one>

[Step 1] (2S,11aR)-2-(Benzyloxy)-6-(((S)-1-((tert-butyldiphenylsilyl)oxy)propan-2-yl)oxy)-7-fluoro-8-methyl-2,3,11,11a-tetrahydro-1H,5H-benzo[f]pyrrolo[2,1-c][1,4]oxazepin-5-one

The same procedures as those in Step 2 of Reference Example 5 were carried out except that the compound obtained in Step 4 of Reference Example 49 (200 mg, 0.56 mmol) and the compound obtained in Step 1 of Reference Example 5 (211 mg, 0.67 mmol) were used as raw materials, to thereby produce the title compound (318 mg).

$^1$H-NMR ($CDCl_3$) δ: 7.61 (2H, dd, J = 7.8, 1.8 Hz), 7.53 (2H, dd, J = 8.0, 1.6 Hz), 7.41-7.28 (11H, m), 6.57 (1H, d, J = 5.9 Hz), 5.01-4.95 (1H, m), 4.55 (1H, d, J = 11.9 Hz), 4.46 (1H, d, J = 11.9 Hz), 4.34-4.31 (1H, m), 4.27-4.23 (1H, m), 4.05 (1H, dd, J = 9.1, 4.6 Hz), 4.00-3.96 (3H, m), 3.94-3.86 (1H, m), 3.80 (1H, dd, J = 12.3, 5.0 Hz), 3.63 (1H, dd, J = 10.5, 5.0 Hz), 2.22 (3H, d, J = 1.8 Hz), 2.18-2.15 (1H, m), 1.83-1.79 (1H, m), 1.26-1.24 (3H, m), 0.95 (9H, s).
LCMS: 654.3 [M+H]+ (Rt= 3.085 min).

[Step 2] (2S,11aR)-2-(Benzyloxy)-7-fluoro-6-(((S)-1-hydroxy propan-2-yl)oxy)-8-methyl-2,3,11,11a-tetrahydro-1H,5H-benzo[f]pyrrolo[2,1-c][1,4]oxazepin-5-one

The same procedures as those in Step 2 of Reference Example 36 were carried out except that the compound obtained in Step 1 (318 mg, 0.49 mmol) was used as a raw material, to thereby produce the title compound (183 mg).

$^1$H-NMR ($CDCl_3$) δ: 7.38-7.29 (6H, m), 6.57 (1H, d, J = 5.5 Hz), 6.36 (1H, dd, J = 8.7, 5.5 Hz), 4.59-4.49 (3H, m), 4.33-4.27 (1H, m), 4.09-3.85 (5H, m), 3.69-3.63 (2H, m), 2.25 (3H, d, J = 2.3 Hz), 2.19 (1H, dd, J = 13.3, 6.9 Hz), 1.89 (1H, dq, J = 13.3, 3.2 Hz), 1.40 (3H, d, J = 6.4 Hz).
LCMS: 416.2 [M+H]+ (Rt= 2.343 min).

[Step 3] (2S,11aR)-2-(Benzyloxy)-7-fluoro-6-(((S)-1-(methoxymethoxy)propan-2-yl)oxy)-8-methyl-2,3,11,11a-tetrahydro-1H,5H-benzo[f]pyrrolo[2,1-c][1,4]oxazepin-5-one

The compound obtained in Step 2 (183 mg, 0.44 mmol) and N,N-diisopropylethylamine (152 μL, 0.88 mmol) were dissolved in dichloromethane (1.00 mL), and to the resulting solution, methoxymethyl chloride (66.8 μL, 0.88 mmol) was added at 0°C, and the reaction mixture was brought to room temperature and agitated overnight. The reaction mixture was combined with water, and extracted with ethyl acetate. The organic phase was washed twice with saturated saline solution, dried with anhydrous magnesium sulfate, and concentrated under reduced pressure to thereby produce the title compound (200 mg).

$^1$H-NMR ($CDCl_3$) δ: 7.36-7.28 (5H, m), 6.60 (1H, d, J = 5.5 Hz), 4.62-4.55 (3H, m), 4.47-4.42 (2H, m), 4.30-4.25 (1H, m), 4.13-4.10 (1H, m), 4.03-4.00 (2H, m), 3.95-3.87 (1H, m), 3.81 (1H, dd, J = 12.3, 5.0 Hz), 3.75 (1H, dd, J = 10.3, 5.3 Hz), 3.61 (1H, dd, J = 10.5, 5.0 Hz), 3.31 (3H, s), 2.28-2.21 (4H, m), 1.85 (1H, dt, J = 13.3, 5.3 Hz), 1.28 (3H, d, J = 6.4 Hz).
LCMS: 460.2 [M+H]+ (Rt= 2.329 min).

[Step 4] (2S,11aR)-7-Fluoro-2-hydroxy-6-(((S)-1-(methoxymethoxy)propan-2-yl)oxy)-8-methyl-2,3,11,11a-tetrahydro-1H,5H-benzo[f]pyrrolo[2,1-c][1,4]oxazepin-5-one

The same procedures as those in Step 6 of Reference Example 49 were carried out except that the compound obtained in Step 3 (200 mg, 0.44 mmol) was used as a raw material, to thereby produce the title compound (143 mg).

$^1$H-NMR ($CDCl_3$) δ:6.62 (1H, d, J = 5.5 Hz), 4.62-4.59 (3H, m), 4.55-4.49 (1H, m), 4.16-4.11 (1H, m), 4.06-4.02 (1H, m), 3.96 (1H, q, J = 10.7 Hz), 3.85-3.81 (3H, m), 3.68 (1H, dd, J = 10.5, 5.5 Hz), 3.33 (3H, s), 2.25 (3H, d, J = 1.4 Hz), 2.17-2.15 (1H, m), 2.05-2.04 (1H, m), 1.86 (1H, dt, J = 13.3, 5.5 Hz), 1.33 (3H, d, J = 5.9 Hz).
LCMS: 370.2 [M+H]+ (Rt= 1.903 min).

<Reference Example 103: (2S,11aR)-7-Fluoro-2-hydroxy-6-(((R)-1-(methoxymethoxy)propan-2-yl)oxy)-8-methyl-2,3,11,11a-tetrahydro-1H,5H-benzo[f]pyrrolo[2,1-c][1,4]oxazepin-5-one>

[Step 1] (S)-1-((tert-Butyldiphenylsilyl)oxy)propan-2-ol

The same procedures as those in Step 1 of Reference Example 5 were carried out except that (S)-propan-1,2-diol (1.00 g, 13.1 mmol) was used as a raw material, to thereby produce the title compound (3.13 g).
$^1$H-NMR ($CDCl_3$) δ: 7.66 (4H, dt, J = 6.6, 1.1 Hz), 7.44-7.40 (6H, m), 3.95-3.87 (1H, m), 3.62 (1H, dd, J = 10.1, 3.7 Hz), 3.44 (1H, dd, J = 10.1, 7.8 Hz), 2.54 (1H, d, J = 3.2 Hz), 1.10 (3H, d, J = 6.4 Hz), 1.07 (9H, s).

[Step 2] (2S,11aR)-7-fluoro-2-hydroxy-6-(((R)-1-(methoxymethoxy)propan-2-yl)oxy)-8-methyl-2,3,11,11a-tetrahydro-1H,5H-benzo[f]pyrrolo[2,1-c][1,4]oxazepin-5-one

The same procedures as those in Reference Example 102 were carried out except that the compound obtained in Step 4 of Reference Example 49 (200 mg, 0.56 mmol) and the compound obtained in Step 1 (194 mg, 0.62 mmol) were used as raw materials, to thereby produce the title compound (175 mg).
LCMS: 370.1 $[M+H]^+$ (Rt= 1.938 min).

<Reference Example 104: (2S,11aR)-6-(2,2-difluoropropoxy)-7-fluoro-2-hydroxy-8-methyl-2,3,11,11a-tetrahydro-1H,5H-benzo[f]pyrrolo[2,1-c][1,4]oxazepin-5-one>

[Step 1] (2S,11aR)-2-(benzyloxy)-7-fluoro-8-methyl-6-(2-oxopropoxy)-2,3,11,11a-tetrahydro-1H,5H-benzo[f]pyrrolo[2,1-c][1,4]oxazepin-5-one

The same procedures as those in Step 5 of Reference Example 49 were carried out except that the compound obtained in Step 4 of Reference Example 49 (200 mg, 0.56 mmol) and bromoacetone (70.5 μL, 0.84 mmol) were used as raw materials, to thereby produce the title compound (235 mg).

$^1$H-NMR ($CDCl_3$) δ: 7.37-7.28 (5H, m), 6.65 (1H, d, J = 5.5 Hz), 4.86-4.82 (1H, m), 4.60-4.56 (2H, m), 4.50-4.47 (1H, m), 4.31-4.26 (1H, m), 4.15-4.02 (2H, m), 3.97-3.89 (2H, m), 3.84 (1H, dd, J = 12.6, 5.3 Hz), 2.29 (3H, s), 2.26-2.22 (4H, m), 1.87 (1H, dt, J = 13.1, 5.3 Hz).
LCMS: 414.2 $[M+H]^+$ (Rt= 2.259 min).

[Step 2] (2S,11aR)-2-(Benzyloxy)-6-(2,2-difluoropropoxy)-7-fluoro-8-methyl-2,3,11,11a-tetrahydro-1H,5H-benzo[f]pyrrolo[2,1-c][1,4]oxazepin-5-one

The same procedures as those in Step 2 of Reference Example 37 were carried out except that the compound obtained in Step 1 (235 mg, 0.57 mmol) was used as a raw material, to thereby produce the title compound (161 mg).

$^1$H-NMR ($CDCl_3$) δ: 7.37-7.28 (5H, m), 6.65 (1H, d, J = 5.5 Hz), 4.57 (1H, d, J = 11.9 Hz), 4.48 (1H, d, J = 11.4 Hz), 4.40-4.26 (2H, m), 4.22-4.09 (3H, m), 4.07-4.00 (1H, m), 3.97-3.92 (2H, m), 3.83 (1H, dd, J = 12.6, 5.3 Hz), 2.29-2.22 (4H, m), 1.89-1.83 (1H, m), 1.78 (3H, t, J = 19.2 Hz).
LCMS: 436.2 $[M+H]^+$ (Rt= 2.417 min).

[Step 3] (2S,11aR)-6-(2,2-Difluoropropoxy)-7-fluoro-2-hydroxy-8-methyl-2,3,11,11a-tetrahydro-1H,5H-benzo[f]pyrrolo[2,1-c][1,4]oxazepin-5-one

The same procedures as those in Step 6 of Reference Example 49 were carried out except that the compound obtained in Step 2 (161 mg, 0.37 mmol) was used as a raw material, to thereby produce the title compound (129 mg).

$^1$H-NMR ($CDCl_3$) δ: 6.67 (1H, d, J = 5.9 Hz), 4.63-4.58 (1H, m), 4.43-4.34 (1H, m), 4.20-4.12 (2H, m), 4.08-4.03 (1H, m), 3.97 (1H, dd, J = 11.2, 9.8 Hz), 3.86 (1H, dq, J = 12.8, 1.7 Hz), 3.80 (1H, dd, J = 12.6, 4.8 Hz), 2.27 (3H, d, J = 1.8 Hz), 2.21-2.15 (1H, m), 1.90-1.76 (5H, m).
LCMS: 346.1 $[M+H]^+$ (Rt= 2.017 min).

<Reference Example 105: (2S,11aR)-7-Fluoro-2-hydroxy-8-methyl-6-(((S)-1,1,1-trifluoropropan-2-yl)oxy)-2,3,11,11a-tetrahydro-1H,5H-benzo[f]pyrrolo[2,1-c][1,4]oxazepin-5-one>

The same procedures as those in Step 1 of Reference Example 7 and Step 6 of Reference Example 49 were carried out except that (R)-1,1,1-trifluoro-2-propanol (160 mg, 1.40 mmol) and the compound obtained in Step 4 of Reference Example 49 (200 mg, 0.56 mmol) were used as raw materials, to thereby produce the title compound (153 mg).
LCMS: 364.1 $[M+H]^+$ (Rt= 2.075 min).

<Reference Example 106: (2S,11aR)-6-Cyclobutoxy-7-fluoro-2-hydroxy-8-methyl-2,3,11,11a-tetrahydro-1H,5H-benzo[f]pyrrolo[2,1-c][1,4]oxazepin-5-one>

The same procedures as those in Step 5 to Step 6 of Reference Example 49 were carried out except that the compound obtained in Step 4 of Reference Example 49 (100 mg, 0.28 mmol) and bromo cyclobutane (39.6 μL, 0.42 mmol) were used as raw materials, to thereby produce the title compound (79.4 mg).
LCMS: 322.1 $[M+H]^+$ (Rt= 2.172 min).

<Reference Example 107: (2S,11aR)-6-((4,4-Difluorocyclohexyl)oxy)-7-fluoro-2-hydroxy-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (100 mg, 0.28 mmol) and 4,4-difluorocyclohexanol (45.7 mg, 0.34 mmol) were used as raw materials, to thereby produce the title compound (74.3 mg).

$^1$H-NMR ($CDCl_3$) δ: 6.63 (1H, d, J = 5.9 Hz), 4.62-4.60 (1H, m), 4.48-4.48 (1H, br m), 4.17 (1H, dd, J = 9.6, 4.1 Hz), 4.08-3.94 (2H, m), 3.90-3.87 (1H, m), 3.77 (1H, dd, J = 12.8, 4.1 Hz), 2.36-2.29 (4H, m), 2.21-2.12 (3H, m), 2.00-1.77 (6H, m), 1.70 (1H, d, J = 3.7 Hz).
LCMS: 386.1 $[M+H]^+$ (Rt= 2.077 min).

<Reference Example 108: (2S,11aR)-6-((3,3-Difluorocyclobutyl)methoxy)-7-fluoro-2-hydroxy-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (100 mg, 0.28 mmol) and (3,3-difluorocyclobutyl)methanol (37.6 μL, 0.36 mmol) were used as raw materials, to thereby produce the title compound (75.9 mg).

$^1$H-NMR ($CDCl_3$) δ: 6.64 (1H, d, J = 5.9 Hz), 4.63-4.61 (1H, m), 4.30 (1H, dd, J = 8.9, 4.8 Hz), 4.16 (1H, dd, J = 9.8, 4.3 Hz), 4.09-4.05 (2H, m), 4.03-3.94 (1H, m), 3.83 (2H, d, J = 4.6 Hz), 2.72-2.45 (5H, m), 2.26 (3H, d, J = 2.3 Hz), 2.20-2.14 (1H, m), 1.87 (1H, dt, J = 13.4, 5.3 Hz), 1.73 (1H, d, J = 4.1 Hz).
LCMS: 372.1 $[M+H]^+$ (Rt= 2.084 min).

<Reference Example 109: (2R,11aR)-6-(3,3-Difluorocyclobutoxy)-7-fluoro-2-hydroxy-8-methyl-2,3,11,11a-tetrahydro-1H,5H-benzo[f]pyrrolo[2,1-c][1,4]oxazepin-5-one>

[Step 1] (2S,11aR)-6-(3,3-Difluorocyclobutoxy)-7-fluoro-2-hydroxy-8-methyl-2,3,11,11a-tetrahydro-1H,5H-benzo[f]pyrrolo[2,1-c][1,4]oxazepin-5-one

The same procedures as those in Reference Example 51 were carried out except that the compound obtained in Step 4 of Reference Example 49 (100 mg, 0.28 mmol) and 3,3-difluorocyclobutanol (35.7 μL, 0.42 mmol) were used as raw materials, to thereby produce the title compound (57.8 mg).
LCMS: 358.1 $[M+H]^+$ (Rt= 2.048 min).

[Step 2] (2R,11aR)-6-(3,3-Difluorocyclobutoxy)-7-fluoro-2-hydroxy-8-methyl-2,3,11,11a-tetrahydro-1H,5H-benzo[f]pyrrolo[2,1-c][1,4]oxazepin-5-one

The same procedures as those in Step 2 of Reference Example 61 were carried out except that the compound obtained in Step 1 (57.8 mg, 0.16 mmol) was used as a raw material, to thereby produce the title compound (41.3 mg).

$^1$H-NMR ($CDCl_3$) δ: 6.68 (1H, d, J = 5.9 Hz), 4.79-4.76 (1H, br m), 4.64-4.61 (2H, m), 4.03 (1H, dd, J = 10.1, 5.5 Hz),

3.93-3.85 (3H, m), 3.13-2.91 (4H, m), 2.29-2.22 (4H, m), 1.83 (2H, d, J = 13.7 Hz).
LCMS: 358.1 $[M+H]^+$ (Rt= 2.079 min).

<Reference Example 110: (2S,11aR)-7-Fluoro-2-hydroxy-6-(((R)-1-methoxy propan-2-yl)oxy)-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (75.0 mg, 0.21 mmol) and (S)-(+)-1-methoxy-2-propanol (31.0 μL, 0.31 mmol) were used as raw materials, to thereby produce the title compound (62.2 mg).
LCMS: 340.1 $[M+H]^+$ (Rt= 1.897 min).

<Reference Example 111: (2S,11aR)-7-Fluoro-2-hydroxy-6-(((S)-1-methoxy propan-2-yl)oxy)-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (75.0 mg, 0.21 mmol) and (R)-(-)-1-methoxy-2-propanol (31.0 μL, 0.31 mmol) were used as raw materials, to thereby produce the title compound (64.0 mg).
LCMS: 340.2 $[M+H]^+$ (Rt= 1.883 min).

<Reference Example 112: (2S,11aR)-7-Fluoro-2-hydroxy-6-(2-methoxy-2-methylpropoxy)-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (100 mg, 0.28 mmol) and 2-methoxy-2-methyl-1-propanol (49.0 μL, 0.42 mmol) were used as raw materials, to thereby produce the title compound (74.7 mg).
LCMS: 354.2 $[M+H]^+$ (Rt= 1.951 min).

<Reference Example 113: (2S,11aR)-7-Fluoro-2-hydroxy-6-(2-methoxyethoxy)-8-methyl-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (75.0 mg, 0.21 mmol) and 2-methoxyethanol (25.0 μL, 0.31 mmol) were used as raw materials, to thereby produce the title compound (58.0 mg).
LCMS: 326.1 $[M+H]^+$ (Rt= 1.856 min).

<Reference Example 114: (2S,11aR)-7-Fluoro-2-hydroxy-8-methyl-6-(2-(pyrrolidin-1-yl)ethoxy)-2,3,11,11a-tetrahydro-1H,5H-benzo[f]pyrrolo[2,1-c][1,4]oxazepin-5-one>

The same procedures as those in Reference Example 51 were carried out except that the compound obtained in Step 4 of Reference Example 49 (100 mg, 0.28 mmol) and 1-(2-hydroxyethyl)pyrrolidine (49.0 μL, 0.42 mmol) were used as raw materials, to thereby produce the title compound (55.2 mg).
LCMS: 365.2 $[M+H]^+$ (Rt= 1.652 min).

<Reference Example 115: (2R,11aR)-7-Fluoro-6-isopropoxy-8-methyl-5-oxo-2,3,11,11a-tetrahydro-1H,5H-benzo[f]pyrrolo[2,1-c][1,4]oxazepin-2-yl methane sulfonate>

The compound obtained in Step 1 of Reference Example 82 (100 mg, 0.32 mmol) and triethylamine (66.0 μL, 0.48 mmol) were dissolved in dichloromethane (1.08 mL), and to the resulting solution, methane sulfonyl chloride (30.0 μL, 0.39 mmol) was added at 0°C, and the mixture was agitated for 2 hours with its temperature maintained at 0°C. The reaction mixture was combined with saturated sodium bicarbonate aqueous solution, and extracted with ethyl acetate. The organic phase was washed with saline solution, dried with anhydrous magnesium sulfate, and concentrated under reduced pressure. The residue was purified with silica gel column chromatography (heptane/ethyl acetate) to thereby produce the title compound (110 mg).

$^1$H-NMR ($CDCl_3$) δ: 6.65 (1H, d, J = 5.9 Hz), 5.42 (1H, t, J = 4.8 Hz), 4.66-4.57 (1H, m), 4.38 (1H, t, J = 11.0 Hz), 4.17 (1H, d, J = 14.6 Hz), 4.03-3.98 (3H, m), 3.10 (3H, s), 2.47-2.40 (1H, m), 2.26-2.21 (4H, m), 1.37 (3H, d, J = 5.9 Hz), 1.31 (3H, d, J = 5.9 Hz).
LCMS: 388.1 $[M+H]^+$ (Rt= 2.063 min) (Method B).

<Reference Example 116: (2R,11aR)-7-Chloro-8-methyl-5-oxo-6-(2,2,2-trifluoroethoxy)-2,3,11,11a-tetrahydro-1H,5H-benzo[f]pyrrolo[2,1-c][1,4]oxazepin-2-yl methane sulfonate>

[Step 1] Methyl 3-chloro-2-(cyclopropylmethoxy)-6-hydroxy-4-methyl benzoate

The same procedures as those in Step 1 of Reference Example 58 were carried out except that the compound obtained in Step 1 of Reference Example 28 (1.97 g, 8.34 mmol) was used as a raw material, to thereby produce the title compound (1.17 g).

$^1$H-NMR ($CDCl_3$) δ: 11.14 (1H, s), 6.70 (1H, s), 3.99 (3H, s), 3.77 (2H, d, J = 6.9 Hz), 2.36 (3H, s), 1.39-1.29 (1H, m), 0.67-0.62 (2H, m), 0.36 (2H, q, J = 5.0 Hz).
LCMS: 271.0 $[M+H]^+$ (Rt= 2.516 min) (Method B).

[Step 2] (2S,11aR)-2-(Benzyloxy)-7-chloro-6-(cyclopropylmethoxy)-8-methyl-2,3,11,11a-tetrahydro-1H,5H-benzo[f]pyrrolo[2,1-c][1,4]oxazepin-5-one

The same procedures as those in Step 2 to Step 5 of Reference Example 27 were carried out except that the compound obtained in Step 1 (1.29 g, 4.76 mmol) and the compound obtained in Reference Example 25 (1.90 g, 6.19 mmol) were used as raw materials, to thereby produce the title compound (1.53 g).

$^1$H-NMR ($CDCl_3$) δ: 7.37-7.28 (5H, m), 6.72 (1H, s), 4.57 (1H, d, J = 11.4 Hz), 4.48 (1H, d, J = 11.4 Hz), 4.30-4.25 (1H, m), 4.13-4.10 (1H, m), 4.06-3.83 (6H, m), 2.36 (3H, s), 2.26-2.19 (1H, m), 1.86 (1H, td, J = 9.0, 4.3 Hz), 1.38-1.31 (1H, m), 0.58-0.50 (2H, m), 0.34-0.30 (2H, m).
LCMS: 428.1 $[M+H]^+$ (Rt= 2.573 min) (Method B).

[Step 3] (2S,11aR)-2-(Benzyloxy)-7-chloro-6-hydroxy-8-methyl-2,3,11,11a-tetrahydro-1H,5H-benzo[f]pyrrolo[2,1-c][1,4]oxazepin-5-one

The same procedures as those in Step 4 of Reference Example 49 were carried out except that the compound obtained in Step 2 (1.53 g, 3.57 mmol) was used as a raw material, to thereby produce the title compound (1.19 g).

$^1$H-NMR ($CDCl_3$) δ: 7.37-7.28 (5H, m), 6.38 (1H, s), 4.58 (1H, d, J = 11.9 Hz), 4.51 (1H, d, J = 11.9 Hz), 4.46 (1H, dd, J = 11.9, 1.4 Hz), 4.25-4.19 (2H, m), 4.03 (1H, dd, J = 14.0, 1.6 Hz), 3.94 (1H, dd, J = 11.9, 8.7 Hz), 3.82 (1H, dd, J = 13.7, 4.1 Hz), 2.38-2.33 (4H, m), 1.74 (1H, ddd, J = 14.2, 10.1, 3.2 Hz).
LCMS: 374.1 $[M+H]^+$ (Rt= 2.625 min) (Method B).

[Step 4] (2S,11aR)-2-(benzyloxy)-7-chloro-8-methyl-6-(2,2,2-trifluoroethoxy)-2,3,11,11a-tetrahydro-1H,5H-benzo[f]pyrrolo[2,1-c][1,4]oxazepin-5-one

The same procedures as those in Step 5 of Reference Example 49 were carried out except that the compound obtained in Step 3 (100 mg, 0.27 mmol) and trifluoromethane sulfonic acid 2,2,2-trifluoroethyl (77.1 μL, 0.54 mmol) were used as raw materials, to thereby produce the title compound (121 mg).

$^1$H-NMR ($CDCl_3$) δ: 7.37-7.30 (5H, m), 6.81 (1H, s), 4.80-4.75 (1H, m), 4.57 (1H, d, J = 11.9 Hz), 4.48 (1H, d, J = 11.4 Hz), 4.43-4.38 (1H, m), 4.30-4.25 (1H, m), 4.17-4.13 (1H, m), 4.04-3.96 (3H, m), 3.81 (1H, dd, J = 12.8, 5.0 Hz), 2.38 (3H, s), 2.29-2.23 (1H, m), 1.86 (1H, dt, J = 13.3, 5.0 Hz).
LCMS: 456.1 $[M+H]^+$ (Rt= 2.572 min) (Method B).

[Step 5] (2S,11aR)-7-Chloro-2-hydroxy-8-methyl-6-(2,2,2-trifluoroethoxy)-2,3,11,11a-tetrahydro-1H,5H-benzo[f]pyrrolo[2,1-c][1,4]oxazepin-5-one

The same procedures as those in Step 4 of Reference Example 59 were carried out except that the compound obtained in Step 4 (121 mg, 0.27 mmol) was used as a raw material, to thereby produce the title compound (97.6 mg). LCMS: 366.1 $[M+H]^+$ (Rt= 2.058 min) (Method B).

[Step 6] (2R,11aR)-7-Chloro-2-hydroxy-8-methyl-6-(2,2,2-trifluoroethoxy)-2,3,11,11a-tetrahydro-1H,5H-benzo[f]pyrrolo[2,1-c][1,4]oxazepin-5-one

The same procedures as those in Step 2 of Reference Example 61 were carried out except that the compound obtained in Step 5 (97.6 mg, 0.27 mmol) was used as a raw material, to thereby produce the title compound (93.5 mg). LCMS: 366.1 $[M+H]^+$ (Rt= 2.097 min) (Method B).

[Step 7] (2R,11aR)-7-Chloro-8-methyl-5-oxo-6-(2,2,2-trifluoroethoxy)-2,3,11,11a-tetrahydro-1H,5H-benzo[f]pyrrolo[2,1-c][1,4]oxazepin-2-yl methane sulfonate

The same procedures as those in Reference Example 115 were carried out except that the compound obtained in Step 6 (93.5 mg, 0.26 mmol) was used as a raw material, to thereby produce the title compound (92.8 mg).

$^1$H-NMR ($CDCl_3$) δ: 6.88 (1H, s), 5.43 (1H, t, J = 4.8 Hz), 4.91-4.86 (1H, m), 4.49-4.34 (2H, m), 4.16-4.11 (2H, m), 4.01-3.97 (2H, m), 3.11 (3H, s), 2.51-2.44 (1H, m), 2.40 (3H, s), 2.25 (1H, d, J = 14.6 Hz).
LCMS: 444.1 $[M+H]^+$ (Rt= 2.270 min) (Method B).

<Reference Example 117: (2R,11aR)-7-Chloro-6-((1-fluorocyclopropyl)methoxy)-8-methyl-5-oxo-2,3,11,11a-tetrahydro-1H,5H-benzo[f]pyrrolo[2,1-c][1,4]oxazepin-2-yl methane sulfonate>

[Step 1] (2S,11aR)-7-Chloro-6-(cyclopropylmethoxy)-2-hydroxy-8-methyl-2,3,11,11a-tetrahydro-1H,5H-benzo[f]pyrrolo[2,1-c][1,4]oxazepin-5-one

The same procedures as those in Step 4 of Reference Example 59 were carried out except that the compound obtained in Step 2 of Reference Example 116 (542 mg, 1.27 mmol) was used as a raw material, to thereby produce the title compound (383 mg). LCMS: 338.1 $[M+H]^+$ (Rt= 2.006 min) (Method B).

[Step 2] (2R,11aR)-7-Chloro-6-(cyclopropylmethoxy)-8-methyl-5-oxo-2,3,11,11a-tetrahydro-1H,5H-benzo[f]pyrrolo[2,1-c][1,4]oxazepin-2-yl benzoate

The compound obtained in Step 1 (383 mg, 1.14 mmol), benzoic acid (216 mg, 1.77 mmol), and triphenylphosphine (464 mg, 1.77 mmol) were dissolved in THF (3.78 mL), combined with DIAD (345 μL, 1.77 mmol), and agitated for 3 hours at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (669 mg).

$^1$H-NMR ($CDCl_3$) δ: 8.04 (2H, d, J = 7.3 Hz), 7.61 (1H, t, J = 7.3 Hz), 7.48 (2H, t, J = 7.8 Hz), 6.77 (1H, s), 5.71 (1H, t, J = 5.0 Hz), 5.03-4.93 (2H, m), 4.47 (1H, dd, J = 11.7, 10.3 Hz), 4.11-3.93 (4H, m), 2.51-2.44 (1H, m), 2.38 (3H, s), 2.07-2.04 (1H, br m), 1.44-1.41 (1H, m), 0.60 (2H, dd, J = 8.0, 1.6 Hz), 0.43 (2H, t, J = 4.6 Hz).
LCMS: 442.1 $[M+H]^+$ (Rt= 2.556 min) (Method B).

[Step 3] (2R,11aR)-7-Chloro-6-hydroxy-8-methyl-5-oxo-2,3,11,11a-tetrahydro-1H,5H-benzo[f]pyrrolo[2,1-c][1,4]oxazepin-2-yl benzoate

The same procedures as those in Step 4 of Reference Example 49 were carried out except that the compound obtained in Step 2 (669 mg, 1.51 mmol) was used as a raw material, to thereby produce the title compound (310 mg).

$^1$H-NMR ($CDCl_3$) δ: 8.04-8.02 (2H, m), 7.61 (1H, t, J = 7.5 Hz), 7.48 (2H, t, J = 7.5 Hz), 6.48 (1H, s), 5.67-5.64 (1H, m), 4.47-4.35 (2H, m), 4.19-4.09 (2H, m), 3.99 (1H, dd, J = 14.0, 4.8 Hz), 2.67-2.60 (1H, m), 2.36 (3H, s), 2.13-2.10 (1H, m).
LCMS: 388.1 $[M+H]^+$ (Rt= 2.567 min) (Method B).

[Step 4] (2R,11aR)-7-Chloro-6-((1-fluorocyclopropyl)methoxy)-8-methyl-5-oxo-2,3,11,11a-tetrahydro-1H,5H-benzo[f]pyrrolo[2,1-c][1,4]oxazepin-2-yl benzoate

The same procedures as those in Step 1 of Reference Example 51 were carried out except that the compound obtained in Step 3 (80.0 mg, 0.21 mmol) and (1-fluorocyclopropyl)methanol (24.5 μL, 0.31 mmol) were used as raw materials, to thereby produce the title compound (66.0 mg).

$^1H$-NMR ($CDCl_3$) δ: 8.04 (2H, d, J = 7.3 Hz), 7.61 (1H, t, J = 7.3 Hz), 7.49 (2H, q, J = 7.6 Hz), 6.81 (1H, s), 5.71 (1H, t, J = 5.0 Hz), 4.55-4.47 (3H, m), 4.10-4.04 (4H, m), 2.53-2.46 (1H, m), 2.39 (3H, s), 2.05-2.04 (1H, m), 1.15-1.07 (3H, m), 0.90-0.85 (1H, m).
LCMS: 460.1 $[M+H]^+$ (Rt= 2.508 min) (Method B).

[Step 5] (2R,11aR)-7-chloro-6-((1-fluorocyclopropyl)methoxy)-2-hydroxy-8-methyl-2,3,11,11a-tetrahydro-1H,5H-benzo[f]pyrrolo[2,1-c][1,4]oxazepin-5-one

The compound obtained in Step 4 (66.0 mg, 0.14 mmol) was dissolved in ethanol (1 mL), combined with 2M sodium hydroxide aqueous solution (140 μL, 0.29 mmol), and agitated for 30 minutes at room temperature. The reaction mixture was combined with water, and extracted with ethyl acetate. The organic phase was washed twice with saline solution, dried with anhydrous magnesium sulfate, and concentrated under reduced pressure to thereby produce the title compound (52.0 mg).
LCMS: 356.1 $[M+H]^+$ (Rt= 2.011 min) (Method B).

[Step 6] (2R,11aR)-7-chloro-6-((1-fluorocyclopropyl)methoxy)-8-methyl-5-oxo-2,3,11,11a-tetrahydro-1H,5H-benzo[f]pyrrolo[2,1-c][1,4]oxazepin-2-yl methane sulfonate

The same procedures as those in Reference Example 115 were carried out except that the compound obtained in Step 5 (52.0 mg, 0.15 mmol) was used as a raw material, to thereby produce the title compound (52.1 mg).

$^1H$-NMR ($CDCl_3$) δ: 6.82 (1H, s), 5.42 (1H, t, J = 4.8 Hz), 4.52-4.46 (3H, m), 4.14-4.09 (2H, m), 4.00-3.95 (2H, m), 3.10 (3H, s), 2.44-2.41 (4H, m), 2.22 (1H, d, J = 15.6 Hz), 1.17 (1H, t, J = 4.8 Hz), 1.12 (1H, t, J = 5.0 Hz), 1.09-1.00 (1H, m), 0.93-0.83 (1H, m).
LCMS: 434.1 $[M+H]^+$ (Rt= 2.200 min) (Method B).

<Reference Example 118: (2R,11aR)-7-Chloro-6-(3-fluoropropoxy)-8-methyl-5-oxo-2,3,11,11a-tetrahydro-1H,5H-benzo[f]pyrrolo[2,1-c][1,4]oxazepin-2-yl methane sulfonate>

The same procedures as those in Step 4 to Step 6 of Reference Example 117 were carried out except that the compound obtained in Step 3 of Reference Example 117 (30.0 mg, 0.08 mmol) and 3-fluoro-1-propanol (8.72 μL, 0.12 mmol) were used as raw materials, to thereby produce the title compound (23.6 mg).

$^1H$-NMR ($CDCl_3$) δ: 6.81 (1H, s), 5.43 (1H, t, J = 5.0 Hz), 4.83-4.59 (2H, m), 4.43 (1H, t, J = 11.0 Hz), 4.36-4.31 (1H, m), 4.23-4.21 (1H, m), 4.11-3.93 (4H, m), 3.11 (3H, s), 2.50-2.42 (1H, m), 2.38 (3H, s), 2.27-2.16 (3H, m).
LCMS: 422.1 $[M+H]^+$ (Rt= 2.155 min) (Method B).

<Reference Example 119: (2R,11aR)-7-Chloro-6-isopropoxy-8-methyl-5-oxo-2,3,11,11a-tetrahydro-1H,5H-benzo[f]pyrrolo[2,1-c][1,4]oxazepin-2-yl methane sulfonate>

The same procedures as those in Step 5 of Reference Example 49 and Step 5 to Step 6 of Reference Example 117 were carried out except that the compound obtained in Step 3 of Reference Example 117 (140 mg, 0.36 mmol) and 2-iodopropane (86.1 μL, 0.87 mmol) were used as raw materials, to thereby produce the title compound (86.1 mg).

$^1H$-NMR ($CDCl_3$) δ: 7.88 (1H, s), 6.77 (1H, s), 5.43 (1H, t, J = 4.8 Hz), 4.54-4.48 (1H, m), 4.42 (1H, dd, J = 11.7, 10.3 Hz), 4.21 (1H, d, J = 14.2 Hz), 4.08-4.01 (2H, m), 3.93 (1H, dd, J = 14.6, 5.0 Hz), 3.11 (3H, s), 2.50-2.43 (1H, m), 2.38 (3H, s), 2.25 (1H, d, J = 15.1 Hz), 1.33 (3H, d, J = 5.9 Hz), 1.28 (3H, d, J = 6.4 Hz).
LCMS: 404.1 $[M+H]^+$ (Rt= 2.139 min) (Method B).

<Reference Example 120: (2R,11aR)-6-((2,2-Difluorocyclopropyl)methoxy)-7-fluoro-8-methyl-5-oxo-2,3,11,11a-tetrahydro-1H,5H-benzo[f]pyrrolo[2,1-c][1,4]oxazepin-2-yl methane sulfonate>

[Step 1] (2S,11aR)-2-(Benzyloxy)-6-((2,2-difluorocyclopropyl)methoxy)-7-fluoro-8-methyl-2,3,11,11a-tetrahydro-1H,5H-benzo[f]pyrrolo[2,1-c][1,4]oxazepin-5-one

The same procedures as those in Step 1 of Reference Example 51 were carried out except that the compound obtained in Step 4 of Reference Example 49 (50.0 mg, 0.14 mmol) and (2,2-difluorocyclopropyl)methanol (45.4 mg, 0.42 mmol) were used as raw materials, to thereby produce the title compound (52.5 mg).

$^1$H-NMR ($CDCl_3$) δ: 7.37-7.30 (5H, m), 6.64 (1H, d, J = 5.5 Hz), 4.57 (1H, d, J = 11.9 Hz), 4.49 (1H, dd, J = 11.7, 2.1 Hz), 4.32-4.29 (2H, m), 4.14-4.09 (1H, m), 4.04-4.01 (2H, m), 3.93-3.85 (3H, m), 2.26-2.07 (5H, m), 1.90-1.83 (1H, m), 1.53-1.39 (1H, m).
LCMS: 448.2 [M+H]$^+$ (Rt= 2.451 min) (Method B).

[Step 2] (2R,11aR)-6-((2,2-Difluorocyclopropyl)methoxy)-7-fluoro-8-methyl-5-oxo-2,3,11,11a-tetrahydro-1H,5H-benzo[f]pyrrolo[2,1-c][1,4]oxazepin-2-yl methane sulfonate

The same procedures as those in Step 5 to Step 7 of Reference Example 116 were carried out except that the compound obtained in Step 1 (52.5 mg, 0.12 mmol) were used as raw materials, to thereby produce the title compound (20.0 mg).

$^1$H-NMR ($CDCl_3$) δ: 6.70 (1H, d, J = 5.9 Hz), 5.42 (1H, t, J = 4.8 Hz), 4.43-4.34 (2H, m), 4.17-3.93 (5H, m), 3.10 (3H, s), 2.45-2.43 (1H, m), 2.28-2.15 (5H, m), 1.53-1.47 (1H, m), 1.40-1.30 (1H, m).
LCMS: 436.1 [M+H]$^+$ (Rt= 2.143 min) (Method B).

<Reference Example 121: (2S,11aR)-7-Fluoro-2-hydroxy-6-isopropoxy-2,3,11,11a-tetrahydro-1H,5H-benzo[f]pyrrolo[2,1-c][1,4]oxazepin-5-one>

The same procedures as those in Reference Example 55 were carried out except that the compound obtained in Reference Example 1 (249 mg, 1.19 mmol) was used as a raw material, to thereby produce the title compound (69.7 mg).

$^1$H-NMR ($CDCl_3$) δ: 7.08 (1H, dd, J = 10.5, 8.7 Hz), 6.74 (1H, dd, J = 8.9, 3.9 Hz), 4.65-4.59 (1H, m), 4.58-4.48 (1H, m), 4.16 (1H, dd, J = 9.6, 4.1 Hz), 4.08-3.92 (2H, m), 3.88-3.81 (2H, m), 2.17 (1H, ddd, J = 12.2, 7.2, 4.0 Hz), 2.04-1.83 (1H, br m), 1.87 (1H, ddd, J = 13.4, 5.3, 5.3 Hz), 1.36 (3H, d, J = 6.4 Hz), 1.29 (3H, d, J = 5.9 Hz).
LCMS: 296.1 [M+H]$^+$ (Rt= 1.722 min) (Method B).

<Reference Example 122: (2S,11aR)-2-Hydroxy-6-methoxy-8-methyl-7-propyl-2,3,11,11a-tetrahydro-1H,5H-benzo[f]pyrrolo[2,1-c][1,4]oxazepin-5-one>

[Step 1] 5-(Allyloxy)-2,2,7-trimethyl-4H-benzo[d][1,3]dioxin-4-one

The same procedures as those in Step 1 of Reference Example 1 were carried out except that the compound obtained in Step 1 of Reference Example 2 (1.04 g, 5.00 mmol) and allyl bromide (730 μL, 8.50 mmol) were used as raw materials, to thereby produce the title compound (1.27 g).

$^1$H-NMR ($CDCl_3$) δ: 6.41 (1H, s), 6.37 (1H, s), 6.13-6.04 (1H, m), 5.59 (1H, dd, J = 17.2, 1.6 Hz), 5.33 (1H, dd, J = 11.0, 1.4 Hz), 2.33 (3H, s), 1.69 (6H, s).
LCMS: 249.1 [M+H]$^+$ (Rt= 2.264 min) (Method B).

[Step 2] 6-Allyl-5-hydroxy-2,2,7-trimethyl-4H-benzo[d][1,3]dioxin-4-one

The compound obtained in Step 1 (200 mg, 0.81 mmol) was dissolved in N,N-diethyl aniline (806 μL), and agitated at 200°C for 3 hours. The reaction mixture was then cooled to room temperature, combined with 1M hydrochloric acid aqueous solution, and extracted three times with ethyl acetate. The organic phase was collected, washed with saturated ammonium chloride aqueous solution, dried with sodium sulfate, and concentrated under reduced pressure. The residue was purified with silica gel column chromatography (heptane/ethyl acetate) to thereby produce the title compound (135 mg).

$^1$H-NMR ($CDCl_3$) δ: 10.54 (1H, s), 6.29 (1H, s), 5.90 (1H, ddt, J = 16.8, 10.8, 5.2 Hz), 5.00 (1H, dd, J = 10.4, 2.0 Hz), 4.96 (1H, dd, J = 17.0, 2.0 Hz), 3.37 (2H, d, J = 5.9 Hz), 2.29 (3H, s), 1.73 (6H, s).
LCMS: 249.1 [M+H]$^+$ (Rt= 2.603 min) (Method B).

[Step 3] Methyl 3-allyl-6-hydroxy-2-methoxy-4-methyl benzoate

The same procedures as those in Reference Example 1 were carried out except that the compound obtained in Step 2 (135 mg, 0.54 mmol) and iodomethane (57.5 μL, 0.92 mmol) were used as raw materials, to thereby produce the title compound (116 mg). $^1$H-NMR ($CDCl_3$) δ: 11.12 (1H, s), 6.65 (1H, s), 5.93 (1H, ddt, J = 16.9, 10.2, 5.4 Hz), 5.01 (1H, ddt, J =

10.2, 1.8, 1.8 Hz), 4.87 (1H, ddt, J = 16.9, 1.8, 1.8 Hz), 3.99 (3H, s), 3.72 (3H, s), 3.36 (2H, ddd, J = 5.4, 1.8, 1.8 Hz), 2.26 (3H, s).
LCMS: 237.1 $[M+H]^+$ (Rt= 2.374 min) (Method B).

[Step 4] (2S,11aR)-2-hydroxy-6-methoxy-8-methyl-7-propyl-2,3,11,11a-tetrahydro-1H,5H-benzo[f]pyrrolo[2,1-c][1,4] oxazepin-5-one

The same procedures as those in Step 2 to Step 6 of Reference Example 27 were carried out except that the compound obtained in Step 3 (130 mg, 0.55 mmol) and the compound obtained in Reference Example 25 (200 mg, 0.66 mmol) were used as raw materials, to thereby produce the title compound (38.9 mg).

$^1$H-NMR ($CDCl_3$) δ: 6.64 (1H, s), 4.63-4.56 (1H, m), 4.16 (1H, dd, J = 9.6, 4.6 Hz), 4.13-4.05 (1H, m), 4.00-3.90 (2H, m), 3.83 (3H, s), 3.80 (1H, dd, J = 12.6, 4.8 Hz), 2.65-2.49 (2H, m), 2.29 (3H, s), 2.20-2.12 (1H, m), 1.84 (1H, ddd, J = 12.2, 6.1, 6.1 Hz), 1.55-1.45 (2H, m), 0.99 (3H, t, J = 7.3 Hz).
LCMS: 306.2 $[M+H]^+$ (Rt= 1.953 min) (Method B).

<Reference Example 123: (2S,11aR)-7-Fluoro-2-hydroxy-8-methyl-6-((1,1,1-trifluoro-2-methylpropan-2-yl) oxy)-2,3,11,11a-tetrahydro-1H,5H-benzo[f]pyrrolo[2,1-c][1,4]oxazepin-5-one>

[Step 1] 1,2,3-Trifluoro-5-(methoxymethoxy)benzene

The same procedures as those in Step 3 of Reference Example 102 were carried out except that 3,4,5-trifluorophenol (4.44 g, 30.0 mmol) was used as a raw material, to thereby produce the title compound (5.68 g).
$^1$H-NMR ($CDCl_3$) δ: 6.73-6.64 (2H, m), 5.10 (2H, s), 3.46 (3H, s).

[Step 2] 1-(Benzyloxy)-2,3-difluoro-5-(methoxymethoxy)benzene

A mixture of the compound obtained in Step 1 (5.68 g, 29.6 mmol), potassium carbonate (16.3 g, 118 mmol), and toluene (59.1 mL) was combined with 18-crown-6-ether (19.5 g, 73.9 mmol) and benzylalcohol (6.09 mL, 59.1 mmol), and agitated at 110°C overnight. The reaction mixture was then cooled to room temperature, combined with water, and extracted with dichloromethane. The organic phase was dried with anhydrous magnesium sulfate, and concentrated under reduced pressure. The residue was purified with silica gel column chromatography (heptane/ethyl acetate) to thereby produce the title compound (6.82 g).
$^1$H-NMR ($CDCl_3$) δ: 7.44-7.32 (5H, m), 6.52-6.49 (2H, m), 5.12 (2H, s), 5.07 (2H, s), 3.45 (3H, s).

[Step 3] 2,3-Difluoro-5-(methoxymethoxy)phenol

The same procedures as those in Step 5 of Reference Example 33 were carried out except that the compound obtained in Step 2 (6.82 g, 24.3 mmol) was used as a raw material, to thereby produce the title compound (4.68 g).
$^1$H-NMR ($CDCl_3$) δ: 6.51-6.42 (2H, m), 5.48 (1H, br s), 5.09 (2H, s), 3.46 (3H, s).

[Step 4] 2,3-Difluoro-5-(methoxymethoxy)phenyl trifluoromethane sulfonate

The same procedures as those in Step 1 of Reference Example 12 were carried out except that the compound obtained in Step 3 (4.68 g, 24.6 mmol) was used as a raw material, to thereby produce the title compound (7.19 g).
$^1$H-NMR ($CDCl_3$) δ: 6.98-6.92 (1H, m), 6.85-6.82 (1H, m), 5.14 (2H, s), 3.48 (3H, s).

[Step 5] 1,2-Difluoro-5-(methoxymethoxy)-3-methyl benzene

The compound obtained in Step 4 (3.92 g, 12.2 mmol), trimethylboroxin (2.04 mL, 14.6 mmol), and potassium carbonate (5.04 g, 36.5 mmol) were dissolved in 1,4-dioxane (60.8 mL), combined with tetrakis(triphenylphosphine) palladium (0) (702 mg, 608 μmol), and agitated under nitrogen atmosphere at 110°C overnight. The reaction mixture was then cooled to room temperature, combined with water, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (1.96 g).
$^1$H-NMR ($CDCl_3$) δ: 6.74-6.68 (1H, m), 6.63-6.60 (1H, m), 5.10 (2H, s), 3.47 (3H, s), 2.27 (3H, d, J = 2.3 Hz).

[Step 6] tert-Butyl 2,3-difluoro-6-(methoxymethoxy)-4-methyl benzoate

The compound obtained in Step 5 (1.96 g, 10.4 mmol) was dissolved in THF (41.6 mL), and to the resulting solution, 1.59M n-butyl lithium /hexane solution (7.86 mL, 12.5 mmol) was added dropwise at -78°C. After the drop was finished, the reaction mixture was agitated at -78°C for 2 hours, combined with di-tert-butyl bicarbonate (3.11 mL, 13.5 mmol), brought to room temperature, and agitated overnight. The reaction mixture was combined with saturated ammonium chloride aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (3.05 g).
$^1$H-NMR ($CDCl_3$) $\delta$: 6.71 (1H, dd, J = 5.3, 1.6 Hz), 5.14 (2H, s), 3.49 (3H, s), 2.28 (3H, d, J = 2.3 Hz), 1.58 (9H, s).

[Step 7] tert-Butyl 3-fluoro-6-(methoxymethoxy)-4-methyl-2-((1,1,1-trifluoro-2-methylpropan-2-yl)oxy)benzoate

To a mixture of 60% sodium hydride (575 mg, 15.0 mmol) and DMF (10.0 mL), 1,1,1-trifluoro-2-methylpropan-2-ol (1.94 mL, 18.0 mmol) was added dropwise, and the mixture was agitated for 1 hour at room temperature. The resulting reaction mixture was combine with the compound obtained in Step 6 (865 mg, 3.00 mmol), and agitated at 100°C overnight. The reaction mixture was then cooled to room temperature, combined with saturated ammonium chloride aqueous solution, extracted twice with heptane/ethyl acetate (1/1). The organic phase was collected, washed with saturated saline solution, dried with anhydrous magnesium sulfate, and concentrated under reduced pressure. The residue was purified with silica gel column chromatography (heptane/ethyl acetate), to thereby produce a crude product of the title compound.
LCMS: 341.1 $[M+H-tBu]^+$ (Rt= 2.722 min) (Method B).

[Step 8] 3-Fluoro-6-hydroxy-4-methyl-2-((1,1,1-trifluoro-2-methylpropan-2-yl)oxy)benzoic acid

The crude product of the compound obtained in Step 7 was dissolved in methanol (30.0 mL), combined with trimethylsilyl chloride (1.90 mL, 15.0 mmol), and refluxed with heating overnight. The reaction mixture was then cooled to room temperature, and concentrated under reduced pressure. The residue was purified with silica gel column chromatography (heptane/ethyl acetate) to thereby produce the title compound (126 mg). LCMS: 297.1 $[M+H]^+$ (Rt= 2.190 min) (Method B).

[Step 9] (2S,11aR)-7-fluoro-2-hydroxy-8-methyl-6-((1,1,1-trifluoro-2-methylpropan-2-yl)oxy)-2,3,11,11a-tetrahydro-1H,5H-benzo[f]pyrrolo[2,1-c][1,4]oxazepin-5-one

The same procedures as those in Step 2 to Step 6 of Reference Example 27 were carried out except that the compound obtained in Step 8 (324 mg, 1.09 mmol) and the compound obtained in Reference Example 25 (1.01 g, 3.28 mmol) were used as raw materials, to thereby produce the title compound (238 mg).
LCMS: 378.1 $[M+H]^+$ (Rt= 1.962 min) (Method B).

<Reference Example 124: N-Benzyl-2,3-difluoro-5-iodopyridin-4-amine>

A mixture of the compound obtained in Step 1 of Reference Example 87 (1.28 g, 5.00 mmol), potassium carbonate (1.38 g, 10.0 mmol), and DMF (10.0 mL) was combined with benzyl bromide (0.90 mL, 7.50 mmol), and agitated at 60°C overnight. The reaction mixture was then cooled to room temperature, combined with water, and extracted twice with heptane/ethyl acetate (1/1). The organic phase was collected and dried with anhydrous magnesium sulfate, and, and concentrated under reduced pressure. The residue was purified with silica gel column chromatography (heptane/ethyl acetate) to thereby produce the title compound (541 mg).
LCMS: 347.0 $[M+H]^+$ (Rt= 2.428 min).

<Reference Example 125: (8aR,10S)-10-Hydroxy-5-methyl-3,4,8a,9,10,11-hexahydro-2H,8H,13H-chromeno[8,7-f]pyrrolo[2,1-c][1,4]oxazepin-13-one>

[Step 1] 5-Hydroxy-6-(3-hydroxy propyl)-2,2,7-trimethyl-4H-benzo[d][1,3]dioxin-4-one

The compound obtained in Step 2 of Reference Example 122 (1.00 g, 4.03 mmol) was dissolved in THF (20.0 mL), combined with 1M borane-THF/THF solution (4.84 mL, 4.84 mmol), and agitated for 2 hours at room temperature. The reaction mixture was combined with sodium peroxoborate tetrahydrate (930 mg, 6.05 mmol) and saturated

ammonium chloride aqueous solution, and extracted three times with ethyl acetate. The organic phase was collected, washed with saturated ammonium chloride aqueous solution, dried with sodium sulfate, and concentrated under reduced pressure. The residue was purified with silica gel column chromatography (heptane/ethyl acetate) to thereby produce the title compound (620 mg).

$^1$H-NMR ($CDCl_3$) δ: 10.71 (1H, s), 6.31 (1H, s), 3.61 (2H, dt, J = 6.1, 6.1 Hz), 2.73 (2H, t, J = 7.3 Hz), 2.32 (3H, s), 2.06 (1H, t, J = 5.5 Hz), 1.77 (2H, tt, J = 7.3, 6.1 Hz), 1.73 (6H, s).
LCMS: 249.1 $[M+H-H_2O]^+$ (Rt= 2.128 min) (Method B).

[Step 2] 5,8,8-Trimethyl-3,4-dihydro-2H,10H-[1,3]dioxyno[4,5-h]chromen-10-one

The compound obtained in Step 1 (551 mg, 2.07 mmol) and triphenylphosphine (652 mg, 2.48 mmol) were dissolved in THF (6.90 mL), and to the resulting solitions, DIAD (483 μL, 2.48 mmol) was added dropwise. After the drop was finished, the reaction mixture was agitated for 5 hours at room temperature, combined with saturated sodium bicarbonate aqueous solution, and extracted with ethyl acetate twice. The organic phase was collected, washed with saturated saline solution, dried with sodium sulfate, and concentrated under reduced pressure. The residue was purified with silica gel column chromatography (heptane/ethyl acetate) to thereby produce the title compound (349 mg). $^1$H-NMR ($CDCl_3$) δ: 6.35 (1H, s), 4.32 (2H, dd, J = 5.6, 5.2 Hz), 2.60 (2H, t, J = 6.6 Hz), 2.22 (3H, s), 2.10-2.01 (2H, m), 1.68 (6H, s).
LCMS: 249.1 $[M+H]^+$ (Rt= 2.131 min) (Method B).

[Step 3] Benzyl 7-hydroxy-5-methyl chroman-8-carboxylate

The same procedures as those in Step 2 of Reference Example 42 were carried out except that the compound obtained in Step 2 (349 mg, 1.41 mmol) was used as a raw material, to thereby produce the title compound (273 mg).

$^1$H-NMR ($CDCl_3$) δ: 11.22 (1H, s), 7.49-7.27 (5H, m), 6.40 (1H, s), 5.41 (2H, s), 4.20 (2H, dd, J = 5.0, 5.0 Hz), 2.57 (2H, t, J = 6.9 Hz), 2.17 (3H, s), 2.06-1.98 (2H, m).
LCMS: 299.1 $[M+H]^+$ (Rt= 2.573 min) (Method B).

[Step 4] tert-Butyl (2R,4S)-2-(((8-((benzyloxy)carbonyl)-5-methyl chroman-7-yl)oxy)methyl)-4-((tert-butyldiphenylsilyl)oxy)pyrrolidin-1-carboxylate

The same procedures as those in Step 2 of Reference Example 27 were carried out except that the compound obtained in Step 3 (273 mg, 0.92 mmol) and the compound obtained in Reference Example 22 (501 mg, 1.10 mmol) were used as raw materials, to thereby produce the title compound (534 mg).
LCMS: 636.4 $[M+H-Boc]^+$ (Rt= 3.497 min) (Method B).

[Step 5] 7-(((2R,4S)-1-(tert-Butoxycarbonyl)-4-((tert-butyldiphenylsilyl)oxy)pyrrolidin-2-yl)methoxy)-5-methyl chroman-8-carbonic acid

The same procedures as those in Step 4 of Reference Example 42 were carried out except that the compound obtained in Step 4 (534 mg, 0.73 mmol) was used as a raw material, to thereby produce a crude product of the title compound.
LCMS: 546.3 $[M+H-Boc]^+$ (Rt= 3.019 min) (Method B).

[Step 6] 7-(((2R,4S)-4-((tert-butyldiphenylsilyl)oxy)pyrrolidin-2-yl)methoxy)-5-methyl chroman-8-carbonic acid

The crude product of the compound obtained in Step 5 was dissolved in dichloromethane (0.73 mL), and to the resulting solution, trifluoroacetic acid (0.73 mL) was added at 0°C, and the mixture was agitated at 0°C for 1 hour. The reaction mixture was concentrated under reduced pressure, the residue was azeotroped with toluene to thereby produce a crude product of the title compound.
LCMS: 546.3 $[M+H]^+$ (Rt= 2.323 min) (Method B).

[Step 7] (8aR,10S)-10-((tert-Butyldiphenylsilyl)oxy)-5-methyl-3,4,8a,9,10,11-hexahydro-2H,8H,13H-chromeno[8,7-f]pyrrolo[2,1-c][1,4]oxazepin-13-one

The same procedures as those in Step 5 of Reference Example 27 were carried out except that the crude product of the compound obtained in Step 6 was used as a raw material, to thereby produce the title compound (107 mg).

$^1$H-NMR ($CDCl_3$) δ: 7.67-7.61 (4H, m), 7.48-7.35 (6H, m), 6.41 (1H, s), 4.52-4.44 (1H, m), 4.35 (1H, ddd, J = 10.0, 6.0, 2.8 Hz), 4.12-4.05 (1H, m), 4.03-3.95 (1H, m), 3.92 (1H, dd, J = 10.4, 4.8 Hz), 3.83 (1H, dd, J = 12.1, 6.2 Hz), 3.74-3.66 (2H, m), 2.67-2.54 (2H, m), 2.17 (3H, s), 2.15-2.06 (2H, m), 2.04-1.94 (1H, m), 1.63 (1H, ddd, J = 12.8, 6.0, 3.2 Hz), 1.03 (9H, s).
LCMS: 528.3 $[M+H]^+$ (Rt= 2.922 min) (Method B).

[Step 8] (8aR,10S)-10-Hydroxy-5-methyl-3,4,8a,9,10,11-hexahydro-2H,8H,13H-chromeno[8,7-f]pyrrolo[2,1-c][1,4]oxazepin-13-one

The same procedures as those in Step 6 of Reference Example 42 were carried out except that the compound obtained in Step 7 (107 mg, 0.20 mmol) was used as a raw material, to thereby produce the title compound (50.4 mg).

$^1$H-NMR ($CDCl_3$) δ: 6.46 (1H, s), 4.63-4.57 (1H, m), 4.32 (1H, ddd, J = 10.4, 6.8, 3.6 Hz), 4.16-4.05 (3H, m), 4.00-3.92 (1H, m), 3.91-3.82 (2H, m), 2.67-2.54 (2H, m), 2.18 (3H, s), 2.17-1.95 (3H, m), 1.86 (1H, ddd, J = 13.3, 5.0, 5.0 Hz).
LCMS: 290.1 $[M+H]^+$ (Rt= 1.671 min) (Method B).

<Example 1: (2S,11aR)-6-isopropoxy-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

[Step 1] tert-Butyl (2R,4S)-2-((3-isopropoxy-2-(methoxycarbonyl)phenoxy)methyl)-4-((2-oxo-1,2,3,4-tetrahydroquinolin-7-yl)oxy)pyrrolidin-1-carboxylate

The compound obtained in Reference Example 1 (40.6 mg, 0.19 mmol), the compound obtained in Reference Example 83 (50.0 mg, 0.14 mmol), and triphenylphosphine (50.7 mg, 0.19 mmol) were dissolved in THF (1 mL), and to the resulting solution, DIAD (37.6 μL, 0.19 mmol) was added dropwise. After the drop was finished, the mixture was agitated at 40°C overnight, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (64.7 mg). LCMS: 455.3 $[M+H-Boc]^+$ (Rt= 2.394 min).

[Step 2] 2-(((2R,4S)-1-(tert-Butoxycarbonyl)-4-((2-oxo-1,2,3,4-tetrahydroquinolin-7-yl)oxy)pyrrolidin-2-yl)methoxy)-6-isopropoxybenzoic acid

The compound obtained in Step 1 (64.7 mg, 0.12 mmol) was dissolved in a mixture solvent of methanol (583 μL), THF (583 μL), and water (583 μL), combined with potassium hydroxide (98.2 mg, 1.75 mmol), and agitated at 90°C overnight. The reaction mixture was then cooled to room temperature, combined with 1M hydrochloric acid aqueous solution, and extracted with ethyl acetate. The organic phase was washed twice with saturated saline solution, dried with anhydrous magnesium sulfate, and concentrated under reduced pressure to thereby produce the title compound (69.7 mg).
LCMS: 441.2 $[M+H-Boc]^+$ (Rt= 2.242 min).

[Step 3] 2-isopropoxy-6-(((2R,4S)-4-((2-oxo-1,2,3,4-tetrahydroquinolin-7-yl)oxy)pyrrolidin-2-yl)methoxy)benzoic acid hydrochloride

The compound obtained in Step 2 (69.7 mg, 0.13 mmol) was dissolved in 1,4-dioxane (645 μL), and to the resulting solution, 4M hydrochloric acid/1,4-dioxane solution (645 μL, 2.58 mmol) was added dropwise. After the drop was finished, the mixture was agitated for 1 hour at room temperature. The reaction mixture was concentrated under reduced pressure to thereby produce the title compound (55.8 mg).
LCMS: 441.2 $[M+H]^+$ (Rt= 1.739 min).

[Step 4] (2S,11aR)-6-Isopropoxy-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one

The compound obtained in Step 3 (55.8 mg, 0.12 mmol) and N,N-diisopropylethylamine (202 μL,1.17 mmol) were dissolved in DMF (2.34 mL), combined with WSC-HCl (33.6 mg, 0.18 mmol) and HOBt (26.9g, 0.18 mmol), and agitated at 40°C for 2.5 hours. The reaction mixture was then cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The

residue was purified with silica gel column chromatography (ethyl acetate/methanol) to thereby produce the title compound (14.7 mg).

$^1$H-NMR ($CDCl_3$) δ: 7.67 (1H, s), 7.30-7.28 (1H, m), 7.06 (1H, d, J = 8.2 Hz), 6.81 (1H, d, J = 8.2 Hz), 6.67 (1H, d, J = 8.2 Hz), 6.49 (1H, dd, J = 8.2, 2.7 Hz), 6.28 (1H, d, J = 2.3 Hz), 4.98-4.94 (1H, m), 4.57-4.48 (1H, m), 4.22-4.15 (2H, m), 4.03-3.99 (3H, m), 2.91 (2H, t, J = 7.5 Hz), 2.62 (2H, t, J = 7.5 Hz), 2.43-2.40 (1H, m), 2.07-2.00 (1H, m), 1.38 (3H, d, J = 5.9 Hz), 1.32 (3H, d, J = 6.4 Hz).
LCMS: 423.2 $[M+H]^+$ (Rt= 2.077 min).

<Example 2: (2S,11aR)-6-(Benzyloxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 2 (120 mg, 0.44 mmol) and the compound obtained in Reference Example 83 (133 mg, 0.37 mmol) were used as raw materials, to thereby produce the title compound (110 mg).

$^1$H-NMR ($CDCl_3$) δ: 7.47 (2H, d, J = 6.9 Hz), 7.35 (2H, t, J = 7.1 Hz), 7.30-7.29 (2H, m), 7.02 (1H, d, J = 8.2 Hz), 6.64 (1H, s), 6.52-6.47 (2H, m), 6.22 (1H, d, J = 2.3 Hz), 5.23 (1H, d, J = 12.0 Hz), 5.12 (1H, d, J = 12.4 Hz), 4.98-4.93 (1H, m), 4.21-3.94 (5H, m), 2.89 (2H, t, J = 7.5 Hz), 2.61 (2H, t, J = 7.3 Hz), 2.44-2.37 (1H, m), 2.30 (3H, s), 2.04-2.00 (1H, m).
LCMS: 485.3 $[M+H]^+$ (Rt= 2.216 min).

<Example 3: (2S,11aR)-6-Hydroxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The compound obtained in Example 2 (105 mg, 0.22 mmol) was dissolved in ethanol (2.2 mL), combined with 10% palladium-activated carbon (10.5 mg), and agitated overnight under hydrogen atmosphere at normal pressure at room temperature. The reaction mixture was combined with dichloromethane, filtered with cerite, and the filtrate was concentrated under reduced pressure. The residue was purified with silica gel column chromatography (ethyl acetate/-methanol) to thereby produce the title compound (74.6 mg). $^1$H-NMR ($CDCl_3$) δ: 13.51 (1H, s), 7.43 (1H, s), 7.08 (1H, d, J = 8.2 Hz), 6.49 (1H, d, J = 2.4 Hz), 6.47 (1H, s), 6.29 (1H, d, J = 1.6 Hz), 6.25 (1H, d, J = 2.4 Hz), 4.93-4.92 (1H, m), 4.48 (1H, dd, J = 11.9, 1.8 Hz), 4.29-4.26 (1H, m), 4.07-3.96 (3H, m), 2.91 (2H, t, J = 7.5 Hz), 2.62 (2H, t, J = 7.5 Hz), 2.47 (1H, dd, J = 13.5, 6.2 Hz), 2.24 (3H, s), 1.92-1.88 (1H, m).
LCMS: 395.2 $[M+H]^+$ (Rt= 2.204 min).

<Example 4: (2S,11aR)-6-Ethoxy-8-methyl-2-((2-oxo-1,2,3,4-tetrahydroquinolin-7-yl)oxy-2,3,11,11a-tetrahydro-1H,5H-benzo[f]pyrrolo[2,1-c][1,4]oxazepin-5-one>

The same procedures as those in Example 1 were carried out except that the compound obtained in Reference Example 3 (97.5 mg, 0.47 mmol) and the compound obtained in Reference Example 83 (53.5 mg, 0.33 mmol) were used as raw materials, to thereby produce the title compound (121 mg).

$^1$H-NMR ($CDCl_3$) δ: 7.97 (1H, s), 7.05 (1H, d, J = 8.7 Hz), 6.58 (1H, s), 6.50-6.48 (2H, m), 6.32 (1H, d, J = 2.3 Hz), 4.94-4.92 (1H, m), 4.19-3.97 (7H, m), 2.90 (2H, t, J = 7.5 Hz), 2.62 (2H, t, J = 7.3 Hz), 2.43-2.36 (1H, m), 2.32 (3H, s), 2.05-1.99 (1H, m), 1.41 (3H, t, J = 7.1 Hz).
LCMS: 423.2 $[M+H]^+$ (Rt= 2.072 min).

<Example 5: (2S,11aR)-6-Isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 4 (170 mg, 0.76 mmol) and the compound obtained in Reference Example 83 (250 mg, 0.69 mmol) were used as raw materials, to thereby produce the title compound (240 mg).

$^1$H-NMR ($CDCl_3$) δ: 7.88 (1H, s), 7.05 (1H, d, J = 8.2 Hz), 6.62 (1H, s), 6.48 (2H, t, J = 5.5 Hz), 6.30 (1H, d, J = 2.3 Hz), 4.97-4.92 (1H, m), 4.55-4.46 (1H, m), 4.19-3.94 (5H, m), 2.90 (2H, t, J = 7.5 Hz), 2.62 (2H, t, J = 7.3 Hz), 2.43-2.36 (1H, m), 2.31 (3H, s), 2.01 (1H, dt, J = 13.6, 5.3 Hz), 1.37 (3H, d, J = 6.4 Hz), 1.31 (3H, d, J = 5.9 Hz).
LCMS: 437.2 $[M+H]^+$ (Rt= 2.124 min).

<Example 6: (2S,11aS)-6-Isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

[Step 1] tert-Butyl (2S,4R)-4-((tert-butyldiphenylsilyl)oxy)-2-(((2,2,7-trimethyl-4-oxo-4H-benzo[d][1,3]dioxin-5-yl)oxy) methyl)pyrrolidin-1-carboxylate

The compound obtained in Step 1 of Reference Example 2 (271 mg, 1.30 mmol), the compound obtained in Reference Example 21 (456 mg, 1.00 mmol), and triphenylphosphine (315 mg, 1.20 mmol) were dissolved in THF (5 mL), and to the resulting solution, DIAD (234 μL, 1.20 mmol) was added dropwise at 0°C. After the drop was finished, the mixture was agitated overnight at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate), to thereby produce a crude product of the title compound.
LCMS: 546.3 $[M+H-Boc]^+$ (Rt= 3.051 min).

[Step 2] tert-Butyl (2S,4R)-4-((tert-butyldiphenylsilyl)oxy)-2-((3-hydroxy-2-(methoxycarbonyl)-5-methylphenoxy) methyl)pyrrolidin-1-carboxylate

The crude product of the compound obtained in Step 1 was dissolved in a mixture solvent of methanol (3.33 mL) and THF (3.33 mL), combined with potassium carbonate (415 mg, 3.00 mmol), and agitated for 4 hours at room temperature. The reaction mixture was combined with 2M hydrochloric acid aqueous solution, and extracted with dichloromethane. The organic phase was dried with anhydrous magnesium sulfate, and concentrated under reduced pressure. The residue was purified with silica gel column chromatography (heptane/ethyl acetate) to thereby produce the title compound (531 mg). LCMS: 520.3 $[M+H-Boc]^+$ (Rt= 3.054 min).

[Step 3] tert-Butyl (2S,4R)-4-((tert-butyldiphenylsilyl)oxy)-2-((3-isopropoxy-2-(methoxycarbonyl)-5-methylphenoxy) methyl)pyrrolidin-1-carboxylate

The compound obtained in Step 2 (531 mg, 0.86 mmol) and potassium carbonate (237 mg, 1.71 mmol) were dissolved in DMF (2.86 mL), combined with 2-iodopropane (171 μL, 1.71 mmol), and agitated at 80°C overnight. The reaction mixture was then cooled to room temperature, combined with water, and extracted with heptane/ethyl acetate (3/1). The organic phase was washed twice with saturated saline solution, dried with anhydrous magnesium sulfate, and concentrated under reduced pressure. The residue was purified with silica gel column chromatography (heptane/ethyl acetate), to thereby produce a crude product of the title compound.
LCMS: 562.4 $[M+H-Boc]^+$ (Rt= 3.014 min).

[Step 4] tert-Butyl (2S,4R)-4-hydroxy-2-((3-isopropoxy-2-(methoxycarbonyl)-5-methylphenoxy)methyl)pyrrolidin-1-carboxylate

The crude product of the compound obtained in Step 3 was dissolved in THF (3.43 mL), and to the resulting solution, 1M tetrabutyl ammonium fluoride/THF solution (1.71 mL, 1.71 mmol) was added dropwise. After the drop was finished, the mixture was agitated for 3 days at room temperature. The reaction mixture was combined with saturated ammonium chloride aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (313 mg). LCMS: 324.2 $[M+H-Boc]^+$ (Rt= 2.268 min).

[Step 5] tert-Butyl (2S,4S)-2-((3-isopropoxy-2-(methoxycarbonyl)-5-methylphenoxy)methyl)-4-((2-oxo-1,2,3,4-tetrahydroquinolin-7-yl)oxy)pyrrolidin-1-carboxylate

The compound obtained in Step 4 (313 mg, 0.74 mmol), 7-hydroxy-3,4-dihydroquinolin-2(1H)-one (169 mg, 1.04 mmol), and triphenylphosphine (252 mg, 0.96 mmol) were dissolved in THF (3.70 mL), and to the resulting solution, DIAD (187 μL, 0.96 mmol) was added dropwise at 0°C. After the drop was finished, the mixture was agitated overnight at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (360 mg). LCMS: 469.3 $[M+H-Boc]^+$ (Rt= 2.428 min).

[Step 6] (2S,11aS)-6-Isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one

The same procedures as those in Step 2 to Step 4 of Example 1 were carried out except that the compound obtained in Step 5 (360 mg, 0.65 mmol) was used as a raw material, to thereby produce the title compound (127 mg).

$^1$H-NMR (DMSO-d6) δ: 10.01 (1H, s), 7.09 (1H, d, J = 8.2 Hz), 6.73 (1H, s), 6.53-6.51 (1H, m), 6.49 (1H, s), 6.45 (1H, s), 5.02 (1H, s), 4.60-4.51 (1H, m), 4.24 (1H, t, J = 10.7 Hz), 4.00-3.98 (1H, m), 3.92-3.88 (1H, m), 3.81-3.75 (2H, m), 2.79 (2H, t, J = 7.5 Hz), 2.44-2.42 (3H, m), 2.27 (3H, s), 1.89 (1H, d, J = 14.6 Hz), 1.23-1.21 (6H, m).
LCMS: 437.2 $[M+H]^+$ (Rt= 2.144 min).

<Example 7: (2S,11aR)-6-(((S)-1-fluoropropan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

[Step 1] tert-Butyl (2R,4S)-2-((3-(((S)-1-((tert-butyldiphenylsilyl)oxy)propan-2-yl)oxy)-2-(methoxycarbonyl)-5-methylphenoxy)methyl)-4-((2-oxo-1,2,3,4-tetrahydroquinolin-7-yl)oxy)pyrrolidin-1-carboxylate

The compound obtained in Reference Example 5 (300 mg, 0.83 mmol), the compound obtained in Reference Example 83 (436 mg, 0.91 mmol), and triphenylphosphine (261 mg, 0.99 mmol) were dissolved in THF (2.49 mL), and to the resulting solution, DIAD (193 μL, 0.99 mmol) was added dropwise. After the drop was finished, the mixture was agitated at 60°C for 1 hour. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (548 mg).
LCMS: 823.4 $[M+H]^+$ (Rt= 2.924 min).

[Step 2] tert-Butyl (2R,4S)-2-((3-(((S)-1-hydroxy propan-2-yl)oxy)-2-(methoxycarbonyl)-5-methylphenoxy)methyl)-4-((2-oxo-1,2,3,4-tetrahydroquinolin-7-yl)oxy)pyrrolidin-1-carboxylate

The compound obtained in Step 1 (548 mg, 0.67 mmol) was dissolved in THF (2 mL), and to the resulting solution, 1M tetrabutyl ammonium fluoride/THF solution (1.00 mL, 1.00 mmol) was added dropwise. After the drop was finished, the mixture was agitated for 1 hour at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (355 mg).
LCMS: 585.3 $[M+H]^+$ (Rt= 2.252 min).

[Step 3] tert-Butyl (2R,4S)-2-((3-(((S)-1-fluoropropan-2-yl)oxy)-2-(methoxycarbonyl)-5-methylphenoxy)methyl)-4-((2-oxo-1,2,3,4-tetrahydroquinolin-7-yl)oxy)pyrrolidin-1-carboxylate

The same procedures as those in Step 3 of Reference Example 36 were carried out except that the compound obtained in Step 2 (139 mg, 0.24 mmol) was used as a raw material, to thereby produce the title compound (43.8 mg).
LCMS: 487.2 $[M+H-Boc]^+$ (Rt= 2.413 min).

[Step 4] (2S,11aR)-6-(((S)-1-Fluoropropan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one

The same procedures as those in Step 2 to Step 4 of Example 1were carried out except that the compound obtained in Step 3 (43.8 mg, 0.75 mmol) was used as a raw material, to thereby produce the title compound (9.5 mg).

$^1$H-NMR ($CDCl_3$) δ: 7.64 (1H, s), 7.05 (1H, d, J = 8.2 Hz), 6.69 (1H, s), 6.57 (1H, s), 6.48 (1H, dd, J = 8.2, 2.3 Hz), 6.26 (1H, d, J = 2.3 Hz), 4.96-4.92 (1H, m), 4.68-4.44 (3H, m), 4.22-4.00 (4H, m), 3.88 (1H, dd, J = 13.0, 4.8 Hz), 2.90 (2H, t, J = 7.5 Hz), 2.61 (2H, t, J = 7.5 Hz), 2.44-2.38 (1H, m), 2.32 (3H, s), 1.99 (1H, dt, J = 13.3, 5.5 Hz), 1.35 (3H, dd, J = 6.2, 1.1 Hz).
LCMS: 455.2 $[M+H]^+$ (Rt= 2.101 min).

<Example 8: (2S,11aR)-6-(((S)-1,1-Difluoropropan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

[Step 1] tert-Butyl (2R,4S)-2-((2-(methoxycarbonyl)-5-methyl-3-(((S)-1-oxopropan-2-yl)oxy)phenoxy)methyl)-4-((2-oxo-1,2,3,4-tetrahydroquinolin-7-yl)oxy)pyrrolidin-1-carboxylate

The compound obtained in Step 2 of Example 7 (120 mg, 0.21 mmol) was dissolved in dichloromethane (1.00 mL), combined with Dess-Martin Periodinane (104 mg, 0.25 mmol), and agitated for 2 hours at room temperature. The reaction mixture was combined with saturated sodium bicarbonate aqueous solution and sodium thiosulfate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (75.7 mg).

$^1$H-NMR ($CDCl_3$) δ: 9.71 (1H, s), 7.56-7.49 (1H, m), 7.07-7.04 (1H, m), 6.55-6.40 (3H, m), 6.24-6.22 (1H, m), 5.03-4.58 (2H, m), 4.45-4.18 (2H, m), 4.06-3.48 (6H, m), 2.91 (2H, t, J = 7.1 Hz), 2.63 (2H, t, J = 7.3 Hz), 2.46-2.40 (1H, m), 2.32-2.18 (4H, m), 1.48-1.44 (12H, m).
LCMS:583.3 $[M+H]^+$ (Rt= 2.257 min).

[Step 2] tert-Butyl (2R,4S)-2-((3-(((S)-1,1-difluoropropan-2-yl)oxy)-2-(methoxycarbonyl)-5-methylphenoxy)methyl)-4-((2-oxo-1,2,3,4-tetrahydroquinolin-7-yl)oxy)pyrrolidin-1-carboxylate

The compound obtained in Step 1 (75.7 mg, 0.13 mmol) was dissolved in dichloromethane (2.60 mL), and to the resulting solution, bis(2-methoxyethyl)aminosulfur trifluoride (68.4 μL, 0.39 mmol) was added at 0°C, and agitated at 0°C for 3.5 hours. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (45.9 mg).

$^1$H-NMR ($CDCl_3$) δ: 7.57-7.51 (1H, m), 7.07-7.03 (1H, m), 6.55-6.51 (1H, m), 6.45-6.38 (3H, m), 5.81 (1H, td, J = 55.7, 3.8 Hz), 5.01-4.94 (1H, m), 4.51-3.94 (4H, m), 3.84-3.82 (3H, m), 3.72-3.47 (2H, m), 2.92-2.88 (2H, m), 2.64-2.60 (2H, m), 2.45-2.39 (1H, m), 2.34-2.32 (3H, m), 2.24-2.17 (1H, m), 1.48-1.44 (9H, m), 1.39 (3H, d, J = 6.4 Hz).
LCMS: 505.3 $[M+H-Boc]^+$ (Rt= 2.397 min).

[Step 3] (2S,11aR)-6-(((S)-1,1-Difluoropropan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one

The same procedures as those in Step 2 to Step 4 of Example 1were carried out except that the compound obtained in Step 2 (45.9 mg, 0.76 mmol) was used as a raw material, to thereby produce the title compound (18.7 mg).

$^1$H-NMR ($CDCl_3$) δ: 7.40 (1H, s), 7.06 (1H, d, J = 8.2 Hz), 6.69 (1H, s), 6.62 (1H, s), 6.48 (1H, dd, J = 8.2, 2.7 Hz), 6.24 (1H, d, J = 2.3 Hz), 6.03 (1H, ddd, J = 57.9, 54.2, 2.5 Hz), 4.97-4.93 (1H, m), 4.43-4.32 (1H, m), 4.24-4.11 (4H, m), 3.87 (1H, dd, J = 13.0, 4.8 Hz), 2.90 (2H, t, J = 7.3 Hz), 2.62 (2H, t, J = 7.5 Hz), 2.46-2.39 (1H, m), 2.32 (3H, s), 2.00 (1H, dt, J = 13.7, 5.7 Hz), 1.39 (3H, d, J = 6.4 Hz).
LCMS: 473.2 $[M+H]^+$ (Rt= 2.149 min).

<Example 9: (2S,11aR)-8-Methyl-2-((2-oxo-1,2,3,4-tetrahydroquinolin-7-yl)oxy)-6-((1,1,1-trifluoropropan-2-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 7 (92.1 mg, 0.33 mmol) and the compound obtained in Reference Example 83 (100 mg, 0.28 mmol) were used as raw materials, to thereby produce the title compound (56.1 mg).

$^1$H-NMR ($CDCl_3$) δ: 7.67-7.63 (1H, 2s), 7.06-7.04 (1H, 2s), 6.72-6.70 (1H, 2s), 6.66-6.63 (1H, 2s), 6.44-6.51 (1H, m), 6.28-6.23 (1H, 2d, J = 2.4 Hz), 4.96-4.96 (1H, m), 4.71-4.44 (1H, m), 4.25-4.03 (4H, m), 3.91-3.80 (1H, m), 2.90 (2H, t, J = 7.5 Hz), 2.62 (2H, t, J = 7.5 Hz), 2.48-2.39 (1H, m), 2.33-2.32 (3H, 2s), 2.02-1.95 (1H, m), 1.57-1.42 (3H, 2d, J =6.4 Hz).
LCMS: 491.2 $[M+H]^+$ (Rt= 2.179 min).

<Example 10: (2S,11aR)-8-methyl-2-((2-oxo-1,2,3,4-tetrahydroquinolin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one>

The compound obtained in Reference Example 39 (20.0 mg, 0.058 mmol), 7-hydroxy-3,4-dihydroquinolin-2(1H)-one (11.3 mg, 0.070 mmol), and triphenylphosphine (29.6 mg, 0.113 mmol) were dissolved in THF (1 mL), and to the resulting solution, DIAD (22.0 μL, 0.113 mmol) was added dropwise. After the drop was finished, the mixture was agitated at 40°C for 4 hours. The reaction mixture was then cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (10.1 mg).

$^1$H-NMR ($CDCl_3$) δ: 7.56 (1H, s), 7.06 (1H, d, J = 8.2 Hz), 6.69 (1H, s), 6.62 (1H, s), 6.50 (1H, d, J = 8.2 Hz), 6.26 (1H, s), 4.95 (1H, br s), 4.70-4.64 (1H, m), 4.17-4.08 (4H, m), 3.87 (1H, dd, J = 13.3, 4.6 Hz), 2.90 (2H, t, J = 7.5 Hz), 2.62 (2H, t, J = 7.5 Hz), 2.46-2.40 (1H, m), 2.33 (3H, s), 2.02-1.96 (1H, m), 1.57 (3H, d, J = 6.9 Hz).
LCMS: 491.2 $[M+H]^+$ (Rt= 2.176 min).

<Example 11: (2S,11aR)-6-(Cyclopentyloxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

[Step 1] tert-Butyl (2R,4S)-4-((2-oxo-1,2,3,4-tetrahydroquinolin-7-yl)oxy)-2-(((2,2,7-trimethyl-4-oxo-4H-benzo[d][1,3]dioxin-5-yl)oxy)methyl)pyrrolidin-1-carboxylate

The compound obtained in Step 1 of Reference Example 2 (632 mg, 3.04 mmol), the compound obtained in Reference Example 83 (846 mg, 2.33 mmol), and triphenylphosphine (735 mg, 2.80 mmol) were dissolved in THF (11.7 mL), and to the resulting solution, DIAD (545 μL, 2.80 mmol) was added dropwise at 0°C. After the drop was finished, the mixture was agitated overnight at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate), to thereby produce a crude product of the title compound.
LCMS: 453.2 $[M+H]^+$ (Rt= 2.421 min).

[Step 2] tert-Butyl (2R,4S)-2-((3-hydroxy-2-(methoxycarbonyl)-5-methylphenoxy)methyl)-4-((2-oxo-1,2,3,4-tetrahydroquinolin-7-yl)oxy)pyrrolidin-1-carboxylate

The crude product of the compound obtained in Step 1 was dissolved in a mixture solvent of THF (7.78 mL) and methanol (7.78 mL), combined with potassium carbonate (968 mg, 7.00 mmol), and agitated overnight at room temperature. The reaction mixture was combined with 2M hydrochloric acid aqueous solution, and extracted with dichloromethane. The organic phase was dried with anhydrous magnesium sulfate, and concentrated under reduced pressure. The residue was purified with silica gel column chromatography (heptane/ethyl acetate) to thereby produce the title compound (738 mg). LCMS: 427.2 $[M+H-Boc]^+$ (Rt= 2.366 min).

[Step 3] tert-Butyl (2R,4S)-2-((3-(cyclopentyloxy)-2-(methoxycarbonyl)-5-methylphenoxy)methyl)-4-((2-oxo-1,2,3,4-tetrahydroquinolin-7-yl)oxy)pyrrolidin-1-carboxylate

The compound obtained in Step 2 (211 mg, 0.40 mmol), cyclopentanol (72.6 μL, 0.80 mmol), and triphenylphosphine (157 mg, 0.60 mmol) were dissolved in THF (2 mL), and to the resulting solution, DIAD (117 μL, 0.60 mmol) was added dropwise at 0°C. After the drop was finished, the mixture was agitated overnight at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (218 mg).
LCMS: 495.3 $[M+H]^+$ (Rt= 2.517 min).

[Step 4] (2S,11aR)-6-(Cyclopentyloxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one

The same procedures as those in Step 2 to Step 4 of Example 1were carried out except that the compound

obtained in Step 3 (218 mg, 0.37 mmol) was used as a raw material, to thereby produce the title compound (77.0 mg).

$^1$H-NMR (DMSO-d6) δ: 9.98 (1H, s), 7.05 (1H, d, J = 8.7 Hz), 6.70 (1H, s), 6.49-6.48 (2H, m), 6.41 (1H, d, J = 2.3 Hz), 5.01-4.97 (1H, m), 4.77-4.75 (1H, m), 4.08 (2H, d, J = 7.8 Hz), 3.97-3.90 (1H, m), 3.77-3.76 (2H, m), 2.77 (2H, t, J = 7.5 Hz), 2.40 (2H, t, J = 7.5 Hz), 2.25-2.21 (4H, m), 2.06-2.03 (1H, m), 1.80-1.49 (8H, m).
LCMS: 463.2 $[M+H]^+$ (Rt= 2.218 min).

<Example 12: (2S,11aR)-6-(((1S,2R,4R)-Bicyclo[2.2.1]heptan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 9 (64.0 mg, 0.23 mmol) and the compound obtained in Reference Example 83 (70.0 mg, 0.19 mmol) were used as raw materials, to thereby produce the title compound (24.1 mg).

$^1$H-NMR ($CDCl_3$) δ: 8.03-8.01 (1H, m), 7.04 (1H, d, J = 8.2 Hz), 6.53-6.48 (3H, m), 6.31-6.31 (1H, m), 4.95-4.94 (1H, m), 4.66-4.62 (1H, m), 4.20-3.88 (5H, m), 2.90 (2H, t, J = 7.5 Hz), 2.62-2.56 (3H, m), 2.41-2.40 (1H, m), 2.29-2.26 (4H, m), 2.14-1.93 (2H, m), 1.75-1.69 (1H, m), 1.57-1.26 (5H, m), 1.16-1.04 (1H, m).
LCMS: 489.3 $[M+H]^+$ (Rt= 2.305 min).

<Example 13: (2S,11aR)-8-Methyl-6-(neopentyloxy)-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 10 (97.5 mg, 0.39 mmol) and the compound obtained in Reference Example 83 (100 mg, 0.28 mmol) were used as raw materials, to thereby produce the title compound (52.9 mg).

$^1$H-NMR ($CDCl_3$) δ: 7.62 (1H, s), 7.04 (1H, d, J = 8.2 Hz), 6.55 (1H, s), 6.49-6.48 (2H, m), 6.25 (1H, d, J = 2.7 Hz), 4.97-4.93 (1H, m), 4.22-4.01 (4H, m), 3.68 (1H, d, J = 8.8 Hz), 3.57 (1H, d, J = 8.4 Hz), 2.90 (2H, t, J = 7.3 Hz), 2.61 (2H, t, J = 7.5 Hz), 2.45-2.39 (1H, m), 2.31 (3H, s), 2.00-1.97 (1H, m), 1.02 (9H, s).
LCMS: 465.3 $[M+H]^+$ (Rt= 2.263 min).

<Example 14: (2S,11aR)-8-Methyl-2-((2-oxo-1,2,3,4-tetrahydroquinolin-7-yl)oxy)-6-((tetrahydrofuran-2-yl)methoxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 11 (47.8 mg, 0.18 mmol) and the compound obtained in Reference Example 83 (50.0 mg, 0.24 mmol) were used as raw materials, to thereby produce the title compound (19.6 mg).

$^1$H-NMR ($CDCl_3$) δ: 8.65-7.96 (1H, 2s), 7.06-7.04 (1H, 2s), 6.60-6.31 (3H, m), 6.38-6.31 (1H, 2d, J = 2.4 Hz), 4.95-4.91 (1H, m), 4.53-3.57 (10H, m), 2.91-2.88 (2H, m), 2.63-2.59 (2H, m), 2.43-2.41 (1H, m), 2.31-2.32 (3H, 2s), 2.21-1.72 (5H, m).
LCMS: 479.3 $[M+H]^+$ (Rt= 2.081 min).

<Example 15: (2S,11aR)-6-Isobutyl-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 12 (110 mg, 0.50 mmol) and the compound obtained in Reference Example 83 (150 mg, 0.41 mmol) were used as raw materials, to thereby produce the title compound (51.5 mg).

$^1$H-NMR ($CDCl_3$) δ: 7.67 (1H, s), 7.05 (1H, d, J = 8.2 Hz), 6.83 (1H, s), 6.73 (1H, s), 6.45 (1H, dd, J = 8.2, 2.3 Hz), 6.21 (1H, d, J = 2.3 Hz), 4.97-4.93 (1H, m), 4.23-4.20 (2H, m), 4.08-3.99 (2H, m), 3.84 (1H, dd, J = 13.3, 4.1 Hz), 3.27 (1H, dd, J = 13.0, 7.1 Hz), 2.90 (2H, t, J = 7.5 Hz), 2.62 (2H, t, J = 7.5 Hz), 2.44-2.39 (1H, m), 2.32-2.27 (4H, m), 1.97 (1H, dt, J = 13.7, 5.7 Hz), 1.79-1.69 (1H, m), 0.84 (6H, t, J = 6.4 Hz).
LCMS: 435.3 $[M+H]^+$ (Rt= 2.288 min).

<Example 16: (2S,11aR)-6-Cyclohexyl-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 13 (89.2 mg, 0.36 mmol) and the compound obtained in Reference Example 83 (156 mg, 0.43 mmol) were used as raw materials, to thereby produce the title compound (23.6 mg).

$^1$H-NMR ($CDCl_3$) δ: 7.63 (1H, s), 7.05 (1H, d, J = 8.2 Hz), 6.98 (1H, s), 6.72 (1H, s), 6.46 (1H, d, J = 8.2 Hz), 6.23 (1H, s), 4.97 (1H, br s), 4.18-3.88 (5H, m), 3.30 (1H, t, J = 10.0 Hz), 2.90 (2H, t, J = 7.3 Hz), 2.62 (2H, t, J = 7.3 Hz), 2.42-2.40 (1H, br m), 2.33 (3H, s), 2.03-1.97 (2H, m), 1.79-1.67 (4H, m), 1.43-1.34 (5H, m).
LCMS: 461.3 $[M+H]^+$ (Rt= 2.386 min).

<Example 17: (2S,11aR)-6-(2-Fluorophenyl)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 14 (47.4 mg, 0.18 mmol) and the compound obtained in Reference Example 83 (60 mg, 0.17 mmol) were used as raw materials, to thereby produce the title compound (44.8 mg).

$^1$H-NMR ($CDCl_3$) δ: 7.55 (1H, s), 7.36 (1H, t, J = 7.3 Hz), 7.29-7.23 (1H, m), 7.14 (1H, t, J = 7.3 Hz), 7.07 (1H, d, J = 8.2 Hz), 6.99 (1H, s), 6.92-6.88 (2H, m), 6.53 (1H, d, J = 8.2 Hz), 6.30 (1H, s), 4.96 (1H, br s), 4.37-4.31 (2H, m), 4.13-4.11 (2H, m), 3.75 (1H, dd, J = 12.8, 4.1 Hz), 2.91 (2H, t, J = 7.5 Hz), 2.63 (2H, t, J = 7.5 Hz), 2.52-2.47 (1H, m), 2.39 (3H, s), 2.02-1.98 (1H, m).
LCMS: 473.2 $[M+H]^+$ (Rt= 2.236 min).

<Example 18: (2S,11aR)-6-(2,6-Difluorophenyl)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 15 (96.7 mg, 0.35 mmol) and the compound obtained in Reference Example 83 (105 mg, 0.29 mmol) were used as raw materials, to thereby produce the title compound (64.1 mg).

$^1$H-NMR ($CDCl_3$) δ: 7.58 (1H, s), 7.24-7.19 (1H, m), 7.06-7.02 (2H, m), 6.98-6.94 (2H, m), 6.73 (1H, t, J = 8.9 Hz), 6.51 (1H, dd, J = 8.2, 2.3 Hz), 6.28 (1H, d, J = 2.3 Hz), 4.95-4.92 (1H, m), 4.35-4.24 (2H, m), 4.15-4.07 (0H, m), 3.71 (1H, dd, J = 12.8, 4.1 Hz), 2.90 (2H, t, J = 7.5 Hz), 2.62 (2H, t, J = 7.5 Hz), 2.50-2.44 (1H, m), 2.39 (3H, s), 2.01-1.95 (1H, m).
LCMS: 491.2 $[M+H]^+$ (Rt= 2.238 min).

<Example 19: (2S,11aR)-6-(2-(Difluoromethyl)phenyl)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 16 (87.1 mg, 0.30 mmol) and the compound obtained in Reference Example 83 (130 mg, 0.36 mmol) were used as raw materials, to thereby produce the title compound (11.6 mg).

$^1$H-NMR ($CDCl_3$) δ: 7.70-7.49 (4H, m), 7.00-6.90 (3H, m), 6.67-6.47 (2H, m), 6.23-6.15 (1H, m), 4.90-4.88 (1H, m), 4.36-3.93 (4H, m), 3.69-3.53 (1H, m), 2.89 (2H, t, J = 7.3 Hz), 2.61 (2H, t, J = 7.5 Hz), 2.46-2.40 (4H, m), 2.01-1.97 (1H, m).
LCMS: 505.2 $[M+H]^+$ (Rt= 2.267 min).

<Example 20: (2S,11aR)-6-(3-Fluoropyridin-2-yl)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 17 (99.5 mg, 0.38 mmol) and the compound obtained in Reference Example 83 (166 mg, 0.46 mmol) were used as raw materials, to thereby produce the title compound (14.4 mg).

$^1$H-NMR (DMSO-$d_6$) δ: 10.04 (1H, s), 8.39-8.38 (1H, m), 7.55-7.52 (1H, m), 7.42-7.37 (1H, m), 7.13 (1H, s), 7.08 (1H, d, J = 8.2 Hz), 7.04 (1H, s), 6.56-6.54 (1H, m), 6.49 (1H, s), 5.04 (1H, s), 4.32-4.07 (3H, m), 3.78 (1H, d, J = 12.3 Hz),

3.64 (1H, dd, J = 12.8, 3.7 Hz), 2.79 (2H, t, J = 7.5 Hz), 2.42 (2H, t, J = 7.3 Hz), 2.36-2.32 (4H, m), 2.12-2.08 (1H, m). LCMS: 474.2 $[M+H]^+$ (Rt= 2.042 min).

<Example 21: (2S,11aR)-8-Methyl-2-((2-oxo-1,2,3,4-tetrahydroquinolin-7-yl)oxy)-6-(2-(trifluoromethyl)pyridin-3-yl)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 18 (9.92 mg, 0.03 mmol) and the compound obtained in Reference Example 83 (10.5 mg, 0.03 mmol) were used as raw materials, to thereby produce the title compound (2.1 mg).
LCMS: 524.2 $[M+H]^+$ (Rt= 2.184 min).

<Example 22: (2S,11aR)-8-Methyl-2-((2-oxo-1,2,3,4-tetrahydroquinolin-7-yl)oxy)-6-(thiophen-2-yl)-2,3,11,11a-tetrahydro-1H,5H-benzo[f]pyrrolo[2,1-c][1,4]oxazepin-5-one>

The same procedures as those in Example 1 were carried out except that the compound obtained in Reference Example 19 (35.6 mg, 0.14 mmol) and the compound obtained in Reference Example 83 (40.0 mg, 0.11 mmol) were used as raw materials, to thereby produce the title compound (14.5 mg).

$^1$H-NMR ($CDCl_3$) δ: 7.60 (1H, s), 7.27-7.26 (1H, m), 7.13 (1H, d, J = 0.9 Hz), 7.07-7.05 (2H, m), 6.95 (1H, dd, J = 5.0, 3.7 Hz), 6.86 (1H, s), 6.52 (1H, dd, J = 8.2, 2.7 Hz), 6.27 (1H, d, J = 2.3 Hz), 4.99-4.99 (1H, m), 4.40-4.11 (4H, m), 3.78 (1H, dd, J = 13.0, 3.9 Hz), 2.91 (2H, t, J = 7.5 Hz), 2.62 (2H, t, J = 7.5 Hz), 2.52-2.50 (1H, m), 2.36 (3H, s), 2.02-1.99 (1H, m).
LCMS: 461.2 $[M+H]^+$ (Rt= 2.211 min).

<Example 23: (2S,11aR)-8-Methyl-2-((2-oxo-1,2,3,4-tetrahydroquinolin-7-yl)oxy)-6-(pyrrolidin-1-yl)-2,3,11,11a-tetrahydro-1H,5H-benzo[f]pyrrolo[2,1-c][1,4]oxazepin-5-one>

[Step 1] (2S,11aR)-8-methyl-5-oxo-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-6-yl trifluoromethane sulfonate

The compound obtained in Example 3 (55.7 mg, 0.14 mmol) and pyridine (13.6 μL, 0.17 mmol) were dissolved in dichloromethane (1.41 mL), and to the resulting solution, trifluoromethane sulfonic anhydride (25.4 μL, 0.16 mmol) was added dropwise at 0°C. After the drop was finished, the mixture was agitated at 0°C for 5 hours. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (23.3 mg).
LCMS: 527.1 $[M+H]^+$ (Rt= 2.219 min).

[Step 2] (2S,11aR)-8-Methyl-2-((2-oxo-1,2,3,4-tetrahydroquinolin-7-yl)oxy)-6-(pyrrolidin-1-yl)-2,3,11,11a-tetrahydro-1H,5H-benzo[f]pyrrolo[2,1-c][1,4]oxazepin-5-one

The compound obtained in Step 1 (23.3 mg, 0.044 mmol), pyrrolidine (22.1 μL, 0.27 mmol), and cesium carbonate (72.1 mg, 0.22 mmol) were dissolved in 1,2-dimethoxyethane (1.00 mL), combined with XPhos (3.38 mg, 7.08μmol) and $Pd_2(dba)_3$ (1.62 mg, 1.77μmol), and agitated at 80°C for 2 hours. The reaction mixture was then cooled to room temperature, combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (1.57 mg).

$^1$H-NMR ($CDCl_3$) δ: 7.32 (1H, s), 7.05 (1H, d, J = 8.2 Hz), 6.43 (1H, dd, J = 8.5, 2.1 Hz), 6.37 (1H, s), 6.25 (1H, s), 6.15 (1H, d, J = 2.3 Hz), 4.99 (1H, br s), 4.41-4.38 (1H, m), 4.22-4.18 (2H, m), 3.98 (1H, t, J = 11.0 Hz), 3.83 (1H, dd, J = 13.0, 3.9 Hz), 3.49 (1H, d, J = 5.5 Hz), 3.41-3.34 (2H, m), 3.02 (2H, t, J = 8.2 Hz), 2.90 (2H, t, J = 7.5 Hz), 2.63-2.61 (2H, m), 2.47-2.45 (1H, m), 2.27 (3H, s), 1.97-1.95 (2H, m), 1.83-1.81 (2H, m).
LCMS: 448.2 $[M+H]^+$ (Rt= 1.840 min).

<Example 24:7-(((2S,11aR)-6-Isopropoxy-8-methyl-2,3,11,11a-tetrahydro-1H,5H-benzo[f]pyrrolo[2,1-c][1,4]oxazepin-2-yl)oxy)-3,4-dihydroquinolin-2(1H)-one>

The same procedures as those in Example 10 were carried out except that the compound obtained in Reference Example 24 (25.0 mg, 0.90 mmol) and 7-hydroxy-3,4-dihydroquinolin-2(1H)-one (44.1 mg, 0.27 mmol) were used as raw materials, to thereby produce the title compound (17.5 mg).
LCMS: 423.2 $[M+H]^+$ (Rt= 1.857 min).

<Example 25: (2S,11aR)-6-(Cyclopentyloxy)-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

[Step 1] (2S,11aR)-6-(Cyclopentyloxy)-2-((2-oxo-1-((2-(trimethylsilyl)ethoxy)methyl)-1,2-dihydro-1,6-naphthyridin-7-yl)oxy)-2,3,11,11a-tetrahydro-1H,5H-benzo[f]pyrrolo[2,1-c][1,4]oxazepin-5-one

The compound obtained in Reference Example 27 (50.0 mg, 0.16 mmol) was dissolved in DMF (1.00 mL), and to the resulting solution, 50% sodium hydride (11.9 mg, 0.25 mmol) was added at 0°C, and the mixture was brought to room temperature, and agitated for 1 hour. To the mixture cooled again to 0°C, the compound obtained in Reference Example 84 (61.5 mg, 0.20 mmol) was added, and agitated for 4 hours at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (70.0 mg).
LCMS: 578.3 $[M+H]^+$ (Rt= 2.548 min).

[Step 2] (2S,11aR)-6-(Cyclopentyloxy)-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one

The compound obtained in Step 1 (70.0 mg, 0.12 mmol) was dissolved in 1,4-dioxane (0.6 mL), combined with 4M hydrochloric acid/1,4-dioxane (0.6 mL, 2.42 mmol), and agitated for 2 hours at room temperature. The reaction mixture was combined with saturated sodium bicarbonate aqueous solution, and extracted with dichloromethane. The organic phase was washed twice with saturated saline solution, dried with anhydrous magnesium sulfate, and concentrated under reduced pressure. The residue was purified with silica gel column chromatography (ethyl acetate/methanol) to thereby produce the title compound (30.6 mg).
LCMS: 448.2 $[M+H]^+$ (Rt= 2.050 min).

[Step 3] (2S,11aR)-6-(Cyclopentyloxy)-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

The compound obtained in Step 2 (30.6 mg, 0.07 mmol) was dissolved in a mixture solvent of dichloromethane (1.14 mL) and methanol (1.14 mL), combined with 20% hydroxide palladium-activated carbon (9.18 mg), and agitated for 4 days under hydrogen atmosphere at normal pressure at room temperature. The reaction mixture was filtered with cerite, and the filtrate was concentrated under reduced pressure. The residue was purified with silica gel column chromatography (ethyl acetate/methanol) to thereby produce the title compound (20.7 mg).

$^1$H-NMR ($CDCl_3$) δ: 8.10 (1H, s), 7.86 (1H, s), 7.29-7.25 (1H, m), 6.77 (1H, d, J = 8.2 Hz), 6.64 (1H, d, J = 7.8 Hz), 6.08 (1H, s), 5.64-5.59 (1H, m), 4.81-4.78 (1H, br m), 4.17-3.92 (5H, m), 2.92 (2H, t, J = 7.5 Hz), 2.68-2.64 (2H, m), 2.37-2.30 (1H, m), 2.12-2.09 (1H, m), 1.99-1.96 (1H, m), 1.86-1.79 (5H, m), 1.57-1.56 (2H, m).
LCMS: 450.2 $[M+H]^+$ (Rt= 1.896 min).

<Example 26: (2S,11aR)-6-(Cyclopropylmethoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 28 (84.1 mg, 0.28 mmol) and the compound obtained in Reference Example 85 (35.0 mg, 0.21 mmol) were used as raw materials, to thereby produce the title compound (7.6 mg).

$^1$H-NMR ($CDCl_3$) δ:7.87 (1H, s), 7.46 (1H, s), 6.59 (1H, s), 6.50 (1H, s), 6.05 (1H, s), 5.64-5.59 (1H, m), 4.19-4.06 (4H, m), 4.00-3.94 (2H, m), 3.83 (1H, dd, J = 10.1, 6.9 Hz), 2.94-2.90 (2H, m), 2.68-2.64 (2H, m), 2.36-2.31 (4H, m),

2.14-2.07 (1H, m), 0.94-0.81 (1H, m), 0.60-0.56 (2H, m), 0.37-0.34 (2H, m).
LCMS: 450.2 $[M+H]^+$ (Rt= 1.887 min).

<Example 27: (2S,11aR)-6-Isobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 25 were carried out except that the compound obtained in Reference Example 29 (70.2 mg, 0.23 mmol) and the compound obtained in Reference Example 84 (89.3 mg, 0.29 mmol) were used as raw materials, to thereby produce the title compound (21.0 mg).

$^1$H-NMR ($CDCl_3$) δ: 7.86 (1H, s), 7.75 (1H, s), 6.56 (1H, s), 6.48 (1H, s), 6.03 (1H, s), 5.64-5.59 (1H, m), 4.19-3.97 (5H, m), 3.83-3.80 (1H, m), 3.73-3.69 (1H, m), 2.91 (2H, t, J = 7.5 Hz), 2.66 (2H, t, J = 7.3 Hz), 2.36-2.32 (4H, m), 2.13-2.08 (2H, m), 1.00 (6H, t, J = 6.9 Hz).
LCMS: 452.2 $[M+H]^+$ (Rt= 1.965 min).

<Example 28: (2S,11aR)-6-(2-Hydroxy-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 25 were carried out except that the compound obtained in Reference Example 30 (30.2 mg, 0.09 mmol) and the compound obtained in Reference Example 84 (35.0 mg, 0.11 mmol) were used as raw materials, to thereby produce the title compound (6.7 mg).

$^1$H-NMR ($CDCl_3$) δ: 7.87 (1H, s), 7.82 (1H, s), 6.59 (1H, s), 6.52 (1H, s), 6.09 (1H, s), 5.63-5.59 (1H, m), 4.23-3.85 (8H, m), 2.92 (2H, t, J = 7.3 Hz), 2.68-2.64 (2H, m), 2.39-2.32 (4H, m), 2.12-2.07 (1H, m), 1.32 (3H, s), 1.22 (3H, s).
LCMS: 468.2 $[M+H]^+$ (Rt= 1.818 min).

<Example 29: (2S,11aR)-6-(2-Fluoro-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The compound obtained in Example 28 (31.9 mg, 0.07 mmol) and N,N-diisopropylethylamine (24.0 μL, 0.14 mmol) were dissolved in dichloromethane (1.71 mL), and to the resulting solution, bis(2-methoxyethyl)aminosulfur trifluoride (19.0 μL, 0.10 mmol) was added dropwise at -78°C. After the drop was finished, the mixture was agitated at 0°C for 7 hours. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (ethyl acetate/methanol) to thereby produce the title compound (7.4 mg). $^1$H-NMR ($CDCl_3$) δ: 7.87 (1H, s), 7.64 (1H, s), 6.58 (1H, s), 6.53 (1H, s), 6.06 (1H, s), 5.64-5.59 (1H, m), 4.21-3.92 (7H, m), 2.91 (2H, t, J = 7.3 Hz), 2.65 (2H, t, J = 7.8 Hz), 2.39-2.32 (4H, m), 2.08-2.05 (1H, m), 1.53 (3H, d, J = 15.6 Hz), 1.47 (3H, d, J = 15.2 Hz). LCMS: 470.2 $[M+H]^+$ (Rt= 1.912 min).

<Example 30: (2S,11aR)-6-Isopropoxy-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one>

The same procedures as those in Step 1 and Step 2 of Example 25 were carried out except that the compound obtained in Reference Example 31 (151 mg, 0.52 mmol) and the compound obtained in Reference Example 84 (193 mg, 0.62 mmol) were used as raw materials, to thereby produce the title compound (122 mg).

$^1$H-NMR (DMSO-$d_6$) δ: 11.89 (1H, s), 8.60 (1H, s), 7.97 (1H, d, J = 9.6 Hz), 6.80 (1H, s), 6.59 (1H, s), 6.50 (1H, s), 6.43 (1H, d, J = 9.6 Hz), 5.69-5.65 (1H, m), 4.55-4.46 (1H, m), 4.20-4.02 (3H, m), 3.90 (2H, d, J = 4.1 Hz), 2.38-2.35 (4H, m), 2.20-2.16 (1H, m), 1.27 (3H, d, J = 6.4 Hz), 1.25 (3H, d, J = 6.0 Hz).
LCMS: 436.2 $[M+H]^+$ (Rt= 2.014 min).

<Example 31: (2S,11aR)-6-Isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Step 3 of Example 25 were carried out except that the compound obtained in Example 30 (258 mg, 0.59 mmol) was used as a raw material, to thereby produce the title compound (205 mg).

$^1$H-NMR (DMSO-$d_6$) δ: 10.41 (1H, s), 7.88 (1H, s), 6.72 (1H, s), 6.51 (1H, s), 6.11 (1H, s), 5.50-5.46 (1H, m), 4.46-4.40

(1H, m), 4.10-4.05 (2H, m), 3.98-3.90 (1H, m), 3.80-3.76 (2H, m), 2.81 (2H, t, J = 7.5 Hz), 2.47-2.46 (2H, m), 2.29-2.23 (4H, m), 2.09-2.03 (1H, m), 1.20-1.17 (6H, m).
LCMS: 438.2 $[M+H]^+$ (Rt= 1.865 min).

<Example 32: (2S,11aR)-8-Chloro-6-isopropoxy-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 32 (31.2 mg, 0.10 mmol) and the compound obtained in Reference Example 85 (24.6 mg, 0.15 mmol) were used as raw materials, to thereby produce the title compound (2.6 mg).
LCMS: 458.0 $[M+H]^+$ (Rt= 1.948 min).

<Example 33: (2S,11aR)-6-Isopropoxy-2-((2-oxo-1,2,3,4-tetrahydro-1,6-naphthyridin-7-yl)oxy)-8-(trifluoromethyl)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 33 (52.0 mg, 0.15 mmol) and the compound obtained in Reference Example 85 (37.0 mg, 0.23 mmol) were used as raw materials, to thereby produce the title compound (2.2 mg).
LCMS: 492.1 $[M+H]^+$ (Rt= 1.993 min).

<Example 34: (2S,11aR)-2-((8-Fluoro-2-oxo-1,2,3,4-tetrahydroquinolin-7-yl)oxy)-6-isopropoxy-8-methyl-2,3,11,11a-tetrahydro-1H,5H-benzo[f]pyrrolo[2,1-c][1,4]oxazepin-5-one>

The same procedures as those in Example 10 were carried out except that the compound obtained in Reference Example 34 (48.2 mg, 0.17 mmol) and the compound obtained in Reference Example 86 (25.0 mg, 0.14 mmol) were used as raw materials, to thereby produce the title compound (26.6 mg).

$^1$H-NMR ($CDCl_3$) δ: 7.53 (1H, s), 6.85 (1H, d, J = 8.2 Hz), 6.62 (1H, s), 6.57 (1H, t, J = 8.0 Hz), 6.49 (1H, s), 5.00-4.96 (1H, m), 4.54-4.45 (1H, m), 4.21-4.15 (3H, m), 4.03-3.97 (1H, m), 3.85 (1H, dd, J = 13.0, 4.8 Hz), 2.95 (2H, t, J = 7.5 Hz), 2.64 (2H, t, J = 7.3 Hz), 2.49-2.46 (1H, m), 2.31 (3H, s), 2.01-1.98 (1H, m), 1.37 (3H, d, J = 5.9 Hz), 1.31 (3H, d, J = 5.9 Hz).
LCMS: 455.2 $[M+H]^+$ (Rt= 2.129 min).

<Example 35: (2S,11aR)-2-((8-Fluoro-2-oxo-1,2,3,4-tetrahydro-1,6-naphthyridin-7-yl)oxy)-6-isopropoxy-8-methyl-2,3,11,11a-tetrahydro-1H,5H-benzo[f]pyrrolo[2,1-c][1,4]oxazepin-5-one>

The same procedures as those in Example 10 were carried out except that the compound obtained in Reference Example 34 (48.0 mg, 0.17 mmol) and the compound obtained in Reference Example 87 (25.0 mg, 0.14 mmol) were used as raw materials, to thereby produce the title compound (32.2 mg).

$^1$H-NMR ($CDCl_3$) δ: 7.67 (2H, s), 6.62 (1H, s), 6.50 (1H, s), 5.69-5.64 (1H, m), 4.53-4.44 (1H, m), 4.18-4.17 (2H, m), 4.11-4.02 (3H, m), 2.99-2.97 (2H, m), 2.69-2.67 (2H, m), 2.46-2.39 (1H, m), 2.31 (3H, s), 2.12-2.10 (1H, m), 1.36 (3H, d, J = 5.9 Hz), 1.29 (3H, d, J = 5.9 Hz).
LCMS: 456.2 $[M+H]^+$ (Rt= 2.093 min).

<Example 36: (2S,11aR)-6-Isopropoxy-8-methyl-2-((2-oxo-1,4-dihydro-2H-benzo[d][1,3]oxazin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 34 (42.3 mg, 0.15 mmol) and the compound obtained in Reference Example 88 (20.0 mg, 0.12 mmol) were used as raw materials, to thereby produce the title compound (18.4 mg).

$^1$H-NMR ($CDCl_3$) δ: 7.36 (1H, s), 7.01 (1H, d, J = 8.2 Hz), 6.63 (1H, s), 6.54 (1H, dd, J = 8.2, 2.3 Hz), 6.50 (1H, s), 6.32 (1H, d, J = 2.3 Hz), 5.25 (2H, s), 4.98-4.94 (1H, m), 4.54-4.48 (1H, m), 4.20-3.95 (5H, m), 2.40 (1H, dt, J = 14.5, 5.1 Hz), 2.31 (3H, s), 2.06-2.00 (1H, m), 1.37 (3H, d, J = 6.4 Hz), 1.31 (3H, d, J = 5.9 Hz).
LCMS: 439.2 $[M+H]^+$ (Rt= 2.103 min).

<Example 37:(7aS,9S)-1-Isopropoxy-3-methyl-9-((2-oxo-1,2,3,4-tetrahydro-1,6-naphthyridin-7-yl)oxy)-6,7,7a,8,9,10-hexahydro-12H-benzo[b]pyrrolo[1,2-e][1,5]oxazocin-12-one>

[Step 1] (7aS,9S)-1-Isopropoxy-3-methyl-9-((2-oxo-1-((2-(trimethylsilyl)ethoxy)methyl)-1,2-dihydro-1,6-naphthyridin-7-yl)oxy)-6,7,7a,8,9,10-hexahydro-12H-benzo[b]pyrrolo[1,2-e][1,5]oxazocin-12-one

The same procedures as those in Step 1 of Example 25 were carried out except that the compound obtained in Reference Example 35 (95.0 mg, 0.31 mmol) and the compound obtained in Reference Example 84 (126 mg, 0.40 mmol) were used as raw materials, to thereby produce the title compound (86.2 mg).
LCMS: 580.3 $[M+H]^+$ (Rt= 2.617 min).

[Step 2] (7aS,9S)-1-Isopropoxy-3-methyl-9-((2-oxo-1,2-dihydro-1,6-naphthyridin-7-yl)oxy)-6,7,7a,8,9,10-hexahydro-12H-benzo[b]pyrrolo[1,2-e][1,5]oxazocin-12-one

The compound obtained in Step 1 (66.0 mg, 0.11 mmol) was dissolved in THF (1.1 mL), combined with 1M tetrabutyl ammonium fluoride/THF solution (1.25 mL,1.25 mmol), and agitated at 60°C for 7 days. The reaction mixture was combined with saturated ammonium chloride aqueous solution, and extracted with ethyl acetate. The organic phase was washed twice with saturated saline solution, dried with anhydrous magnesium sulfate, and concentrated under reduced pressure. The residue was purified with silica gel column chromatography (ethyl acetate/methanol) to thereby produce the title compound (18.5 mg). LCMS: 450.2 $[M+H]^+$ (Rt= 2.004 min).

[Step 3] (7aS,9S)-1-Isopropoxy-3-methyl-9-((2-oxo-1,2,3,4-tetrahydro-1,6-naphthyridin-7-yl)oxy)-6,7,7a,8,9,10-hexahydro-12H-benzo[b]pyrrolo[1,2-e][1,5]oxazocin-12-one

The same procedures as those in Step 3 of Example 25 were carried out except that the compound obtained in Step 2 (18.5 mg, 0.04 mmoL) was used as a raw material, to thereby produce the title compound (7.5 mg).

$^1$H-NMR ($CDCl_3$) δ: 7.86 (1H, s), 7.55 (1H, br s), 6.54 (1H, s), 6.46 (1H, s), 6.04 (1H, s), 5.64-5.57 (1H, m), 4.56-4.46 (2H, m), 4.15-4.08 (1H, m), 4.04 (1H, t, J = 8.5 Hz), 3.90 (1H, dd, J = 14.0, 6.4 Hz), 3.89-3.80 (1H, m), 2.94-2.88 (2H, m), 2.69-2.63 (2H, m), 2.42-2.33 (1H, m), 2.30 (3H, s), 2.16-2.05 (2H, m), 1.60-1.57 (1H, m), 1.36-1.27 (6H, m).
LCMS: 452.3 $[M+H]^+$ (Rt= 1.865 min).

<Example 38: (2S,11aR)-6-(((R)-1-Fluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 25 were carried out except that the compound obtained in Reference Example 36 (63.3 mg, 0.21 mmol) and the compound obtained in Reference Example 84 (76.3 mg, 0.25 mmol) were used as raw materials, to thereby produce the title compound (43.2 mg).

$^1$H-NMR ($CDCl_3$) δ: 7.86 (1H, s), 7.51 (1H, s), 6.68 (1H, s), 6.55 (1H, s), 6.06 (1H, s), 5.64-5.60 (1H, m), 4.68-4.38 (3H, m), 4.19-4.04 (3H, m), 3.99 (2H, d, J = 4.6 Hz), 2.92 (2H, t, J = 7.5 Hz), 2.68-2.64 (2H, m), 2.37-2.30 (4H, m), 2.09-2.03 (1H, m), 1.37 (3H, dd, J = 6.4, 1.4 Hz).
LCMS: 456.2 $[M+H]^+$ (Rt= 1.843 min).

<Example 39: (2S,11aR)-6-(((R)-1,1-Difluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 25 were carried out except that the compound obtained in Reference Example 37 (127 mg, 0.39 mmol) and the compound obtained in Reference Example 84 (145 mg, 0.47 mmol) were used as raw materials, to thereby produce the title compound (89.4 mg).

$^1$H-NMR ($CDCl_3$) δ: 8.02 (1H, s), 7.85 (1H, s), 6.67 (1H, s), 6.60 (1H, s), 6.07 (1H, s), 5.94-5.59 (2H, m), 4.44-4.40 (1H, m), 4.20-3.96 (5H, m), 2.91 (2H, t, J = 7.3 Hz), 2.65 (2H, td, J = 7.4, 2.0 Hz), 2.38-2.34 (4H, m), 2.09-2.06 (1H, m), 1.45 (3H, d, J = 6.4 Hz).
LCMS: 474.2 $[M+H]^+$ (Rt= 1.885 min).

<Example 40: (2S,11aR)-8-Methyl-2-((2-oxo-1,2-dihydro-1,6-naphthyridin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one>

The same procedures as those in Step 1 to Step 2 of Example 25were carried out except that the compound obtained in Reference Example 38 (5.60 g, 16.2 mmol) and the compound obtained in Reference Example 84 (5.55 g, 17.8 mmol) were used as raw materials, to thereby produce the title compound (4.61 g).

$^1$H-NMR ($CDCl_3$) δ: 10.21 (1H, s), 8.36 (1H, s), 7.72 (1H, d, J = 9.6 Hz), 6.71 (1H, s), 6.63 (1H, s), 6.51 (1H, d, J = 9.6 Hz), 6.46 (1H, s), 5.74-5.69 (1H, m), 4.70-4.64 (1H, m), 4.22-4.01 (5H, m), 2.44-2.36 (1H, m), 2.33 (3H, s), 2.14-2.08 (1H, m), 1.57 (3H, d, J = 6.9 Hz).
LCMS: 490.2 $[M+H]^+$ (Rt= 2.080 min).

<Example 41: (2S,11aR)-8-Methyl-2-((2-oxo-1,2,3,4-tetrahydro-1,6-naphthyridin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one>

The same procedures as those in Step 3 of Example 25 were carried out except that the compound obtained in Example 40 (160 mg, 0.26 mmol) was used as a raw material, to thereby produce the title compound (71.1 mg).

$^1$H-NMR ($CDCl_3$) δ: 7.90 (1H, s), 7.85 (1H, s), 6.70 (1H, s), 6.63 (1H, s), 6.08 (1H, s), 5.64-5.59 (1H, m), 4.72-4.62 (1H, m), 4.19-4.18 (1H, m), 4.11-4.07 (2H, m), 4.05-3.97 (2H, m), 2.93-2.89 (2H, m), 2.67-2.63 (2H, m), 2.38-2.33 (4H, m), 2.10-2.04 (1H, m), 1.57 (3H, d, J = 6.9 Hz).
LCMS: 492.2 $[M+H]^+$ (Rt= 1.940 min).

<Example 42: (2S,11aR)-8-Methyl-2-((2-oxo-1,2,3,4-tetrahydro-1,6-naphthyridin-7-yl)oxy)-6-(((S)-1,1,1-trifluoropropan-2-yl)oxy)-2,3,11,11a-tetrahydro-1H,5H-benzo[f]pyrrolo[2,1-c][1,4]oxazepin-5-one>

The same procedures as those in Example 25 were carried out except that the compound obtained in Reference Example 40 (153 mg, 0.44 mmol) and the compound obtained in Reference Example 84 (165 mg, 0.531 mmol) were used as raw materials, to thereby produce the title compound (61.3 mg).

$^1$H-NMR ($CDCl_3$) δ: 7.94 (1H, s), 7.86 (1H, s), 6.71 (1H, s), 6.66 (1H, s), 6.01 (1H, s), 5.65-5.61 (1H, m), 4.57-4.48 (1H, m), 4.25-4.17 (1H, m), 4.11-4.06 (3H, m), 3.96 (1H, dd, J = 13.3, 5.0 Hz), 2.94-2.90 (2H, m), 2.67-2.63 (2H, m), 2.41-2.35 (1H, m), 2.32 (3H, s), 2.09-2.03 (1H, m), 1.43 (3H, d, J = 6.4 Hz).
LCMS: 492.2 $[M+H]^+$ (Rt= 1.939 min).

<Example 43: (2S,11aR)-2-((8-Fluoro-2-oxo-1,2,3,4-tetrahydro-1,6-naphthyridin-7-yl)oxy)-8-methyl-6-(((R)-1,1,1-trifluoropropan-2-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 39 (37.5 mg, 0.11 mmol) and the compound obtained in Reference Example 87 (18.0 mg, 0.10 mmol) were used as raw materials, to thereby produce the title compound (17.6 mg).

$^1$H-NMR ($CDCl_3$) δ: 7.66 (1H, s), 7.64 (1H, s), 6.70 (1H, s), 6.63 (1H, s), 5.67-5.62 (1H, m), 4.69-4.59 (1H, m), 4.22-4.02 (5H, m), 3.00-2.96 (2H, m), 2.70-2.66 (2H, m), 2.48-2.41 (1H, m), 2.33 (3H, s), 2.10 (1H, dt, J = 13.6, 5.4 Hz), 1.55 (3H, d, J = 6.9 Hz).
LCMS: 510.2 $[M+H]^+$ (Rt= 2.124 min).

<Example 44: (2S,11aR)-8-Methyl-2-((2-oxo-1,4-dihydro-2H-benzo[d][1,3]oxazin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 39 (20.0 mg, 0.06 mmol) and the compound obtained in Reference Example 88 (11.5 mg, 0.07 mmol) were used as raw materials, to thereby produce the title compound (9.8 mg).

$^1$H-NMR ($CDCl_3$) δ: 7.31 (1H, s), 7.02 (1H, d, J = 8.2 Hz), 6.70 (1H, s), 6.63 (1H, s), 6.55 (1H, dd, J = 8.2, 2.3 Hz), 6.31 (1H, d, J = 2.3 Hz), 5.25 (2H, s), 4.98-4.95 (1H, m), 4.70-4.64 (1H, m), 4.21 (1H, dd, J = 9.6, 4.1 Hz), 4.13-4.07 (3H, m), 3.88 (1H, dd, J = 13.0, 4.8 Hz), 2.46-2.40 (1H, m), 2.33 (3H, s), 2.02-1.99 (1H, m), 1.58 (3H, s).
LCMS: 493.2 $[M+H]^+$ (Rt= 2.139 min).

<Example 45: (2S,11aR)-8-Methyl-2-((3-oxo-3,4-dihydro-2H-benzo[b][1,4]oxazin-6-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 39 (30.0 mg, 0.09 mmol) and the compound obtained in Reference Example 89 (21.5 mg, 0.13 mmol) were used as raw materials, to thereby produce the title compound (2.0 mg).
LCMS: 493.2 $[M+H]^+$ (Rt= 2.168 min).

<Example 46: (2S,11aR)-6-((R)-sec-Butoxy)-8-methyl-2-((2-oxo-1,2,3,4-tetrahydro-1,6-naphthyridin-7-yl) oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 42 (111 mg, 0.36 mmol) and the compound obtained in Reference Example 85 (89.5 mg, 0.55 mmol) were used as raw materials, to thereby produce the title compound (61.7 mg).

$^1$H-NMR (DMSO-$d_6$) δ: 10.42 (1H, s), 7.87 (1H, s), 6.71 (1H, s), 6.50 (1H, s), 6.09 (1H, s), 5.49-5.45 (1H, m), 4.24-4.21 (1H, m), 4.13-4.03 (2H, m), 3.93-3.90 (1H, m), 3.83-3.80 (1H, m), 3.77-3.72 (1H, m), 2.80 (2H, t, J = 7.3 Hz), 2.47-2.45 (2H, m), 2.25-2.22 (4H, m), 2.06-2.03 (1H, m), 1.54-1.47 (2H, m), 1.18 (3H, d, J = 6.4 Hz), 0.80 (3H, t, J = 7.5 Hz).
LCMS: 452.2 $[M+H]^+$ (Rt= 1.950 min).

<Example 47: (2S,11aR)-6-((S)-sec-Butoxy)-8-methyl-2-((2-oxo-1,2,3,4-tetrahydro-1,6-naphthyridin-7-yl) oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 43 (109 mg, 0.36 mmol) and the compound obtained in Reference Example 85 (87.9 mg, 0.54 mmol) were used as raw materials, to thereby produce the title compound (57.1 mg).

$^1$H-NMR (DMSO-$d_6$) δ: 10.41 (1H, s), 7.88 (1H, s), 6.70 (1H, s), 6.49 (1H, s), 6.12 (1H, s), 5.49-5.47 (1H, m), 4.27-4.24 (1H, m), 4.10-4.05 (2H, m), 3.96-3.93 (1H, m), 3.80-3.75 (2H, m), 2.81 (2H, t, J = 7.5 Hz), 2.48-2.46 (2H, m), 2.25-2.22 (4H, m), 2.10-2.03 (1H, m), 1.63-1.46 (2H, m), 1.15 (3H, d, J = 5.9 Hz), 0.91 (3H, t, J = 7.3 Hz).
LCMS: 452.2 $[M+H]^+$ (Rt= 1.963 min).

<Example 48: (2S,11aR)-6-Cyclobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 25 were carried out except that the compound obtained in Reference Example 44 (88.4 mg, 0.29 mmol) and the compound obtained in Reference Example 84 (109 mg, 0.35 mmol) were used as raw materials, to thereby produce the title compound (22.8 mg).

$^1$H-NMR ($CDCl_3$) δ: 8.01 (1H, s), 7.86 (1H, s), 6.47 (1H, s), 6.43 (1H, s), 6.11 (1H, s), 5.63-5.58 (1H, m), 4.69-4.62 (1H, m), 4.18-4.03 (4H, m), 3.91 (1H, dd, J = 12.8, 4.6 Hz), 2.91 (2H, t, J = 7.5 Hz), 2.68-2.64 (2H, m), 2.45-2.36 (2H, m), 2.34-2.10 (7H, m), 1.86-1.77 (1H, m), 1.69-1.57 (1H, m).
LCMS: 450.2 $[M+H]^+$ (Rt= 1.920 min).

<Example 49: (2S,11aR)-6-(Cyclopentyloxy)-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one>

[Step 1] tert-Butyl (2R,4S)-4-((tert-Butyldiphenylsilyl)oxy)-2-((3-hydroxy-2-(methoxycarbonyl)-5-methylphenoxy) methyl)pyrrolidin-1-carboxylate

The same procedures as those in Step 1 to Step 2 of Reference Example 30 were carried out except that the compound obtained in Step 1 of Reference Example 2 (1.84 g, 8.84 mmol) and the compound obtained in Reference Example 22 (3.10 g, 6.80 mmol) were used as raw materials, to thereby produce the title compound (3.59 g).
LCMS: 520.3 $[M+H-Boc]^+$ (Rt= 3.075 min).

[Step 2] tert-Butyl (2R,4S)-4-((tert-butyldiphenylsilyl)oxy)-2-((3-(cyclopentyloxy)-2-(methoxycarbonyl)-5-methylphenoxy)methyl)pyrrolidin-1-carboxylate

The compound obtained in Step 1 (620 mg, 1.00 mmol), cyclopentanol (272 μL, 3.00 mmol), and triphenylphosphine (525 mg, 2.00 mmol) were dissolved in THF (5 mL), and to the resulting solution, DIAD (389 μL, 2.00 mmol) was added dropwise at 0°C. After the drop was finished, the mixture was agitated overnight at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (671 mg).
LCMS: 588.4 $[M+H-Boc]^+$ (Rt= 3.147 min).

[Step 3] tert-Butyl (2R,4S)-2-((3-(cyclopentyloxy)-2-(methoxycarbonyl)-5-methylphenoxy)methyl)-4-hydroxy pyrrolidin-1-carboxylate

The same procedures as those in Step 4 of Example 6 were carried out except that the compound obtained in Step 2 (671 mg, 0.98 mmol) was used as a raw material, to thereby produce the title compound (401 mg).
LCMS: 350.2 $[M+H-Boc]^+$ (Rt= 2.362 min).

[Step 4] tert-Butyl (2R,4S)-2-((3-(cyclopentyloxy)-2-(methoxycarbonyl)-5-methylphenoxy)methyl)-4-((2-oxo-1-((2-(trimethylsilyl)ethoxy)methyl)-1,2-dihydro-1,6-naphthyridin-7-yl)oxy)pyrrolidin-1-carboxylate

The same procedures as those in Step 1 of Example 25 were carried out except that the compound obtained in Step 3 (401 mg, 0.89 mmol) and the compound obtained in Reference Example 84 (333 mg, 1.07 mmol) were used as raw materials, to thereby produce the title compound (749 mg).
LCMS: 724.4 $[M+H]^+$ (Rt= 2.877 min).

[Step 5] 2-(((2R,4S)-1-(tert-Butoxycarbonyl)-4-((2-oxo-1-((2-(trimethylsilyl)ethoxy)methyl)-1,2-dihydro-1,6-naphthyridin-7-yl)oxy)pyrrolidin-2-yl)methoxy)-6-(cyclopentyloxy)-4-methyl benzoic acid

The same procedures as those in Step 2 of Example 1 were carried out except that the compound obtained in Step 4 (749 mg, 1.04 mmol) was used as a raw material, to thereby produce a crude product of the title compound.
LCMS: 710.4 $[M+H]^+$ (Rt= 2.650 min).

[Step 6] 2-(Cyclopentyloxy)-4-methyl-6-(((2R,4S)-4-((2-oxo-1,2-dihydro-1,6-naphthyridin-7-yl)oxy)pyrrolidin-2-yl)methoxy)benzoic acid

The crude product of the compound obtained in Step 5 was dissolved in 1,4-dioxane (5.17 mL), and to the resultant solution, 4M hydrochloric acid/1,4-dioxane solution (5.17 mL, 20.7 mmol) was added dropwise at 0°C. After the drop was finished, the mixture was agitated overnight at room temperature, the reaction mixture was concentrated under reduced pressure. The residue was 1,4-dioxane azeotroped with to thereby produce a crude product of the title compound.
LCMS: 480.2 $[M+H]^+$ (Rt= 1.803 min).

[Step 7] (2S,11aR)-6-(Cyclopentyloxy)-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one

The same procedures as those in Step 4 of Example 1 were carried out except that the crude product of the compound obtained in Step 6 was used as a raw material, to thereby produce the title compound (265 mg).

$^1$H-NMR (DMSO-$d_6$) δ: 11.82 (1H, s), 8.52 (1H, s), 7.90 (1H, d, J = 9.6 Hz), 6.71 (1H, s), 6.49 (1H, s), 6.43 (1H, s), 6.36 (1H, d, J = 9.6 Hz), 5.60-5.57 (1H, m), 4.78-4.75 (1H, m), 4.13-4.05 (2H, m), 3.99-3.94 (1H, m), 3.85-3.80 (2H, m), 2.31-2.28 (4H, m), 2.13-2.09 (1H, m), 1.66-1.60 (8H, m).
LCMS: 462.2 $[M+H]^+$ (Rt= 2.103 min).

<Example 50: (2S,11aR)-6-(Cyclopentyloxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Step 3 of Example 25 were carried out except that the compound obtained in

Example 49 (207 mg, 0.45 mmol) was used as a raw material, to thereby produce the title compound (152 mg).

$^1$H-NMR (DMSO-$d_6$) δ: 10.41 (1H, s), 7.88 (1H, s), 6.70 (1H, s), 6.49 (1H, s), 6.12 (1H, s), 5.49-5.46 (1H, m), 4.77-4.75 (1H, m), 4.09-4.05 (2H, m), 3.98-3.90 (1H, m), 3.80-3.74 (2H, m), 2.81 (2H, t, J = 7.5 Hz), 2.48-2.46 (2H, m), 2.25-2.21 (4H, m), 2.08-2.05 (1H, m), 1.81-1.44 (8H, m).
LCMS: 464.2 $[M+H]^+$ (Rt= 1.971 min).

<Example 51: (2S,11aR)-6-((2,2-Difluorocyclopentyl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 45 (70.7 mg, 0.20 mmol) and the compound obtained in Reference Example 85 (49.2 mg, 0.30 mmol) were used as raw materials, to thereby produce the title compound (30.0 mg).

$^1$H-NMR (DMSO-$d_6$) δ: 10.36 (1H, s), 7.82 (1H, s), 6.78 (1H, s), 6.53 (1H, d, J = 9.6 Hz), 6.05 (1H, d, J = 18.3 Hz), 5.44-5.42 (1H, m), 4.71-4.60 (1H, m), 4.07-4.03 (2H, m), 3.92-3.88 (1H, m), 3.80-3.66 (2H, m), 2.75 (2H, t, J = 7.5 Hz), 2.42-2.40 (2H, m), 2.26-1.89 (8H, m), 1.84-1.53 (3H, m).
LCMS: 500.2 $[M+H]^+$ (Rt= 1.978 min).

<Example 52: (2S,11aR)-6-Isobutyl-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 25 were carried out except that the compound obtained in Reference Example 46 (78.6 mg, 0.27 mmol) and the compound obtained in Reference Example 84 (101 mg, 0.33 mmol) were used as raw materials, to thereby produce the title compound (45.4 mg).

$^1$H-NMR ($CDCl_3$) δ: 8.08 (1H, s), 7.87 (1H, s), 6.82 (1H, s), 6.72 (1H, s), 6.00 (1H, s), 5.64-5.60 (1H, m), 4.24-3.90 (5H, m), 3.26 (1H, dd, J = 12.8, 7.3 Hz), 2.97-2.85 (2H, m), 2.71-2.59 (2H, m), 2.38-2.30 (5H, m), 2.07-2.01 (1H, m), 1.80-1.70 (1H, m), 0.85 (3H, d, J = 6.4 Hz), 0.83 (3H, d, J = 6.4 Hz).
LCMS: 436.3 $[M+H]^+$ (Rt= 2.041 min).

<Example 53: (2S,11aR)-6-(2,6-Difluorophenyl)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 25 were carried out except that the compound obtained in Reference Example 47 (77.4 mg, 0.22 mmol) and the compound obtained in Reference Example 84 (83.6 mg, 0.27 mmol) were used as raw materials, to thereby produce the title compound (52.0 mg).

$^1$H-NMR ($CDCl_3$) δ: 7.85 (1H, s), 7.70 (1H, s), 7.25-7.18 (1H, m), 7.02 (1H, s), 6.99-6.94 (2H, m), 6.71 (1H, t, J = 8.9 Hz), 6.10 (1H, s), 5.64-5.60 (1H, m), 4.33-4.22 (2H, m), 4.16-4.14 (1H, m), 4.00-3.96 (1H, m), 3.84 (1H, dd, J = 13.0, 4.8 Hz), 2.92 (2H, t, J = 7.5 Hz), 2.68-2.65 (2H, m), 2.43-2.37 (4H, m), 2.09-2.03 (1H, m).
LCMS: 492.2 $[M+H]^+$ (Rt= 2.007 min).

<Example 54: (2S,11aR)-8-Methyl-2-((2-oxo-1,2,3,4-tetrahydro-1,6-naphthyridin-7-yl)oxy)-6-(pyrrolidin-1-yl)-2,3,11,11a-tetrahydro-1H,5H-benzo[f]pyrrolo[2,1-c][1,4]oxazepin-5-one>

The same procedures as those in Example 25 were carried out except that the compound obtained in Reference Example 48 (30.1 mg, 0.10 mmol) and the compound obtained in Reference Example 84 (40.2 mg, 0.13 mmol) were used as raw materials, to thereby produce the title compound (8.9 mg).

$^1$H-NMR ($CDCl_3$) δ: 7.88 (1H, s), 7.47 (1H, s), 6.37 (1H, s), 6.25 (1H, s), 5.91 (1H, s), 5.66-5.62 (1H, m), 4.36-4.40 (1H, m), 4.13-3.93 (4H, m), 3.39-3.37 (2H, m), 3.02 (2H, t, J = 8.2 Hz), 2.93 (2H, t, J = 7.3 Hz), 2.68-2.66 (2H, m), 2.39-2.35 (1H, m), 2.27 (3H, s), 2.09-2.03 (1H, m), 1.96-1.95 (2H, m), 1.83-1.81 (2H, m).
LCMS: 449.2 $[M+H]^+$ (Rt= 1.722 min).

<Example 55: (2S,11aR)-7-Fluoro-6-methoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

[Step 1] (2S,11aR)-7-Fluoro-6-methoxy-8-methyl-2-((2-oxo-1-((2-(trimethylsilyl)ethoxy)methyl)-1,2-dihydro-1,6-naphthyridin-7-yl)oxy)-2,3,11,11a-tetrahydro-1H,5H-benzo[f]pyrrolo[2,1-c][1,4]oxazepin-5-one

The compound obtained in Reference Example 49 (40.6 mg, 0.14 mmol) was dissolved in DMF (1 mL), and to the resulting solution, 50% sodium hydride (10.4 mg, 0.22 mmol) was added at 0°C, and the mixture was brought to room temperature, and agitated for 1 hour. The reaction mixture was combined with the compound obtained in Reference Example 84 (58.3 mg, 0.19 mmol), and agitated for 4 hours at room temperature. The reaction mixture was combined with saturated sodium bicarbonate aqueous solution, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (42.9 mg).

$^1$H-NMR ($CDCl_3$) δ: 8.32 (1H, s), 7.64 (1H, d, J = 9.6 Hz), 6.75 (1H, s), 6.64 (1H, d, J = 5.5 Hz), 6.51 (1H, d, J = 9.6 Hz), 5.75-5.70 (1H, m), 5.63 (1H, d, J = 10.5 Hz), 5.58 (1H, d, J = 11.0 Hz), 4.22-4.04 (5H, m), 4.01 (3H, s), 3.67-3.63 (2H, m), 2.44-2.37 (1H, m), 2.27 (3H, d, J = 1.8 Hz), 2.13 (1H, td, J = 9.5, 4.7 Hz), 0.96-0.92 (2H, m), -0.04 (9H, s).
LCMS: 556.3 $[M+H]^+$ (Rt= 2.491 min).

[Step 2] (2S,11aR)-7-Fluoro-6-methoxy-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one

The compound obtained in Step 1 (42.9 mg, 0.08 mmol) was dissolved in THF (0.40 mL), combined with 6M hydrochloric acid aqueous solution (0.40 mL, 2.32 mmol), and agitated for 2 days at room temperature. The reaction mixture was combined with saturated sodium bicarbonate aqueous solution, and extracted with dichloromethane. The organic phase was washed twice with saturated saline solution, dried with anhydrous magnesium sulfate, and concentrated under reduced pressure. The residue was purified with silica gel column chromatography (ethyl acetate/methanol) to thereby produce the title compound (35.6 mg).

$^1$H-NMR ($CDCl_3$) δ: 9.75 (1H, s), 8.36 (1H, s), 7.72 (1H, d, J = 9.6 Hz), 6.64 (1H, d, J = 5.9 Hz), 6.51 (1H, d, J = 9.6 Hz), 6.43 (1H, s), 5.74-5.70 (1H, m), 4.22-4.01 (8H, m), 2.43-2.35 (1H, m), 2.27 (3H, d, J = 1.8 Hz), 2.12 (1H, dt, J = 13.6, 5.3 Hz).
LCMS: 426.2 $[M+H]^+$ (Rt= 2.020 min).

[Step 3] (2S,11aR)-7-Fluoro-6-methoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

The compound obtained in Step 2 (35.6 mg, 0.08 mmol) was dissolved in THF (1.67 mL), combined with 20% hydroxide palladium-activated carbon (17.8 mg), and agitated for 5 days under hydrogen atmosphere at normal pressure at room temperature. The reaction mixture was filtered with cerite, and the filtrate was concentrated under reduced pressure. The residue was purified with silica gel column chromatography (ethyl acetate/methanol) to thereby produce the title compound (19.2 mg).

$^1$H-NMR ($CDCl_3$) δ: 7.85 (1H, s), 7.75 (1H, s), 6.63 (1H, d, J = 5.9 Hz), 6.07 (1H, s), 5.64-5.59 (1H, m), 4.18 (1H, dd, J = 8.9, 3.9 Hz), 4.14-3.98 (7H, m), 2.93-2.90 (2H, m), 2.68-2.64 (2H, m), 2.38-2.32 (1H, m), 2.27 (3H, d, J = 2.3 Hz), 2.11-2.06 (1H, m).
LCMS: 428.2 $[M+H]^+$ (Rt= 1.860 min).

<Example 56: (2S,11aR)-6-Ethoxy-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 50 (40.2 mg, 0.14 mmol) and the compound obtained in Reference Example 84 (55.0 mg, 0.18 mmol) were used as raw materials, to thereby produce the title compound (23.5 mg).

$^1$H-NMR ($CDCl_3$) δ: 7.86 (1H, s), 7.54 (1H, s), 6.63 (1H, d, J = 5.9 Hz), 6.05 (1H, s), 5.64-5.59 (1H, m), 4.31-3.97 (7H, m), 2.92 (2H, t, J = 7.8 Hz), 2.68-2.64 (2H, m), 2.37-2.30 (1H, m), 2.26 (3H, d, J = 2.3 Hz), 2.09 (1H, dt, J = 13.6, 5.3 Hz),

1.38 (3H, t, J = 6.9 Hz).
LCMS: 442.2 $[M+H]^+$ (Rt= 1.899 min).

<Example 57: (2S,11aR)-7-Fluoro-8-methyl-2-((2-oxo-1,2,3,4-tetrahydro-1,6-naphthyridin-7-yl)oxy)-6-propoxy-2,3,11,11a-tetrahydro-1H,5H-benzo[f]pyrrolo[2,1-c][1,4]oxazepin-5-one>

The same procedures as those in Example 55 were carried out except that the compound obtained in Reference Example 51 (104 mg, 0.34 mmol) and the compound obtained in Reference Example 84 (136 mg, 0.44 mmol) were used as raw materials, to thereby produce the title compound (19.8 mg).

$^1$H-NMR ($CDCl_3$) δ: 7.86 (1H, s), 7.52 (1H, s), 6.61 (1H, d, J = 5.9 Hz), 6.04 (1H, s), 5.64-5.59 (1H, m), 4.21-4.15 (2H, m), 4.13-3.97 (5H, m), 2.92 (2H, t, J = 7.5 Hz), 2.68-2.64 (2H, m), 2.39-2.35 (1H, m), 2.26 (3H, d, J = 2.3 Hz), 2.12-2.05 (1H, m), 1.84-1.75 (2H, m), 0.99 (3H, t, J = 7.3 Hz).
LCMS: 456.2 $[M+H]^+$ (Rt= 1.979 min).

<Example 58: (2S,11aR)-6-Butoxy-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 52 (135 mg, 0.42 mmol) and the compound obtained in Reference Example 84 (169 mg, 0.54 mmol) were used as raw materials, to thereby produce the title compound (21.4 mg).

$^1$H-NMR ($CDCl_3$) δ: 7.98 (1H, s), 7.86 (1H, s), 6.61 (1H, d, J = 5.5 Hz), 6.08 (1H, s), 5.64-5.59 (1H, m), 4.24-3.90 (7H, m), 2.92 (2H, t, J = 7.5 Hz), 2.67-2.65 (2H, m), 2.36-2.30 (1H, m), 2.26 (3H, d, J = 1.4 Hz), 2.11-2.07 (1H, m), 1.80-1.72 (2H, m), 1.50-1.40 (2H, m), 0.93 (3H, t, J = 7.3 Hz).
LCMS: 470.2 $[M+H]^+$ (Rt= 2.033 min).

<Example 59: (2S,11aR)-7-Fluoro-8-methyl-2-((2-oxo-1,2,3,4-tetrahydro-1,6-naphthyridin-7-yl)oxy)-6-(pentyloxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 53 (85.3 mg, 0.25 mmol) and the compound obtained in Reference Example 84 (102 mg, 0.33 mmol) were used as raw materials, to thereby produce the title compound (37.9 mg).

$^1$H-NMR ($CDCl_3$) δ: 7.86 (1H, s), 7.69 (1H, s), 6.61 (1H, d, J = 5.9 Hz), 6.06 (1H, s), 5.64-5.59 (1H, m), 4.23-3.90 (7H, m), 2.92 (2H, t, J = 7.5 Hz), 2.68-2.64 (2H, m), 2.36-2.30 (1H, m), 2.26 (3H, d, J = 1.8 Hz), 2.14-2.07 (1H, m), 1.83-1.72 (2H, m), 1.43-1.31 (4H, m), 0.89 (3H, t, J = 7.1 Hz).
LCMS: 484.2 $[M+H]^+$ (Rt= 2.106 min).

<Example 60: (2S,11aR)-7-Fluoro-6-(3-fluoropropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 54 (50.8 mg, 0.16 mmol) and the compound obtained in Reference Example 84 (62.7 mg, 0.20 mmol) were used as raw materials, to thereby produce the title compound (30.6 mg).

$^1$H-NMR ($CDCl_3$) δ: 7.86 (1H, s), 7.62 (1H, s), 6.64 (1H, d, J = 5.9 Hz), 6.06 (1H, s), 5.65-5.60 (1H, m), 4.73-4.70 (1H, m), 4.62-4.57 (1H, m), 4.32-4.29 (1H, m), 4.24-4.05 (4H, m), 4.02-3.97 (2H, m), 2.92 (2H, t, J = 7.5 Hz), 2.68-2.64 (2H, m), 2.39-2.33 (1H, m), 2.26 (3H, d, J = 2.3 Hz), 2.18-2.09 (3H, m).
LCMS: 474.2 $[M+H]^+$ (Rt= 1.924 min).

<Example 61: (2S,11aR)-7-Fluoro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 55 (60.0 mg, 0.19 mmol) and the compound obtained in Reference Example 84 (72.3 mg, 0.23 mmol) were used as raw materials, to thereby produce the title compound (29.5 mg).

$^1$H-NMR ($CDCl_3$) δ: 8.20 (1H, s), 7.86 (1H, s), 6.62 (1H, d, J = 5.5 Hz), 6.03 (1H, s), 5.64-5.59 (1H, m), 4.51-4.42 (1H, m), 4.17 (1H, dd, J = 8.5, 3.4 Hz), 4.13-4.03 (3H, m), 3.99 (1H, dd, J = 13.3, 5.0 Hz), 2.98-2.86 (2H, m), 2.71-2.59 (2H, m), 2.38-2.32 (1H, m), 2.26 (3H, d, J = 1.8 Hz), 2.06 (1H, dt, J = 13.3, 5.4 Hz), 1.36 (3H, d, J = 5.9 Hz), 1.26 (3H, d, J = 6.4 Hz).
LCMS: 456.2 $[M+H]^+$ (Rt= 1.925 min).

<Example 62: (2S,11aR)-7-Fluoro-6-isopropoxy-8-methyl-2-((2-oxo-2,3,4,5-tetrahydro-1H-pyrido[4,3-b]azepin-8-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Step 1 of Reference Example 82 (15.0 mg, 0.05 mmol) and the compound obtained in Reference Example 90 (7.5 mg, 0.04 mmol) were used as raw materials, to thereby produce the title compound (3.9 mg).

$^1$H-NMR (CD3OD) δ: 7.97 (1H, s), 6.74 (1H, d, J = 5.9 Hz), 6.28 (1H, s), 5.63-5.58 (1H, m), 4.33-4.22 (2H, m), 4.19-4.07 (3H, m), 3.86 (1H, dd, J = 13.0, 4.3 Hz), 2.77-2.71 (2H, m), 2.45-2.32 (3H, m), 2.27 (3H, d, J = 2.3 Hz), 2.24-2.10 (3H, m), 1.29 (3H, d, J = 6.4 Hz), 1.18 (3H, d, J = 5.9 Hz).
LCMS: 470.2 $[M+H]^+$ (Rt= 2.057 min).

<Example 63: (2S,11aR)-9-Fluoro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 56 (236 mg, 0.76 mmol) and the compound obtained in Reference Example 84 (308 mg, 0.99 mmol) were used as raw materials, to thereby produce the title compound (147 mg).

$^1$H-NMR ($CDCl_3$) δ: 7.87 (1H, s), 7.61 (1H, s), 6.60 (1H, d, J = 5.5 Hz), 6.03 (1H, s), 5.65-5.60 (1H, m), 4.48-4.39 (1H, m), 4.26-4.23 (1H, m), 4.15-3.96 (4H, m), 2.92 (2H, t, J = 7.5 Hz), 2.68-2.64 (2H, m), 2.38-2.31 (1H, m), 2.28 (3H, d, J = 1.8 Hz), 2.13-2.11 (1H, m), 1.35 (3H, d, J = 5.9 Hz), 1.29 (3H, d, J = 6.4 Hz).
LCMS: 456.2 $[M+H]^+$ (Rt= 1.920 min).

<Example 64: (2S,11aR)-7,9-Difluoro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 57 (106 mg, 0.32 mmol) and the compound obtained in Reference Example 84 (121 mg, 0.39 mmol) were used as raw materials, to thereby produce the title compound (65.7 mg).

$^1$H-NMR ($CDCl_3$) δ: 7.87 (1H, s), 7.76 (1H, s), 6.00 (1H, s), 5.65-5.61 (1H, m), 4.45-4.38 (1H, m), 4.31-4.24 (1H, m), 4.10-4.05 (3H, m), 4.00 (1H, dd, J = 13.3, 5.0 Hz), 2.94-2.90 (2H, m), 2.68-2.64 (2H, m), 2.40-2.34 (1H, m), 2.24 (3H, t, J = 1.8 Hz), 2.09 (1H, dt, J = 13.7, 5.1 Hz), 1.35 (3H, d, J = 5.9 Hz), 1.24 (3H, d, J = 6.4 Hz).
LCMS: 474.2 $[M+H]^+$ (Rt= 1.970 min).

<Example 65: (2S,11aR)-7-Chloro-6-ethoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 58 (50.0 mg, 0.16 mmol) and the compound obtained in Reference Example 85 (39.4 mg, 0.24 mmol) were used as raw materials, to thereby produce the title compound (26.5 mg).

$^1$H-NMR ($CDCl_3$) δ: 8.60 (1H, s), 7.85 (1H, s), 6.75 (1H, s), 6.11 (1H, s), 5.64-5.59 (1H, m), 4.29-4.21 (1H, m), 4.18-4.16 (2H, m), 4.09-4.08 (2H, m), 4.04-4.02 (2H, m), 2.93-2.90 (2H, m), 2.67-2.63 (2H, m), 2.36-2.32 (4H, m), 2.11-2.06 (1H, m), 1.39 (3H, t, J = 7.1 Hz).
LCMS: 458.1 $[M+H]^+$ (Rt= 1.949 min).

<Example 66: (2S,11aR)-7-Chloro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference

Example 59 (69.0 mg, 0.21 mmol) and the compound obtained in Reference Example 85 (52.2 mg, 0.32 mmol) were used as raw materials, to thereby produce the title compound (34.4 mg).

$^1$H-NMR ($CDCl_3$) δ: 7.87 (1H, s), 7.67 (1H, br s), 6.73 (1H, s), 5.94 (1H, s), 5.65-5.61 (1H, m), 4.42-4.33 (1H, m), 4.22 (1H, dd, J = 8.9, 3.9 Hz), 4.13-3.91 (4H, m), 2.94-2.91 (2H, m), 2.68-2.64 (2H, m), 2.40-2.38 (4H, m), 2.07-2.02 (1H, m), 1.34 (3H, d, J = 6.4 Hz), 1.24 (3H, d, J = 5.9 Hz).
LCMS: 472.2 $[M+H]^+$ (Rt= 1.998 min).

<Example 67: (2S,11aR)-7,9-Dichloro-6-isopropoxy-8-methyl-2-((2-oxo-1,2,3,4-tetrahydro-1,6-naphthyridin-7-yl) oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 60 (27.4 mg, 0.08 mmol) and the compound obtained in Reference Example 85 (18.7 mg, 0.11 mmol) were used as raw materials, to thereby produce the title compound (12.4 mg).

$^1$H-NMR ($CDCl_3$) δ: 7.87-7.82 (2H, br m), 5.95 (1H, s), 5.65-5.63 (1H, m), 4.41-4.35 (2H, m), 4.15-4.03 (3H, m), 3.96 (1H, dd, J = 13.3, 4.6 Hz), 2.94-2.91 (2H, m), 2.68-2.64 (2H, m), 2.52 (3H, s), 2.43-2.37 (1H, m), 2.11-2.06 (1H, m), 1.34 (3H, d, J = 6.4 Hz), 1.23 (3H, d, J = 5.9 Hz).
LCMS: 506.1 $[M+H]^+$ (Rt= 2.106 min).

<Example 68: (2S,11aR)-6-(Cyclopropylmethoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The compound obtained in Reference Example 61 (109 mg, 0.23 mmol) and potassium carbonate (41.1 mg, 0.30 mmol) were dissolved in DMSO (2.29 mL), and to the resulting solution, the compound obtained in Reference Example 85 (37.5 mg, 0.23 mmol) was added, and agitated at 50°C for 2 days. The reaction mixture was then cooled to room temperature, combined with saturated sodium bicarbonate aqueous solution, and extracted with ethyl acetate. The organic phase was washed twice with saturated saline solution, dried with sodium sulfate, and concentrated under reduced pressure. The residue was purified with silica gel column chromatography (ethyl acetate/methanol) to thereby produce the title compound (19.9 mg).

$^1$H-NMR ($CDCl_3$) δ: 7.86 (1H, s), 7.62 (1H, s), 6.63 (1H, d, J = 5.5 Hz), 6.06 (1H, s), 5.64-5.59 (1H, m), 4.17-3.85 (7H, m), 2.92 (2H, t, J = 7.5 Hz), 2.66 (2H, t, J = 7.3 Hz), 2.36-2.30 (1H, m), 2.26 (3H, s), 2.14-2.06 (1H, m), 1.34-1.31 (1H, m), 0.56-0.55 (2H, m), 0.33-0.33 (2H, m).
LCMS: 468.2 $[M+H]^+$ (Rt= 1.982 min).

<Example 69: (2S,11aR)-7-Fluoro-8-methyl-2-((2-oxo-1,2,3,4-tetrahydro-1,6-naphthyridin-7-yl)oxy)-6-(2,2,2-trifluoroethoxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 62 (99.3 mg, 0.28 mmol) and the compound obtained in Reference Example 84 (115 mg, 0.37 mmol) were used as raw materials, to thereby produce the title compound (55.8 mg).

$^1$H-NMR ($CDCl_3$) δ: 7.86 (1H, s), 7.54 (1H, s), 6.72 (1H, d, J = 5.9 Hz), 6.05 (1H, s), 5.65-5.61 (1H, m), 4.71-4.66 (1H, m), 4.38-4.34 (1H, m), 4.21 (1H, d, J = 5.5 Hz), 4.09-3.96 (4H, m), 2.93-2.90 (2H, m), 2.67-2.65 (2H, m), 2.41-2.35 (1H, m), 2.28 (3H, d, J = 1.8 Hz), 2.10-2.05 (1H, m).
LCMS: 496.2 $[M+H]^+$ (Rt= 1.989 min).

<Example 70: (2S,11aR)-7-Fluoro-6-((1-fluorocyclopropyl)methoxy)-8-methyl-2-((2-oxo-1,2,3,4-tetrahydro-1,6-naphthyridin-7-yl)oxy)-2,3, 11,11a-tetrahydro- 1H,5H-benzo[f]pyrrolo[2,1-c][1,4]oxazepin-5-one>

The same procedures as those in Example 68 were carried out except that the compound obtained in Reference Example 63 (49.5 mg, 0.10 mmol) and the compound obtained in Reference Example 85 (17.1 mg, 0.10 mmol) were used as raw materials, to thereby produce the title compound (9.0 mg).

$^1$H-NMR ($CDCl_3$) δ: 7.86 (1H, s), 7.63 (1H, s), 6.66 (1H, d, J = 5.9 Hz), 6.08 (1H, s), 5.65-5.60 (1H, m), 4.54-4.30 (2H, m), 4.19-3.95 (5H, m), 2.92 (2H, t, J = 7.5 Hz), 2.68-2.64 (2H, m), 2.37-2.31 (1H, m), 2.27 (3H, d, J = 2.3 Hz), 2.12-2.06 (1H, m), 1.14 (1H, t, J = 5.3 Hz), 1.09 (1H, t, J = 5.3 Hz), 0.97-0.95 (1H, m), 0.83-0.79 (1H, m).

LCMS: 486.2 $[M+H]^+$ (Rt= 1.957 min).

<Example 71: (2S,11aR)-7-Fluoro-6-isobutoxy-8-methyl-2-((2-oxo-1,2,3,4-tetrahydro-1,6-naphthyridin-7-yl) oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 64 (132 mg, 0.41 mmol) and the compound obtained in Reference Example 84 (165 mg, 0.53 mmol) were used as raw materials, to thereby produce the title compound (49.6 mg).

$^1$H-NMR ($CDCl_3$) $\delta$: 7.88 (1H, s), 7.86 (1H, s), 6.61 (1H, d, J = 5.9 Hz), 6.06 (1H, s), 5.64-5.59 (1H, m), 4.13-3.97 (6H, m), 3.80-3.76 (1H, m), 2.92 (2H, t, J = 7.3 Hz), 2.67-2.64 (2H, m), 2.38-2.31 (1H, m), 2.26-2.25 (3H, m), 2.11-2.08 (2H, m), 1.00 (3H, d, J = 6.4 Hz), 0.97 (3H, d, J = 7.2 Hz).
LCMS: 470.2 $[M+H]^+$ (Rt= 2.044 min).

<Example 72: (2S,11aR)-7-Fluoro-6-(2-fluoro-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 65 (39.6 mg, 0.12 mmol) and the compound obtained in Reference Example 84 (46.9 mg, 0.15 mmol) were used as raw materials, to thereby produce the title compound (10.8 mg).

$^1$H-NMR ($CDCl_3$) $\delta$: 7.86 (1H, s), 7.63 (1H, s), 6.64 (1H, d, J = 5.9 Hz), 6.06 (1H, s), 5.65-5.61 (1H, m), 4.18-3.90 (7H, m), 2.92 (2H, t, J = 7.3 Hz), 2.67-2.63 (2H, m), 2.39-2.35 (1H, m), 2.26 (3H, d, J = 2.3 Hz), 2.09-2.06 (1H, m), 1.49 (3H, d, J = 4.6 Hz), 1.43 (3H, d, J = 4.1 Hz).
LCMS: 488.2 $[M+H]^+$ (Rt= 1.976 min).

<Example 73: (2S,11aR)-6-((1,3-Difluoropropan-2-yl)oxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 66 (20.7 mg, 0.06 mmol) and the compound obtained in Reference Example 85 (14.8 mg, 0.09 mmol) were used as raw materials, to thereby produce the title compound (9.1 mg).

$^1$H-NMR ($CDCl_3$) $\delta$: 7.86 (1H, s), 7.58 (1H, s), 6.70 (1H, d, J = 5.9 Hz), 6.05 (1H, s), 5.65-5.60 (1H, m), 4.87-4.84 (1H, m), 4.77-4.72 (2H, m), 4.67-4.55 (2H, m), 4.22-4.15 (1H, m), 4.11-4.02 (3H, m), 3.97 (1H, dd, J = 13.3, 5.0 Hz), 2.94-2.90 (2H, m), 2.66 (2H, td, J = 7.4, 2.1 Hz), 2.39-2.33 (1H, m), 2.28 (3H, d = 2.0 Hz), 2.06 (1H, td, J = 9.5, 4.7 Hz).
LCMS: 492.2 $[M+H]^+$ (Rt= 1.906 min).

<Example 74: (2S,11aR)-6-((R)-sec-Butoxy)-7-fluoro-8-methyl-2-((2-oxo-1,2,3,4-tetrahydro-1,6-naphthyridin-7-yl) oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 67 (103 mg, 0.32 mmol) and the compound obtained in Reference Example 84 (128 mg, 0.41 mmol) were used as raw materials, to thereby produce the title compound (39.1 mg).

$^1$H-NMR ($CDCl_3$) $\delta$: 7.87 (1H, s), 7.56 (1H, s), 6.60 (1H, d, J = 5.9 Hz), 6.00 (1H, s), 5.64-5.59 (1H, m), 4.28-4.25 (1H, m), 4.16 (1H, dd, J = 9.1, 3.7 Hz), 4.08-3.98 (4H, m), 2.92 (2H, t, J = 7.3 Hz), 2.67-2.64 (2H, m), 2.37-2.34 (1H, m), 2.25 (3H, d, J = 2.3 Hz), 2.10-2.05 (1H, m), 1.74-1.71 (1H, m), 1.60-1.53 (1H, m), 1.30 (3H, d, J = 5.9 Hz), 0.90 (3H, t, J = 7.5 Hz).
LCMS: 470.3 $[M+H]^+$ (Rt= 2.001 min).

<Example 75: (2S,11aR)-6-((S)-sec-Butoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 68 (73.5 mg, 0.23 mmol) and the compound obtained in Reference Example 84 (91.8 mg, 0.30 mmol) were used as raw materials, to thereby produce the title compound (45.0 mg).

$^1$H-NMR ($CDCl_3$) $\delta$: 7.87 (1H, s), 7.57 (1H, s), 6.62 (1H, d, J = 5.9 Hz), 5.96 (1H, s), 5.64-5.60 (1H, m), 4.23-4.19 (2H,

m), 4.14-3.93 (4H, m), 2.93-2.90 (2H, m), 2.68-2.64 (2H, m), 2.36-2.35 (1H, m), 2.26 (3H, d, J = 2.3 Hz), 2.08-2.02 (1H, m), 1.85-1.78 (1H, m), 1.67-1.60 (1H, m), 1.17 (3H, d, J = 5.9 Hz), 1.01-0.97 (3H, m).
LCMS: 470.2 $[M+H]^+$ (Rt= 1.998 min).

<Example 76: (2S,11aR)-7-Fluoro-6-(((R)-1-fluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 69 (115 mg, 0.35 mmol) and the compound obtained in Reference Example 84 (141 mg, 0.45 mmol) were used as raw materials, to thereby produce the title compound (46.3 mg).

$^1$H-NMR ($CDCl_3$) δ: 7.86 (1H, s), 7.78 (1H, s), 6.65 (1H, d, J = 5.9 Hz), 6.06 (1H, s), 5.64-5.60 (1H, m), 4.64-4.32 (3H, m), 4.18-4.16 (1H, m), 4.05-3.97 (4H, m), 2.93-2.90 (2H, m), 2.70-2.63 (2H, m), 2.38-2.32 (1H, m), 2.27 (3H, d, J = 1.8 Hz), 2.08-2.02 (1H, m), 1.40-1.38 (3H, m).
LCMS: 474.2 $[M+H]^+$ (Rt= 1.901 min).

<Example 77: (2S,11aR)-6-(((R)-1,1-Difluoropropan-2-yl)oxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 70 (116 mg, 0.34 mmol) and the compound obtained in Reference Example 84 (119 mg, 0.38 mmol) were used as raw materials, to thereby produce the title compound (40.4 mg).

$^1$H-NMR ($CDCl_3$) δ: 7.86 (1H, s), 7.53 (1H, s), 6.67 (1H, d, J = 5.9 Hz), 6.03 (1H, s), 5.88-5.60 (2H, m), 4.33-4.29 (1H, m), 4.20-4.02 (4H, m), 3.92 (1H, dd, J = 13.3, 4.6 Hz), 2.93-2.90 (2H, m), 2.66 (2H, td, J = 7.4, 2.6 Hz), 2.39-2.36 (1H, m), 2.27 (3H, d, J = 2.3 Hz), 2.06-2.00 (1H, m), 1.47 (3H, d, J = 6.4 Hz).
LCMS: 492.2 $[M+H]^+$ (Rt= 1.960 min).

<Example 78: (2S,11aR)-7-Fluoro-8-methyl-2-((2-oxo-1,2,3,4-tetrahydro-1,6-naphthyridin-7-yl)oxy)-6-(((R)-1,1,1-trifluoropropan-2-yl)oxy)-2,3,11,11 a-tetrahydro-1H,5H-benzo[f]pyrrolo[2,1-c][1,4]oxazepin-5-one>

The same procedures as those in Example 55 were carried out except that the compound obtained in Reference Example 71 (71.8 mg, 0.20 mmol) and the compound obtained in Reference Example 84 (73.7 mg, 0.24 mmol) were used as raw materials, to thereby produce the title compound (33.9 mg).

$^1$H-NMR ($CDCl_3$) δ: 7.86 (1H, s), 7.46 (1H, s), 6.69 (1H, d, J = 5.9 Hz), 6.03 (1H, s), 5.65-5.60 (1H, m), 4.63-4.56 (1H, m), 4.23-4.15 (1H, m), 4.08-3.83 (4H, m), 2.94-2.90 (2H, m), 2.68-2.64 (2H, m), 2.40-2.37 (1H, m), 2.27 (3H, d, J = 1.8 Hz), 2.06-2.00 (1H, m), 1.59 (3H, d, J = 6.4 Hz).
LCMS: 510.2 $[M+H]^+$ (Rt= 1.962 min).

<Example 79: (2S,11aR)-6-(Cyclopentyloxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 72 (41.4 mg, 0.12 mmol) and the compound obtained in Reference Example 84 (49.9 mg, 0.16 mmol) were used as raw materials, to thereby produce the title compound (8.6 mg).

$^1$H-NMR ($CDCl_3$) δ: 7.87 (1H, s), 7.55 (1H, s), 6.60 (1H, d, J = 5.9 Hz), 5.99 (1H, s), 5.64-5.59 (1H, m), 4.84-4.80 (1H, m), 4.13-3.97 (5H, m), 2.93-2.91 (2H, m), 2.68-2.64 (2H, m), 2.39-2.33 (1H, m), 2.25 (3H, d, J = 2.3 Hz), 2.09-2.06 (1H, m), 2.04-2.02 (1H, m), 1.89-1.49 (7H, m).
LCMS: 482.3 $[M+H]^+$ (Rt= 2.029 min).

<Example 80: (2S,11aR)-7-Fluoro-8-methyl-2-((2-oxo-1,2,3,4-tetrahydro-1,6-naphthyridin-7-yl)oxy)-6-(((S)-tetrahydrofuran-3-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 73 (33.8 mg, 0.10 mmol) and the compound obtained in Reference Example 84 (40.5 mg, 0.13 mmol) were used as raw materials, to thereby produce the title compound (7.8 mg).

$^{1}H$-NMR ($CDCl_3$) δ: 7.87 (1H, s), 7.79 (1H, s), 6.65 (1H, d, J = 5.9 Hz), 6.04 (1H, s), 5.63-5.59 (1H, m), 5.01-4.99 (1H, m), 4.20-3.83 (9H, m), 2.93-2.90 (2H, m), 2.67-2.65 (2H, m), 2.40-2.34 (1H, m), 2.26-2.24 (4H, m), 2.08-2.00 (2H, m).
LCMS: 484.2 $[M+H]^+$ (Rt= 1.862 min).

<Example 81: (2S,11aR)-7-Fluoro-8-methyl-2-((2-oxo-1,2,3,4-tetrahydro-1,6-naphthyridin-7-yl)oxy)-6-(((R)-tetrahydrofuran-3-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 74 (59.6 mg, 0.18 mmol) and the compound obtained in Reference Example 84 (71.4 mg, 0.23 mmol) were used as raw materials, to thereby produce the title compound (8.2 mg).

$^{1}H$-NMR ($CDCl_3$) δ: 7.86 (1H, s), 7.67 (1H, s), 6.64 (1H, d, J = 5.9 Hz), 6.07 (1H, s), 5.65-5.61 (1H, m), 5.00-4.96 (1H, m), 4.19-3.87 (9H, m), 2.93-2.90 (2H, m), 2.66-2.65 (2H, m), 2.47-2.44 (1H, m), 2.39-2.37 (1H, m), 2.27 (3H, d, J = 2.3 Hz), 2.09-2.01 (2H, m).
LCMS: 484.2 $[M+H]^+$ (Rt= 1.843 min).

<Example 82: (2S,11S,11aR)-7-Fluoro-6-isopropoxy-8,11-dimethyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 75 (155 mg, 0.48 mmol) and the compound obtained in Reference Example 85 (118 mg, 0.72 mmol) were used as raw materials, to thereby produce the title compound (127 mg).
LCMS: 470.2 $[M+H]^+$ (Rt= 2.002 min).

<Example 83: (2S,11R,11aR)-7-Fluoro-6-isopropoxy-8,11-dimethyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 76 (13.0 mg, 0.04 mmol) and the compound obtained in Reference Example 85 (19.8 mg, 0.12 mmol) were used as raw materials, to thereby produce the title compound (5.1 mg).
LCMS: 470.3 $[M+H]^+$ (Rt= 1.973 min).

<Example 84: (2S,11aR)-7-Fluoro-8-methyl-2-((2-oxo-1,2,3,4-tetrahydro-1,6-naphthyridin-7-yl)oxy)-6-(pyrrolidin-1-yl)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 77 (42.1 mg, 0.13 mmol) and the compound obtained in Reference Example 84 (53.1 mg, 0.17 mmol) were used as raw materials, to thereby produce the title compound (10.2 mg).

$^{1}H$-NMR ($CDCl_3$) δ: 7.88 (1H, s), 7.75 (1H, s), 6.26 (1H, d, J = 5.5 Hz), 5.95 (1H, s), 5.66-5.62 (1H, m), 4.32-4.26 (1H, m), 4.11-3.93 (4H, m), 3.66-3.62 (2H, m), 3.14 (2H, t, J = 7.5 Hz), 2.92 (2H, t, J = 7.3 Hz), 2.66 (2H, t, J = 7.3 Hz), 2.42-2.36 (1H, m), 2.19 (3H, d, J = 2.7 Hz), 2.08-2.07 (1H, m), 1.88-1.74 (4H, m).
LCMS: 467.2 $[M+H]^+$ (Rt= 1.707 min).

<Example 85: (2S,11aR)-7-Fluoro-6-isopropyl-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 78 (38.3 mg, 0.13 mmol) and the compound obtained in Reference Example 84 (52.8 mg, 0.17 mmol) were used as raw materials, to thereby produce the title compound (22.1 mg).

$^{1}H$-NMR ($CDCl_3$) δ7.87 (1H, s), 7.49 (1H, s), 6.74 (1H, d, J = 5.9 Hz), 6.01 (1H, s), 5.66-5.61 (1H, m), 4.15-3.90 (5H, m), 3.50-3.43 (1H, m), 2.94-2.91 (2H, m), 2.68-2.64 (2H, m), 2.39-2.32 (1H, m), 2.24 (3H, d, J = 2.3 Hz), 2.13-2.10 (1H, m), 1.38 (3H, d, J = 6.9 Hz), 1.29 (3H, d, J = 7.3 Hz).
LCMS: 440.2 $[M+H]^+$ (Rt= 2.032 min).

<Example 86: (2S,11aR)-6-Cyclopentyl-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 79 (72.0 mg, 0.23 mmol) and the compound obtained in Reference Example 84 (91.1 mg, 0.29 mmol) were used as raw materials, to thereby produce the title compound (45.5 mg).

$^1$H-NMR ($CDCl_3$) δ: 7.87 (1H, s), 7.47 (1H, s), 6.73 (1H, d, J = 5.9 Hz), 6.02 (1H, s), 5.66-5.61 (1H, m), 4.10-3.93 (5H, m), 3.50-3.48 (1H, m), 2.92 (2H, t, J = 7.3 Hz), 2.68-2.64 (2H, m), 2.39-2.32 (1H, m), 2.23 (3H, d, J = 1.8 Hz), 2.17-2.10 (1H, m), 1.83 (5H, br s), 1.63-1.61 (3H, br m).
LCMS: 466.2 $[M+H]^+$ (Rt= 2.094 min).

<Example 87: (2S,11aS)-7-Fluoro-6-isopropoxy-8-methyl-2-((2-oxo-1,2,3,4-tetrahydro-1,6-naphthyridin-7-yl)oxy)-1,2,3,10,11,11a-hexahydro-5H-benzo[e]pyrrolo[1,2-a]azepin-5-one>

The same procedures as those in Example 55 were carried out except that the compound obtained in Reference Example 81 (54.7 mg, 0.18 mmol) and the compound obtained in Reference Example 84 (71.9 mg, 0.23 mmol) were used as raw materials, to thereby produce the title compound (11.0 mg).

$^1$H-NMR ($CDCl_3$) δ: 7.90 (1H, s), 7.79 (1H, s), 6.60 (1H, d, J = 6.4 Hz), 5.94 (1H, s), 5.55-5.50 (1H, m), 4.38-4.32 (1H, m), 3.97-3.88 (2H, m), 3.70-3.63 (1H, m), 2.86-2.82 (2H, m), 2.72 (1H, td, J = 13.0, 7.8 Hz), 2.60-2.56 (2H, m), 2.51 (1H, dd, J = 13.7, 6.4 Hz), 2.33-2.26 (1H, m), 2.18 (3H, d, J = 2.3 Hz), 2.08 (1H, dt, J = 13.3, 5.3 Hz), 2.01-1.92 (1H, m), 1.83 (1H, td, J = 12.1, 7.5 Hz), 1.28 (3H, d, J = 5.9 Hz), 1.17 (3H, d, J = 5.9 Hz).
LCMS: 454.2 $[M+H]^+$ (Rt= 1.957 min).

<Example 88: (2S,11aR)-7-Fluoro-2-((8-fluoro-2-oxo-1,2,3,4-tetrahydro-1,6-naphthyridin-7-yl)amino)-6-isopropoxy-8-methyl-2,3,11,11a-tetrahydro-1H,5H-benzo[f]pyrrolo[2,1-c][1,4]oxazepin-5-one>

[Step 1] tert-Butyl (E)-3-(4-amino-5-fluoro-6-(((2S,11aR)-7-fluoro-6-isopropoxy-8-methyl-5-oxo-2,3,11,11a-tetrahydro-1H,5H-benzo[f]pyrrolo[2,1-c][1,4]oxazepin-2-yl)amino)pyridin-3-yl)acrylate

The compound obtained in Reference Example 82 (227 mg, 0.74 mmol) and potassium carbonate (203 mg, 1.47 mmol) were dissolved in DMF (3.68 mL), combined with the compound obtained in Reference Example 91 (226 mg, 0.88 mmol), and agitated at 100°C for 6 days. The reaction mixture was combined with water, and extracted with ethyl acetate. The organic phase was washed twice with saturated saline solution, dried with anhydrous magnesium sulfate, and concentrated under reduced pressure. The residue was purified with silica gel column chromatography (heptane/ethyl acetate) to thereby produce the title compound (401 mg).
LCMS: 545.3 $[M+H]^+$ (Rt= 1.920 min).

[Step 2] tert-Butyl 3-(4-amino-5-fluoro-6-(((2S,11aR)-7-fluoro-6-isopropoxy-8-methyl-5-oxo-2,3,11,11a-tetrahydro-1H,5H-benzo[f]pyrrolo[2,1-c][1,4]oxazepin-2-yl)amino)pyridin-3-yl)propanoate

The same procedures as those in Step 3 of Reference Example 85 were carried out except that the compound obtained in Step 1 (401 mg, 0.74 mmol) was used as a raw material, to thereby produce the title compound (402 mg).
LCMS: 547.3 $[M+H]^+$ (Rt= 1.890 min).

[Step 3] (2S,11aR)-7-Fluoro-2-((8-fluoro-2-oxo-1,2,3,4-tetrahydro-1,6-naphthyridin-7-yl)amino)-6-isopropoxy-8-methyl-2,3,11,11a-tetrahydro-1H,5H-benzo[f]pyrrolo[2,1-c][1,4]oxazepin-5-one

The same procedures as those in Step 4 of Reference Example 85 were carried out except that the compound obtained in Step 2 (402 mg, 0.74 mmol) was used as a raw material, to thereby produce the title compound (46.3 mg).

$^1$H-NMR (DMSO-$d_6$) δ: 10.40 (1H, s), 7.63 (1H, s), 6.76 (1H, d, J = 5.9 Hz), 6.53 (1H, d, J = 5.9 Hz), 4.63-4.51 (1H, m), 4.33-4.21 (1H, m), 4.11-3.93 (3H, m), 3.87 (1H, dd, J = 11.9, 6.4 Hz), 3.46 (1H, dd, J = 11.9, 6.4 Hz), 2.80 (2H, t, J = 7.3 Hz), 2.50-2.43 (2H, m), 2.22 (3H, d, J = 1.8 Hz), 2.19-2.08 (1H, m), 2.03-1.95 (1H, m), 1.18 (3H, d, J = 5.9 Hz), 1.12 (3H, d, J = 5.9 Hz).
LCMS: 473.2 $[M+H]^+$ (Rt= 1.762 min).

<Example 89: (2S,11aR)-6-(Cyclopropylmethoxy)-8-methyl-2-((2-oxo-1,2,3,4-tetrahydroquinolin-8-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 28 (25.0 mg, 0.08 mmol) and 8-hydroxy-3,4-dihydroquinolin-2(1H)-one (16.1 mg, 0.10 mmol) were used as raw materials, to thereby produce the title compound (12.0 mg).

$^1$H-NMR ($CDCl_3$) δ: 7.61 (1H, s), 6.93 (1H, t, J = 7.8 Hz), 6.81 (1H, d, J = 7.8 Hz), 6.75 (1H, d, J = 8.2 Hz), 6.57 (1H, s), 6.50 (1H, s), 5.07-5.02 (1H, m), 4.21-4.18 (2H, m), 4.07-4.02 (3H, m), 3.93 (1H, dd, J = 10.1, 6.9 Hz), 3.81 (1H, dd, J = 10.1, 6.9 Hz), 2.98-2.94 (2H, m), 2.61 (2H, td, J = 7.5, 2.6 Hz), 2.48-2.42 (1H, m), 2.31 (3H, s), 2.13-2.06 (1H, m), 1.30-1.28 (1H, m), 0.58-0.54 (2H, m), 0.35-0.31 (2H, m).
LCMS: 449.2 $[M+H]^+$ (Rt= 2.172 min).

<Example 90: (2S,11aR)-6-(Cyclopropylmethoxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 28 (25.0 mg, 0.08 mmol) and 7-hydroxy-3,4-dihydroquinolin-2(1H)-one (16.1 mg, 0.10 mmol) were used as raw materials, to thereby produce the title compound (10.9 mg).

$^1$H-NMR ($CDCl_3$) δ: 7.60 (1H, s), 7.06 (1H, d, J = 8.2 Hz), 6.59 (1H, s), 6.50-6.49 (2H, m), 6.28 (1H, d, J = 2.3 Hz), 4.96-4.92 (1H, m), 4.20-3.93 (6H, m), 3.83 (1H, dd, J = 10.1, 6.9 Hz), 2.90 (2H, t, J = 7.5 Hz), 2.62 (2H, t, J = 7.3 Hz), 2.44-2.37 (1H, m), 2.31 (3H, s), 2.03-1.99 (1H, m), 1.32-1.28 (1H, m), 0.60-0.57 (2H, m), 0.36-0.35 (2H, m).
LCMS: 449.2 $[M+H]^+$ (Rt= 2.151 min).

<Example 91: (2S,11aR)-6-(Cyclopropylmethoxy)-8-methyl-2-((2-oxo-1,2,3,4-tetrahydroquinolin-6-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 28 (25.0 mg, 0.08 mmol) and 6-hydroxy-3,4-dihydroquinolin-2(1H)-one (16.1 mg, 0.10 mmol) were used as raw materials, to thereby produce the title compound (5.8 mg).

$^1$H-NMR ($CDCl_3$) δ: 7.29 (1H, s), 6.71-6.70 (2H, m), 6.61 (2H, t, J = 10.3 Hz), 6.50 (1H, s), 4.94-4.90 (1H, m), 4.17-4.12 (3H, m), 4.06-4.02 (1H, m), 3.97-3.95 (2H, m), 3.83 (1H, dd, J = 10.1, 6.9 Hz), 2.93 (2H, t, J = 7.3 Hz), 2.62-2.60 (2H, m), 2.42-2.40 (1H, m), 2.31 (3H, s), 2.03-1.99 (1H, m), 1.31-1.28 (1H, m), 0.60-0.56 (2H, m), 0.38-0.34 (2H, m).
LCMS: 449.2 $[M+H]^+$ (Rt= 2.121 min).

<Example 92: (2S,11aR)-6-(Cyclopropylmethoxy)-8-methyl-2-((2-oxo-1,2,3,4-tetrahydroquinolin-5-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 28 (50.0 mg, 0.17 mmol) and 5-hydroxy-3,4-dihydroquinolin-2(1H)-one (32.3 mg, 0.20 mmol) were used as raw materials, to thereby produce the title compound (29.4 mg).

$^1$H-NMR ($CDCl_3$) δ: 7.55 (1H, s), 7.11 (1H, t, J = 8.2 Hz), 6.59 (1H, s), 6.56 (1H, d, J = 8.2 Hz), 6.50 (1H, s), 6.39 (1H, d, J = 7.8 Hz), 5.05-5.01 (1H, m), 4.22-4.14 (3H, m), 4.05-4.03 (1H, m), 3.96-3.92 (2H, m), 3.83 (1H, dd, J = 10.1, 6.9 Hz), 2.89-2.84 (2H, m), 2.61-2.53 (2H, m), 2.46-2.44 (1H, m), 2.31 (3H, s), 2.07-2.01 (1H, m), 1.30-1.28 (1H, m), 0.59-0.58 (2H, m), 0.35 (2H, br s).
LCMS: 449.2 $[M+H]^+$ (Rt= 2.121 min).

<Example 93: (2S,11aR)-7-Fluoro-8-methyl-2-((2-oxo-1,2,3,4-tetrahydro-1,6-naphthyridin-7-yl)oxy)-6-pentyl-2,3,11,11a-tetrahydro-1H,5H-benzo[f]pyrrolo[2,1-c][1,4]oxazepin-5-one>

The same procedures as those in Example 55 were carried out except that the compound obtained in Reference Example 98 (40.2 mg, 0.13 mmol) and the compound obtained in Reference Example 84 (50.5 mg, 0.16 mmol) were used as raw materials, to thereby produce the title compound (1.6 mg).

$^1$H-NMR ($CDCl_3$) δ: 7.87 (1H, s), 7.42 (1H, s), 6.73 (1H, d, J = 6.4 Hz), 5.99 (1H, s), 5.65-5.60 (1H, m), 5.35-5.31 (3H,

m), 4.06-4.00 (4H, m), 3.07-3.03 (1H, m), 2.92 (2H, t, J = 7.5 Hz), 2.72-2.64 (3H, m), 2.38-2.32 (1H, m), 2.25-2.20 (5H, m), 2.01-1.99 (3H, m), 1.64 (3H, t, J = 7.3 Hz).
LCMS: 468.2 $[M+H]^+$ (Rt= 2.169 min).

<Example 94: (2S,11aR)-7-Fluoro-6-(2-fluorophenyl)-8-methyl-2-((2-oxo-1,2,3,4-tetrahydro-1,6-naphthyridin-7-yl) oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 99 (56.2 mg, 0.16 mmol) and the compound obtained in Reference Example 84 (65.8 mg, 0.21 mmol) were used as raw materials, to thereby produce the title compound (31.7 mg).

$^1$H-NMR ($CDCl_3$) δ: 8.02-7.85 (2H, m), 7.51-7.47 (1H, m), 7.32-7.27 (1H, m), 7.21-7.12 (1H, m), 6.97-6.90 (2H, m), 6.14-6.08 (1H, m), 5.64-5.60 (1H, m), 4.30-4.25 (2H, m), 4.15-4.06 (1H, m), 3.98-3.94 (1H, m), 3.87-3.78 (1H, m), 2.93-2.89 (2H, m), 2.68-2.65 (2H, m), 2.44-2.38 (1H, m), 2.32 (3H, d, J = 1.8 Hz), 2.07 (1H, dt, J = 13.6, 5.7 Hz).
LCMS: 492.2 $[M+H]^+$ (Rt= 1.996 min).

<Example 95: (2S,11aR)-6-isopropoxy-8-methyl-2-((2-oxo-1,2,3,4-tetrahydro-1,6-naphthyridin-7-yl)amino)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 100 (58.1 mg, 0.20 mmol) and the compound obtained in Reference Example 84 (124 mg, 0.40 mmol) were used as raw materials, to thereby produce the title compound (4.1 mg).
LCMS: 437.3 $[M+H]^+$ (Rt= 1.705 min).

<Example 96: (6aR,9S)-1-(Cyclopropylmethoxy)-3-methyl-9-((2-oxo-1,2,3,4-tetrahydroquinolin-7-yl) oxy)-6,6a,7,8,9,10-hexahydro-12H-benzo[f]pyrido[2,1-c][1,4]oxazepin-12-one>

The same procedures as those in Example 1 were carried out except that the compound obtained in Step 1 of Reference Example 28 (29.9 mg, 0.13 mmol) and the compound obtained in Reference Example 92 (39.7 mg, 0.11 mmol) were used as raw materials, to thereby produce the title compound (0.6 mg).

$^1$H-NMR ($CDCl_3$) δ: 7.41 (1H, s), 7.06 (1H, d, J = 8.2 Hz), 6.60-6.57 (2H, m), 6.47-6.44 (1H, m), 6.36 (1H, d, J = 2.3 Hz), 4.75 (1H, dt, J = 14.8, 2.1 Hz), 4.59-4.59 (1H, m), 4.32 (1H, dd, J = 12.3, 10.5 Hz), 4.18-4.17 (1H, m), 3.97 (1H, dd, J = 10.1, 6.4 Hz), 3.89-3.84 (2H, m), 3.21 (1H, dd, J = 14.6, 2.7 Hz), 2.91 (2H, t, J = 7.3 Hz), 2.63-2.61 (2H, m), 2.31 (3H, s), 2.21-2.16 (1H, m), 2.00-1.98 (1H, m), 1.89-1.85 (1H, m), 1.41-1.31 (2H, m), 0.61 (2H, dt, J = 11.7, 3.0 Hz), 0.41-0.37 (2H, m).
LCMS: 463.3 $[M+H]^+$ (Rt= 2.220 min).

<Example 97: (6aR,8R)-1-(Cyclopropylmethoxy)-3-methyl-8-((2-oxo-1,2,3,4-tetrahydroquinolin-7-yl) oxy)-6,6a,7,8,9,10-hexahydro-12H-benzo[f]pyrido[2,1-c][1,4]oxazepin-12-one>

The same procedures as those in Example 1 were carried out except that the compound obtained in Step 1 of Reference Example 28 (56.4 mg, 0.24 mmol) and the compound obtained in Reference Example 93 (74.9 mg, 0.20 mmol) were used as raw materials, to thereby produce the title compound (19.4 mg).

$^1$H-NMR ($CDCl_3$) δ: 7.61 (1H, s), 7.06 (1H, d, J = 8.2 Hz), 6.60 (1H, s), 6.50 (1H, d, J = 8.7 Hz), 6.46 (1H, s), 6.28 (1H, s), 4.68 (1H, d, J = 14.2 Hz), 4.43-4.37 (3H, m), 4.12 (1H, q, J = 6.9 Hz), 3.93-3.87 (3H, m), 3.18 (1H, t, J = 12.1 Hz), 2.90 (2H, t, J = 7.5 Hz), 2.62 (2H, t, J = 7.3 Hz), 2.29-2.26 (4H, m), 1.98-1.94 (2H, m), 1.75-1.70 (1H, m), 1.33-1.30 (1H, m), 0.64-0.59 (2H, m), 0.41-0.33 (2H, m).
LCMS: 463.2 $[M+H]^+$ (Rt= 2.197 min).

<Example 98: (2S,11aR)-2-((3,3-Dimethyl-2-oxo-1,2,3,4-tetrahydroquinolin-7-yl)oxy)-7-fluoro-6-isopropoxy-8-methyl-2,3,11,11a-tetrahydro-1H,5H-benzo[f]pyrrolo[2,1-c][1,4]oxazepin-5-one>

The same procedures as those in Example 68 were carried out except that the compound obtained in Reference Example 94 (14.9 mg, 0.08 mmol) and the compound obtained in Reference Example 101 (30.0 mg, 0.06 mmol) were used as raw materials, to thereby produce the title compound (7.2 mg).

$^1$H-NMR ($CDCl_3$) δ: 7.38 (1H, br s), 7.03 (1H, d, J = 8.2 Hz), 6.62 (1H, d, J = 5.9 Hz), 6.44 (1H, dd, J = 8.2, 2.3 Hz), 6.18 (1H, d, J = 2.3 Hz), 4.98-4.91 (1H, m), 4.46-4.35 (1H, m), 4.24-4.16 (2H, m), 4.16-4.06 (1H, m), 4.01 (1H, dd, J = 11.2, 9.8 Hz), 3.86 (1H, dd, J = 13.3, 4.6 Hz), 2.73 (2H, s), 2.47-2.39 (1H, m), 2.26 (3H, d, J = 2.3 Hz), 1.99 (1H, ddd, J = 13.9, 5.8, 5.8 Hz), 1.35 (3H, d, J = 5.9 Hz), 1.24 (3H, d, J = 5.7 Hz), 1.20 (3H, s), 1.19 (3H, s).
LCMS: 483.3 [M+H]$^+$ (Rt= 2.316 min).

<Example 99: (2S,11aR)-7-Fluoro-6-(((S)-1-hydroxy propan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 102 (143 mg, 0.39 mmol) and the compound obtained in Reference Example 84 (156 mg, 0.50 mmol) were used as raw materials, to thereby produce the title compound (75.8 mg).

$^1$H-NMR ($CDCl_3$) δ: 7.86 (1H, s), 7.63 (1H, s), 6.60 (1H, d, J = 5.9 Hz), 6.34 (1H, dd, J = 8.2, 5.9 Hz), 6.10 (1H, s), 5.65-5.59 (1H, m), 4.52-4.48 (1H, m), 4.16-4.01 (5H, m), 3.94 (1H, dd, J = 13.3, 4.6 Hz), 3.68-3.62 (2H, m), 2.92 (2H, t, J = 7.5 Hz), 2.68-2.64 (2H, m), 2.34-2.30 (1H, m), 2.26 (3H, d, J = 1.8 Hz), 2.15-2.12 (1H, m), 1.40 (3H, d, J = 6.4 Hz).
LCMS: 472.2 [M+H]$^+$ (Rt= 1.857 min).

<Example 100: (2S,11aR)-7-Fluoro-6-(((R)-1-hydroxy propan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 103 (175 mg, 0.47 mmol) and the compound obtained in Reference Example 84 (192 mg, 0.62 mmol) were used as raw materials, to thereby produce the title compound (80.7 mg).

$^1$H-NMR ($CDCl_3$) δ: 8.00 (1H, s), 7.86 (1H, s), 6.65 (1H, d, J = 5.5 Hz), 6.09 (1H, s), 5.63-5.58 (1H, m), 4.40-4.38 (1H, m), 4.22 (1H, dd, J = 8.2, 2.7 Hz), 4.13-4.08 (3H, m), 3.93 (1H, dd, J = 13.3, 5.0 Hz), 3.80-3.78 (2H, m), 3.62-3.59 (1H, m), 2.98-2.85 (2H, m), 2.69-2.63 (2H, m), 2.41-2.38 (1H, m), 2.28 (3H, d, J = 1.8 Hz), 2.06 (1H, dt, J = 13.6, 5.6 Hz), 1.34 (3H, d, J = 6.4 Hz).
LCMS: 472.2 [M+H]$^+$ (Rt= 1.816 min).

<Example 101: (2S,11aR)-6-(2,2-Difluoropropoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 104 (129 mg, 0.37 mmol) and the compound obtained in Reference Example 84 (151 mg, 0.49 mmol) were used as raw materials, to thereby produce the title compound (46.4 mg).

$^1$H-NMR ($CDCl_3$) δ: 7.86 (1H, s), 7.46 (1H, s), 6.67 (1H, d, J = 5.9 Hz), 6.04 (1H, s), 5.66-5.62 (1H, m), 4.29-4.03 (6H, m), 3.95 (1H, dd, J = 13.3, 4.6 Hz), 2.92 (2H, t, J = 7.3 Hz), 2.68-2.63 (2H, m), 2.40-2.37 (1H, m), 2.27 (3H, d, J = 2.3 Hz), 2.05 (1H, dt, J = 13.9, 5.4 Hz), 1.77 (3H, t, J = 19.2 Hz).
LCMS: 492.2 [M+H]$^+$ (Rt= 1.977 min).

<Example 102: (2S,11aR)-7-Fluoro-6-isopropoxy-8-methyl-2-((2-oxo-1,2-dihydro-1,6-naphthyridin-7-yl) oxy)-2,3,11,11a-tetrahydro-1H,5H-benzo[f]pyrrolo[2,1-c][1,4]oxazepin-5-one>

The same procedures as those in Step 1 to Step 2 of Example 55were carried out except that the compound obtained in Reference Example 55 (60.0 mg, 0.19 mmol) and the compound obtained in Reference Example 84 (72.3 mg, 0.23 mmol) were used as raw materials, to thereby produce the title compound (40.2 mg).

$^1$H-NMR ($CDCl_3$) δ: 10.36 (1H, s), 8.38 (1H, s), 7.74 (1H, d, J = 9.6 Hz), 6.63 (1H, d, J = 5.9 Hz), 6.50 (1H, d, J = 9.1 Hz), 6.41 (1H, s), 5.74-5.70 (1H, m), 4.52-4.43 (1H, m), 4.19 (1H, dd, J = 8.9, 3.9 Hz), 4.13-4.01 (4H, m), 2.42-2.36 (1H, m), 2.27 (3H, d, J = 1.8 Hz), 2.10 (1H, dt, J = 13.7, 5.5 Hz), 1.37 (3H, d, J = 6.4 Hz), 1.27 (3H, d, J = 6.4 Hz).
LCMS: 454.2 [M+H]$^+$ (Rt= 2.171 min).

<Example 103: (2S,11aR)-7-Fluoro-8-methyl-2-((2-oxo-1,2,3,4-tetrahydro-1,6-naphthyridin-7-yl)oxy)-6-(((S)-1,1,1-trifluoropropan-2-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 105 (153 mg, 0.42 mmol) and the compound obtained in Reference Example 84 (171 mg, 0.55 mmol) were used as raw materials, to thereby produce the title compound (95.0 mg).

$^1$H-NMR ($CDCl_3$) δ: 7.87 (1H, s), 7.64 (1H, s), 6.71 (1H, d, J = 5.9 Hz), 5.99 (1H, s), 5.66-5.62 (1H, m), 4.70-4.61 (1H, m), 4.21 (1H, dd, J = 16.9, 11.9 Hz), 4.15-4.04 (3H, m), 3.93 (1H, dd, J = 13.3, 4.6 Hz), 2.93-2.90 (2H, m), 2.67-2.63 (2H, m), 2.41-2.39 (1H, m), 2.28 (3H, d, J = 1.8 Hz), 2.08-2.02 (1H, m), 1.42 (3H, d, J = 6.4 Hz).
LCMS: 510.2 $[M+H]^+$ (Rt= 2.034 min).

<Example 104: (2S,11aR)-6-Cyclobutoxy-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 106 (79.4 mg, 0.25 mmol) and the compound obtained in Reference Example 84 (99.9 mg, 0.32 mmol) were used as raw materials, to thereby produce the title compound (19.3 mg).

$^1$H-NMR ($CDCl_3$) δ: 7.87 (1H, s), 7.56 (1H, s), 6.61 (1H, d, J = 5.9 Hz), 6.01 (1H, s), 5.64-5.60 (1H, m), 4.72-4.64 (1H, m), 4.16 (1H, dd, J = 8.7, 3.7 Hz), 4.08-4.03 (4H, m), 2.93-2.91 (2H, m), 2.68-2.64 (2H, m), 2.38-2.17 (9H, m), 2.11-2.06 (1H, m), 1.72-1.66 (1H, m), 1.42 (1H, td, J = 19.1, 9.6 Hz).
LCMS: 468.2 $[M+H]^+$ (Rt= 1.960 min).

<Example 105: (2S,11aR)-6-((4,4-Difluorocyclohexyl)oxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 107 (74.3 mg, 0.19 mmol) and the compound obtained in Reference Example 84 (77.9 mg, 0.25 mmol) were used as raw materials, to thereby produce the title compound (34.7 mg).

$^1$H-NMR ($CDCl_3$) δ: 7.86 (1H, s), 7.62 (1H, s), 6.64 (1H, d, J = 5.9 Hz), 6.01 (1H, s), 5.63-5.59 (1H, m), 4.39-4.38 (1H, br m), 4.19 (1H, d, J = 5.0 Hz), 4.06-4.03 (3H, m), 4.00-3.97 (1H, m), 2.93-2.91 (2H, m), 2.68-2.63 (2H, m), 2.41-2.35 (1H, m), 2.31-2.07 (7H, m), 1.98-1.76 (5H, m).
LCMS: 532.2 $[M+H]^+$ (Rt= 2.057 min).

<Example 106: (2S,11aR)-7-Fluoro-6-isopropoxy-8-methyl-2-((3-methyl-2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 68 were carried out except that the compound obtained in Reference Example 95 (17.3 mg, 0.10 mmol) and the compound obtained in Reference Example 101 (30.0 mg, 0.06 mmol) were used as raw materials, to thereby produce the title compound (6.8 mg).

$^1$H-NMR ($CDCl_3$) δ: 7.85 (1H, s), 7.62 (1H, br s), 6.62 (1H, d, J = 5.9 Hz), 5.98 (1H, s), 5.64-5.59 (1H, m), 4.51-4.41 (1H, m), 4.17 (1H, dd, J = 8.7, 3.2 Hz), 4.13-4.01 (3H, m), 3.99 (1H, dd, J = 13.0, 4.8 Hz), 3.01-2.92 (1H, m), 2.71-2.59 (2H, m), 2.39-2.31 (1H, m), 2.26 (3H, d, J = 2.3 Hz), 2.10-2.02 (1H, m), 1.36 (3H, d, J = 5.9 Hz), 1.30 (3H, d, J = 5.0 Hz), 1.25 (3H, d, J = 6.4 Hz).
LCMS: 470.2 $[M+H]^+$ (Rt= 1.982 min).

<Example 107: (2S,11aR)-6-((3,3-Difluorocyclobutyl)methoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 108 (75.9 mg, 0.20 mmol) and the compound obtained in Reference Example 84 (82.6 mg, 0.27 mmol) were used as raw materials, to thereby produce the title compound (31.2 mg).

$^1$H-NMR ($CDCl_3$) δ: 7.86 (1H, s), 7.58 (1H, s), 6.65 (1H, d, J = 5.9 Hz), 6.06 (1H, s), 5.64-5.60 (1H, m), 4.33 (1H, dd, J = 8.9, 4.3 Hz), 4.18 (1H, dd, J = 8.2, 3.2 Hz), 4.09-4.01 (5H, m), 2.92 (2H, t, J = 7.8 Hz), 2.62-2.51 (7H, m), 2.39-2.35 (1H,

m), 2.27 (3H, s), 2.08 (1H, td, J = 9.3, 4.3 Hz).
LCMS: 518.2 $[M+H]^+$ (Rt= 2.033 min).

<Example 108: (2S,11aR)-6-(3,3-Difluorocyclobutoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Reference Example 109 (41.3 mg, 0.12 mmol) and the compound obtained in Reference Example 85 (37.9 mg, 0.23 mmol) were used as raw materials, to thereby produce the title compound (3.1 mg).

$^1$H-NMR ($CDCl_3$) δ: 7.86 (1H, s), 7.43 (1H, s), 6.67 (1H, d, J = 5.9 Hz), 6.03 (1H, s), 5.66-5.61 (1H, m), 4.71-4.68 (1H, m), 4.20-4.19 (1H, m), 4.09-4.07 (3H, m), 3.96 (1H, dd, J = 13.0, 4.8 Hz), 2.97-2.92 (6H, m), 2.66 (2H, td, J = 7.4, 3.7 Hz), 2.42-2.35 (1H, m), 2.27 (3H, d, J = 2.3 Hz), 2.09-2.07 (1H, m).
LCMS: 504.2 $[M+H]^+$ (Rt= 1.974 min).

<Example 109: (2S,11aR)-7-Fluoro-6-(((R)-1-methoxy propan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 110 (62.2 mg, 0.18 mmol) and the compound obtained in Reference Example 84 (74.1 mg, 0.24 mmol) were used as raw materials, to thereby produce the title compound (29.5 mg).

$^1$H-NMR ($CDCl_3$) δ: 7.86 (1H, s), 7.73 (1H, s), 6.61 (1H, d, J = 5.9 Hz), 6.07 (1H, s), 5.64-5.59 (1H, m), 4.61-4.58 (1H, m), 4.16-3.93 (5H, m), 3.66 (1H, dd, J = 10.1, 6.9 Hz), 3.43 (1H, dd, J = 10.5, 4.1 Hz), 3.37 (3H, s), 2.92 (2H, t, J = 7.5 Hz), 2.68-2.64 (2H, m), 2.36-2.29 (1H, m), 2.26 (3H, d, J = 1.8 Hz), 2.09 (1H, dt, J = 13.4, 5.1 Hz), 1.33 (3H, d, J = 6.4 Hz).
LCMS: 486.2 $[M+H]^+$ (Rt= 1.883 min).

<Example 110: (2S,11aR)-7-Fluoro-6-(((S)-1-methoxy propan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 111 (64.0 mg, 0.19 mmol) and the compound obtained in Reference Example 84 (76.2 mg, 0.25 mmol) were used as raw materials, to thereby produce the title compound (32.4 mg).

$^1$H-NMR ($CDCl_3$) δ: 7.95 (1H, s), 7.86 (1H, s), 6.64 (1H, d, J = 5.9 Hz), 6.06 (1H, s), 5.64-5.60 (1H, m), 4.40 (1H, td, J = 11.9, 5.9 Hz), 4.18 (1H, dd, J = 9.1, 3.7 Hz), 4.07-4.04 (3H, m), 3.93 (1H, dd, J = 13.3, 4.6 Hz), 3.70 (1H, dd, J = 10.1, 5.0 Hz), 3.51 (1H, dd, J = 9.8, 6.2 Hz), 3.37 (3H, s), 2.91 (2H, t, J = 7.3 Hz), 2.65 (2H, td, J = 7.4, 2.0 Hz), 2.38-2.34 (1H, m), 2.26 (3H, d, J = 1.8 Hz), 2.04 (1H, dt, J = 13.4, 5.6 Hz), 1.27 (3H, d, J = 6.4 Hz).
LCMS: 486.2 $[M+H]^+$ (Rt= 1.865 min).

<Example 111: (2S,11aR)-7-Fluoro-6-(2-methoxy-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 112 (74.7 mg, 0.21 mmol) and the compound obtained in Reference Example 84 (85.4 mg, 0.28 mmol) were used as raw materials, to thereby produce the title compound (22.9 mg).

$^1$H-NMR ($CDCl_3$) δ: 7.86 (2H, s), 6.63 (1H, d, J = 5.9 Hz), 6.12 (1H, s), 5.64-5.60 (1H, m), 4.18-4.13 (2H, m), 4.07-3.97 (5H, m), 3.33 (3H, s), 2.91 (2H, t, J = 7.5 Hz), 2.67-2.63 (2H, m), 2.37-2.33 (1H, m), 2.26 (3H, d, J = 2.3 Hz), 2.06 (1H, dt, J = 13.6, 5.4 Hz), 1.29 (6H, d, J = 8.2 Hz).
LCMS: 500.3 $[M+H]^+$ (Rt= 1.909 min).

<Example 112: (2S,11aR)-7-Fluoro-6-(2-methoxyethoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 113 (58.0 mg, 0.18 mmol) and the compound obtained in Reference Example 84 (72.0 mg, 0.23 mmol) were used

as raw materials, to thereby produce the title compound (25.0 mg).

$^1$H-NMR ($CDCl_3$) δ: 7.86 (1H, s), 7.71 (1H, s), 6.64 (1H, d, J = 5.9 Hz), 6.10 (1H, s), 5.64-5.59 (1H, m), 4.32-4.28 (2H, m), 4.17 (1H, dd, J = 8.5, 3.0 Hz), 4.13-3.95 (4H, m), 3.87-3.82 (1H, m), 3.69 (1H, td, J = 7.7, 3.5 Hz), 3.44 (3H, s), 2.92 (2H, t, J = 7.3 Hz), 2.66 (2H, td, J = 7.4, 1.8 Hz), 2.36-2.30 (1H, m), 2.26 (3H, d, J = 1.8 Hz), 2.10-2.04 (1H, m).
LCMS: 472.2 $[M+H]^+$ (Rt= 1.827 min).

<Example 113: (2S,11aR)-7-Fluoro-6-isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 10 were carried out except that the compound obtained in Step 1 of Reference Example 82 (50.0 mg, 0.16 mmol) and 7-hydroxy-3,4-dihydroquinolin-2(1H)-one (31.7 mg, 0.19 mmol) were used as raw materials, to thereby produce the title compound (12.8 mg).

$^1$H-NMR ($CDCl_3$) δ: 7.64 (1H, s), 7.05 (1H, d, J = 8.2 Hz), 6.62 (1H, d, J = 5.9 Hz), 6.45 (1H, dd, J = 8.2, 1.8 Hz), 6.23 (1H, d, J = 2.3 Hz), 4.95-4.94 (1H, br m), 4.46-4.37 (1H, m), 4.21-3.98 (4H, m), 3.86 (1H, dd, J = 13.3, 4.6 Hz), 2.90 (2H, t, J = 7.3 Hz), 2.62 (2H, t, J = 7.3 Hz), 2.44-2.41 (1H, br m), 2.26 (3H, d, J = 1.8 Hz), 1.99 (1H, dt, J = 13.6, 5.8 Hz), 1.35 (3H, d, J = 6.4 Hz), 1.24 (3H, d, J = 6.4 Hz).
LCMS: 455.2 $[M+H]^+$ (Rt= 2.143 min).

<Example 114: (2S,11aR)-7-Fluoro-8-methyl-2-((2-oxo-1,2,3,4-tetrahydro-1,6-naphthyridin-7-yl)oxy)-6-(2-(pyrrolidin-1-yl)ethoxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 114 (55.2 mg, 0.15 mmol) and the compound obtained in Reference Example 84 (61.2 mg, 0.20 mmol) were used as raw materials, to thereby produce the title compound (14.3 mg).

$^1$H-NMR ($CDCl_3$) δ: 7.92 (1H, br s), 7.86 (1H, s), 6.62 (1H, d, J = 5.5 Hz), 6.11 (1H, s), 5.64-5.59 (1H, m), 4.30-4.22 (2H, m), 4.17 (1H, d, J = 5.5 Hz), 4.08-4.04 (2H, m), 3.99 (2H, d, J = 4.1 Hz), 3.08-3.02 (1H, m), 2.91 (2H, t, J = 7.5 Hz), 2.76 (1H, dt, J = 12.8, 5.3 Hz), 2.67-2.61 (6H, m), 2.35-2.31 (1H, m), 2.26 (3H, d, J = 1.8 Hz), 2.07-2.05 (1H, m), 1.79 (4H, br s).
LCMS: 511.2 $[M+H]^+$ (Rt= 1.661 min) (Method B).

<Example 115: (2S,11aR)-7-Fluoro-2-((8-fluoro-2-oxo-1,2,3,4-tetrahydroquinolin-7-yl)oxy)-6-isopropoxy-8-methyl-2,3,11,11a-tetrahydro-1H,5H-benzo[f]pyrrolo[2,1-c][1,4]oxazepin-5-one>

The same procedures as those in Example 10 were carried out except that the compound obtained in Step 1 of Reference Example 82 (25.0 mg, 0.08 mmol) and the compound obtained in Reference Example 86 (17.6 mg, 0.10 mmol) were used as raw materials, to thereby produce the title compound (7.2 mg).

$^1$H-NMR ($CDCl_3$) δ: 7.47 (1H, br s), 6.85 (1H, d, J = 8.2 Hz), 6.62 (1H, d, J = 5.5 Hz), 6.55 (1H, t, J = 8.2 Hz), 5.00-4.96 (1H, m), 4.43-4.35 (1H, m), 4.28-4.12 (3H, m), 4.00 (1H, t, J = 10.5 Hz), 3.81 (1H, dd, J = 13.3, 4.1 Hz), 2.95 (2H, t, J = 7.3 Hz), 2.65-2.63 (2H, m), 2.50-2.48 (1H, m), 2.26 (3H, d, J = 2.3 Hz), 2.01-1.95 (1H, m), 1.33 (3H, d, J = 5.9 Hz), 1.25 (3H, d, J = 6.4 Hz).
LCMS: 473.2 $[M+H]^+$ (Rt= 2.094 min) (Method B).

<Example 116: (2S,11aR)-7-Fluoro-2-((8-fluoro-2-oxo-1,2,3,4-tetrahydro-1,6-naphthyridin-7-yl)oxy)-6-isopropoxy-8-methyl-2,3,11,11a-tetrahydro-1H,5H-benzo[f]pyrrolo[2,1-c][1,4]oxazepin-5-one>

The same procedures as those in Example 68 were carried out except that the compound obtained in Reference Example 115 (54.8 mg, 0.14 mmol) and the compound obtained in Reference Example 87 (25.8 mg, 0.14 mmol) were used as raw materials, to thereby produce the title compound (18.3 mg).

$^1$H-NMR ($CDCl_3$) δ: 7.67 (1H, s), 7.61 (1H, s), 6.62 (1H, d, J = 5.5 Hz), 5.70-5.65 (1H, m), 4.40-4.34 (1H, m), 4.21-4.16 (3H, m), 4.06-3.96 (2H, m), 3.00-2.96 (2H, m), 2.70-2.66 (2H, m), 2.47-2.44 (1H, m), 2.26 (3H, d, J = 2.3 Hz), 2.11-2.06 (1H, m), 1.33 (3H, d, J = 5.9 Hz), 1.23 (3H, d, J = 5.9 Hz).
LCMS: 474.2 $[M+H]^+$ (Rt= 2.042 min) (Method B).

<Example 117: (2S,11aR)-2-((8-chloro-2-oxo-1,2,3,4-tetrahydro-1,6-naphthyridin-7-yl)oxy)-7-fluoro-6-isopropoxy-8-methyl-2,3,11,11a-tetrahydro-1H,5H-benzo[f]pyrrolo[2,1-c][1,4]oxazepin-5-one>

[Step 1] (2S,11aR)-2-((4-(Benzylamino)-3-chloro-5-iodopyridin-2-yl)oxy)-7-fluoro-6-isopropoxy-8-methyl-2,3,11,11a-tetrahydro-1H,5H-benzo[f]pyrrolo[2,1-c][1,4]oxazepin-5-one

A mixture of the compound obtained in Reference Example 55 (500 mg, 1.62 mmol), potassium carbonate (670 mg, 4.85 mmol), and toluene (8.08 mL) was combined with 18-crown-6-ether (641 mg, 2.42 mmol) and the compound obtained in Reference Example 96 (703 mg, 1.94 mmol), and agitated at 100°C overnight. The reaction mixture was then cooled to room temperature, combined with water, and extracted with ethyl acetate. The organic phase was washed with water twice, dried with sodium sulfate, and concentrated under reduced pressure. The residue was purified with silica gel column chromatography (heptane/ethyl acetate) to thereby produce the title compound (963 mg). $^1$H-NMR ($CDCl_3$) δ: 8.10 (1H, s), 7.38-7.30 (4H, m), 6.61 (1H, d, J = 5.9 Hz), 5.60-5.56 (1H, m), 4.78 (3H, s), 4.38-4.32 (1H, m), 4.21-4.10 (3H, m), 4.05-3.96 (2H, m), 2.46-2.40 (1H, m), 2.25 (3H, d, J = 2.3 Hz), 2.07 (1H, dt, J = 13.7, 5.5 Hz), 1.30 (3H, d, J = 5.9 Hz), 1.24 (3H, d, J = 5.9 Hz).
LCMS: 652.1 $[M+H]^+$ (Rt= 2.810 min) (Method B).

[Step 2] (2S,11aR)-2-((8-Chloro-2-oxo-1,2,3,4-tetrahydro-1,6-naphthyridin-7-yl)oxy)-7-fluoro-6-isopropoxy-8-methyl-2,3,11,11a-tetrahydro-1H,5H-benzo[f]pyrrolo[2,1-c][1,4]oxazepin-5-one

The same procedures as those in Step 2 to Step 4 of Reference Example 85 were carried out except that the compound obtained in Step 1 (100 mg, 0.15 mmol) and ethyl acrylate (33.4 μL, 0.31 mmol) were used as raw materials, to thereby produce the title compound (26.0 mg).

$^1$H-NMR ($CDCl_3$) δ: 7.81 (1H, br s), 7.78 (1H, s), 6.62 (1H, d, J = 5.9 Hz), 5.68-5.62 (1H, br m), 4.43-4.33 (1H, m), 4.23-4.00 (5H, m), 3.00-2.93 (2H, m), 2.72-2.64 (2H, m), 2.50-2.42 (1H, m), 2.26 (3H, d, J = 2.3 Hz), 2.15-2.06 (1H, m), 1.32 (3H, d, J = 5.9 Hz), 1.24 (3H, d, J = 5.9 Hz).
LCMS: 490.2 $[M+H]^+$ (Rt= 2.141 min) (Method B).

<Example 118: (2S,11aR)-7-Fluoro-6-isopropoxy-8-methyl-2-((4-methyl-2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Step 2 to Step 4 of Reference Example 85 were carried out except that the compound obtained in Step 1 of Example 117 (100 mg, 0.15 mmol) and ethyl crotonate (38.0 μL, 0.31 mmol) were used as raw materials, to thereby produce the title compound (1.8 mg).

$^1$H-NMR ($CDCl_3$) δ: 7.89 (1H, s), 7.47 (1H, br s), 6.62 (1H, d, J = 5.5 Hz), 5.98 (1H, s), 5.66-5.59 (1H, m), 4.52-4.43 (1H, m), 4.20-4.14 (1H, m), 4.10-3.96 (4H, m), 3.19-3.10 (1H, m), 2.77-2.68 (1H, m), 2.49-2.40 (1H, m), 2.39-2.31 (1H, m), 2.26 (3H, d, J = 1.8 Hz), 2.14-2.05 (1H, m), 1.36 (3H, d, J = 6.4 Hz), 1.35-1.29 (3H, m), 1.25 (3H, d, J = 5.9 Hz).
LCMS: 470.2 $[M+H]^+$ (Rt= 1.933 min) (Method B).

<Example 119: (2S,11aR)-7-Fluoro-6-isopropoxy-8-methyl-2-((3-oxo-3,4-dihydro-2H-benzo[b][1,4]oxazin-6-yl)oxy)-2,3,11,11a-tetrahydro-1H,5H-benzo[f]pyrrolo[2,1-c][1,4]oxazepin-5-one>

The same procedures as those in Example 68 were carried out except that the compound obtained in Reference Example 115 (40.0 mg, 0.10 mmol) and the compound obtained in Reference Example 89 (17.1 mg, 0.10 mmol) were used as raw materials, to thereby produce the title compound (5.5 mg).

$^1$H-NMR ($CDCl_3$) δ: 7.58 (1H, br s), 6.89 (1H, d, J = 8.7 Hz), 6.63 (1H, d, J = 5.9 Hz), 6.45 (1H, dd, J = 8.9, 2.5 Hz), 6.29 (1H, d, J = 2.3 Hz), 4.88 (1H, br s), 4.57 (2H, s), 4.46-4.43 (1H, m), 4.20-4.16 (3H, m), 4.00 (1H, t, J = 10.5 Hz), 3.84 (1H, dd, J = 13.0, 4.3 Hz), 2.45-2.38 (1H, m), 2.26 (3H, d, J = 2.3 Hz), 1.97 (1H, dt, J = 13.4, 5.8 Hz), 1.36 (3H, d, J = 6.4 Hz), 1.27 (3H, d, J = 6.4 Hz).
LCMS: 457.2 $[M+H]^+$ (Rt= 2.129 min) (Method B).

<Example 120: (2S,11aR)-7-Fluoro-2-((6-fluoro-2-oxo-1,2,3,4-tetrahydroquinolin-7-yl)oxy)-6-isopropoxy-8-methyl-2,3,11,11a-tetrahydro-1H,5H-benzo[f]pyrrolo[2,1-c][1,4]oxazepin-5-one>

The same procedures as those in Example 10 were carried out except that the compound obtained in Step 1 of

Reference Example 82 (30.9 mg, 0.10 mmol) and the compound obtained in Reference Example 97 (27.2 mg, 0.15 mmol) were used as raw materials, to thereby produce the title compound (28.6 mg).

$^1$H-NMR ($CDCl_3$) δ: 8.58 (1H, s), 6.88 (1H, d, J = 11.0 Hz), 6.63 (1H, d, J = 5.5 Hz), 6.52 (1H, d, J = 7.3 Hz), 4.97-4.91 (1H, br m), 4.40-4.10 (4H, m), 4.05-3.96 (1H, m), 3.79 (1H, dd, J = 13.3, 4.1 Hz), 2.88 (2H, t, J = 7.5 Hz), 2.66-2.58 (2H, m), 2.53-2.43 (1H, m), 2.26 (3H, d, J = 2.3 Hz), 2.01-1.91 (1H, m), 1.31 (3H, d, J = 6.4 Hz), 1.24 (3H, d, J = 6.4 Hz).
LCMS: 473.2 $[M+H]^+$ (Rt= 2.169 min) (Method B).

<Example 121: (2S,11aR)-7-Chloro-8-methyl-2-((2-oxo-1,2,3,4-tetrahydro-1,6-naphthyridin-7-yl)oxy)-6-(2,2,2-trifluoroethoxy)-2,3,11,11a-tetrahydro-1H,5H-benzo[f]pyrrolo[2,1-c][1,4]oxazepin-5-one>

The same procedures as those in Example 68 were carried out except that the compound obtained in Reference Example 116 (92.8 mg, 0.21 mmol) and the compound obtained in Reference Example 85 (34.3 mg, 0.21 mmol) were used as raw materials, to thereby produce the title compound (18.2 mg).

$^1$H-NMR ($CDCl_3$) δ: 7.86 (1H, s), 7.63 (1H, s), 6.83 (1H, s), 6.05 (1H, s), 5.66-5.61 (1H, m), 4.77-4.72 (1H, m), 4.42-4.38 (1H, m), 4.22 (1H, dd, J = 16.7, 11.7 Hz), 4.12-3.96 (4H, m), 2.92 (2H, t, J = 7.1 Hz), 2.68-2.64 (2H, m), 2.41-2.37 (4H, m), 2.11-2.06 (1H, m).
LCMS: 512.1 $[M+H]^+$ (Rt= 2.023 min) (Method B).

<Example 122: (2S,11aR)-7-Chloro-6-((1-fluorocyclopropyl)methoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 68 were carried out except that the compound obtained in Reference Example 117 (52.1 mg, 0.12 mmol) and the compound obtained in Reference Example 85 (19.7 mg, 0.12 mmol) were used as raw materials, to thereby produce the title compound (14.2 mg).

$^1$H-NMR ($CDCl_3$) δ: 7.86 (1H, s), 7.47 (1H, s), 6.78 (1H, s), 6.07 (1H, s), 5.65-5.60 (1H, m), 4.53 (1H, dd, J = 18.8, 11.0 Hz), 4.39 (1H, dd, J = 25.6, 11.0 Hz), 4.19 (1H, d, J = 5.0 Hz), 4.10-3.95 (4H, m), 2.92 (2H, t, J = 7.3 Hz), 2.68-2.64 (2H, m), 2.37-2.33 (4H, m), 2.08 (1H, dt, J = 11.1, 3.8 Hz), 1.14-1.11 (2H, m), 1.01-0.85 (2H, m).
LCMS: 502.2 $[M+H]^+$ (Rt= 1.975 min) (Method B).

<Example 123: (2S,11aR)-7-Chloro-6-(3-fluoropropoxy)-8-methyl-2-((2-oxo-1,2,3,4-tetrahydro-1,6-naphthyridin-7-yl) oxy)-2,3,11,11a-tetrahydro-1H,5H-benzo[f]pyrrolo[2,1-c][1,4]oxazepin-5-one>

The same procedures as those in Example 68 were carried out except that the compound obtained in Reference Example 118 (61.1 mg, 0.15 mmol) and the compound obtained in Reference Example 85 (23.8 mg, 0.15 mmol) were used as raw materials, to thereby produce the title compound (13.2 mg).

$^1$H-NMR ($CDCl_3$) δ: 7.86 (1H, s), 7.71 (1H, s), 6.76 (1H, s), 6.04 (1H, s), 5.65-5.61 (1H, m), 4.74 (1H, t, J = 5.9 Hz), 4.62 (1H, t, J = 5.9 Hz), 4.22-4.20 (3H, m), 4.12-3.95 (4H, m), 2.93-2.90 (2H, m), 2.66 (2H, td, J = 7.5, 2.1 Hz), 2.40-2.34 (4H, m), 2.25-2.12 (2H, m), 2.08-2.04 (1H, m).
LCMS: 490.2 $[M+H]^+$ (Rt= 1.927 min) (Method B).

<Example 124: (2S,11aR)-7-Chloro-2-((8-fluoro-2-oxo-1,2,3,4-tetrahydro-1,6-naphthyridin-7-yl)oxy)-6-isopropoxy-8-methyl-2,3,11,11a-tetrahydro-1H,5H-benzo[f]pyrrolo[2,1-c][1,4]oxazepin-5-one>

The same procedures as those in Example 68 were carried out except that the compound obtained in Reference Example 119 (43.0 mg, 0.11 mmol) and the compound obtained in Reference Example 87 (19.4 mg, 0.11 mmol) were used as raw materials, to thereby produce the title compound (13.2 mg).

$^1$H-NMR ($CDCl_3$) δ: 7.67 (1H, s), 7.60 (1H, br s), 6.73 (1H, s), 5.71-5.68 (1H, m), 4.31-4.08 (5H, m), 3.91 (1H, dd, J = 13.5, 4.3 Hz), 2.98 (2H, td, J = 7.2, 2.1 Hz), 2.68 (2H, td, J = 7.5, 2.0 Hz), 2.52-2.45 (1H, m), 2.36 (3H, s), 2.09-2.02 (1H, m), 1.29 (3H, d, J = 5.9 Hz), 1.23 (3H, d, J = 6.4 Hz).
LCMS: 490.2 $[M+H]^+$ (Rt= 2.112 min) (Method B).

<Example 125: (2S,11aR)-7-Chloro-6-isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one>

The same procedures as those in Example 68 were carried out except that the compound obtained in Reference Example 119 (43.0 mg, 0.11 mmol) and 7-hydroxy-3,4-dihydroquinolin-2(1H)-one (17.4 mg, 0.11 mmol) were used as raw materials, to thereby produce the title compound (10.5 mg).

$^1$H-NMR ($CDCl_3$) $\delta$: 7.58 (1H, s), 7.05 (1H, d, J = 8.7 Hz), 6.73 (1H, s), 6.42 (1H, dd, J = 8.2, 2.3 Hz), 6.20 (1H, d, J = 2.3 Hz), 4.95 (1H, br s), 4.31-4.26 (3H, m), 4.19-4.16 (1H, m), 4.06 (1H, dd, J = 19.9, 9.4 Hz), 3.82 (1H, dd, J = 13.3, 4.1 Hz), 2.90 (2H, t, J = 7.3 Hz), 2.62 (2H, t, J = 7.5 Hz), 2.46 (1H, dd, J = 13.7, 7.8 Hz), 2.37 (3H, s), 2.01-1.95 (1H, m), 1.32 (3H, d, J = 6.4 Hz), 1.23 (3H, d, J = 5.9 Hz).
LCMS: 471.2 $[M+H]^+$ (Rt= 2.193 min) (Method B).

<Example 126: (2S,11aR)-6-((2,2-Difluorocyclopropyl)methoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 68 were carried out except that the compound obtained in Reference Example 120 (20.0 mg, 0.05 mmol) and the compound obtained in Reference Example 85 (9.1 mg, 0.06 mmol) were used as raw materials, to thereby produce the title compound (5.5 mg).

$^1$H-NMR ($CDCl_3$) $\delta$: 7.86 (1H, s), 7.39 (1H, br s), 6.67 (1H, d, J = 5.9 Hz), 6.04 (1H, d, J = 5.0 Hz), 5.65-5.60 (1H, m), 4.45-4.42 (1H, m), 4.26-4.16 (2H, m), 4.11-3.97 (5H, m), 2.92 (2H, t, J = 7.1 Hz), 2.66-2.64 (2H, m), 2.36-2.33 (1H, m), 2.27 (3H, d, J = 1.4 Hz), 2.16-2.10 (3H, m).
LCMS: 504.2 $[M+H]^+$ (Rt= 1.939 min) (Method B).

<Example 127: (2S,11aR)-2-((8-Chloro-3-fluoro-2-oxo-1,2,3,4-tetrahydro-1,6-naphthyridin-7-yl)oxy)-7-fluoro-6-isopropoxy-8-methyl-2,3,11,11a-tetrahydro-1H,5H-benzo[f]pyrrolo[2,1-c][1,4]oxazepin-5-one>

[Step 1] 8-Chloro-7-(((2S,11aR)-7-fluoro-6-isopropoxy-8-methyl-5-oxo-2,3,11,11a-tetrahydro-1H,5H-benzo[f]pyrrolo[2,1-c][1,4]oxazepin-2-yl)oxy)-2-oxo-1,2,3,4-tetrahydro-1,6-naphthyridin-3-yl benzoate

The same procedures as those in Step 2 to Step 4 of Reference Example 85 were carried out except that the compound obtained in Step 1 of Example 117 (400 mg, 0.61 mmol) and (3-ethoxy-3-oxo-1-propen-2-yl) benzoate (180 $\mu$L, 0.92 mmol) were used as raw materials, to thereby produce the title compound (34.2 mg).
LCMS: 610.2 $[M+H]^+$ (Rt= 2.438 min) (Method B).

[Step 2] (2S,11aR)-2-((8-Chloro-3-hydroxy-2-oxo-1,2,3,4-tetrahydro-1,6-naphthyridin-7-yl)oxy)-7-fluoro-6-isopropoxy-8-methyl-2,3,11,11a-tetrahydro-1H,5H-benzo[f]pyrrolo[2,1-c][1,4]oxazepin-5-one

The compound obtained in Step 1 (34.2 mg, 0.06 mmol) was dissolved in methanol (2.00 mL), combined with potassium carbonate (25.0 mg, 0.18 mmol), and agitated overnight at room temperature. The reaction mixture was concentrated under reduced pressure, and the residue was combined with water and extracted three times with ethyl acetate. The organic phase was collected, washed with saturated saline solution, dried with sodium sulfate, and concentrated under reduced pressure to thereby produce the title compound (27.6 mg).
LCMS: 506.2 $[M+H]^+$ (Rt= 1.996 min) (Method B).

[Step 3] (2S,11aR)-2-((8-Chloro-3-fluoro-2-oxo-1,2,3,4-tetrahydro-1,6-naphthyridin-7-yl)oxy)-7-fluoro-6-isopropoxy-8-methyl-2,3,11,11a-tetrahydro-1H,5H-benzo[f]pyrrolo[2,1-c][1,4]oxazepin-5-one

The compound obtained in Step 2 (21.1 mg, 0.04 mmol) was dissolved in dichloromethane (0.83 mL), combined with morpholinosulfur trifluoride (20.4 $\mu$L, 0.17 mmol), and agitated overnight at room temperature. The reaction mixture was combined with saturated sodium bicarbonate aqueous solution, and extracted three times with ethyl acetate. The organic phase was collected, washed with saturated saline solution, dried with sodium sulfate, and concentrated under reduced pressure. The residue was purified with silica gel column chromatography (heptane/ethyl acetate), then with amino silica gel column chromatography (ethyl acetate/methanol) to thereby produce the title compound (2.5 mg).

$^1$H-NMR ($CDCl_3$) $\delta$: 7.88 (1H, br s), 7.85 (1H, s), 6.62 (1H, d, J = 5.5 Hz), 5.69-5.62 (1H, m), 5.26-5.05 (1H, m), 4.44-4.34 (1H, m), 4.23-4.09 (3H, m), 4.08-4.00 (2H, m), 3.42-3.21 (2H, m), 2.51-2.41 (1H, m), 2.26 (3H, d, J = 2.3 Hz),

2.16-2.07 (1H, m), 1.32 (3H, d, J = 5.9 Hz), 1.24 (3H, d, J = 5.9 Hz).
LCMS: 508.2 $[M+H]^+$ (Rt= 2.167 min) (Method B).

<Example 128: (2S,11aR)-7-Fluoro-6-isopropoxy-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 121 (69.7 mg, 0.24 mmol) and the compound obtained in Reference Example 84 (95.4 mg, 0.31 mmol) were used as raw materials, to thereby produce the title compound (30.5 mg).

$^1$H-NMR ($CDCl_3$) $\delta$: 7.87 (1H, s), 7.78 (1H, br s), 7.09 (1H, dd, J = 10.7, 8.9 Hz), 6.75 (1H, dd, J = 8.9, 3.9 Hz), 6.01 (1H, s), 5.66-5.59 (1H, m), 4.54-4.46 (1H, m), 4.21-4.16 (1H, m), 4.15-3.98 (4H, m), 2.95-2.89 (2H, m), 2.69-2.63 (2H, m), 2.40-2.32 (1H, m), 2.12-2.04 (1H, m), 1.37 (3H, d, J = 5.9 Hz), 1.26 (3H, d, J = 6.0 Hz).
LCMS: 442.2 $[M+H]^+$ (Rt= 1.770 min) (Method B).

<Example 129: (2S,11aR)-6-Methoxy-8-methyl-2-((2-oxo-1,2,3,4-tetrahydro-1,6-naphthyridin-7-yl)oxy)-7-propyl-2,3,11,11a-tetrahydro-1H,5H-benzo[f]pyrrolo[2,1-c][1,4]oxazepin-5-one>

The same procedures as those in Example 55 were carried out except that the compound obtained in Reference Example 122 (38.9 mg, 0.13 mmol) and the compound obtained in Reference Example 84 (51.5 mg, 0.17 mmol) were used as raw materials, to thereby produce the title compound (6.6 mg).

$^1$H-NMR ($CDCl_3$) $\delta$: 7.86 (1H, s), 7.72 (1H, br s), 6.64 (1H, s), 5.99 (1H, s), 5.66-5.59 (1H, m), 4.21-3.97 (5H, m), 3.82 (3H, s), 2.94-2.87 (2H, m), 2.69-2.49 (4H, m), 2.39-2.31 (1H, m), 2.29 (3H, s), 2.11-2.03 (1H, m), 1.55-1.45 (2H, m), 1.00 (3H, t, J = 7.2 Hz).
LCMS: 452.2 $[M+H]^+$ (Rt= 1.949 min) (Method B).

<Example 130: (2S,11aR)-7-Fluoro-8-methyl-2-((2-oxo-1,2,3,4-tetrahydro-1,6-naphthyridin-7-yl)oxy)-6-((1,1,1-trifluoro-2-methylpropan-2-yl)oxy)-2,3,11,11a-tetrahydro-1H,5H-benzo[f]pyrrolo[2,1-c][1,4]oxazepin-5-one>

The same procedures as those in Example 55 were carried out except that the compound obtained in Reference Example 123 (94.3 mg, 0.25 mmol) and the compound obtained in Reference Example 84 (101 mg, 0.33 mmol) were used as raw materials, to thereby produce the title compound (50.2 mg).

$^1$H-NMR ($CDCl_3$) $\delta$: 7.88 (1H, s), 7.86 (1H, s), 6.72 (1H, d, J = 5.9 Hz), 5.99 (1H, s), 5.67-5.60 (1H, m), 4.23 (1H, dd, J = 9.6, 4.1 Hz), 4.18-4.00 (3H, m), 3.92 (1H, dd, J = 13.3, 4.6 Hz), 2.99-2.85 (2H, m), 2.72-2.58 (2H, m), 2.46-2.36 (1H, m), 2.27 (3H, d, J = 2.3 Hz), 2.10-2.00 (1H, m), 1.54 (3H, s), 1.39 (3H, s).
LCMS: 524.2 $[M+H]^+$ (Rt= 1.937 min) (Method B).

<Example 131: (2S,11aR)-7-Fluoro-2-((8-fluoro-2-oxo-1,2,3,4-tetrahydro-1,6-naphthyridin-7-yl)oxy)-8-methyl-6-((1,1,1-trifluoro-2-methylpropan-2-yl)oxy)-2,3,11,11a-tetrahydro-1H,5H-benzo[f]pyrrolo[2,1-c][1,4]oxazepin-5-one>

The same procedures as those in Example 117 were carried out except that the compound obtained in Reference Example 123 (71.7 mg, 0.19 mmol) and the compound obtained in Reference Example 124 (85.5 mg, 0.25 mmol) were used as raw materials, to thereby produce the title compound (69.0 mg).

$^1$H-NMR ($CDCl_3$) $\delta$: 7.66 (1H, s), 7.62 (1H, s), 6.73 (1H, d, J = 5.5 Hz), 5.72-5.65 (1H, m), 4.30-4.15 (3H, m), 4.06 (1H, t, J = 10.5 Hz), 3.90 (1H, dd, J = 13.7, 4.1 Hz), 3.05-2.90 (2H, m), 2.75-2.61 (2H, m), 2.54-2.45 (1H, m), 2.27 (3H, d, J = 1.8 Hz), 2.10-2.01 (1H, m), 1.52 (3H, s), 1.35 (3H, s).
LCMS: 542.2 $[M+H]^+$ (Rt= 2.118 min) (Method B).

<Example 132: (2S,11aR)-2-((8-Chloro-2-oxo-1,2,3,4-tetrahydro-1,6-naphthyridin-7-yl)oxy)-7-fluoro-8-methyl-6-((1,1,1-trifluoro-2-methylpropan-2-yl)oxy)-2,3,11,11a-tetrahydro-1H,5H-benzo[f]pyrrolo[2,1-c][1,4]oxazepin-5-one>

The same procedures as those in Example 117 were carried out except that the compound obtained in Reference Example 123 (71.7 mg, 0.19 mmol) and the compound obtained in Reference Example 96 (89.6 mg, 0.25 mmol) were used

as raw materials, to thereby produce the title compound (56.9 mg).

$^1$H-NMR ($CDCl_3$) δ: 7.81 (1H, s), 7.78 (1H, s), 6.73 (1H, d, J = 5.9 Hz), 5.65-5.59 (1H, m), 4.30-4.16 (3H, m), 4.06 (1H, t, J = 10.3 Hz), 3.99 (1H, dd, J = 13.7, 4.6 Hz), 3.04-2.90 (2H, m), 2.75-2.61 (2H, m), 2.55-2.46 (1H, m), 2.27 (3H, d, J = 2.3 Hz), 2.14-2.04 (1H, m), 1.52 (3H, s), 1.34 (3H, s).
LCMS: 558.2 $[M+H]^+$ (Rt= 2.198 min) (Method B).

<Example 133:(8aR,10S)-5-Methyl-10-((2-oxo-1,2,3,4-tetrahydro-1,6-naphthyridin-7-yl)oxy)-3,4,8a,9,10,11-hexahydro-2H,8H, 13H-chromeno[8,7-f]pyrrolo[2,1-c][1,4]oxazepin-13-one>

[Step 1] (8aR,10S)-10-((4-(Benzylamino)-3-chloro-5-iodopyridin-2-yl)oxy)-5-methyl-3,4,8a,9,10,11-hexahydro-2H,8H,13H-chromeno[8,7-f]pyrrolo[2,1-c][1,4]oxazepin-13-one

The same procedures as those in Step 1 of Example 117 were carried out except that the compound obtained in Reference Example 125 (50.4 mg, 0.17 mmol) and the compound obtained in Reference Example 96 (75.8 mg, 0.21 mmol) were used as raw materials, to thereby produce the title compound (82.7 mg).

$^1$H-NMR ($CDCl_3$) δ: 8.09 (1H, s), 7.40-7.28 (5H, m), 6.47 (1H, s), 5.61-5.53 (1H, m), 4.80-4.71 (3H, m), 4.33 (1H, ddd, J = 10.0, 6.4, 3.2 Hz), 4.21-3.98 (6H, m), 2.68-2.54 (2H, m), 2.38 (1H, ddd, J = 14.0, 8.4, 4.8 Hz), 2.18 (3H, s), 2.15-2.05 (2H, m), 2.04-1.94 (1H, m).
LCMS: 632.2 $[M+H]^+$ (Rt= 2.656 min) (Method B).

[Step 2] Ethyl (E)-3-(4-(benzylamino)-5-chloro-6-(((8aR,10S)-5-methyl-13-oxo-3,4,8a,9,10,11-hexahydro-2H,8H,13H-chromeno[8,7-f]pyrrolo[2,1-c][1,4]oxazepin-10-yl)oxy)pyridin-3-yl)acrylate

The same procedures as those in Step 1 of Reference Example 84 were carried out except that the compound obtained in Step 1 (50.0 mg, 0.08 mmol) and ethyl acrylate (17.2 μL, 0.16 mmol) were used as raw materials, to thereby produce the title compound (33.7 mg).

$^1$H-NMR ($CDCl_3$) δ: 7.91 (1H, s), 7.82 (1H, d, J = 16.0 Hz), 7.39-7.27 (5H, m), 6.48 (1H, s), 6.21 (1H, d, J = 16.0 Hz), 5.69-5.63 (1H, m), 5.04 (1H, dd, J = 6.2, 6.2 Hz), 4.54 (2H, d, J = 6.4 Hz), 4.36-4.30 (1H, m), 4.23 (2H, q, J = 7.3 Hz), 4.22-4.01 (7H, m), 2.68-2.55 (2H, m), 2.45-2.34 (1H, m), 2.19 (3H, s), 2.17-2.05 (2H, m), 2.04-1.95 (1H, m), 1.30 (3H, t, J = 7.1 Hz).
LCMS: 604.3 $[M+H]^+$ (Rt= 2.591 min) (Method B).

[Step 3] Ethyl 3-(4-amino-6-(((8aR,10S)-5-methyl-13-oxo-3,4,8a,9,10,11-hexahydro-2H,8H,13H-chromeno[8,7-f]pyrrolo[2,1-c][1,4]oxazepin-10-yl)oxy)pyridin-3-yl)propanoate

The same procedures as those in Step 3 of Reference Example 85 were carried out except that the compound obtained in Step 2 (33.7 mg, 0.06 mmol) was used as a raw material, to thereby produce the title compound (18.5 mg).

$^1$H-NMR ($CD_3OD$) δ: 7.56 (1H, s), 6.50 (1H, s), 6.07 (1H, s), 5.38 (1H, br s), 4.24-4.01 (8H, m), 3.90 (1H, dd, J = 13.0, 4.8 Hz), 2.76 (2H, t, J = 7.3 Hz), 2.69-2.62 (2H, m), 2.57 (2H, t, J = 7.3 Hz), 2.42-2.33 (1H, m), 2.21 (3H, s), 2.19-2.10 (1H, m), 2.06-1.97 (2H, m), 1.23 (3H, t, J = 7.1 Hz).
LCMS: 482.2 $[M+H]^+$ (Rt= 1.745 min) (Method B).

[Step 4] (8aR,10S)-5-Methyl-10-((2-oxo-1,2,3,4-tetrahydro-1,6-naphthyridin-7-yl)oxy)-3,4,8a,9,10,11-hexahydro-2H,8H,13H-chromeno[8,7-f]pyrrolo[2,1-c][1,4]oxazepin-13-one

The same procedures as those in Step 4 of Reference Example 85 were carried out except that the compound obtained in Step 3 (7.60 mg, 0.02 mmol) was used as a raw material, to thereby produce the title compound (4.6 mg).

$^1$H-NMR ($CDCl_3$) δ: 7.85 (1H, s), 7.75 (1H, br s), 6.47 (1H, s), 6.07 (1H, s), 5.63-5.56 (1H, m), 4.32 (1H, ddd, J = 10.0, 6.4, 3.2 Hz), 4.19-3.95 (6H, m), 2.91 (2H, t, J = 7.5 Hz), 2.69-2.54 (4H, m), 2.36-2.24 (1H, m), 2.19 (3H, s), 2.14-1.96 (3H, m).
LCMS: 436.2 $[M+H]^+$ (Rt= 1.704 min) (Method B).

<Example 134:(8aR,10S)-10-((8-Chloro-2-oxo-1,2,3,4-tetrahydro-1,6-naphthyridin-7-yl)oxy)-5-methyl-3,4,8a,9,10,11-hexahydro-2H,8H,13H-chromeno[8,7-f]pyrrolo[2,1-c][1,4]oxazepin-13-one>

[Step 1] Ethyl 3-(4-amino-5-chloro-6-(((8aR,10S)-5-methyl-13-oxo-3,4,8a,9,10,11-hexahydro-2H,8H,13H-chromeno[8,7-f]pyrrolo[2,1-c][1,4]oxazepin-10-yl)oxy)pyridin-3-yl)propanoate

The same procedures as those in Step 3 of Reference Example 85 were carried out except that the compound obtained in Step 2 of Example 133 (33.7 mg, 0.06 mmol) was used as a raw material, to thereby produce the title compound (2.8 mg).
LCMS: 516.2 $[M+H]^+$ (Rt= 2.066 min) (Method B).

[Step 2] (8aR,10S)-10-((8-Chloro-2-oxo-1,2,3,4-tetrahydro-1,6-naphthyridin-7-yl)oxy)-5-methyl-3,4,8a,9,10,11-hexahydro-2H,8H,13H-chromeno[8,7-f]pyrrolo[2,1-c][1,4]oxazepin-13-one

The same procedures as those in Step 4 of Reference Example 85 were carried out except that the compound obtained in Step 1 (2.8 mg, 5.4 μmol) was used as a raw material, to thereby produce the title compound (2.7 mg).

$^1$H-NMR ($CDCl_3$) δ: 7.86 (1H, s), 7.77 (1H, s), 6.48 (1H, s), 5.67-5.60 (1H, m), 4.33 (1H, ddd, J = 10.4, 6.4, 3.2 Hz), 4.25-4.14 (2H, m), 4.13-4.01 (4H, m), 3.03-2.90 (2H, m), 2.74-2.55 (4H, m), 2.46-2.37 (1H, m), 2.19 (3H, s), 2.15-1.95 (3H, m).
LCMS: 470.2 $[M+H]^+$ (Rt= 2.001 min) (Method B).

The structural formulae and IUPAC names of the compounds in the above examples are shown in the tables below.

[Table 1]

| Example | IUPAC name | Structural formula |
|---|---|---|
| 1 | (2S,11aR)-6-isopropoxy-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 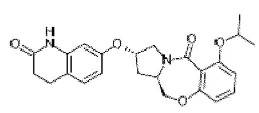 |
| 2 | (2S,11aR)-6-(benzyloxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 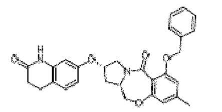 |
| 3 | (2S,11aR)-6-hydroxy-8-methyl-2-((2-oxo-1,2,3,4-tetrahydroquinolin-7-yl]oxy)-2,3,11,11a-tetrahydro-1H,5H-benzo[f]pyrrolo[2,1-c][1,4]oxazepin-5-one | 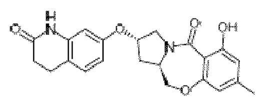 |
| 4 | (2S,11aR)-6-ethoxy-8-methyl-2-((2-oxo-1,2,3,4-tetrahydroquinolin-7-yl)oxy-2,3,11,11a-tetrahydro-1H,5H-benzo[f]pyrrolo[2,1-c][1,4]oxazepin-5-one | 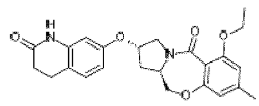 |
| 5 | (2S,11aR)-6-isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 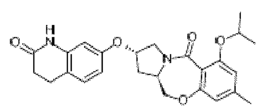 |
| 6 | (2S,11aS)-6-isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 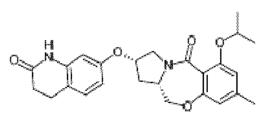 |
| 7 | (2S,11aR)-6-(((S)-1-fluoropropan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 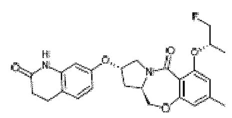 |

(continued)

| Example | IUPAC name | Structural formula |
|---|---|---|
| 8 | (2S,11aR)-6-(((S)-1,1-difluoropropan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 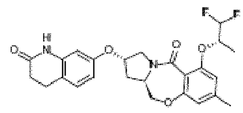 |
| 9 | (2S,11aR)-8-methyl-2-((2-oxo-1,2,3,4-tetrahydroquinolin-7-yl) oxy)-6-((1,1,1-trifluoropropan-2-yl)oxy)-2,3,11,11a-tetrahydro-1H,5H-benzo[f]pyrrolo[2,1-c][1,4]oxazepin-5-one | 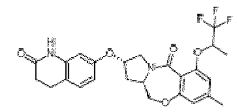 |
| 10 | (2S,11aR)-8-methyl-2-((2-oxo-1,2,3,4-tetrahydroquinolin-7-yl) oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one | 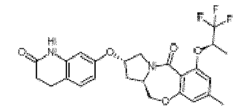 |
| 11 | (2S,11aR)-6-(cyclopentyloxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 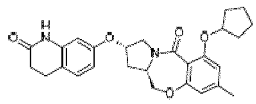 |
| 12 | (2S,11aR)-6-(((1S,2R,4R)-bicyclo[2. 2. 1]heptan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 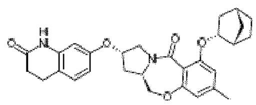 |
| 13 | (2S,11aR)-8-methyl-6-(neopentyloxy)-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 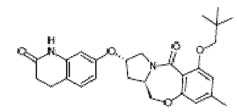 |
| 14 | (2S,11aR)-8-methyl-2-((2-oxo-1,2,3,4-tetrahydroquinolin-7-yl)oxy)-6-((tetrahydrofuran-2-yl)methoxy)-2,3,11,11a-tetrahydro-1H,5H-benzo[f]pyrrolo[2,1-c][1,4]oxazepin-5-one | 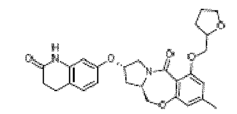 |

[Table 2]

| Example | IUPAC name | Structural formula |
|---|---|---|
| 15 | [2S,11aR)-6-isobutyl-8-methyl-2-((2-oxo-1,2,3,4-tetrahydroquinolin-7-yl) oxy)-2,3,1 1,11a-tetrahydro-1H,5H-benzo[f]pyrrolo[2,1-c][1,4]oxazepin-5-one | 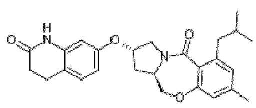 |
| 16 | (2S,11aR)-6-cyclohexyl-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 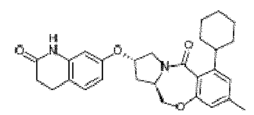 |
| 17 | (2S,11aR)-6-(2-fluorophenyl)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 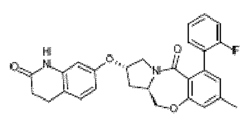 |
| 18 | (2S,11aR)-6-(2,6-difluorophenyl)-8-methyl-2-((2-oxa-1,2,3,4-tetrahydroquinolin-7-yl)oxy)-2,3,11,11a-tetrahydro-1H,5H-benzo[f]pyrrolo[2,1-c][1,4] oxazepin-5-one | 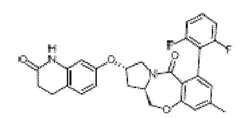 |
| 19 | (2S,11aR)-6-(2-(difluoromethyl)phenyl)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 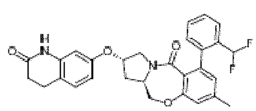 |

(continued)

| Example | IUPAC name | Structural formula |
|---|---|---|
| 20 | [2S,11aR)-6-(3-fluoropyridin-2-yl)-8-methyl-2-((2-oxo-1 ,2,3,4-tetrahydroquinolin-7-yl)oxy)-2,3,11,11a-tetrahydro-1H,5H-benzo[f]pyrrolo[2,1-c][1,4] oxazepin-5-one | 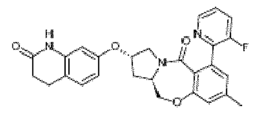 |
| 21 | (2S,11aR)-8-methyl-2-((2-oxo-1,2,3,4-tetrahydroquinolin-7-yl) oxy)-6-(2-(trifluoromethyl)pyridin-3-yl)-2,3,11,11a-tetrahydro-1H,5H-benzo [f]pyrrolo[2,1-c][1,4]oxazepin-5-one | 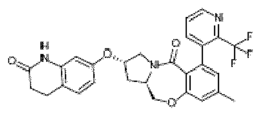 |
| 22 | [2S,11aR)-8-methyl-2-((2-oxo-1,2,3,4-tetrahydroquinolin-7-yl)oxy)-6-(thiophen-2-yl)-2,3,11,11a-tetrahydro-1H,5H-benzo[f]pyrrolo[2,1-c][1,4]oxaze-pin-5-one | 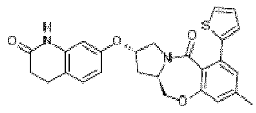 |
| 23 | (2S,11aR)-8-methyl-2-((2-oxo-1,2,3,4-tetrahydroquinolin-7-yl)oxy)-6-(pyrrolidin-1-yl)-2,3,11,11a-tetrahydro-1H,5H-benzo[f]pyrrolo[2,1-c][1,4]oxaze-pin-5-one | 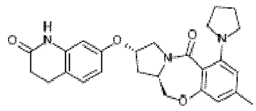 |
| 24 | 7-(((2S,11aR)-6-isopropoxy-8-methyl-2,3,11,11a-tetrahydro-1H,5H-benzo [f]pyrrolo[2,1-c][1,4]oxazepin-2-yl)oxy)-3,4-dihydroquinolin-2(1H)-one | 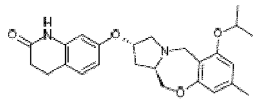 |
| 25 | (2S,11aR)-6-(cyclopentyloxy)-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxa-zepin-5-one | 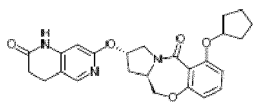 |
| 26 | (2S,11aR)-6-(cyclopropylmethoxy)--B-methyl-2-((2-oxo-1,2,3,4-tetrahydro-1,6-naphithyridin-7-yl)oxy)-2,3,11,11a-tetrahydro-1H,5H-benzo[f]pyrrolo[2,1-c][1,4]oxazepin-5-one | 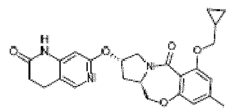 |
| 27 | (2S,11aR)-6-isobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c] [1,4]oxazepin-5-one | 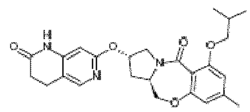 |
| 28 | (2S,11aR)-6-(2-hydroxy-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f] pyrrolo[2,1-c][1,4]oxazepin-5-one | 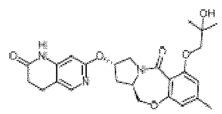 |

[Table 3]

| Example | IUPAC name | Structural formula |
|---|---|---|
| 29 | (2S,11aR)-6-(2-fluoro-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f] pyrrolo[2,1-c][1,4]oxazepin-5-one | 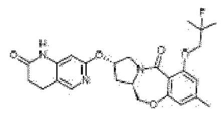 |
| 30 | (2S,11aR)-6-isopropoxy-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxa-zepin-5-one | 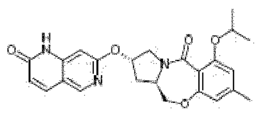 |
| 31 | (2S,11aR)-6-isopropoxy-8-methyl-2-((2-oxo-1 ,2,3,4-tetrahydro-1,6-naphtyridin-7-yl)oxy)-2,3,11,11a-tetrahydro-1H,5H-benzo[f]pyrrolo[2,1-c][1,4] oxazepin-5-one | 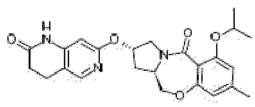 |

(continued)

| Example | IUPAC name | Structural formula |
|---|---|---|
| 32 | (2S,11aR)-8-chloro-6-isopropoxy-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 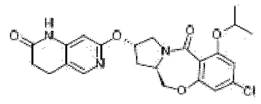 |
| 33 | (2S,11aR)-6-isopropoxy-2-((2-oxo-1,2,3,4-tetrahydro-1,6-naphthyridin-7-yl)oxy)-8-(trifluoromethyl)-2,3,11,11a-tetrahydro-1H,5H-benzo[f]pyrrolo[2,1-c][1,4]oxazepin-5-one | 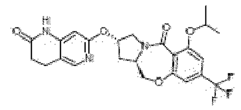 |
| 34 | (2S,11aR)-2-((8-fluoro-2-oxo-1,2,3,4-tetrahydroquinolin-7-yl)oxy)-6-isopropoxy-8-methyl-2,3,11,11a-tetrahydro-1H,5H-benzo[f]pyrrolo[2,1-c][1,4]oxazepin-5-one | 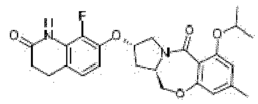 |
| 35 | (2S,11aR)-2-((8-fluoro-2-oxo-1,2,3,4-tetrahydro-1,6-naphthyridin-7-yl)oxy)-6-isopropoxy-8-methyl-2,3,11,11a-tetrahydro-1H,5H-benzo[f]pyrrolo[2,1-c][1,4]oxazepin-5-one | 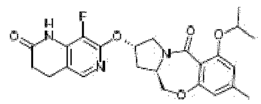 |
| 36 | (2S,1 1aR)-6-isopropoxy-8-methyl-2-((2-oxo-1,4-dihydro-2H-benzo[d][1,3]oxadin-7-yl)oxy)-2,3,11,11a-tetrahydro-1H,5H-benzo[f]pyrrolo[2,1-c][1,4]oxazepin-5-one | 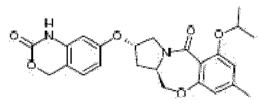 |
| 37 | (7aS,9S)-1-isopropoxy-3-methyl-9-((2-oxo-1,2,3,4-tetrahydro-1,6-naphthyridin-7-yl)oxy)-6,7,7a,8,9,10-hexahydro-12H-benzo[b]pyrrolo[1,2-e][1,5]oxazocin-12-one | 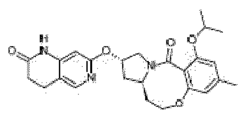 |
| 38 | (2S,11aR)-6-(((R)-1-fluoropropan-2-yl)oxy)-8-methyl-2-((2-oxo-1,2,3,4-tetrahydro-1,6-naphthyridin-7-yl)oxy)-2,3,11,11a-tetrahydro-1 H,5H-benzo[f]pyrrolo[2,1-c][1,4]oxazepin-5-one | 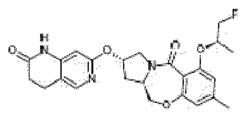 |
| 39 | (2S,11aR)-6-(((R)-1,1-difluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 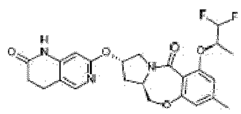 |
| 40 | (2S,11aR)-8-methyl-2-((2-oxo-1,2-dihydro-1,6-naphthyridin-7-yl)oxy)-6-(((R)-1,1,1-trifluoropropan-2-yl)oxy)-2,3,11,11a-tetrahydro- 1H,5H-benzo[f]pyrrolo[2,1-c][1,4]oxazepin-5-one | 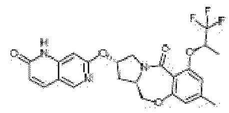 |
| 41 | (2S,11aR)-8-methyl-2-((2-oxo-1,2,3,4-tetrahydro-1,6-naphthyridin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one | 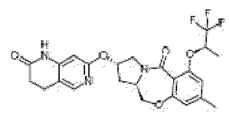 |
| 42 | (2S,11aR)-8-methyl-2-((2-oxo-1,2,3,4-tetrahydro-1,6-naphthyridin-7-yl)oxy)-6-(((S)-1,1,1-trifluoropropan-2-yl)oxy)-2,3,11,11a-tetrahydro-1H,5H-benzo[f]pyrrolo[2,1-c][1,4]oxazepin-5-one | 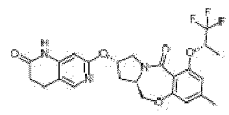 |

[Table 4]

| Example | IUPAC name | Structural formula |
|---|---|---|
| 43 | (2S,11aR)-2-((8-fluoro-2-oxo-1,2,3,4-tetrahydro-1,6-naphthyridin-7-yl)oxy)-8-methyl-6-(((R)-1,1,1-trifluoropropan-2-yl)oxy)-2,3,11,11a-tetrahydro-1H,5H-benzo[f]pyrrolo[2,1-c][1,4]oxazepin-5-one | 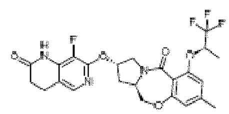 |

(continued)

| Example | IUPAC name | Structural formula |
|---|---|---|
| 44 | (2S,11aR)-8-methyl-2-((2-oxo-1,4-dihydro-2H-benzo[d][1,3]oxadin-7-yl) oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one | 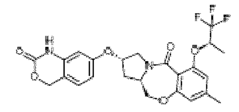 |
| 45 | (2S,11aR)-8-methyl-2-((3-oxo-3,4-dihydro-2H-benzo[b][1,4]oxadin-6-yl) oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one | 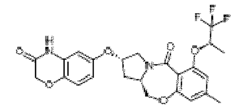 |
| 46 | (2S,11aR)-6-((R)-sec-butoxy)-8-methyl-2-(2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 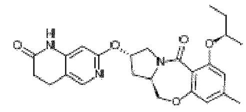 |
| 47 | (2S,11aR)-6-((S)-sec-butoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 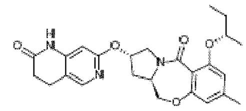 |
| 48 | (2S,11aR)-6-cyclobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 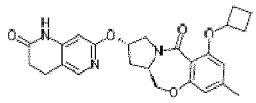 |
| 49 | (2S,11aR)-6-(cyclopentyloxy)-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one | 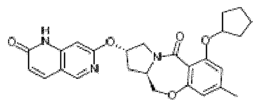 |
| 50 | (2S,11aR)-6-(cyclopentyloxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 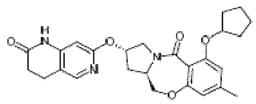 |
| 51 | (2S,11aR)-6-((2,2-difluorocyclopentyl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 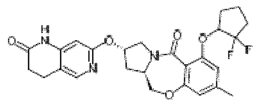 |
| 52 | (2S,11aR)-6-isobutyl-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 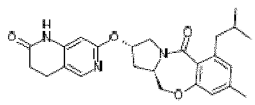 |
| 53 | (2S,11aR)-6-(2,6-difluorophenyl)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 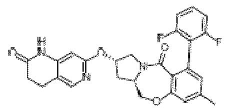 |
| 54 | (2S,11aR)-8-methyl-2-((2-oxo-1,2,3,4-tetrahydro-1,6-naphthyridin-7-yl) oxy)-6-(pyrrolidin-1-yl)-2,3,11,11a-tetrahydro-1H,5H-benzo[f]pyrrolo[2,1-c][1,4]oxazepin-5-one | 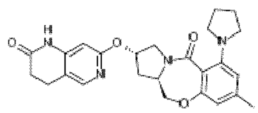 |
| 55 | (2S,11aR)-7-fluoro-6-methoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 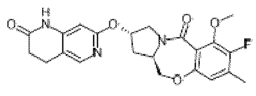 |
| 56 | (2S,11aR)-6-ethoxy-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 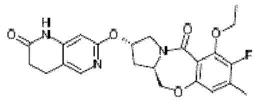 |

[Table 5]

| Example | IUPAC name | Structural formula |
|---|---|---|
| 57 | (2S,11aR)-7-fluoro-8-methyl-2-((2-oxo-1,2,3,4-tetrahydro-1,6-naphthyridin-7-yl)oxy)-6-propoxy-2,3,11,11a-tetrahydro-1H,5H-benzo[f]pyrrolo[2,1-c][1,4]oxazepin-5-one | 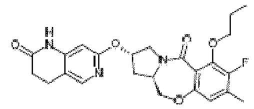 |
| 58 | (2S,11aR)-6-butoxy-7-fluoro-8-methyl-2-((2-oxo-1,2,3,4-tetrahydro-1,6-naphthyridin-7-y1)oxy)-2,3,11,11a-tetrahydro-1H,5H-benzo[f]pyrrolo[2,1-c][1,4]oxazepin-5-one | 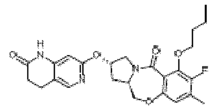 |
| 59 | (2S,11aR)-7-fluoro-8-methyl-2-((2-oxo-1,2,3,4-tetrahydro-1,6-naphthyridin-7-yl)oxy)-6-(pentylioxy)-2,3,11,11a-tetrahydro-1H,5H-benzo[f]pyrrolo[2,1-c][1,4]oxazepin-5-one | 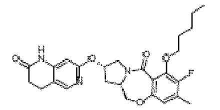 |
| 60 | (2S,11aR)-7-fluoro-6-(3-fluoropropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 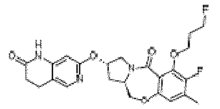 |
| 61 | (2S,11aR)-7-fluoro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 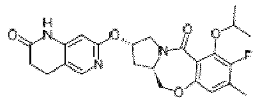 |
| 62 | (2S,11aR)-7-fluoro-6-isopropoxy-8-methyl-2-((2-oxo-2,3,4,5-tetrahydro-1H-pyrido[4,3-b]azepin-8-yl)oxy)-2,3,11,11a-tetrahydro-1H,5H-benzo[f]pyrrolo[2,1-c][1,4]oxazepin-5-one | 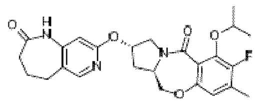 |
| 63 | (2S,11aR)-9-fluoro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 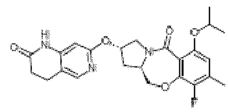 |
| 64 | (2S,11aR)-7,9-difluoro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 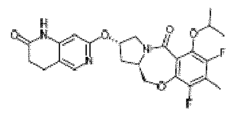 |
| 65 | (2S,11aR)-7-chloro-6-ethoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 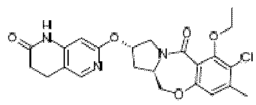 |
| 66 | (2S,11aR)-7-chloro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 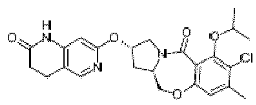 |
| 67 | (2S,11aR)-7,9-dichloro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 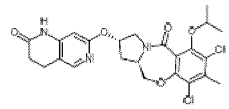 |
| 68 | (2S,11aR)-6-(cyclopropylmethoxy)-7-fluoro-8-methyl-2-((2-oxo-1,2,3,4-tetrahydro-1,6-naphthyridin-7-yl)oxy)-2,3,11,11a-tetrahydro-1H,5H-benizo[f]pyrrolo[2,1-c][1,4]oxazepin-5-one | 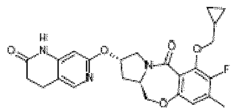 |
| 69 | (2S,11aR)-7-fluoro-8-methyl-2-((2-oxo-1,2,3,4-tetrahydro-1,6-naphthyridin-7-yl)oxy)-6-(2,2,2-trifluoroethoxy)-2,3,11,11a-tetrahydro-1H,5H- benzo[f]pyrrolo[2,1-c][1,4]oxazepin-5-one | 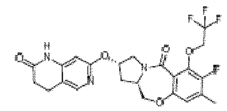 |

(continued)

| Example | IUPAC name | Structural formula |
|---|---|---|
| 70 | (2S,11aR)-7-fluoro-6-((1-fluorocyclopropyl)methoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 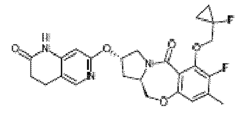 |

[Table 6]

| Example | IUPAC name | Structural formula |
|---|---|---|
| 71 | (2S,11aR)-7-fluoro-6-isobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 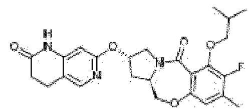 |
| 72 | (2S,11aR)-7-fluoro-6-(2-fluoro-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 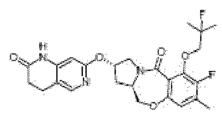 |
| 73 | (2S,11aR)-6-((1,3-difluoropropan-2-yl)oxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 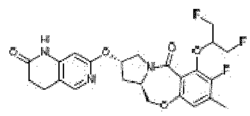 |
| 74 | (2S,11aR)-6-((R)-sec-butoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 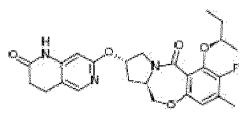 |
| 75 | (2S,11aR)-6-((S)-sec-butoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 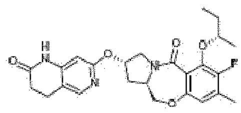 |
| 76 | (2S,11aR)-7-fluoro-6-(((R)-1-fluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 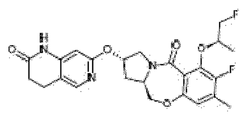 |
| 77 | (2S,11aR)-6-(((R)-1,1-difluoropropan-2-yl)oxy)-7-fluoro-8-methyl-2-((2-oxo-1,2,3,4-tetrahydro-1,6-naphthyridin-7-yl)oxy}-2,3,11,11a-tetrahydro-1H,5H-benzo[f]pyrrolo[2,1-c][1,4]oxazepin-5-one | 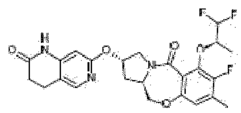 |
| 78 | (2S,11aR)-7-fluoro-8-methyl-2-((2-oxo-1,2,3,4-tetrahydro-1,6-naphthyridin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one | 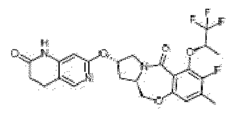 |
| 79 | (2S,11aR)-6-(cyclopentyloxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 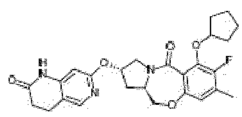 |
| 80 | (2S,11aR)-7-fluoro-8-methyl-2-((2-oxo-1,2,3,4-tetrahydro-1,6-naphthyridin-7-yl)oxy)-6-(((S)-tetrahydrofuran-3-yl)oxy)-2,3,11,11a-tetrahydro-1H,5H-benzo[f]pyrrolo[2,1-c][1,4]oxazepin-5-one | 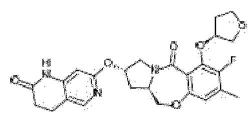 |
| 81 | (2S,11aR)-7-fluoro-8-methyl-2-((2-oxo-1,2,3,4-tetrahydro-1,6-naphthyridin-7-yl)oxy)-6-(((R)-tetrahydrofuran-3-yl)oxy)-2,3,11,11a-tetrahydro-1H,5H-benzo[f]pyrrolo[2,1-c][1,4]oxazepín-5-one | 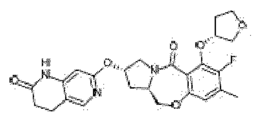 |

(continued)

| Example | IUPAC name | Structural formula |
|---|---|---|
| 82 | (2S,11S,11aR)-7-fluoro-6-isopropoxy-8,11-dimethyl-2-((2-oxo-1,2,3,4-tetrahydro-1,6-naphthyridin-7-yl)oxy)-2,3,11,11a-tetrahydro-1H.5H-benzo[f]pyrrolo[2,1-c][1,4]oxazepin-5-one | 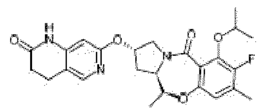 |
| 83 | (2S,11R,11aR)-7-fluoro-6-isopropoxy-8,11-dimethyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 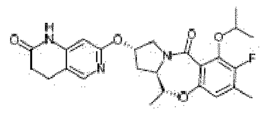 |
| 84 | (2S,11aR)-7-fluoro-8-methyl-2-((2-oxo-1,2,3,4-tetrahydro-1,6-naphthyridin-7-yl)oxy)-6-(pyrrolidin-1-yl)2,3,11,11a-tetrahydro-1H,5H-benzo[f]pyrrolo[2,1-c][1,4]oxazepin-5-one | 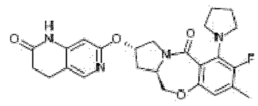 |

[Table 7]

| Example | IUPAC name | Structural formula |
|---|---|---|
| 85 | (2S,11 aR)-7-fluoro-6-isopropyl-β-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 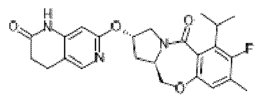 |
| 86 | (2S,11aR)-6-cyclopentyl-7-fluoro-8-methyl-2-((2-oxa-1,2,3,4-tetrahydro-1,6-naphthyridin-7-yl)oxy)-2,3,11,11a-tetrahydro-1H,5H-benzo[f]pyrrolo[2,1-c][1,4]oxazepini-5-one | 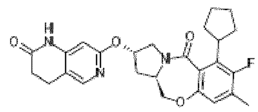 |
| 87 | (2S,11as)-7-fluoro-6-isopropoxy-8-methyl-2-((2-oxo-1,2,3,4-tetrahydro-1,6-naphthyridin-7-yl)oxy)-1,2,3,10,11,11a-hexahydro-5H-benzo[e]pyrrolo[1,2-a]azepin-5-one | 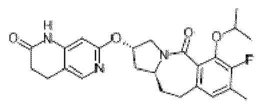 |
| 88 | (2S,11aR)-7-fluoro-2-((8-fluoro-2-oxo-1,2,3,4-tetrahydro-1,6-naphthyridin-7-yl)amino)-6-isopropoxy-8-methyl-2,3,11,11a-tetrahydro-1H,5H-benzo[f]pyrrolo[2,1-c][1,4]oxazepin-5-one | 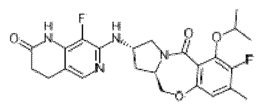 |
| 89 | (2S,11aR)-6-(cyclopropylmethoxy)-8-methyl-2-((2-oxo-1,2,3,4-tetrahydroquinolin-8-yl)oxy)-2,3,11,11a-tetrahydro-1H,5H-benzo[f]pyrrolo[2,1-c][1 ,4]oxazepin-5-one | 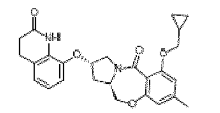 |
| 90 | (2S,11aR)-6-(cyclopropylmethoxy)-8-methyl-2-((2-oxo-1,2,3,4-tetrahydraquinolin-7-yl)oxy)-2,3,11,11a-tetrahydro-1H,5H-benzo[f]pyrrolo[2,1-c][1,4]oxazepin-5-one | 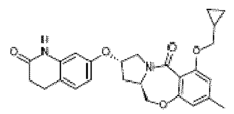 |
| 91 | (2S,11aR)-6-(cyclopropylmethoxy)-8-methyl-2-((2-oxo-1,2,3,4-tetrahydroquinolin-6-yl)oxy)-2,3,11,11a-tetrahydro-1H,5H-benzo[f]pyrrolo[2,1-c][1,4]oxazepin-5-one | 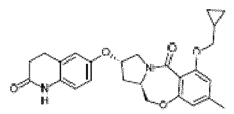 |
| 92 | (2S,11aR)-6-(cyclopropylmethoxy)-8-methyl-2-((2-oxo-1,2,3,4-tetrahydroquinolin-5-yl)oxy)-2,3,11,11a-tetrahydro-1H,5H-benzo[f]pyrrolo[2,1-c][1,4]oxazepin-5-one | 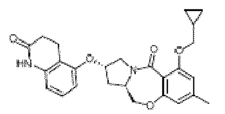 |
| 93 | (2S,11aR)-7-fluoro-8-methyl-2-((2-oxo-1,2,3,4-tetrahydro-1,6-naphthyridin-7-yl)oxy)-6-pentyl-2,3,11,11a-tetrahydro-1H,5H-benzo[f]pyrrolo[2,1-c][1,4]oxazepin-5-one | 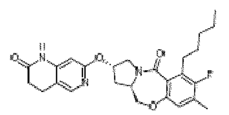 |

(continued)

| Example | IUPAC name | Structural formula |
|---|---|---|
| 94 | (2S,11aR)-7-fluoro-6-(2-fluorophenyl)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 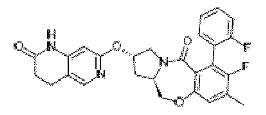 |
| 95 | (2S,11aR)-6-isopropoxy-8-methyl-2-((2-oxo-1,2,3,4-tetrahydro-1,6-naphthyridin-7-yl)amino)-2,3,11,11a-tetrahydro-1H,5H-benzo[f]pyrrolo [2,1-c][1,4]oxazepin-5-one | 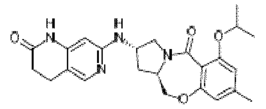 |
| 96 | (6aR,9S)-1-(cyclopropylmethoxy)-3-methyl-0-((2-oxo-1,2,3,4-tetrahydroquinolin-7-yl)oxy)-6,6a,7,8,9,10-hexahydro-12H-benzo[f]pyrido[2,1-c][1,4] oxazepin-12-one | 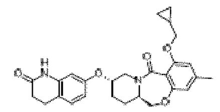 |
| 97 | (6aR,8R)-1-(cyclopropylmethoxy)-3-methyl-8-((2-oxo-1,2,3,4-tetrahydroquinolin-7-yl)oxy)-6,6a,7,8,9,10-hexahydro-12H-benzo[f]pyrido[2,1-c][1,4] oxazepin-12-one | 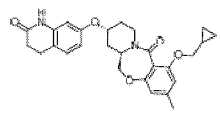 |
| 98 | (2S,11aR)-2-((3,3-dimethyl-2-oxo-1,2,3,4-tetrahydroquinolin-7-yl)oxy)-7-fluoro-6-isopropoxy-8-methyl-2,3,11,11a-tetrahydro-1H,5H-benzo[f]pyrrolo[2,1-c][1,4]oxazepin-5-one | 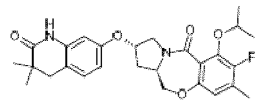 |

[Table 8]

| Example | IUPAC name | Structural formula |
|---|---|---|
| 99 | (2S,11aR)-7-fluoro-6-(((S)-1-hydroxypropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 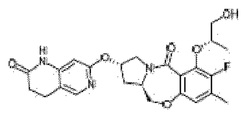 |
| 100 | (2S,11aR)-7-fluoro-6-(((R)-1-hydroxypropan-2-yl)oxy)-8-methyl-2-((2-oxo-1,2,3,4-tetrahydro-1,6-napthyridin-7- yl)oxy)-2,3,11,11a-tetrahydro-1H,5H-benzo[f]pyrrolo[2,1-c][1,4}oxazepin-5-one | 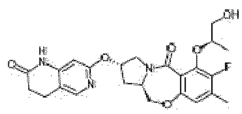 |
| 101 | (2S,11aR)-6-(2,2-difluoropropoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f] pyrrolo[2,1-c][1,4]oxazepin-5-one | 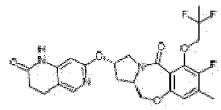 |
| 102 | (2S,11aR)-7-fluoro-6-isopropoxy-8-methyl-2-((2-oxo-1,2-dihydro-1,6-naphthyridin-7-yl)oxy)-2,3,11,11a-tetrahydro-1H,5H-benzo[f]pyrrolo[2,1-c] [1,4]oxazepin-5-one | 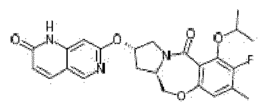 |
| 103 | (2S,11aR)-7-fluoro-8-methyl-2-((2-oxo-1,2,3,4-tetrahydro-1,6-naphthyridin-7-yl)oxy)-6-(((S)-1,1,1-trifluoropropan-2-yl)oxy)-2,3,11,11a-tetrahydro-1H,5H-benzo[f]pyrrolo[2,1-c][1,4]oxazepin-5-one | 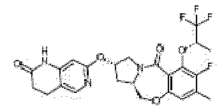 |
| 104 | (2S,11aR)-6-cyclobutoxy-7-fluoro-8-methyl-2-((2-oxo-1,2,3,4-tetrahydro-1,6-naphthyrídin-7-yl)oxy)-2,3,11,11a-tetrahydro-1H,5H-benzo[f]pyrrolo[2,1-c][1,4}oxazepin-5-one | 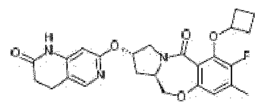 |
| 105 | (2S,11aR)-6-((4,4-difluorocyclohexyl)oxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 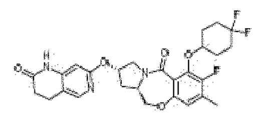 |

(continued)

| Example | IUPAC name | Structural formula |
|---|---|---|
| 106 | (2S,11aR)-7-fluoro-6-isopropoxy-8-methyl-2-((3-methyl-2-oxo-1,2,3,4-tetrahydro-1,6-naphthyridin-7-yl)oxy)-2,3.,11,11a-tetrahydro-1H,5H-benzo[f]pyrrolo[2,1-c][1,4]oxazepin-5-one | 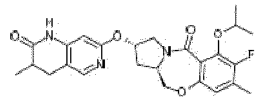 |
| 107 | (2S,11aR)-6-((3,3-difluorocyclobutyl)methoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 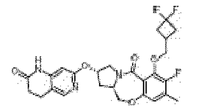 |
| 108 | (2S,11aR)-6-(3,3-difluorocyclobutoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1 ,4]oxazepin-5-one | 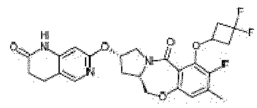 |
| 109 | (2S,11aR)-7-fluoro-6-(((R)-1-methoxypropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 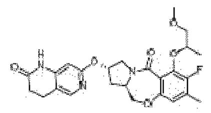 |
| 110 | (2S,11aR)-7-fluoro-6-(((S)-1-methoxypropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 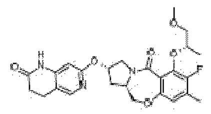 |
| 111 | (2S,11aR)-7-fluoro-6-(2-methoxy-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 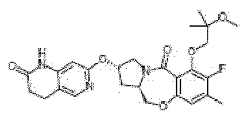 |
| 112 | (2S,11aR)-7-fluoro-6-(2-methoxyethoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 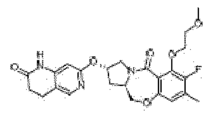 |

[Table 9]

| Example | IUPAC name | Structural formula |
|---|---|---|
| 113 | (2S,11aR)-7-fluoro-6-isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one | 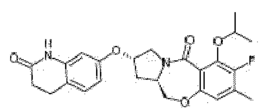 |
| 114 | (2S,11aR)-7-fluoro-8-methyl-2-((2-oxo-1,2,3,4-tetrahydro-1,6-naphtyridin-7-yl)oxy)-6-(2-(pyrrolidin-1-yl)ethoxy)-2,3,11,11a-tetrahydro-1H,5H-benzo[f]pyrrolo[2,1-c][1,4]oxazepin-5-one | 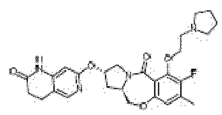 |
| 115 | (2S,11aR)-7-fluoro-2-(8-fluoro-2-oxo-1,2,3,4-tetrahydroquinolin-7-yl)oxy)-6-isopropoxy-8-methyl-2,3,11,11a-tetrahydro-1H,5H-benzo[f]pyrrolo[2,1-c][1,4]oxazepin-5-one | 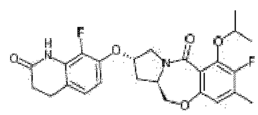 |
| 116 | (2S,11aR)-7-fluoro-2-((8-fluoro-2-oxo-1,2,3,4-tetrahydro-1,6-naphthyridin-7-yl)oxy)-6-isopropoxy-8-methyl-2,3,11,11a-tetrahydro-1H,5H-benzo[f]pyrrolo[2,1-c][1,4]oxazepin-5-one | 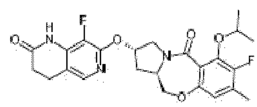 |
| 117 | (2S,11aR)-2-((8-chloro-2-oxo-1,2,3,4-tetrahydro-1,6-naphthyridin-7-yl)oxy)-7-fluoro-6-isopropoxy-8-methyl-2,3,11,11a-tetrahydro-1H,5H-benzo[f]pyrrolo[2,1-c][1,4]oxazepin-5-one | 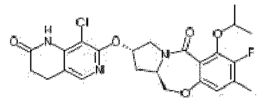 |

(continued)

| Example | IUPAC name | Structural formula |
|---|---|---|
| 118 | (2S,11aR)-7-fluoro-6-isopropoxy-8-methyl-2-((4-methyl-2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 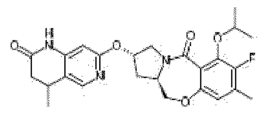 |
| 119 | (2S,11aR)-7-fluoro-6-isopropoxy-8-methyl-2-((3-oxo-3,4-dihydro-2H-benzo[b][1,4]oxadin-6-yl)oxy)-2,3,11,11a-tetrahydro-1H,5H-benzo[f]pyrrolo[2,1-c][1,4]oxazepin-5-one | 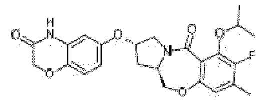 |
| 120 | (2S,11aR)-7-fluoro-2-((6-fluoro-2-oxo-1,2,3,4-tetrahydroquinolin-7-yl)oxy)-6-isopropoxy-8-methyl-2,3,11,11a-tetrahydro-1H,5H-benzo[f]pyrrolo[2,1-c][1,4]oxazepin-5-one | 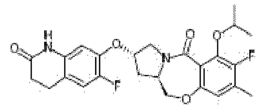 |
| 121 | (2S,11aR)-7-chloro-8-methyl-2-(2-oxo-1,2,3,4-tetrahydro-1,6-naphthyridin-7-yl)oxy)-6-(2,2,2-trifluoroethoxy)-2,3,11,11a-tetrahydro-1H,5H-benzo[f]pyrrolo[2,1-c][1,4]oxazepin-5-one | 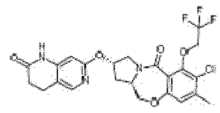 |
| 122 | (2S,11aR)-7-chloro-6-((1-fluorocyclopropyl)methoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 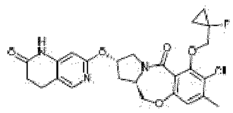 |
| 123 | (2S,11aR)-7-chloro-6-(3-fluoropropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 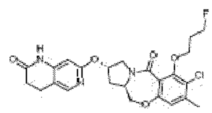 |
| 124 | (2S,11aR)-7-chloro-2-((8-fluoro-2-oxo-1,2,3,4-tetrahydro-1,6-naphthyridin-7-yl)oxy)-6-isopropoxy-8-methyl-2,31,11a-tetrahydro-1H,5H-benzo[f]pyrrolo[2,1-c][1,4]oxazepin-5-one | 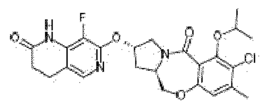 |
| 125 | (2S,11aR)-7-chloro-6-isopropoxy-8-methyl-2-((2-oxo-1,2,3,4-tetrahydroquinolin-1-yl)oxy)-2,3,11,11a-tetrahydro-1H,5H-benzo[f]pyrrolo[2,1-c][1,4]oxazepin-5-one | 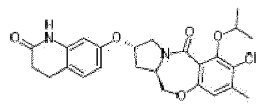 |
| 126 | (2S,11aR)-6-((2,2-difluorocyclopropyl)methoxy)-7-fluoro-8-methyl-2-((2-oxo-1,2,3 4-tetrahydro-1,6-naphthyridin-7-yl)oxy)-2,3,11,11a-tetrahydro-1H,5H-benzo[f]pyrrolo[2,1-c][1,4]oxazepin-5-one | 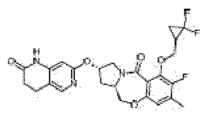 |

[Table 10]

| Example | IUPAC name | Structural formula |
|---|---|---|
| 127 | (2S,11aR)-2-((8-chloro-3-fluoro-2-oxo-1,2,3,4-tetrahydro-1,6-naphthyridin-7-yl)oxy)-7-fluoro-6-isopropoxy-8-methyl-2,3,11,11a-tetrahydro-1H,5H-benzo[f]pyrrolo[2,1-c][1,4]oxazepin-5-one | 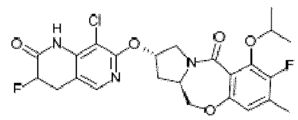 |
| 128 | (2S,11aR)-7-fluoro-6-isopropoxy-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one | 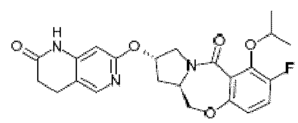 |
| 129 | (2S,11aR)-6-methoxy-8-methyl-2-((2-oxo-1,2,3,4-tetrahydro-1,6-naphthyridin-7-yl)oxy)-7-propyl-2,3,11,11a-tetrahydro-1H,5H-benzo[f]pyrrolo[2,1-c][1,4]oxazepin-5-one | 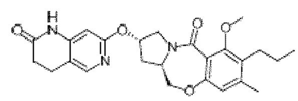 |

(continued)

| Example | IUPAC name | Structural formula |
|---|---|---|
| 130 | (2S,11aR)-7-fluoro-8-methyl-2-((2-oxo-1,2,3,4-tetrahydro-1,6-naphthyridin-7-yl)oxy)-6-((1,1,1-trifluoro-2-methylpropan-2-yl)oxy)-2,3,11,11a-tetrahydro-1H,5H-benzo[f]pyrrolo[2,1-c][1,4]oxazepin-5-one | 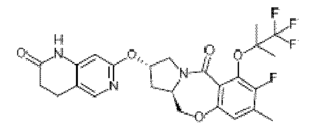 |
| 131 | (2S,11aR)-7-fluoro-2-((8-fluoro-2-oxo-1,2,3,4-tetrahydro-1,6-naphthyridin-7-yl)oxy)-8-methyl-6-((1,1,1-trifluoro-2-methylpropan-2-yl)oxy)-2,3,11,11a-tetrahydro-1H,5H-benzo[f]pyrrolo[2,1-c][1,4]oxazepin-5-one | 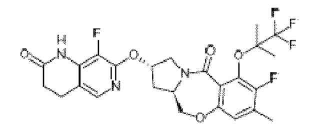 |
| 132 | (2S,11aR)-2-((8-chloro-2-oxo-1 ,2,3,4-tetrahydro-1,6-naphthyridin-7-yl)oxy)-7-fluoro-6-methyl-8-((1,1,1-trifluoro-2-methylpropan-2-yl)oxy))-2,3,11,11a-tetrahydro-1H,5H-benzo[f]pyrrolo[2,1-c][1,4]oxazepin-5-one | 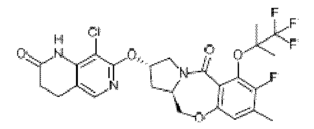 |
| 133 | (8aR,10S)-5-methyl-10-((2-oxo-1,2,3,4-tetrahydro-1,6-naphthyridin-7-yl)oxy)-3,4,8a,9,10,11-hexahydro-2H,8H,13H-chromeno[8,7-f]pyrrolo[2,1-c][1,4]oxazepin-13-one | 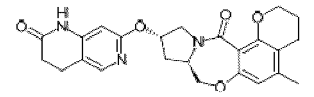 |
| 134 | (8aR,10S)-10-((8-chloro-2-oxo-1,2,3,4-tetrahydro-1,6-naphthyridin-7-yl)oxy)-5-methyl-3,4,8a,9,10,11-hexahydro-2H,8H,13H-chromeno[8,7-f]pyrrolo[2,1-c][1,4]oxazepin-13-one | 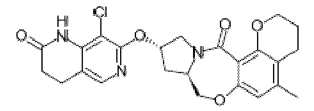 |

<Preparation of cells forcibly expressing OX1R or OX2R>

In order to obtain cell clones stably expressing human orexin type 1 receptor (hOX1R, NP_001516.2) or human orexin type 2 receptor (hOX2R, NP_001371201.1), hOX1R or hOX2R cDNAs were inserted into the pcDNA5/FRT plasmid vector (Invitrogen) to generate plasmid DNAs for hOX1R or hOX2R expression (pcDNA5/FRT/hOX1R or pcDNA5/FRT/hOX2R), respectively. Each of the plasmid DNAs was transfected into Flp-In 293 cells (Invitrogen) with Lipofectamine 2000 (ThermoFisher Scientific), and limiting dilution was carried out using Hygromycin drug resistance as a selection marker to thereby produce clone cells forcibly expressing hOX1R and hOX2R.

<Evaluation of OX1R and OX2R agonist activity>

Flp-In 293 cells forcibly expressing human orexin type 1 receptor (hOX1R) or human orexin type 2 receptor (hOX2R) were seeded in each well of a 384-well black plate (BD Falcon) at 5,000 cells/25 μL in 10% FBS (Corning) and Glutamax-supplemented DMEM (ThermoFisher Scientific) supplemented with 1% Penicillin-Streptomycin (ThermoFisher Scientific), and incubated for 24 h (37°C, 5% $CO_2$). Then, 25 μL of assay buffer A (20 mM HEPES (ThermoFisher Scientific), 5 mM Probenecid (Sigma-Aldrich), and Hank's balanced salt solution (Fujifilm Wako Pure Chemicals)), which contains Calcium 5 Dye (Molecular Devices), was added to each well, and incubated (37°C, 5% $CO_2$) for 60 minutes. Next, fluorescence intensity measurement was started using a FLIPR Tetra (Molecular Devices) to assess changes in intracellular calcium ion concentration. 10 seconds later, 12.5 μL of Orexin A (R&D Systems, Cat # 1455) or each of the test compounds was added. Fluorescence intensity was measured up to 170 seconds after the start of the measurement. Orexin A and the test compounds were each dissolved in DMSO and diluted in assay buffer B (20 mM HEPES, Hank's balanced salt solution, 0.05% BSA (Sigma-Aldrich), 2.5 mM Probenecid) such that the final concentration of DMSO was 0.5%. The difference between the minimum fluorescence intensity before the addition of the test compound and the maximum fluorescence intensity after the addition of the test compound was used as the signal for the change in intracellular calcium ion concentration. The 50% agonist effect concentration of the test compound ($EC_{50}$ value) was calculated using XLFit ver. 5.5 (IDBS Software), defining the signal in the well with solvent control (DMSO 0.5%) as 0% and the average signal in the well with a final concentration of 5 nM of Orexin A as 100%. The calculation formula is shown below.

[Fitting method]

$$y = (A+((B-A)/(1+(10^((C-x)*D)))))$$

(fitting fomula: 4 parameter logistic model (xlfit 208))

*x = log conc(M) of compounds
*y = % of control
*A= min(0%), B = max(100%), C = -log(EC50), D = slope

<Evaluation results of OX2R agonist activity>

The evaluation results of the agonist activity of orexin type 2 receptors are shown in the tables below. The activity of each compound is indicated on the following scale.

*: $10\ nM < EC_{50}$

**: $1\ nM < EC_{50} \leqq 10\ nM$

***: $0.1\ nM < EC_{50} \leqq 1\ nM$

s****: $EC_{50} \leqq 0.1\ nM$

The activity of Orexin A was also evaluated several times based on this evaluation system. The resulting activity values ($EC_{50}$ values) ranged from 0.02 nM to 0.05 nM.

[Table 11]

| Example | $EC_{50}$ |
|---|---|
| 1 | ** |
| 2 | * |
| 3 | * |
| 4 | * |
| 5 | *** |
| 6 | * |
| 7 | *** |
| 8 | ** |
| 9 | ** |
| 10 | *** |
| 11 | **** |
| 12 | ** |
| 13 | ** |
| 14 | * |
| 15 | ** |
| 16 | * |
| 17 | * |
| 18 | ** |
| 19 | * |
| 20 | * |
| 21 | * |
| 22 | * |

(continued)

| Example | $EC_{50}$ |
|---|---|
| 23 | ** |
| 24 | * |
| 25 | *** |
| 26 | *** |
| 27 | **** |
| 28 | * |
| 29 | *** |
| 30 | *** |
| 31 | **** |
| 32 | *** |
| 33 | *** |
| 34 | *** |
| 35 | **** |
| 36 | *** |
| 37 | *** |
| 38 | *** |
| 39 | *** |
| 40 | *** |
| 41 | *** |
| 42 | *** |
| 43 | *** |
| 44 | *** |
| 45 | * |
| 46 | **** |
| 47 | **** |
| 48 | *** |
| 49 | **** |
| 50 | **** |
| 51 | *** |
| 52 | *** |
| 53 | *** |
| 54 | *** |
| 55 | * |
| 56 | *** |
| 57 | **** |
| 58 | **** |
| 59 | *** |
| 60 | **** |
| 61 | **** |

(continued)

| Example | $EC_{50}$ |
|---|---|
| 62 | **** |
| 63 | *** |
| 64 | **** |
| 65 | *** |
| 66 | **** |
| 67 | **** |
| 68 | **** |
| 69 | *** |
| 70 | **** |
| 71 | **** |
| 72 | **** |
| 73 | **** |
| 74 | **** |
| 75 | **** |
| 76 | **** |
| 77 | **** |
| 78 | **** |
| 79 | **** |
| 80 | *** |
| 81 | **** |
| 82 | ** |
| 83 | *** |
| 84 | *** |
| 85 | ** |
| 86 | * |
| 87 | **** |
| 88 | *** |
| 89 | * |
| 90 | ** |
| 91 | * |
| 92 | * |
| 93 | * |
| 94 | *** |
| 95 | ** |
| 96 | ** |
| 97 | * |
| 98 | *** |
| 99 | *** |
| 100 | *** |

(continued)

| Example | $EC_{50}$ |
|---|---|
| 101 | **** |
| 102 | **** |
| 103 | **** |
| 104 | **** |
| 105 | *** |
| 106 | **** |
| 107 | *** |
| 108 | **** |
| 109 | **** |
| 110 | *** |
| 111 | *** |
| 112 | ** |
| 113 | **** |
| 114 | * |
| 115 | **** |
| 116 | **** |
| 117 | **** |
| 118 | **** |
| 119 | *** |
| 120 | *** |

[Table 12]

| Example | $E^{C}_{50}$ |
|---|---|
| 121 | *** |
| 122 | **** |
| 123 | *** |
| 124 | **** |
| 125 | *** |
| 126 | **** |
| 127 | **** |
| 128 | *** |
| 129 | * |
| 130 | *** |
| 131 | *** |
| 132 | **** |
| 133 | * |
| 134 | * |

**INDUSTRIUAL APPLICABILITY**

The present invention can be used in the production industry for pharmaceuticals and other products containing compounds with orexin type 2 receptor agonist activity.

**Claims**

1. A compound represented by Formula (I) or a pharmaceutically acceptable salt thereof.

[Formula 1]

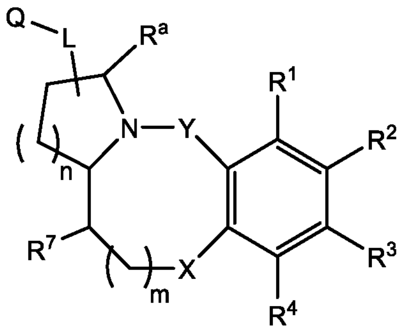

(I)

[In the formula,

$R^1$ represents a hydrogen atom, $C_{1-6}$ alkyl group, $C_{3-8}$ cycloalkyl group, 3- to 8-membered heterocyclyl group, hydroxy group, halogen, amino group, cyano group, carboxyl group, $C_{6-10}$ aryl group, 5- to 12-membered heteroaryl group, $C_{1-6}$ alkoxy group, $C_{3-8}$ cycloalkoxy group, 3- to 8-membered heterocyclyloxy group, $C_{6-10}$ aryloxy group, 5-to 12-membered heteroaryloxy group, $C_{3-8}$ cycloalkyl $C_{1-6}$ alkoxy group, 3- to 8-membered heterocyclyl $C_{1-6}$ alkoxy group, $C_{6-10}$ aryl $C_{1-6}$ alkoxy group, or 5- to 12-membered heteroaryl $C_{1-6}$ alkoxy group;
$R^1$ may be substituted with 1 to 6 [halogens, hydroxy groups, carboxyl groups, oxo groups, amino groups, cyano groups, $C_{1-6}$ alkyl groups which may be substituted with 1 to 3 halogens, or $C_{1-6}$ alkoxy groups which may be substituted with 1 to 3 halogens];
$R^2$, $R^3$ and $R^4$ each represent, independently of each other, a hydrogen atom, $C_{1-6}$ alkyl group which may be substituted with 1 to 6 halogens, $C_{3-8}$ cycloalkyl group which may be substituted with 1 to 6 halogens, hydroxy group, or halogen;
each of $R^2$, $R^3$ and $R^4$ may be substituted with 1 to 6 [halogens, hydroxy groups, carboxyl groups, oxo groups, amino groups, cyano groups, $C_{1-6}$ alkyl groups which may be substituted with 1 to 3 halogens, or $C_{1-6}$ alkoxy groups which may be substituted with 1 to 3 halogens];
each of the pairs of adjacent groups $R^1$ and $R^2$, $R^2$ and $R^3$, and $R^3$ and $R^4$ may each be linked to each other via a single bond to form a 5- to 8-membered ring;
$R^7$ represents a hydrogen atom, halogen, or $C_{1-6}$ alkyl group which may be substituted with 1 to 6 halogens;
$R^a$ represents a hydrogen atom, $C_{1-6}$ alkyl group which may be substituted with 1 to 6 halogens, hydroxy group, or halogen;
X represents -O-, or $-CR^bR^c-$,
$R^b$ and $R^c$ each represent, independently of each other, a hydrogen atom, $C_{1-6}$ alkyl group which may be substituted with 1 to 6 halogens, or halogen,
Y represents $-CH_2-$ or -CO-,
n represents 1 or 2,
m represents 0 or 1,
L represents -O- or -NH-,
Q represents a 3- to 10-membered heterocyclyl group which may be substituted with 1 to 4 $R^{11}$, $C_{6-10}$ aryl group which may be substituted with 1 to 4 $R^{12}$, or 5- to 12-membered heteroaryl group which may be substituted with 1 to 4 $R^{13}$, and
$R^{11}$, $R^{12}$ and $R^{13}$ each represent, independently of each other, a halogen, hydroxy group, oxo group, $C_{1-6}$ alkyl group which may be substituted with 1 to 6 halogens, $C_{3-8}$ cycloalkyl group which may be substituted with 1 to 6 halogens, or $C_{1-6}$ alkoxy group which may be substituted with 1 to 6 halogens.]

**2.** A compound or a pharmaceutically acceptable salt thereof according to claim 1, wherein

$R^1$ is $C_{1-6}$ alkyl group, $C_{3-8}$ cycloalkyl group, 3- to 8-membered heterocyclyl group, hydroxy group, $C_{6-10}$ aryl group, 5- to 12-membered heteroaryl group, $C_{1-6}$ alkoxy group, $C_{3-8}$ cycloalkoxy group, 3- to 8-membered heterocyclyloxy group, $C_{3-8}$ cycloalkyl $C_{1-6}$ alkoxy group, 3- to 8-membered heterocyclyl $C_{1-6}$ alkoxy group, or $C_{6-10}$ aryl $C_{1-6}$ alkoxy group, and
$R^1$ may be substituted with 1 to 3 [halogens, hydroxy groups, oxo groups, $C_{1-6}$ alkyl groups which may be substituted with 1 to 3 halogens, or $C_{1-6}$ alkoxy groups which may be substituted with 1 to 3 halogens].

**3.** A compound or a pharmaceutically acceptable salt thereof according to claim 1 or 2, wherein $R^2$ is a hydrogen atom or halogen.

**4.** A compound or a pharmaceutically acceptable salt thereof according to any one of claims 1 to 3, wherein $R^3$ is a hydrogen atom, $C_{1-6}$ alkyl group which may be substituted with 1 to 6 halogens, or halogen.

**5.** A compound or a pharmaceutically acceptable salt thereof according to any one of claims 1 to 4, wherein $R^4$ is a hydrogen atom or halogen.

**6.** A compound or a pharmaceutically acceptable salt thereof according to any one of claims 1 to 5, wherein $R^a$ is a hydrogen atom.

**7.** A compound or a pharmaceutically acceptable salt thereof according to any one of claims 1 to 6, wherein n is 1.

**8.** A compound or a pharmaceutically acceptable salt thereof according to any one of claims 1 to 7, wherein m is 0.

**9.** A compound or a pharmaceutically acceptable salt thereof according to any one of claims 1 to 8, wherein L is -O-.

**10.** A compound or a pharmaceutically acceptable salt thereof according to any one of claims 1 to 9, wherein Y is -CO-.

**11.** A compound or a pharmaceutically acceptable salt thereof according to any one of claims 1 to 10, wherein Q is a 5- to 12-membered bicyclic heteroaryl group which may be substituted with 1 to 4 $R^{13}$.

**12.** A compound or a pharmaceutically acceptable salt thereof according to any one of claims 1 to 11, wherein Q is a structure represented by any one selected from the group consisting of Formulae (II-1), (II-2), (II-3), and (II-4).

[Formula 2]

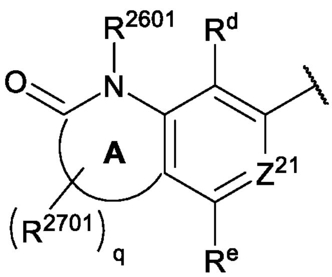

(II-1)

[Formula 3]

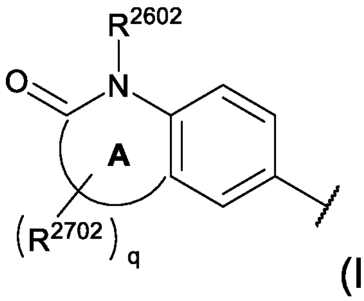

(II-2)

[Formula 4]

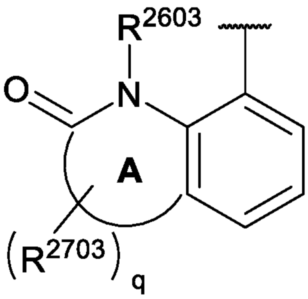

(II-3)

[Formula 5]

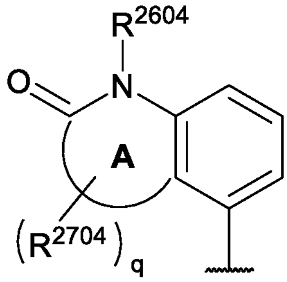

(II-4)

[In Formulae (II-1), (II-2), (II-3), and (II-4),

$Z^{21}$ represents a $CR^f$ or N,
Ring A represents a 6- to 8-membered monocyclic heterocyclyl group or 6- to 7-membered monocyclic heteroaryl group,
$R^{2701}$, $R^{2702}$, $R^{2703}$ and $R^{2704}$ each represent, independently of each other, a halogen or $C_{1-6}$ alkyl group which may be substituted with 1 to 3 halogens,
$R^d$, $R^e$, and $R^f$ each represent, independently of each other, a hydrogen atom, halogen, or $C_{1-6}$ alkyl group which may be substituted with 1 to 3 halogens,
$R^{2601}$, $R^{2602}$, $R^{2603}$ and $R^{2604}$ each represent, independently of each other, a hydrogen atom, $C_{1-6}$ alkyl group which may be substituted with 1 to 6 halogens, or $C_{3-8}$ cycloalkyl group which may be substituted with 1 to 6 halogens, and
q represents, independently of each other, an integer of 0 to 4.]

**13.** A compound or a pharmaceutically acceptable salt thereof according to claim 1, which is a compound represented by Formula (III) or a pharmaceutically acceptable salt thereof.

[Formula 6]

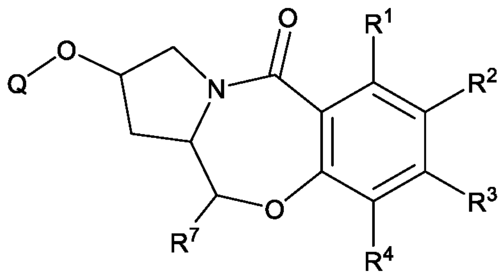

(III)

[In the formula,

$R^1$ represents a hydrogen atom, $C_{1-6}$ alkyl group, $C_{3-8}$ cycloalkyl group, 3- to 8-membered heterocyclyl group, hydroxy group, halogen, $C_{6-10}$ aryl group, 5- to 12-membered heteroaryl group, $C_{1-6}$ alkoxy group, $C_{3-8}$ cycloalkoxy group, 3- to 8-membered heterocyclyloxy group, $C_{6-10}$ aryloxy group, 5- to 12-membered heteroaryloxy group, $C_{3-8}$ cycloalkyl $C_{1-6}$ alkoxy group, 3- to 8-membered heterocyclyl $C_{1-6}$ alkoxy group, $C_{6-10}$ aryl $C_{1-6}$ alkoxy group, or 5- to 12-membered heteroaryl $C_{1-6}$ alkoxy group;
$R^1$ may be substituted with 1 to 6 [halogens, hydroxy groups, carboxyl groups, oxo groups, amino groups, cyano groups, $C_{1-6}$ alkyl groups which may be substituted with 1 to 3 halogens, or $C_{1-6}$ alkoxy groups which may be substituted with 1 to 3 halogens];
$R^2$, $R^3$ and $R^4$ each represent, independently of each other, a hydrogen atom, $C_{1-6}$ alkyl group which may be substituted with 1 to 6 halogens, or halogen;
$R^7$ represents a hydrogen atom, halogen, or $C_{1-6}$ alkyl group which may be substituted with 1 to 3 halogens;
Q represents a 3- to 10-membered heterocyclyl group which may be substituted with 1 to 4 $R^{11}$, $C_{6-10}$ aryl group which may be substituted with 1 to 4 $R^{12}$, or 5- to 12-membered heteroaryl group which may be substituted with 1 to 4 $R^{13}$, and
$R^{11}$, $R^{12}$ and $R^{13}$ each represent, independently of each other, a halogen, oxo group, or $C_{1-6}$ alkyl group which may be substituted with 1 to 3 halogens.]

**14.** A compound or a pharmaceutically acceptable salt thereof according to any one of claims 1 to 11, wherein Q is a structure represented by any one selected from the group consisting of Formulae (IV), (V), and (VI).

[Formula 7]

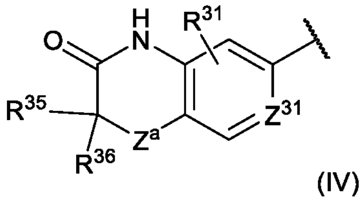

(IV)

[Formula 8]

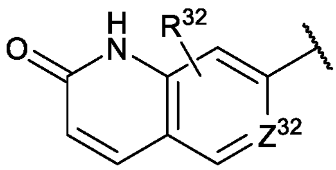

(V)

[Formula 9]

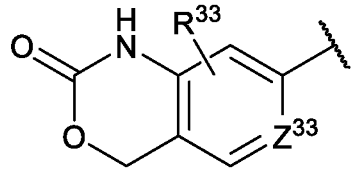

(VI)

[In Formulae (IV), (V), and (VI),

$R^{31}$, $R^{32}$, $R^{33}$, $R^{35}$ and $R^{36}$ each represent, independently of each other, a hydrogen atom, $C_{1-6}$ alkyl group which may be substituted with 1 to 6 halogens, hydroxy group, or halogen,
$Z^{31}$, $Z^{32}$and $Z^{33}$ each represent, independently of each other, CH or N,
$Z^a$ represents O or $CR^g{}_2$, and
$R^g$ represents, independently of each other, a hydrogen atom, or $C_{1-6}$ alkyl group which may be substituted with 1 to 6 halogens.]

**15.** A compound which is at least one selected from (1) to (50) below or a pharmaceutically acceptable salt thereof.

(1) (2S,11aR)-6-isopropoxy-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one
(2) (2S,11aR)-6-(cyclopentyloxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one
(3) (2S,11aR)-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(4) (2S,11aR)-6-(((S)-1-fluoropropan-2-yl)oxy)-8-methyl-2-((2-oxo-1,2,3,4-tetrahydroquinolin-7-yl)oxy)-2,3,11,11a-tetrahydro-1H,5H-benzo[f]pyrrolo[2,1-c][1,4]oxazepin-5-one
(5) (2S,11aR)-6-isopropoxy-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one
(6) (2S,11aR)-6-(cyclopentyloxy)-8-methyl-2-((2-oxo-1,2-dihydro-1,6-naphthyridin-7-yl)oxy)-2,3,11,11a-tetrahydro-1H,5H-benzo[f]pyrrolo[2,1-c][1,4]oxazepin-5-one
(7) (2S,11aR)-6-(cyclopentyloxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(8) (2S,11aR)-8-methyl-2-((2-oxo-1,2,3,4-tetrahydro-1,6-naphthyridin-7-yl)oxy)-6-(((S)-1,1,1-trifluoropropan-2-yl)oxy)-2,3,11,11a-tetrahydro-1H,5H-benzo[f]pyrrolo[2,1-c][1,4]oxazepin-5-one
(9) (2S,11aR)-6-cyclobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(10) (2S,11aR)-8-methyl-2-((2-oxo-1,2,3,4-tetrahydro-1,6-naphthyridin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one
(11) (2S,11aR)-6-(((R)-1-fluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(12) (2S,11aR)-6-(((R)-1,1-difluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(13) (2S,11aR)-6-(2,6-difluorophenyl)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(14) (2S,11aR)-2-((8-fluoro-2-oxo-1,2,3,4-tetrahydro-1,6-naphthyridin-7-yl)oxy)-6-isopropoxy-8-methyl-2,3,11,11a-tetrahydro-1H,5H-benzo[f]pyrrolo[2,1-c][1,4]oxazepin-5-one
(15) (2S,11aR)-2-((8-fluoro-2-oxo-1,2,3,4-tetrahydroquinolin-7-yl)oxy)-6-isopropoxy-8-methyl-2,3,11,11a-tetrahydro-1H,5H-benzo[f]pyrrolo[2,1-c][1,4]oxazepin-5-one
(16) (2S,11aR)-7-fluoro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(17) (2S,11aR)-6-isobutyl-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(18) (2S,11aR)-6-(cyclopentyloxy)-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

(19) (2S,11aR)-8-methyl-2-((2-oxo-1,2-dihydro-1,6-naphthyridin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one
(20) (2S,11aR)-8-methyl-2-((2-oxo-1,2,3,4-tetrahydroquinolin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one
(21) (2S,11aR)-2-((8-fluoro-2-oxo-1,2,3,4-tetrahydro-l,6-naphthyridin-7-yl)oxy)-8-methyl-6-(((R)-1,1,1-trifluoropropan-2-yl)oxy)-2,3,11,11a-tetrahydro-1H,5H-benzo[f]pyrrolo[2,1-c][1,4]oxazepin-5-one
(22) (2S,11aR)-8-chloro-6-isopropoxy-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(23) (2S,11aR)-6-isobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(24) (2S,11aR)-6-((R)-sec-butoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(25) (2S,11aR)-6-((S)-sec-butoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(26) (2S,11aR)-6-((2,2-difluorocyclopentyl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(27) (2S,11aR)-7,9-difluoro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(28) (2S,11aR)-7-fluoro-8-methyl-2-((2-oxo-1,2,3,4-tetrahydro-1,6-naphthyridin-7-yl)oxy)-6-(((R)-1,1,1-trifluoropropan-2-yl)oxy)-2,3,11,11a-tetrahydro-1H,5H-benzo[f]pyrrolo[2,1-c][1,4]oxazepin-5-one
(29) (2S,11aR)-6-(2-fluoro-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(30) (2S,11aR)-8-methyl-2-((2-oxo-1,2,3,4-tetrahydro-1,6-naphthyridin-7-yl)oxy)-6-(pyrrolidin-1-yl)-2,3,11,11a-tetrahydro-1H,5H-benzo[f]pyrrolo[2,1-c][1,4]oxazepin-5-one
(31) (2S,11aR)-6-(cyclopropylmethoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(32) (2S,11aR)-7-fluoro-6-(2-fluoro-2-methylpropoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(33) (2S,11aR)-7-fluoro-8-methyl-2-((2-oxo-1,2,3,4-tetrahydro-1,6-naphthyridin-7-yl)oxy)-6-(pyrrolidin-1-yl)-2,3,11,11a-tetrahydro-1H,5H-benzo[f]pyrrolo[2,1-c][1,4]oxazepin-5-one
(34) (2S,11aR)-6-isopropoxy-2-((2-oxo-1,2,3,4-tetrahydro-1,6-naphthyridin-7-yl)oxy)-8-(trifluoromethyl)-2,3,11,11a-tetrahydro-1H,5H-benzo[f]pyrrolo[2,1-c][1,4]oxazepin-5-one
(35) (2S,11aR)-6-(cyclopropylmethoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(36) (2S,11aR)-7-fluoro-6-isobutoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(37) (2S,11aR)-7-fluoro-6-((1-fluorocyclopropyl)methoxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(38) (2S,11aR)-9-fluoro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(39) (2S,11aR)-6-((R)-sec-butoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(40) (2S,11aR)-6-((S)-sec-butoxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(41) (2S,11aR)-6-(cyclopentyloxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(42) (2S,11aR)-6-ethoxy-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(43) (2S,11aR)-7-fluoro-8-methyl-2-((2-oxo-1,2,3,4-tetrahydro-1,6-naphthyridin-7-yl)oxy)-6-propoxy-2,3,11,11a-tetrahydro-1H,5H-benzo[f]pyrrolo[2,1-c][1,4]oxazepin-5-one
(44) (2S,11aR)-7-chloro-6-isopropoxy-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(45) (2S,11aR)-6-(((R)-1,1-difluoropropan-2-yl)oxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(46) (2S,11aR)-7-fluoro-6-(((R)-1-fluoropropan-2-yl)oxy)-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one
(47) (2S,11aR)-7-fluoro-8-methyl-2-((2-oxo-1,2,3,4-tetrahydro-1,6-naphthyridin-7-yl)oxy)-6-(2,2,2-trifluoroethoxy)-2,3,11,11a-tetrahydro-1H,5H-benzo[f]pyrrolo[2,1-c][1,4]oxazepin-5-one

(48) (2S,11aR)-7-fluoro-8-methyl-2-((2-oxo-1,2,3,4-tetrahydro-1,6-naphthyridin-7-yl)oxy)-6-(((S)-tetrahydrofuran-3-yl)oxy)-2,3,11,11a-tetrahydro-1H,5H-benzo[f]pyrrolo[2,1-c][1,4]oxazepin-5-one
(49) (2S,11aR)-7-fluoro-8-methyl-2-((2-oxo-1,2,3,4-tetrahydro-1,6-naphthyridin-7-yl)oxy)-6-(((R)-tetrahydrofuran-3-yl)oxy)-2,3,11,11a-tetrahydro-1H,5H-benzo[f]pyrrolo[2,1-c][1,4]oxazepin-5-one
(50) (2S,11aR)-6-((1,3-difluoropropan-2-yl)oxy)-7-fluoro-8-methyl-2-((2-oxo-1,2,3,4-tetrahydro-1,6-naphthyridin-7-yl)oxy)-2,3,11,11a-tetrahydro-1H,5H-benzo[f]pyrrolo[2,1-c][1,4]oxazepin-5-one

**16.** An orexin type 2 receptor agonist comprising a compound or a pharmaceutically acceptable salt thereof according to any one of claims 1 to 15.

**17.** A pharmaceutical composition comprising a compound or a pharmaceutically acceptable salt thereof according to any one of claims 1 to 15..

**18.** A preventive drug or therapeutic drug for narcolepsy, idiopathic hypersomnia, hypersomnia, sleep apnea syndrome, narcolepsy syndrome with narcolepsy-like symptoms, hypersomnia associated with Parkinson's disease, hypersomnia associated with Guillain-Barre syndrome, hypersomnia associated with Kleine-Levin syndrome, or hypersomnia associated with Lewy body dementia, comprising a compound or a pharmaceutically acceptable salt thereof according to any one of claims 1 to 15.

## INTERNATIONAL SEARCH REPORT

International application No.
**PCT/JP2023/015990**

**A. CLASSIFICATION OF SUBJECT MATTER**

***C07D 498/04***(2006.01)i; ***A61K 31/55***(2006.01)i; ***A61K 31/553***(2006.01)i; ***A61P 25/02***(2006.01)i; ***A61P 25/16***(2006.01)i; ***A61P 25/26***(2006.01)i; ***A61P 25/28***(2006.01)i; ***A61P 43/00***(2006.01)i; ***C07D 519/00***(2006.01)i
FI: C07D498/04; A61K31/55; A61K31/553; A61P25/02; A61P25/16; A61P25/26; A61P25/28; A61P43/00 111; C07D498/04 CSP; C07D519/00 301; C07D519/00 311

According to International Patent Classification (IPC) or to both national classification and IPC

**B. FIELDS SEARCHED**

Minimum documentation searched (classification system followed by classification symbols)

C07D498/04; A61K31/55; A61K31/553; A61P25/02; A61P25/16; A61P25/26; A61P25/28; A61P43/00; C07D519/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2014-522847 A (GILEAD SCIENCES, INC.) 08 September 2014 (2014-09-08)<br>claims, paragraphs [1368]-[1369], [1372]-[1373] | 1-18 |
| A | JP 2009-527532 A (BOEHRINGER INGELHEIM INTERNATIONAL GMBH) 30 July 2009 (2009-07-30)<br>claims, examples 1-91 | 1-18 |
| A | JP 2009-500406 A (ASTRAZENECA AB) 08 January 2009 (2009-01-08)<br>claims, examples 1-6 | 1-18 |
| A | WO 2012/061019 A2 (MERCK SHARP & DOHME CORP.) 10 May 2012 (2012-05-10)<br>claims, examples 1-32 | 1-18 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 June 2023** | **20 June 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
PCT/JP2023/015990

C. DOCUMENTS CONSIDERED TO BE RELEVANT

| Category* | Citation of document, with indication, where appropriate, of the relevant passages | Relevant to claim No. |
|---|---|---|
| A | TURLINGTON, M. et al. Exploration of Allosteric Agonism Structure-Activity Relationships within an Acetylene Series of Metabotropic Glutamate Receptor 5 (mGlu5) Positive Allosteric Modulators (PAMs): Discovery of 5-((3-Fluorophenyl)ethynyl)-N-(3-methyloxetan-3-yl)picolinamide (ML254). Journal of Medicinal Chemistry. 2013, vol. 56, no. 20, pp. 7976-7996, DOI: 10.1021/jm401028t<br>fig. 4 | 1-18 |

Form PCT/ISA/210 (second sheet) (January 2015)

**INTERNATIONAL SEARCH REPORT**
**Information on patent family members**

International application No.
**PCT/JP2023/015990**

| Patent document cited in search report | Publication date (day/month/year) | Patent family member(s) | Publication date (day/month/year) |
|---|---|---|---|
| JP 2014-522847 A | 08 September 2014 | US 2013/0012492 A1<br>claims, paragraphs [2419], [2421]<br>WO 2013/006463 A1<br>EP 2966067 A1<br>CN 103635467 A<br>KR 10-2014-0033413 A | |
| JP 2009-527532 A | 30 July 2009 | US 2007/0207999 A1<br>claims, examples 1-91<br>WO 2007/096351 A1<br>EP 1989198 A1 | |
| JP 2009-500406 A | 08 January 2009 | US 2010/0173890 A1<br>claims, examples 1-6<br>WO 2007/004960 A1<br>EP 1902041 A1<br>CN 101258136 A | |
| WO 2012/061019 A2 | 10 May 2012 | EP 2632469 A2<br>US 2013/0210804 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021048822 A1


### Non-patent literature cited in the description


- *Cell*, 1998, vol. 92, 573-585
- *Proc. Natl. Acad. Sci. USA*, 2004, vol. 101, 4649-4654
- *Cell*, 1998, vol. 98, 437-451
- *Cell*, 1999, vol. 98, 365-376
- *Neuron*, 2003, vol. 38, 715-730
- *The Lancet*, 2000, vol. 355, 39-40
- *Respiration*, 2004, vol. 71, 575-579
- *Brain*, 2007, vol. 130, 1586-1595
- *CNS Drugs*, 2013, vol. 27, 83-90
- **T. W. GREENE** ; **P. G. M. WUTS**. Protective Groups in Organic Synthesis. John Wiley & Sons Inc., 2014
- Palladium Reagents and Catalysts. John Wiley & Sons Inc., 2004
- Palladium Reagents and Catalysts. John Wiley & Sons Inc, 2004